(19) Europäisches Patentamt
European Patent Office
Office européen des brevets

(11) EP 3 109 239 A1

(12) EUROPEAN PATENT APPLICATION
published in accordance with Art. 153(4) EPC

(43) Date of publication:
**28.12.2016 Bulletin 2016/52**

(21) Application number: **15752236.8**

(22) Date of filing: **17.02.2015**

(51) Int Cl.:
***C07D 231/50*** (2006.01) ***A61K 31/4152*** (2006.01)
***A61K 31/4155*** (2006.01) ***A61K 31/423*** (2006.01)
***A61K 31/4439*** (2006.01) ***A61K 31/444*** (2006.01)
***A61K 31/4709*** (2006.01) ***A61K 31/5377*** (2006.01)
***A61K 31/538*** (2006.01) ***A61P 35/00*** (2006.01)
***A61P 43/00*** (2006.01) ***C07D 401/04*** (2006.01)
***C07D 401/12*** (2006.01) ***C07D 401/14*** (2006.01)
***C07D 403/12*** (2006.01) ***C07D 405/12*** (2006.01)
***C07D 413/12*** (2006.01)

(86) International application number:
**PCT/JP2015/054318**

(87) International publication number:
**WO 2015/125786 (27.08.2015 Gazette 2015/34)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **18.02.2014 JP 2014028990**

(71) Applicant: **Daiichi Sankyo Company, Limited**
**Tokyo 103-8426 (JP)**

(72) Inventors:
- **EBISAWA, Masayuki**
  **Tokyo 140-8710 (JP)**
- **SUZUKI, Takashi**
  **Tokyo 140-8710 (JP)**
- **HAGINOYA, Noriyasu**
  **Tokyo 140-8710 (JP)**
- **HAMADA, Tomoaki**
  **Tokyo 140-8710 (JP)**
- **MURATA, Takeshi**
  **Tokyo 140-8710 (JP)**
- **UOTO, Kouichi**
  **Tokyo 140-8710 (JP)**
- **MURAKAMI, Ryo**
  **Tokyo 140-8710 (JP)**
- **TAKATA, Takehiko**
  **Tokyo 140-8710 (JP)**

(74) Representative: **Fairbairn, Angus Chisholm**
**Marks & Clerk LLP**
**90 Long Acre**
**London WC2E 9RA (GB)**

# (54) AMINOPYRAZOLONE DERIVATIVE

(57) The present invention is intended to provide a compound or a pharmacologically acceptable salt thereof which has an excellent inhibitory action on the ATPase activity of a TIP48/TIP49 complex and as such, is useful for the treatment of tumors.

[Solution]

The present invention provides a compound having a structure represented by the general formula (I) or a pharmacologically acceptable salt thereof, and a pharmaceutical composition comprising the compound. In the formula, $R^1$, $R^2$, $R^3$, $R^4$, $R^5$, $R^6$, $R^7$, and W are as defined in the present specification.

EP 3 109 239 A1

## Description

### Technical Field

The present invention relates to a compound having a specific chemical structure or a pharmacologically acceptable salt thereof which has an excellent inhibitory action on the ATPase activity of TIP48/TIP49 complex.

### Background Art

TIP48 (also called CGI-46, ECP51, INO80J, REPTIN, RUVBL2, RUVB-LIKE2, RVB2, TIH2, or TIP49B) and TIP49 (also called ECP54, INO80H, NMP238, PONTIN, Pontin 52, RUVBL1, RUVB-LIKE1, RVB1, TIH1, or TIP49A) are ATPases of the Walker superfamily, which is classified in the AAA+ (adenosine triphosphatase associated with diverse cellular activities) ATPase family. TIP48 and TIP49 form a ring-shaped multimer, which forms a complex with various protein groups involved in chromatin remodeling, telomerase or tubulin, etc., and participates in the control of various intracellular molecular mechanisms such as regulation of gene expression by transcriptional factors, DNA damage repair, and telomerase activity (Non Patent Literatures 1 and 2).

For example, an oncogene c-Myc, which promotes the malignant transformation of cells, is known as a factor that interacts with the TIP48/TIP49 complex. c-Myc is a transcriptional factor that induces, in response to various stresses, the expression of genes involved in cell cycle or cell apoptosis. c-Myc is considered to promote the malignant transformation of cells through abnormality in its own transcription regulation functions. Translocation of the c-Myc gene or mutation in this gene has actually been observed in human lymphoma. One factor involved in such ability of c-Myc to promote malignant transformation is the ATPase activity of the TIP48/TIP49 complex. Inhibition of the ATPase activity of the TIP48/TIP49 complex has been reported to suppress the ability of c-Myc to promote malignant transformation (Non Patent Literature 3). Also, some transcriptional factors such as the β-catenin, ATF-2, and E2F families have been reported to interact with the TIP48/TIP49 complex and participate in the malignant transformation of cells (Non Patent Literature 2).

TIP48/TIP49 has been reported to be overexpressed in the tissue sites of tumors such as liver cancer, colorectal cancer, and lymphoma compared with normal tissues, suggesting the association of TIP48/TIP49 with malignant transformation (Non Patent Literatures 4 to 6).

Focusing on such functions of the TIP48/TIP49 complex, substances inhibiting the ATPase activity of the TIP48/TIP49 complex have been regarded as candidates for antitumor agents. For example, 4 types of low-molecular compounds inhibiting the ATPase activity of TIP49 have been reported (Non Patent Literature 7).

### Citation List

### Non Patent Literature

Non Patent Literature 1: Sci. Signal., 2013, 12, mr1
Non Patent Literature 2: Biochim. Biophys. Acta. 2011, 1815, 147-157
Non Patent Literature 3: Mol. Cell, 2000, 5, 321-330
Non Patent Literature 4: Hepatology, 2009, 50, 1871-1883
Non Patent Literature 5: Oncol. Rep., 2012, 28, 1619-1624
Non Patent Literature 6: Jpn. J. Cancer Res. 2002, 93, 894-901
Non Patent Literature 7: Biochem. J., 2012, 443, 549-559


### Summary of Invention

### Technical Problem

An object of the present invention is to provide a novel low-molecular compound that has a potent inhibitory action on the ATPase activity of a TIP48/TIP49 complex and exhibits an antitumor effect.

### Solution to Problem

The present invention relates to the following (1) to (14) :

(1) A compound represented by the general formula (I) or a pharmacologically acceptable salt thereof:

[Formula 1]

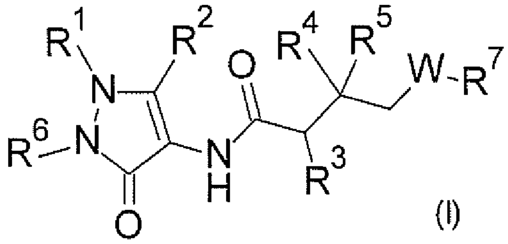

(I)

wherein

$R^1$ represents a hydrogen atom, a $C_1$-$C_6$ alkyl group optionally having 1 to 3 substituents independently selected from group A given below, a $C_2$-$C_6$ alkenyl group optionally having 1 to 3 substituents independently selected from group A given below, or a $C_2$-$C_6$ alkynyl group optionally having 1 to 3 substituents independently selected from group A given below;
$R^2$ represents a hydrogen atom, a halogen atom, a cyano group, a $C_2$-$C_6$ alkenyl group, a $C_1$-$C_6$ alkyl group, or - $CR^{21}R^{22}$-$(CR^{23}R^{24})_m$-$(CR^{25}R^{26})_n$-$(CR^{27}R^{28})_q$-$R^{29}$;
m, n, and q each independently represent an integer of 0 or 1, wherein
when m represents 0, n and q each represent 0, and when n represents 0, q represents 0;
$R^{21}$, $R^{22}$, $R^{23}$, $R^{24}$, $R^{25}$, $R^{26}$, $R^{27}$, and $R^{28}$ each independently represent a hydrogen atom, a hydroxy group, a halogen atom, a cyano group, a $C_1$-$C_6$ alkyl group, or a $C_1$-$C_6$ alkoxy group, or
$R^{21}$ together with $R^{22}$, $R^{23}$ together with $R^{24}$, $R^{25}$ together with $R^{26}$, and $R^{27}$ together with $R^{28}$ each independently optionally form an oxo group;
$R^{29}$ represents a halogen atom, a hydroxy group, a cyano group, a $C_1$-$C_6$ alkoxy group, -$NR^{291}R^{292}$, -$OR^{293}$, - $COR^{294}$, or -$SO_2R^{294}$;
$R^{291}$ and $R^{292}$ each independently represent a hydrogen atom, a $C_1$-$C_6$ alkylcarbonyl group optionally substituted by 1 to 3 halogen atoms, a $C_1$-$C_6$ alkoxycarbonyl group, a $C_3$-$C_6$ cycloalkyl group, a $C_1$-$C_6$ alkyl group optionally having 1 to 3 substituents independently selected from group B given below, or a phenyl-$C_1$-$C_6$ alkyl group optionally having, on the benzene ring, 1 to 3 substituents independently selected from group B given below;
$R^{293}$ represents a $C_1$-$C_6$ alkyl group, a $C_3$-$C_6$ cycloalkyl group, a 5- or 6-membered aromatic heterocyclic group optionally having, in the ring, 1 to 3 heteroatoms independently selected from the group consisting of a nitrogen atom, an oxygen atom, and a sulfur atom, or a 5- or 6-membered aliphatic heterocyclic group optionally having, in the ring, 1 to 3 heteroatoms independently selected from the group consisting of a nitrogen atom, an oxygen atom, and a sulfur atom, wherein
the 5- or 6-membered aromatic heterocyclic group and the 5- or 6-membered aliphatic heterocyclic group are each optionally substituted by 1 to 3 $C_1$-$C_6$ alkyl groups;
$R^{294}$ represents a hydroxy group, a $C_1$-$C_6$ alkoxy group, a phenyl group optionally having 1 to 3 substituents independently selected from group B given below, -$NR^{296}R^{297}$, or -$OR^{293}$;

$R^{296}$ and $R^{297}$ each independently represent a hydrogen atom, a $C_3$-$C_6$ cycloalkyl group, or a $C_1$-$C_6$ alkyl group optionally having 1 to 3 substituents independently selected from group B given below, or
$R^{296}$ and $R^{297}$ optionally together form a 3-to 6-membered aliphatic heterocyclic ring optionally having, in the ring, 1 to 3 heteroatoms independently selected from the group consisting of a nitrogen atom, an oxygen atom, and a sulfur atom;

$R^3$ represents a hydrogen atom, a halogen atom, a hydroxy group, a $C_1$-$C_6$ alkyl group, or a $C_1$-$C_6$ alkoxy group;
$R^4$ and $R^5$ each independently represent a hydrogen atom, a hydroxy group, a halogen atom, a $C_1$-$C_6$ alkyl group optionally having 1 to 3 halogen atoms, a $C_1$-$C_6$ alkoxy group, or a $C_1$-$C_6$ alkylcarbonyloxy group, or
$R^4$ and $R^5$ optionally together form a 3- to 6-membered cycloalkyl ring, or
a 5- or 6-membered aliphatic heterocyclic ring optionally having, in the ring, 1 to 3 heteroatoms independently selected from the group consisting of a nitrogen atom, an oxygen atom, and a sulfur atom;
W represents any of the following $W^1$ to $W^3$:

[Formula 2]

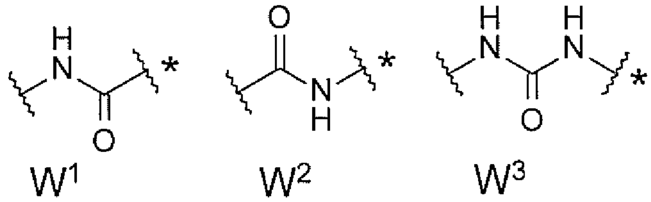

(* binds to $R^7$) ;

$R^6$ represents a phenyl group optionally having 1 to 5 substituents independently selected from group D given below, a $C_3$-$C_7$ cycloalkyl group optionally having 1 to 3 substituents independently selected from group D given below, or a 5- or 6-membered aromatic heterocyclic group optionally having, in the ring, 1 to 3 heteroatoms independently selected from the group consisting of a nitrogen atom, an oxygen atom, and a sulfur atom, wherein the 5- or 6-membered aromatic heterocyclic ring optionally has 1 to 4 substituents independently selected from group D given below; and

$R^7$ represents a phenyl group optionally having 1 to 5 substituents independently selected from group C given below, a naphthyl group optionally having 1 to 7 substituents independently selected from group C given below, a 5- or 6-membered aromatic heterocyclic group optionally having, in the ring, 1 to 3 heteroatoms independently selected from the group consisting of a nitrogen atom, an oxygen atom, and a sulfur atom, an 8-to 10-membered bicyclic aromatic heterocyclic group optionally having, in the ring, 1 to 3 heteroatoms independently selected from the group consisting of a nitrogen atom, an oxygen atom, and a sulfur atom, or an 8- to 10-membered bicyclic partially unsaturated-ring aliphatic heterocyclic group optionally having, in the ring, 1 to 3 heteroatoms independently selected from the group consisting of a nitrogen atom, an oxygen atom, and a sulfur atom, wherein the 5- or 6-membered aromatic heterocyclic group, the 8-to 10-membered bicyclic aromatic heterocyclic group, and the bicyclic partially unsaturated-ring aliphatic heterocyclic group each optionally have 1 to 4 substituents independently selected from group C given below:

group A consists of a hydroxy group, a $C_1$-$C_6$ alkoxy group, an amino group, a $C_1$-$C_6$ alkylamino group, a di-$C_1$-$C_6$ alkylamino group, and a 5- or 6-membered aliphatic heterocyclic group optionally having, in the ring, 1 to 3 heteroatoms independently selected from the group consisting of a nitrogen atom, an oxygen atom, and a sulfur atom,

group B consists of a halogen atom, a hydroxy group, a cyano group, a $C_1$-$C_6$ alkyl group, and a $C_1$-$C_6$ alkoxy group, group C consists of a halogen atom, a hydroxy group, a $C_1$-$C_6$ alkyl group, a $C_1$-$C_6$ alkoxy group optionally substituted by 1 to 3 halogen atoms, a $C_1$-$C_6$ alkoxy-$C_1$-$C_6$ alkyl group, a $C_3$-$C_6$ cycloalkoxy group, a $C_1$-$C_6$ alkoxy-$C_1$-$C_6$ alkoxy group, and an oxy group bonded to a 3- to 6-membered aliphatic heterocyclic group optionally having, in the ring, 1 to 3 heteroatoms independently selected from the group consisting of a nitrogen atom, an oxygen atom, and a sulfur atom, and

group D consists of a halogen atom, a $C_1$-$C_6$ alkyl group optionally substituted by 1 to 3 halogen atoms, and a $C_1$-$C_6$ alkoxy group.

(2) The compound according to (1) or a pharmacologically acceptable salt thereof, wherein in the formula (I), $R^1$ represents a hydrogen atom, or a $C_1$-$C_6$ alkyl group optionally having 1 to 3 substituents independently selected from the group consisting of a hydroxy group and a di-$C_1$-$C_6$ alkylamino group.

(3) The compound according to (1) or (2) or a pharmacologically acceptable salt thereof, wherein in the formula (I), $R^2$ represents a $C_1$-$C_6$ alkyl group or $-CR^{21a}R^{22a}-(CR^{23a}R^{24a})_{ma}-(CR^{25a}R^{26a})_{na}-(CR^{27a}R^{28a})_{qa}-R^{29a}$;

ma, na, and qa each independently represent an integer of 0 or 1, wherein when ma represents 0, na and qa each represent 0, and when na represents 0, qa represents 0;

$R^{21a}$, $R^{22a}$, $R^{23a}$, $R^{24a}$, $R^{25a}$, $R^{26a}$, $R^{27a}$, and $R^{28a}$ each independently represent a hydrogen atom, a $C_1$-$C_6$ alkyl group, or a $C_1$-$C_6$ alkoxy group;

$R^{29a}$ represents a halogen atom, a hydroxy group, a $C_1$-$C_6$ alkoxy group, $-NR^{291a}R^{292a}$, or $-COR^{294a}$;

$R^{291a}$ and $R^{292a}$ each independently represent a $C_1$-$C_6$ alkyl group optionally having 1 to 3 substituents independently selected from the group consisting of a halogen atom and a hydroxy group, or a hydrogen atom;

$R^{294a}$ represents a $C_1$-$C_6$ alkoxy group or $-NR^{296a}R^{297a}$; and

$R^{296a}$ and $R^{297a}$ each independently represent a hydrogen atom or a $C_1$-$C_6$ alkyl group, or

$R^{296a}$ and $R^{297a}$ optionally together form a 3-to 6-membered aliphatic heterocyclic ring optionally having, in the ring, 1 to 3 heteroatoms independently selected from the group consisting of a nitrogen atom, an oxygen atom, and a sulfur atom.

(4) The compound according to any one of (1) to (3) or a pharmacologically acceptable salt thereof, wherein in the formula (I),
W represents any of the following $W^1$ and $W^2$:

[Formula 3]

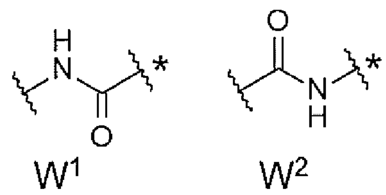

(* binds to $R^7$).
(5) The compound according to any one of (1) to (4) or a pharmacologically acceptable salt thereof, wherein in the formula (I),
$R^6$ represents a phenyl group or a pyridyl group, wherein
the phenyl group and the pyridyl group each optionally have one substituent independently selected from group E given below:

group E consists of a halogen atom, a $C_1$-$C_6$ alkyl group, a trifluoromethyl group, and a $C_1$-$C_6$ alkoxy group.

(6) The compound according to any one of (1) to (5) or a pharmacologically acceptable salt thereof, wherein in the formula (I),
$R^7$ is represented by the following formula (II):

[Formula 4]

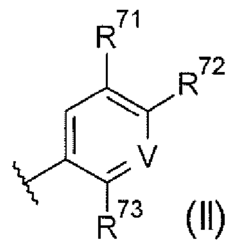

wherein

$R^{71}$ represents a halogen atom;
$R^{72}$ represents a hydrogen atom or a halogen atom;
$R^{73}$ represents a $C_1$-$C_6$ alkoxy group optionally substituted by 1 to 3 halogen atoms, a $C_1$-$C_6$ alkoxy-$C_1$-$C_6$ alkoxy group, or a $C_3$-$C_6$ cycloalkoxy group;
V represents a nitrogen atom or $CR^{74}$; and

$R^{74}$ represents a hydrogen atom, or
$R^{73}$ and $R^{74}$ optionally together form a pyridine ring, a morpholine ring, a tetrahydrofuran ring, a tetrahydropyran ring, a dioxane ring, an oxazole ring, or a furan ring, wherein
the pyridine ring, the morpholine ring, the tetrahydrofuran ring, the tetrahydropyran ring, the dioxane ring, the oxazole ring, and the furan ring each optionally have, on the ring, 1 or 2 substituents independently selected from group F given below: group F consists of a $C_1$-$C_6$ alkyl group, a $C_1$-$C_6$ alkoxy group, a $C_1$-$C_6$ alkoxy-$C_1$-$C_6$ alkyl group, and a $C_1$-$C_6$ alkoxy-$C_1$-$C_6$ alkoxy group.

(7) A compound represented by the general formula (III) or a pharmacologically acceptable salt thereof:

[Formula 5]

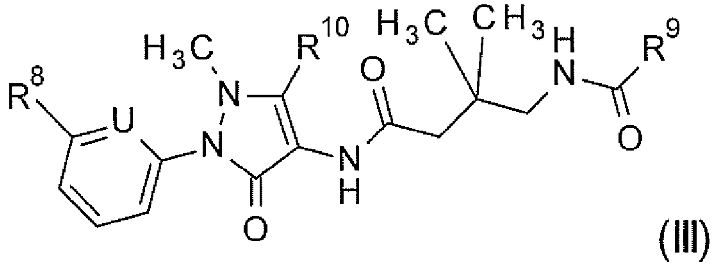

(III)

wherein

$R^8$ represents a hydrogen atom or a methyl group;
U represents CH or a nitrogen atom;
$R^9$ represents any of the following formulas (VII) to (IX) :

[Formula 6]

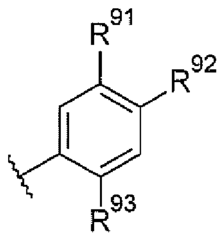

(VII)

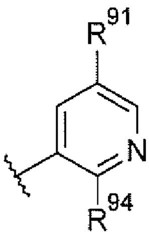

(VIII)

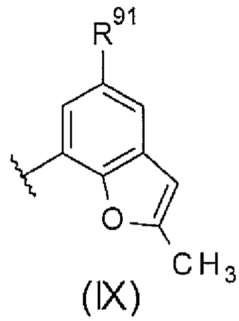

(IX)

wherein

$R^{91}$ represents a halogen atom;
$R^{92}$ represents a hydrogen atom or a halogen atom;
$R^{93}$ represents a methoxy group, an ethoxy group, or a 2-methoxyethoxy group; and
$R^{94}$ represents a methoxy group or an ethoxy group; and

$R^{10}$ represents a methyl group or any of the following formulas (IV) to (VI):

[Formula 7]

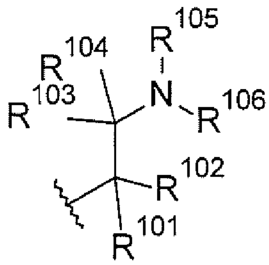

(IV)

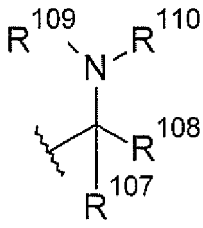

(V)

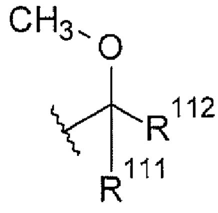

(VI)

wherein

$R^{101}$ and $R^{102}$ each independently represent a hydrogen atom, a methyl group, or a methoxy group; and $R^{103}$, $R^{104}$, $R^{105}$, $R^{106}$, $R^{107}$, $R^{108}$, $R^{109}$, $R^{110}$, $R^{111}$, and $R^{112}$ each independently represent a hydrogen atom or a methyl group.

(8) Any one compound selected from the following group or a pharmacologically acceptable salt thereof:

(+)-5-chloro-N-[2,2-dimethyl-4-({1-methyl-5-[1-(methylamino)ethyl]-3-oxo-2-phenyl-2,3-dihydro-1H-pyrazol-4-yl}amino)-4-oxobutyl]-2-ethoxypyridine-3-carboxamide,
(-)-5-chloro-N-[2,2-dimethyl-4-({1-methyl-5-[1-(methylamino)ethyl]-3-oxo-2-phenyl-2,3-dihydro-1H-pyrazol-4-yl}amino)-4-oxobutyl]-2-ethoxypyridine-3-carboxamide,
5-chloro-N-[4-({5-[2-(dimethylamino)ethyl]-1-methyl-3-oxo-2-phenyl-2,3-dihydro-1H-pyrazol-4-yl}amino)-2,2-dimethyl-4-oxobutyl]-2-ethoxybenzamide,
5-chloro-N-[4-({5-[(1R)-2-(dimethylamino)-1-methylethyl]-1-methyl-3-oxo-2-phenyl-2,3-dihydro-1H-pyrazol-4-yl}amino)-2,2-dimethyl-4-oxobutyl]-2-ethoxybenzamide, 5-chloro-N-[4-({5-[(1R)-2-(dimethylamino)-1-methylethyl]-1-methyl-3-oxo-2-phenyl-2,3-dihydro-1H-pyrazol-4-yl}amino)-2,2-dimethyl-4-oxobutyl]-2-ethoxypyridine-3-carboxamide,
5-chloro-2-ethoxy-4-fluoro-N-(4-{[5-(methoxymethyl)-1-methyl-3-oxo-2-phenyl-2,3-dihydro-1H-pyrazol-4-yl]amino}-2,2-dimethyl-4-oxobutyl)benzamide,
5-chloro-2-ethoxy-4-fluoro-N-(4-{[5-((1S)-1-methoxyethyl)-1-methyl-3-oxo-2-phenyl-2,3-dihydro-1H-pyrazol-4-yl]amino}-2,2-dimethyl-4-oxobutyl)benzamide, and
5-chloro-2-ethoxy-4-fluoro-N-(4-{[5-((1R)-1-methoxyethyl)-1-methyl-3-oxo-2-phenyl-2,3-dihydro-1H-pyrazol-4-yl]amino}-2,2-dimethyl-4-oxobutyl)benzamide.

(9) A pharmaceutical composition comprising a compound according to any one of (1) to (8) or a pharmacologically acceptable salt thereof as an active ingredient.
(10) An inhibitor of the ATPase activity of a TIP48/TIP49 complex comprising a compound according to any one of (1) to (8) or a pharmacologically acceptable salt thereof as an active ingredient.
(11) An antitumor agent comprising a compound according to any one of (1) to (8) or a pharmacologically acceptable salt thereof as an active ingredient.
(12) The antitumor agent according to (11), wherein the tumor is bladder cancer, breast cancer, brain tumor, colorectal cancer, ovary cancer, stomach cancer, head and neck cancer, kidney cancer, leukemia, multiple myeloma, lymphoma, liver cancer, lung cancer, pancreatic cancer, prostate cancer, skin cancer, or bone and soft tissue tumor.
(13) A therapeutic agent for a tumor found to have an increased expression level of a TIP48/TIP49 complex, comprising a compound according to any one of (1) to (8) or a pharmacologically acceptable salt thereof as an active ingredient.
(14) A therapeutic agent for a tumor that is treatable by inhibiting the ATPase activity of a TIP48/TIP49 complex, comprising a compound according to any one of (1) to (8) or a pharmacologically acceptable salt thereof as an active ingredient.

Description of Embodiments

In the present invention, a "halogen atom" is a fluorine atom, a chlorine atom, a bromine atom, or an iodine atom.

In the present invention, "$C_1$-$C_6$ alkyl group" refers to a linear or branched alkyl group having 1 to 6 carbon atoms. The $C_1$-$C_6$ alkyl group is, for example, a methyl group, an ethyl group, a propyl group, an isopropyl group, a butyl group, an isobutyl group, a sec-butyl group, a tert-butyl group, a pentyl group, an isopentyl group, a 2-methylbutyl group, a neopentyl group, a 1-ethylpropyl group, a hexyl group, an isohexyl group, or a 4-methylpentyl group.

In the present invention, a "$C_3$-$C_6$ cycloalkyl group" is a cyclopropyl group, a cyclobutyl group, a cyclopentyl group, or a cyclohexyl group.

In the present invention, a "$C_3$-$C_7$ cycloalkyl group" is a cyclopropyl group, a cyclobutyl group, a cyclopentyl group, a cyclohexyl group, or a cycloheptyl group.

In the present invention, "$C_1$-$C_6$ alkoxy group" refers to a $C_1$-$C_6$ alkoxy group formed from the aforementioned $C_1$-$C_6$ alkyl group. Examples thereof include a methoxy group, an ethoxy group, a n-propoxy group, an isopropoxy group, a butoxy group, an isobutoxy group, a sec-butoxy group, a tert-butoxy group, a pentoxy group, an isopentoxy group, a 2-methylbutoxy group, hexyloxy, and an isohexyloxy group.

In the present invention, "$C_3$-$C_6$ cycloalkoxy group" refers to a $C_3$-$C_6$ cycloalkoxy group formed from the aforementioned $C_3$-$C_6$ cycloalkyl group. Examples thereof include a cyclopropoxy group, a cyclobutoxy group, a cyclopentyloxy group, and a cyclohexyloxy group.

In the present invention, "phenyl-$C_1$-$C_6$ alkyl group" refers to the aforementioned $C_1$-$C_6$ alkyl group substituted by one phenyl group. Examples thereof include a benzyl group, a 1-phenylethyl group, a 2-phenylethyl group, a 1-methyl-2-phenylethyl group, a 2-phenylpropyl group, and a 3-phenylpropyl group.

In the present invention, "$C_1$-$C_6$ alkylamino group" refers to a group in which an amino group is substituted by one aforementioned $C_1$-$C_6$ alkyl group. Examples thereof include a methylamino group, an ethylamino group, a propylamino group, an isopropylamino group, a butylamino group, an isobutylamino group, a sec-butylamino group, a tert-butylamino group, a pentylamino group, an isopentylamino group, a 2-methylbutylamino group, a neopentylamino group, a 1-ethylpropylamino group, a hexylamino group, and an isohexylamino group.

In the present invention, "di-$C_1$-$C_6$ alkylamino group" refers to a group in which an amino group is substituted by two identical or different aforementioned $C_1$-$C_6$ alkyl groups. Examples thereof include a dimethylamino group, a diethylamino group, a dipropylamino group, a diisopropylamino group, a dibutylamino group, a diisobutylamino group, a dipentylamino group, a dineopentylamino group, a dihexylamino group, a N-ethyl-N-methylamino group, a N-methyl-N-propylamino group, a N-isopropyl-N-methylamino group, a N-butyl-N-methylamino group, a N-isobutyl-N-methylamino group, a N-ethyl-N-propylamino group, a N-ethyl-N-isopropylamino group, a N-butyl-N-ethylamino group, and a N-ethyl-N-isopentylamino group.

In the present invention, "$C_1$-$C_6$ alkylcarbonyl group" refers to a group in which a carbonyl group is substituted by one aforementioned $C_1$-$C_6$ alkyl group. Examples thereof include an acetyl group, an ethylcarbonyl group, a propylcarbonyl group, and an isopropylcarbonyl group.

In the present invention, "$C_1$-$C_6$ alkoxycarbonyl group" refers to a group in which a carbonyl group is substituted by one aforementioned $C_1$-$C_6$ alkoxy group. Examples thereof include a methoxycarbonyl group, an ethoxycarbonyl group, a propoxycarbonyl group, an isopropoxycarbonyl group, and a tert-butoxycarbonyl group.

In the present invention, "$C_1$-$C_6$ alkylcarbonyloxy group" refers to a group in which one aforementioned $C_1$-$C_6$ alkylcarbonyl group is bonded to an oxy group. Examples thereof include a methylcarbonyloxy group, an ethylcarbonyloxy group, a propylcarbonyloxy group, and an isopropylcarbonyloxy group.

In the present invention, "$C_2$-$C_6$ alkenyl group" refers to a linear or branched alkenyl group having 2 to 6 carbon atoms. Examples thereof include a vinyl group, an allyl group, a 1-propenyl group, an isopropenyl group, a 2-methyl-1-propenyl group, a 2-methyl-2-propenyl group, a 1-butenyl group, a 2-butenyl group, a 3-butenyl group, a 1-pentenyl group, a 1-hexenyl group, a 1,3-hexadienyl group, and a 1,5-hexadienyl group.

In the present invention, "$C_2$-$C_6$ alkynyl group" refers to a linear or branched alkynyl group having 2 to 6 carbon atoms. Examples thereof include an ethynyl group, a 1-propynyl group, a 2-propynyl group, a 1-butynyl group, a 2-butynyl group, a 3-butynyl group, a 1-ethynyl-2-propynyl group, a 1-methyl-2-propynyl group, a 1-pentynyl group, a 1-hexynyl group, a 1,3-hexadiynyl group, and a 1,5-hexadiynyl group.

In the present invention, "$C_1$-$C_6$ alkoxy-$C_1$-$C_6$ alkyl group" refers to a group in which the aforementioned $C_1$-$C_6$ alkyl group is substituted by one aforementioned $C_1$-$C_6$ alkoxy group. Examples thereof include a methoxymethyl group, an ethoxymethyl group, a propoxymethyl group, an isopropoxymethyl group, a methoxyethyl group, an ethoxyethyl group, a propoxyethyl group, and an isopropoxyethyl group.

In the present invention, "$C_1$-$C_6$ alkoxy-$C_1$-$C_6$ alkoxy group" refers to a group in which the aforementioned $C_1$-$C_6$ alkoxy group is substituted by one aforementioned $C_1$-$C_6$ alkoxy group. Examples thereof include a methoxymethoxy group, an ethoxymethoxy group, a propoxymethoxy group, an isopropoxymethoxy group, a methoxyethoxy group, an ethoxyethoxy group, a propoxyethoxy group, and an isopropoxyethoxy group.

In the present invention, "aromatic heterocyclic group" refers to a group derived from a monocyclic aromatic compound containing a heteroatom in the ring-constituting atoms. Examples thereof include a furyl group, a thienyl group, a pyrrolyl group, an oxazolyl group, an isoxazolyl group, a thiazolyl group, an isothiazolyl group, an imidazolyl group, an oxadiazolyl group, a thiadiazolyl group, a triazinyl group, a pyrazolyl group, a pyridyl group, a pyrazyl group, a pyrimidinyl group, and a pyridazinyl group.

In the present invention, "aliphatic heterocyclic group" refers to a group derived from a monocyclic aliphatic cyclic compound containing a heteroatom in the ring-constituting atoms. Examples thereof include an oxiranyl group, an aziridinyl group, a thiiranyl group, an oxetanyl group, an azetidinyl group, a thietanyl group, a tetrahydrofuranyl group, a pyrrolidinyl group, a tetrahydrothiophenyl group, a tetrahydropyranyl group, a piperazinyl group, a tetrahydrothiopyranyl group, a morpholino group, a morpholinyl group, and a piperidinyl group.

In the present invention, "bicyclic aromatic heterocyclic group" refers to a group derived from a fused aromatic cyclic compound containing a heteroatom in the ring-constituting atoms. Examples thereof include an indolyl group, an isoindolyl group, a benzofuryl group, a benzothienyl group, a benzimidazolyl group, a benzoxazolyl group, a benzothiazolyl group, a benzo[b]pyridyl group, an imidazopyridyl group, and a benzo[c]pyridyl group.

In the present invention, "bicyclic partially unsaturated-ring aliphatic heterocyclic group" refers to a group derived from a fused aliphatic cyclic compound having an unsaturated bond in a portion of the ring and containing a heteroatom in the ring-constituting atoms. Examples thereof include an indolyl group, a 2,3-dihydrobenzofuryl group, a 2,3-dihydrobenzothienyl group, a 1,3-benzodioxolyl group, a benzopyranyl group, a 1,2,3,4-tetrahydroquinolyl group, a 3,4-dihydro-2H-1,4-benzoxazinyl group, a 2,3-dihydro-1,4-benzodioxy group, and a 4H-1,4-benzoxazinyl group.

In the present invention, "tumor" includes not only malignant tumors but every type of tumor and includes, for example, carcinoma, sarcoma, and benign tumors. Particularly, a malignant tumor is also referred to as a "cancer".

In the present invention, "overexpression of a TIP48/TIP49 complex" means that the mRNA expression level of the TIP48 gene or the TIP49 gene and the expression level of its protein is increased by enhanced gene transcription activity, promoted translation, suppressed proteolysis, improved protein stabilization, or the like.

In the present invention, "ATPase activity of a TIP48/TIP49 complex" refers to enzymatic activity that catalyzes ATP hydrolysis when the TIP48 protein and the TIP49 protein coexist with each other.

Next, preferred examples of each substituent in the general formula (I) will be described.

$R^1$ is preferably a hydrogen atom, a $C_1$-$C_6$ alkyl group, a hydroxy-$C_1$-$C_6$ alkyl group, a di-$C_1$-$C_6$ alkylamino group, a morpholino-$C_1$-$C_6$ alkyl group, an azetidino-$C_1$-$C_6$ alkyl group, or a $C_2$-$C_6$ alkenyl group, more preferably a hydrogen atom, a methyl group, an ethyl group, a propyl group, a 2-hydroxyethyl group, a 2-azetidinoethyl group, a 2-dimethylaminoethyl group, or a 2-morpholinoethyl group, further preferably a methyl group.

$R^2$ is preferably any of the following $R^{2A}$ to $R^{2AI}$:

[Formula 8-1]

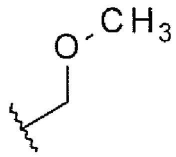

$R^{2A}$

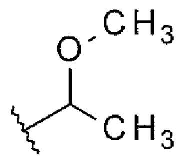

$R^{2B}$

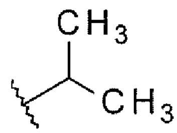

$R^{2C}$

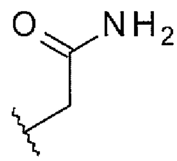

$R^{2D}$

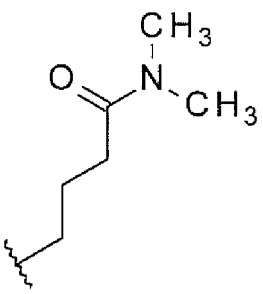

$R^{2E}$

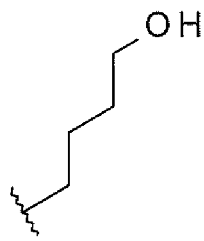

$R^{2F}$

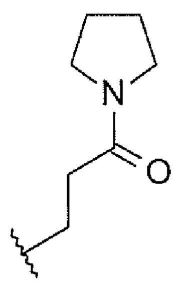

$R^{2G}$

[Formula 8-2]

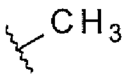
$R^{2H}$

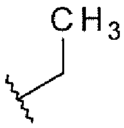
$R^{2I}$

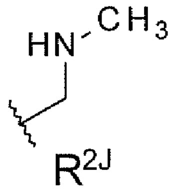
$R^{2J}$

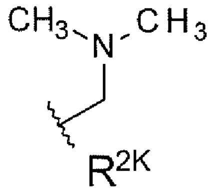
$R^{2K}$

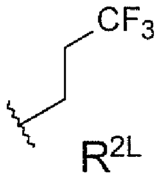
$R^{2L}$

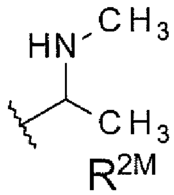
$R^{2M}$

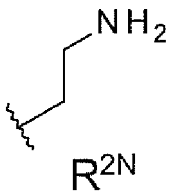
$R^{2N}$

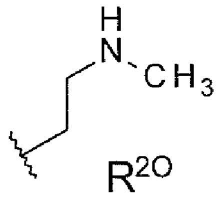
$R^{2O}$

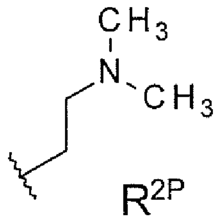
$R^{2P}$

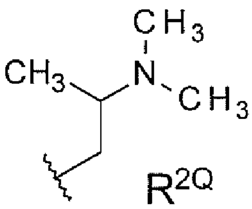
$R^{2Q}$

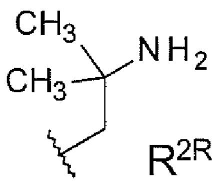
$R^{2R}$

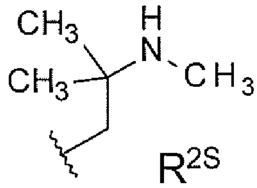
$R^{2S}$

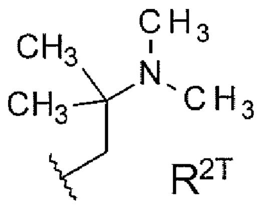
$R^{2T}$

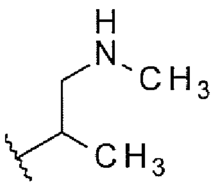
$R^{2U}$

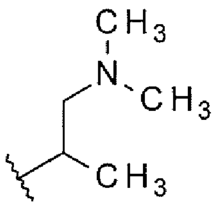
$R^{2V}$

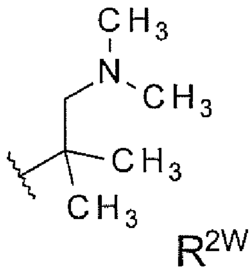
$R^{2W}$

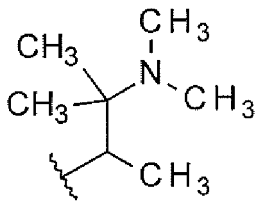
$R^{2X}$

[Formula 8-3]

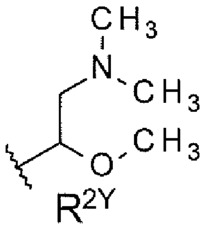

$R^{2Y}$

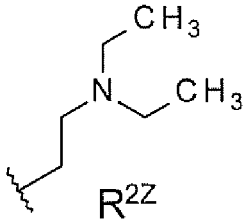

$R^{2Z}$

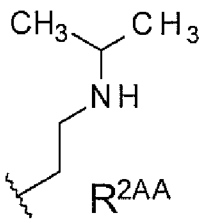

$R^{2AA}$

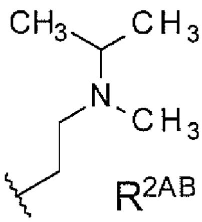

$R^{2AB}$

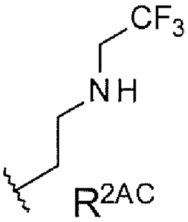

$R^{2AC}$

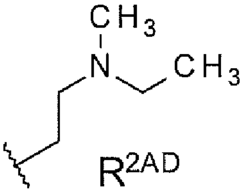

$R^{2AD}$

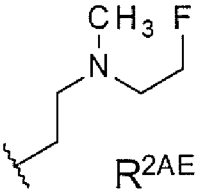

$R^{2AE}$

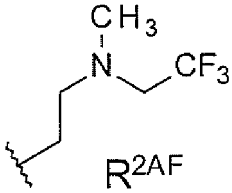

$R^{2AF}$

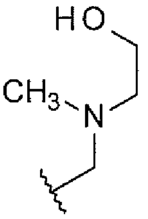

$R^{2AG}$

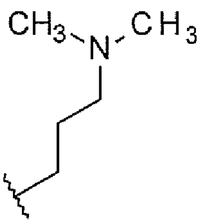

$R^{2AH}$

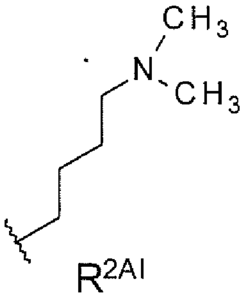

$R^{2AI}$

$R^2$ is more preferably any of the following $R^{2A}$, $R^{2B}$, $R^{2M}$, $R^{2P}$, $R^{2V}$, $R^{2AK}$, $R^{2AL}$, and $R^{2AM}$:

[Formula 9]

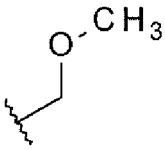

$R^{2A}$

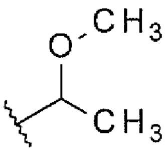

$R^{2B}$

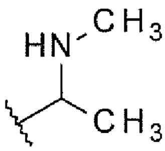

$R^{2M}$

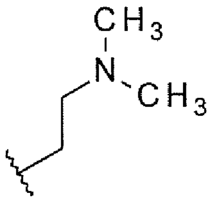

$R^{2P}$

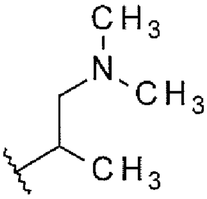

$R^{2V}$

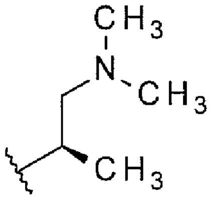

$R^{2AK}$

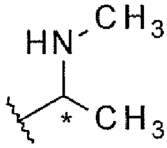

$R^{2AL}$

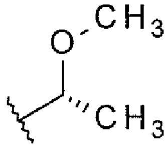

$R^{2AM}$

$R^3$ is preferably a hydrogen atom, a fluorine atom, a hydroxy group, or a methyl group, more preferably a hydrogen atom.

$R^4$ and $R^5$ are, each independently, preferably a $C_1$-$C_6$ alkyl group optionally having 1 to 3 halogen atoms.

More preferably, both of $R^4$ and $R^5$ are methyl groups.

W is preferably any of the following $W^1$ and $W^2$ (* binds to $R^7$):

[Formula 10]

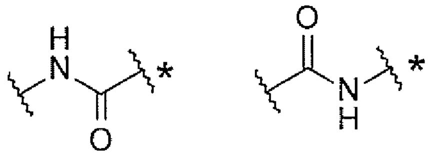

$W^1$ $W^2$

W is more preferably $W^1$.

$R^6$ is preferably a phenyl group, a 2-pyridyl group, a 2-fluorophenyl group, a 6-chloro-2-pyridyl group, a 3-methylphenyl group, a 3-methoxyphenyl group, a 3-trifluorophenyl group, a 6-methyl-2-pyridyl group, or a 4-methoxy-2-pyridyl group, more preferably a phenyl group or a 6-methyl-2-pyridyl group, further preferably a phenyl group.

$R^7$ is preferably any of the following $R^{7A}$ to $R^{7T}$:

[Formula 11]

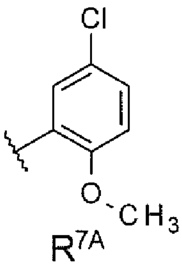
$R^{7A}$
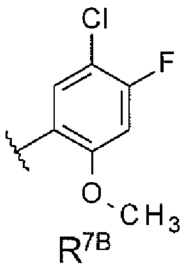
$R^{7B}$
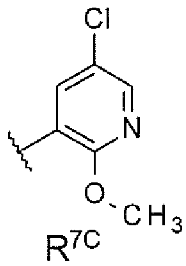
$R^{7C}$
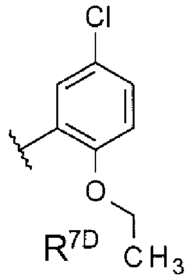
$R^{7D}$
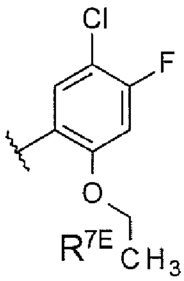
$R^{7E}$

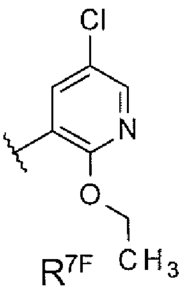
$R^{7F}$
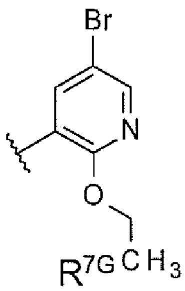
$R^{7G}$
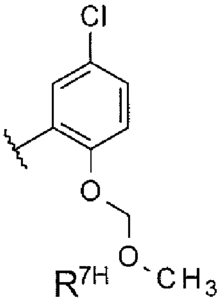
$R^{7H}$
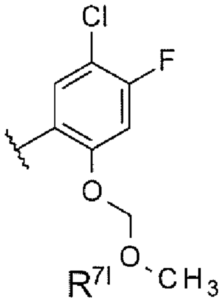
$R^{7I}$
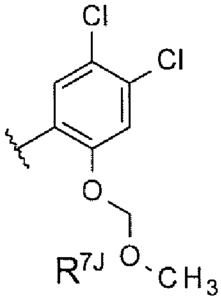
$R^{7J}$

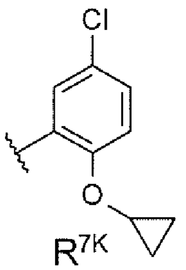
$R^{7K}$
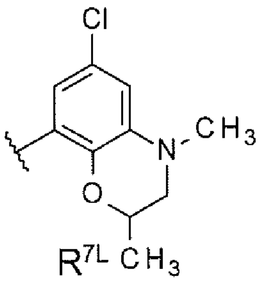
$R^{7L}$
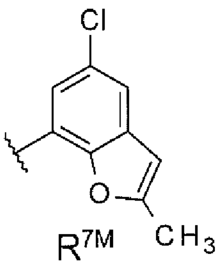
$R^{7M}$
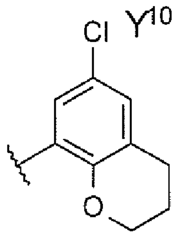
$R^{7N}$
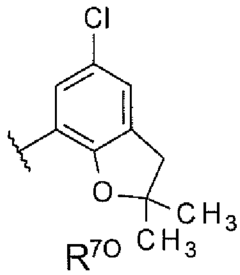
$R^{7O}$

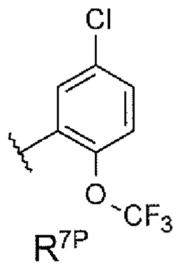
$R^{7P}$
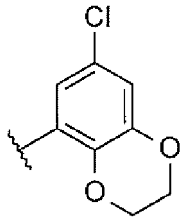
$R^{7Q}$
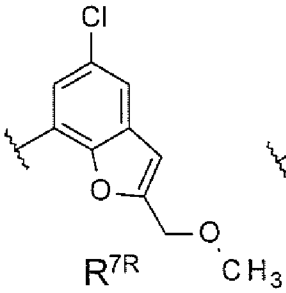
$R^{7R}$
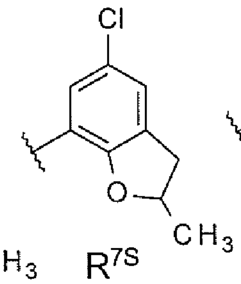
$R^{7S}$
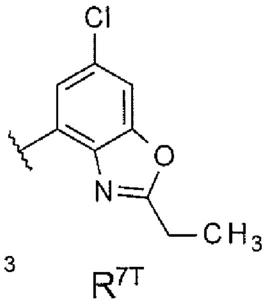
$R^{7T}$

$R^7$ is more preferably any of $R^{7D}$, $R^{7E}$, $R^{7F}$, $R^{7H}$, and $R^{7M}$, further preferably any of $R^{7D}$, $R7^E$, and $R^{7F}$.

A preferred combination of the substituents in the general formula (I) is as follows:

$R^1$ is a hydrogen atom, a $C_1$-$C_6$ alkyl group, a hydroxy-$C_1$-$C_6$ alkyl group, a di-$C_1$-$C_6$ alkylamino group, a morpholino-$C_1$-$C_6$ alkyl group, an azetidino-$C_1$-$C_6$ alkyl group, or a $C_2$-$C_6$ alkenyl group, more preferably a hydrogen atom, a methyl group, an ethyl group, a propyl group, a 2-hydroxyethyl group, a 2-azetidinoethyl group, a 2-dimethylaminoethyl group, or a 2-morpholinoethyl group,
$R^2$ is any of $R^{2A}$ to $R^{2AI}$,
$R^3$ is a hydrogen atom, a fluorine atom, a hydroxy group, or a methyl group,
both of $R^4$ and $R^5$ are methyl groups,
$R^6$ is a phenyl group, a 2-pyridyl group, a 2-fluorophenyl group, a 6-chloro-2-pyridyl group, a 2-methylphenyl group, a 2-methoxyphenyl group, a 2-trifluorophenyl group, a 6-methyl-2-pyridyl group, or a 4-methoxy-2-pyridyl group, and
$R^7$ is any of $R^{7A}$ to $R^{7T}$.

The compound represented by the general formula (I) of the present invention can be converted to a pharmaceutically acceptable salt, if desired. The pharmaceutically acceptable salt refers to a salt that has no significant toxicity and can be used as a drug. The compound represented by the general formula (I) of the present invention can form a salt through reaction with an acid when having a basic group.

Examples of salts based on a basic group can include: hydrohalides such as hydrofluoride, hydrochloride, hydrobromide, and hydroiodide; inorganic acid salts such as nitrate, perchlorate, sulfate, and phosphate; $C_1$-$C_6$ alkylsulfonates such as methanesulfonate, trifluoromethanesulfonate, and ethanesulfonate; arylsulfonates such as benzenesulfonate and p-toluenesulfonate; organic acid salts such as acetate, malate, fumarate, succinate, citrate, ascorbate, tartrate, oxalate, adipate, and maleate; and amino acid salts such as glycine salt, lysine salt, arginine salt, ornithine salt, glutamate, and aspartate.

The compound represented by the general formula (I) of the present invention or the salt thereof, when left in air or recrystallized, may incorporate water molecules to form a hydrate. Such hydrates are also included in the salts of the present invention.

The compound represented by the general formula (I) of the present invention or the salt thereof, when left in a solvent or recrystallized, may absorb certain kinds of solvents to form solvates. Such solvates are also included in the salts of the present invention.

The compound represented by the general formula (I) of the present invention or the pharmacologically acceptable salt thereof encompasses all isomers (diastereomers, optical isomers, geometric isomers, rotational isomers, etc.).

For the compound of the present invention, these isomers and mixtures of these isomers are all represented by a single formula, i.e., the general formula (I). Thus, the present invention includes all of these isomers and even mixtures of these isomers at arbitrary ratios.

The compound of the present invention may also contain unnatural proportions of atomic isotopes at one or more of the atoms constituting such a compound. Examples of atomic isotopes include deuterium ($^2H$), tritium ($^3H$), iodine-125 ($^{125}I$), and carbon-14 ($^{14}C$). The compound may be radiolabeled with a radioisotope such as tritium ($^3H$), iodine-125 ($^{125}I$), or carbon-14 ($^{14}C$). The radiolabeled compound is useful as a therapeutic or prophylactic agent, a research reagent (e.g., an assay reagent), and a diagnostic agent (e.g., an *in vivo* diagnostic imaging agent). All isotopic variants of the compound of the present invention are included in the scope of the present invention, regardless of being radioactive or not.

The ATPase activity of the TIP48/TIP49 complex can be measured by use of an ATPase assay described below in Test Examples 1 and 2. The ATPase activity of the TIP48/TIP49 complex can be measured, as described below in the Test Examples, for example, by using recombinant human TIP48 and TIP49 proteins (hereinafter, referred to as rTIP48 and rTIP49) and ATP in the presence or absence of a test compound and measuring the amount of ADP formed through hydrolysis by the ATPase activity of the TIP48/TIP49 complex, using ADP-Glo. Alternatively, the ATPase activity of the TIP48/TIP49 complex can be measured by a method described in, for example, J. Mol. Biol. 366, 172-179 (2007).

The cell growth inhibitory activity of the compound of the present invention can be examined by use of a growth inhibition testing method usually used by those skilled in the art. The cell growth inhibitory activity can be determined, as described below in Test Example 3, for example, by comparing the degree of growth of cells between in the presence of and in the absence of a test compound. The degree of growth can be examined using, for example, a test system for determining live cells. Examples of methods for determining live cells include [$^3H$]-thymidine uptake test, BrdU method, and MTT assay.

The *in vivo* antitumor activity can be examined by use of an antitumor testing method usually used by those skilled in the art. The *in vivo* antitumor activity according to the present invention can be confirmed, for example, by: transplanting various tumor cells to mice, rats, or the like; after confirmation of engraftment of the transplanted cells, administering the compound of the present invention through an oral route, an intravenous route, or the like to the animals; and a few days to a few weeks later, comparing tumor growth in a vehicle group with tumor growth in the compound administration group.

The compound of the present invention is used in the treatment of a tumor, for example, bladder cancer, breast cancer, brain tumor, colorectal cancer, ovary cancer, stomach cancer, head and neck cancer, kidney cancer, leukemia, multiple myeloma, lymphoma, liver cancer, lung cancer, pancreatic cancer, prostate cancer, skin cancer, or bone and soft tissue tumor.

Since the involvement of the TIP48/TIP49 complex in cancer growth, survival, etc. has been suggested, the compound of the present invention is preferably used for a tumor having the overexpression of TIP48/TIP49. Liver cancer, colorectal cancer, lymphoma, and the like are known as tumors overexpressing TIP48/TIP49.

The overexpression of TIP48/TIP49 can be confirmed by examining TIP48/TIP49 in a tissue sample (collected by, for example, blood collection or biopsy) from a patient by use of a method known in the art, such as Southern blotting, Northern blotting, Western blotting, ELISA, DNA chips, FISH assay, immunohistochemical staining, analysis using other gene analysis methods known in the art {e.g., PCR, LCR (ligase chain reaction), SDA (strand displacement amplification), NASBA (nucleic acid sequence-based amplification), ICAN (isothermal and chimeric primer-initiated amplification), and

LAMP (loop-mediated isothermal amplification)} or the like, or a pathological approach.

The compound of the present invention may be used in combination with an additional antitumor agent. Examples thereof include antitumor antibiotics, antitumor plant components, BRM (biological response modifiers), hormones, vitamins, antitumor antibodies, molecular target drugs, and other antitumor agents.

More specifically, examples of alkylating agents include: alkylating agents such as nitrogen mustard, nitrogen mustard N-oxide, and chlorambucil; aziridine alkylating agents such as carboquone and thiotepa; epoxide alkylating agents such as dibromomannitol and dibromodulcitol; nitrosourea alkylating agents such as carmustine, lomustine, semustine, nimustine hydrochloride, streptozocin, chlorozotocin, and ranimustine; and others such as busulfan, improsulfan tosylate, and dacarbazine.

Examples of various antimetabolites include: purine antimetabolites such as 6-mercaptopurine, 6-thioguanine, and thioinosine; pyrimidine antimetabolites such as fluorouracil, tegafur, tegafur uracil, carmofur, doxifluridine, broxuridine, cytarabine, and enocitabine; and antifolates such as methotrexate and trimetrexate.

Examples of antitumor antibiotics include: anthracycline antibiotic antitumor agents such as mitomycin C, bleomycin, peplomycin, daunorubicin, aclarubicin, doxorubicin, pirarubicin, THP-adriamycin, 4'-epidoxorubicin, and epirubicin; and others such as chromomycin A3 and actinomycin D.

Examples of antitumor plant components include: vinca alkaloids such as vindesine, vincristine, and vinblastine; taxanes such as paclitaxel and docetaxel; and epipodophyllotoxins such as etoposide and teniposide.

Examples of BRM include tumor necrosis factors and indomethacin.

Examples of hormones include hydrocortisone, dexamethasone, methylprednisolone, prednisolone, prasterone, betamethasone, triamcinolone, oxymetholone, nandrolone, metenolone, fosfestrol, ethynyl estradiol, chlormadinone, and medroxyprogesterone.

Examples of vitamins include vitamin C and vitamin A.

Examples of antitumor antibodies and molecular target drugs include trastuzumab, rituximab, cetuximab, nimotuzumab, denosumab, bevacizumab, infliximab, imatinib mesylate, gefitinib, erlotinib, sunitinib, lapatinib, and sorafenib.

Examples of other antitumor agents include cisplatin, carboplatin, oxaliplatin, tamoxifen, camptothecin, ifosfamide, cyclophosphamide, melphalan, L-asparaginase, aceglatone, sizofiran, picibanil, procarbazine, pipobroman, neocarzinostatin, hydroxyurea, ubenimex, and krestin.

Next, typical methods for producing the compound represented by the general formula () will be described. The compound of the present invention can be produced by various production methods. The production methods shown below are given for illustrative purposes. It should be understood that the present invention is not limited by these examples. The compound represented by the general formula (I) and intermediates for production thereof can be produced through the use of various reactions known in the art as described below. In this respect, functional groups in starting materials or intermediates may be protected with appropriate protective groups. Examples of such functional groups can include a hydroxy group, a carboxy group, and an amino group. For the types of their protective groups as well as conditions for the introduction and removal of these protective groups, see those described in, for example, Protective Groups in Organic Synthesis (T.W. Greene and P.G.M. Wuts, John Wiley & Sons, Inc., New York, 2006).

Production method

Next, typical methods for producing the compound represented by the general formula (I) will be described. The compound of the present invention can be produced by various production methods. The production methods shown below are given for illustrative purposes. It should be understood that the present invention is not limited by these examples. Each reaction can be carried out with a substituent protected with an appropriate protective group, if necessary. The type of the protective group is not particularly limited.

General formula (I)

[Formula 12]

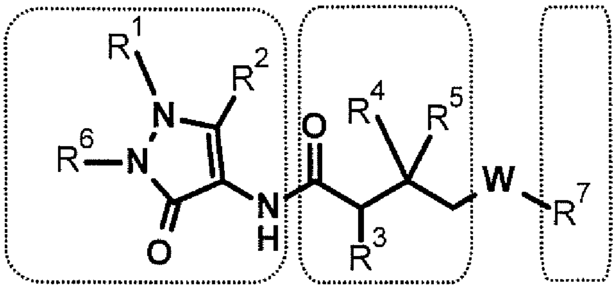

(A) (B) (C)

(I)

In the general formula (I), $R^2$ that is not a hydrogen atom, a halogen atom, a cyano group, a $C_2$-$C_6$ alkenyl group, or a $C_1$-$C_6$ alkyl group can be represented by the general formula (2). X represents $-CR^{21}R^{22}-(CR^{23}R^{24})_m-(CR^{25}R^{26})_n-(CR^{27}R^{28})_q-$, and, m, n, q, $R^{21}$, $R^{22}$, $R^{23}$, $R^{24}$, $R^{25}$, $R^{26}$, $R^{27}$, and $R^{28}$ are as defined above.

General formula (2)

[Formula 13]

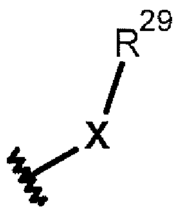

(2)

Typical examples of synthesis procedures include, but are not particularly limited to, a method which involves constructing W between site (B) and site (C) shown in the general formula (I) and bonding this complex to site (A), and a method which involves first bonding site (A) to site (B) through an amide bond and then constructing W between this complex and site (C). Substructure W (3) in the general formula (I) (* binds to $R^7$)

[Formula 14]

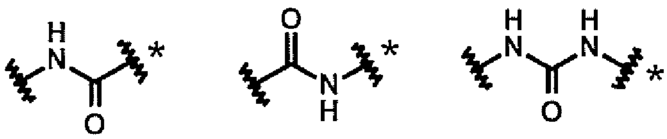

($W^1$) ($W^2$) ($W^3$)

Specifically, the general formula (I) can be represented by the general formula (4).

General formula (4)

[Formula 15]

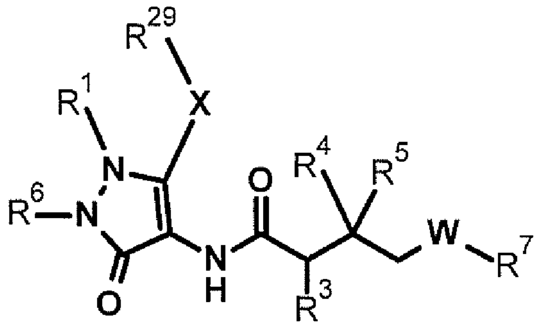

(4)

[Production method 1]

First, the synthesis of aminopyrazolone derivative (12) constituting site (A) will be described.

[Formula 16]

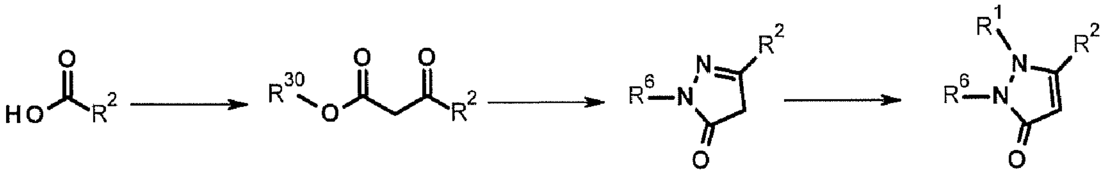

(5) (6) (7) (8)

[Formula 17]

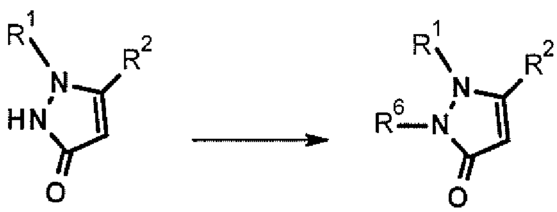

(9) (8)

[Formula 18]

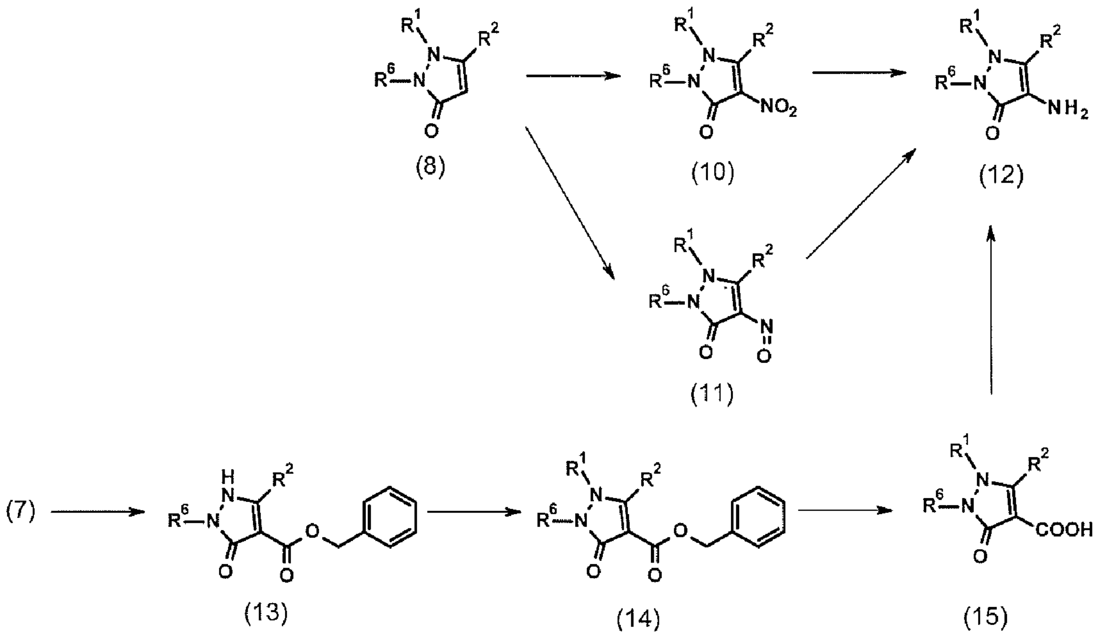

(8) (10) (12)

(11)

(7) (13) (14) (15)

wherein $R^1$, $R^2$, and $R^6$ are as defined above, and $R^{30}$ represents a protective group for the carboxy group, and in this case, a lower alkyl group such as methyl or ethyl is preferred.

Synthesis of compound (6)

A commercially available product corresponding to compound (6) can be used, if available. The commercially available product corresponding to compound (6) can be purchased from, for example, Tokyo Chemical Industry Co., Ltd. If such a commercially available product is not available, a commercially available carboxylic acid or appropriately synthesized carboxylic acid (5) can be activated and reacted with malonic acid monoester in the presence of magnesium chloride to obtain the compound. Examples of methods for activating the carboxylic acid can include a method using 1,1'-carbonyldiimidazole and a method via acid chloride. The reaction can be carried out by the addition of a base, if necessary. Examples of the base can include triethylamine. Examples of the solvent used in the reaction include, but are not particularly limited to, tetrahydrofuran, dioxane, acetonitrile, N,N-dimethylformamide, toluene, and mixed solvents thereof. The reaction temperature is usually in the range of -78°C to 100°C or the boiling point of the solvent, preferably in the range of around room temperature to 100°C.

When an unprotected amino group is present in $R^2$ of compound (5), compound (6) can be obtained by introducing a protective group to the amino group and then the resulting compound to the conditions described above. The conditions for the introduction of the protective group differ depending on the protective group. When the protective group is, for example, a tert-butoxycarbonyl group or a trifluoroacetyl group, the amino group can be reacted with the corresponding acid anhydride in the presence or absence of a base to introduce the protective group thereto. Examples of the base used can include triethylamine and pyridine. Examples of the solvent used in the reaction include, but are not particularly limited to, tetrahydrofuran, dioxane, acetonitrile, N,N-dimethylformamide, methylene chloride, toluene, and mixed solvents thereof. The reaction temperature is usually in the range of -78°C to 100°C or the boiling point of the solvent, preferably in the range of 0°C to 100°C. When the protective group is a benzoyl group, a tosyl group, or the like, the amino group can be reacted with the corresponding acid chloride in the presence of a base to introduce the protective group thereto. Examples of the base used can include triethylamine and pyridine. Examples of the solvent used in the reaction include, but are not particularly limited to, tetrahydrofuran, dioxane, acetonitrile, N,N-dimethylformamide, methylene chloride, toluene, and mixed solvents thereof. The reaction temperature is usually in the range of -78°C to 100°C or the boiling point of the solvent, preferably in the range of 0°C to 100°C.

Synthesis of compound (7)

Compound (7) can be obtained by reacting compound (6) with an appropriate hydrazine derivative in the presence or absence of a base. The appropriate hydrazine derivative can be purchased from, for example, Tokyo Chemical Industry Co., Ltd. Examples of the solvent used in the reaction include, but are not particularly limited to, ethanol, tetrahydrofuran, dioxane, acetonitrile, N,N-dimethylformamide, toluene, acetic acid, and mixed solvents thereof.

Examples of the base used can include potassium carbonate and potassium tert-butoxide. The reaction temperature is usually in the range of -78°C to 180°C or the boiling point of the solvent, preferably in the range of around room temperature to 150°C.

Synthesis of compound (8)

Compound (8) can be obtained by reacting compound (7) with alkyl halide or dialkylsulfuric acid, alkyl trifluoromethanesulfonate, or the like. Examples of the solvent used in the reaction include, but are not particularly limited to, tetrahydrofuran, dioxane, acetonitrile, N,N-dimethylformamide, methylene chloride, toluene, and mixed solvents thereof. The reaction temperature is usually in the range of -78°C to 180°C or the boiling point of the solvent, preferably in the range of 0°C to 150°C.

Alternatively, compound (8) can be obtained by reacting compound (7) with the corresponding alcohol in the presence of phosphine and azodicarboxylic acid ester or azodicarboxylic acid amide. Examples of the phosphine used can include triphenylphosphine and tri-n-butylphosphine. Examples of the azodicarboxylic acid ester or the azodicarboxylic acid amide used can include diethyl azodicarboxylate, diisopropyl azodicarboxylate, and 1,1'-(azodicarbonyl)dipiperidine. Examples of the solvent used in the reaction include, but are not particularly limited to, tetrahydrofuran, dioxane, toluene, and mixed solvents thereof. The reaction temperature is usually in the range of -78°C to 100°C or the boiling point of the solvent, preferably in the range of 0°C to 100°C.

Compound (8) can also be obtained by reacting compound (9) with appropriate aryl halide in the presence of a copper catalyst, a ligand, and a base. Compound (9) can be synthesized by a method shown in, for example, WO2007/10015, or can be purchased from Enamine Ltd. or the like. Examples of the copper catalyst used can include copper iodide. Examples of the ligand can include trans-N,N'-dimethylcyclohexane-1,2-diamine. Examples of the base can include potassium carbonate and sodium carbonate. Examples of the solvent used in the reaction include, but are not particularly limited to, tetrahydrofuran, dioxane, N,N-dimethylformamide, toluene, or mixed solvents thereof. The reaction temperature is usually in the range of around room temperature to 200°C or the boiling point of the solvent, preferably in the range of around room temperature to 180°C.

Synthesis of compound (10)

Compound (10) can be obtained by reacting compound (8) with a nitrating agent such as nitric acid or niter. Examples of the solvent used in the reaction include, but are not particularly limited to, sulfuric acid, trifluoroacetic acid, acetic acid, and mixed solvents thereof. When nitric acid is used, this nitric acid may be used instead of the solvent. The reaction temperature is usually in the range of -78°C to 100°C or the boiling point of the solvent, preferably in the range of -20°C to 100°C. If a protective group labile under acidic conditions is present in $R^2$, this protective group may first be exchanged with a protective group stable under acidic conditions at this point, followed by nitration by the method mentioned above.

Synthesis of compound (12) from compound (10)

Compound (12) can be obtained by adding a reduction catalyst such as palladium carbon to compound (10) dissolved or suspended in a solvent and reducing the compound through reaction under a hydrogen atmosphere. Examples of the solvent used in the reaction include, but are not particularly limited to, methanol, ethanol, tetrahydrofuran, N,N-dimethylformamide, acetic acid, and mixed solvents thereof. The reaction temperature is usually in the range of -78°C to 100°C or the boiling point of the solvent, preferably in the range of around room temperature to 100°C.

When compound (12) is a racemic compound having asymmetric carbon, the compound (12) may be optically resolved at this point by liquid chromatography using a chiral column under appropriate conditions, or a compound obtained later may be resolved.

Synthesis of compound (11)

Compound (11) can be obtained by reacting compound (8) with sodium nitrite under acidic conditions or with alkyl nitrite in an organic solvent. Examples of the alkyl nitrite used can include tert-butyl nitrite and isoamyl nitrite. In the case of using sodium nitrite, examples of the solvent used in the reaction include, but are not particularly limited to, hydrochloric acid, acetic acid, sulfuric acid, and mixed solvents thereof with water or alcohols. In the case of using alkyl nitrite, examples of the solvent used in the reaction include, but are not particularly limited to, ethanol, tetrahydrofuran, dioxane, acetonitrile, methylene chloride, toluene, and mixed solvents thereof. The reaction temperature is usually in the range of -78°C to 100°C or the boiling point of the solvent, preferably in the range of -20°C to 100°C. If a protective group labile under acidic conditions is present in $R^2$, this protective group may first be exchanged with a protective group

stable under acidic conditions at this point, followed by nitrosation by the method mentioned above.

Synthesis of compound (12) from compound (11)

Compound (12) can be obtained by allowing compound (11) to coexist with a metal under acidic conditions. Examples of the metal used can include tin and zinc. Examples of the solvent used in the reaction include, but are not particularly limited to, hydrochloric acid, a mixed solvent of ethanol and water, and acetic acid. The reaction temperature is usually in the range of -78°C to 100°C or the boiling point of the solvent, preferably in the range of 0°C to 100°C.
Compound (12) can also be obtained by adding a reduction catalyst such as palladium carbon to compound (11) dissolved or suspended in a solvent and reducing the compound through reaction under a hydrogen atmosphere. Examples of the solvent used in the reaction include, but are not particularly limited to, methanol, ethanol, tetrahydrofuran, N,N-dimethylformamide, acetic acid, and mixed solvents thereof. The reaction temperature is usually in the range of -78°C to 100°C or the boiling point of the solvent, preferably in the range of around room temperature to 100°C.
When compound (12) is a racemic compound having asymmetric carbon, the compound (12) may be optically resolved at this point by liquid chromatography using a chiral column under appropriate conditions, or a compound obtained later may be resolved.

Synthesis of compound (13)

Compound (13) can be obtained by reacting compound (7) with benzyl chloroformate in the presence of calcium hydroxide. Examples of the solvent used in the reaction include, but are not particularly limited to, tetrahydrofuran, dioxane, toluene, and mixed solvents thereof. The reaction temperature is usually in the range of -78°C to 150°C or the boiling point of the solvent, preferably in the range of 0°C to 100°C.

Synthesis of compound (14)

Compound (14) can be obtained by subjecting compound (13) to conditions appropriately selected from the reaction conditions described in the method for producing compound (8) from compound (7).

Synthesis of compound (15)

Compound (15) can be obtained by adding a reduction catalyst such as palladium carbon to compound (14) dissolved or suspended in a solvent and deprotecting the compound through reaction under a hydrogen atmosphere. Examples of the solvent used in the reaction include, but are not particularly limited to, methanol, ethanol, tetrahydrofuran, N,N-dimethylformamide, and mixed solvents thereof. The reaction temperature is usually in the range of -78°C to 100°C or the boiling point of the solvent, preferably in the range of around room temperature to 100°C.

Synthesis of compound (12) from compound (15)

Compound (12) can be obtained by reacting compound (15) with diphenylphosphoric acid azide under basic conditions. Examples of the base used include triethylamine and N,N-diisopropylethylamine. Examples of the solvent used in the reaction include, but are not particularly limited to, tetrahydrofuran, dioxane, acetonitrile, N,N-dimethylformamide, water, and mixed solvents thereof. The reaction temperature is usually in the range of -78°C to 150°C or the boiling point of the solvent, preferably in the range of around room temperature to 150°C.
When compound (12) is a racemic compound having asymmetric carbon, the compound (12) may be optically resolved at this point by liquid chromatography using a chiral column under appropriate conditions, or a compound obtained later may be resolved.

[Production method 2]

In the case of compound (8) in which $R^2$ is represented by the general formula (2), and $R^{29}$ is an amino group monosubstituted by a protective group $R^{31}$ (compound (8h)), a lower alkyl group can be introduced to the amino group by a method as described below.

[Formula 19]

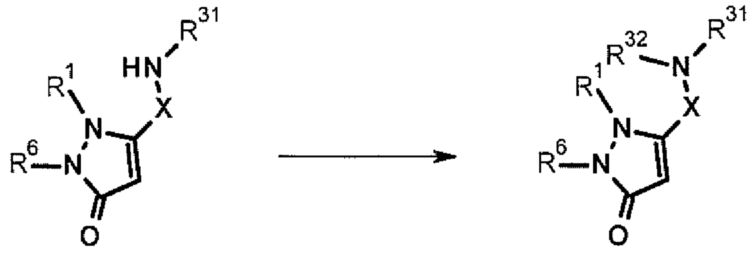

(8h) (8a)

wherein $R^{31}$ represents a protective group for the amino group, $R^{32}$ represents a lower alkyl group, and X, $R^1$, and $R^6$ are as defined above.

Examples of the protective group for the amino group include carbamate groups typified by a tert-butoxycarbonyl group, alkanoyl groups typified by an acetyl group and a trifluoroacetyl group, aralkyl groups typified by a benzyl group, arylcarbonyl groups typified by a benzoyl group, and sulfonyl groups typified by a tosyl group.

Synthesis of compound (8a)

Compound (8a) can be obtained by reacting compound (8h) with appropriate alkyl halide in the presence of a base. Examples of the base used can include potassium carbonate. Examples of the solvent used in the reaction include, but are not particularly limited to, tetrahydrofuran, dioxane, acetonitrile, N,N-dimethylformamide, and mixed solvents thereof. The reaction temperature is usually in the range of -78°C to 100°C or the boiling point of the solvent, preferably in the range of around room temperature to 100°C.

[Production method 3]

In the case of compound (12) in which $R^2$ is represented by the general formula (2), and $R^{29}$ is an amino group monosubstituted by a protective group $R^{31}$ (compound (12h)), a lower alkyl group can be introduced to the amino group by a method as described below.

[Formula 20]

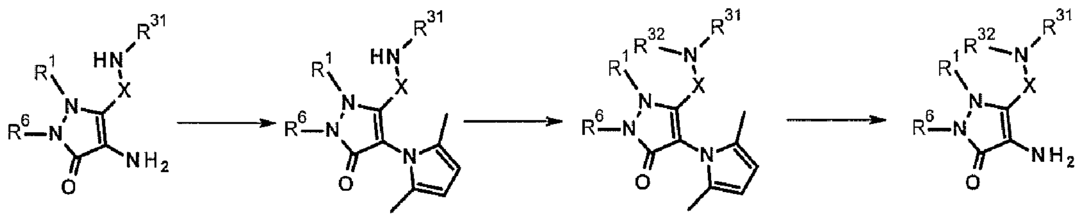

(12h) (16) (17) (12a)

wherein $R^{31}$ represents a protective group for the amino group, $R^{32}$ represents a lower alkyl group and is as defined above, and X, $R^1$, and $R^6$ are as defined above.

Synthesis of compound (16)

Compound (16) can be obtained by reacting compound (12h) with acetonylacetone in the presence of an acid catalyst. Examples of the acid catalyst can include tosylic acid. Examples of the solvent used in the reaction include, but are not particularly limited to, benzene, toluene, and mixed solvents thereof. The reaction temperature is usually in the range of -78°C to 100°C or the boiling point of the solvent, preferably in the range of around room temperature to 100°C.

Synthesis of compound (17)

Compound (17) can be obtained by reacting compound (16) with alkyl halide in the presence of a base. Examples of the base can include sodium hydride. Examples of the solvent used in the reaction include, but are not particularly limited to, tetrahydrofuran, dioxane, acetonitrile, N,N-dimethylformamide, and mixed solvents thereof. The reaction temperature is usually in the range of -78°C to 100°C or the boiling point of the solvent, preferably in the range of 0°C to 100°C.

Synthesis of compound (12a)

Compound (12a) can be obtained by reacting compound (17) with hydroxylamine hydrochloride in the presence of a base. Examples of the base can include triethylamine. Examples of the solvent used in the reaction include, but are not particularly limited to, tetrahydrofuran, ethanol, water, and mixed solvents thereof. An organic solvent that may be mixed with water at an arbitrary ratio is preferred. The reaction temperature is usually in the range of -78°C to 150°C or the boiling point of the solvent, preferably in the range of room temperature to 150°C.

[Production method 4]

Compound (12) in which $R^2$ is represented by the general formula (2), and $R^{29}$ is $-CO_2R^{33}$ (compound (12b)) can be converted to compound (12c) or compound (12d) by a method as described below.

[Formula 21]

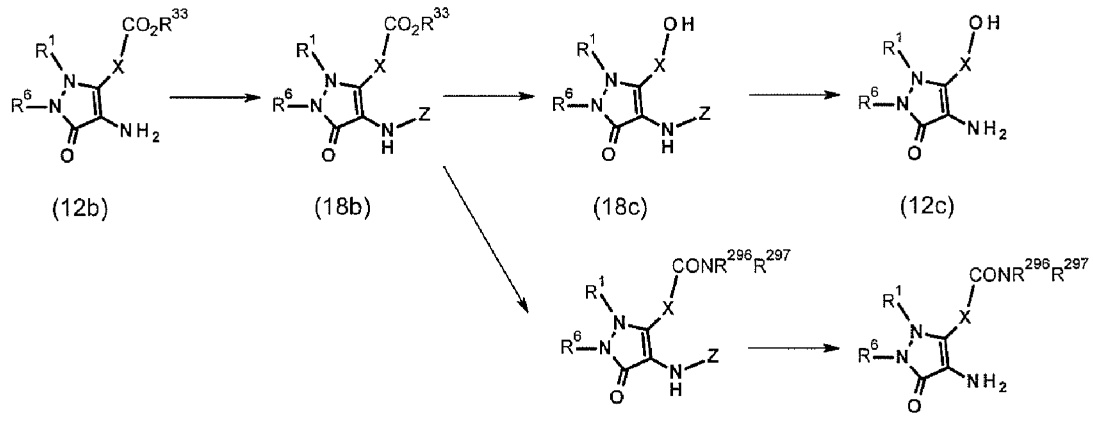

(12b) (18b) (18c) (12c) (18d) (12d)

wherein Z represents a benzyloxycarbonyl group, and X, $R^1$, $R^6$, $R^{296}$, are $R^{297}$ are as defined above.

$R^{33}$ is a protective group for the carboxyl group, and in this case, lower alkyl such as methyl or ethyl is preferred.

Synthesis of compound (18b)

Compound (18b) can be obtained by reacting compound (12b) with benzyloxycarbonyl chloride in the presence of a base. Examples of the base used can include N,N-diisopropylethylamine and triethylamine. Examples of the reaction solvent used include, but are not particularly limited to, tetrahydrofuran, dioxane, acetonitrile, N,N-dimethylformamide, methylene chloride, toluene, and mixed solvents thereof. The reaction temperature is usually in the range of -78°C to 100°C or the boiling point of the solvent, preferably in the range of 0°C to 100°C. Synthesis of compound (18c)

Compound (18c) can be obtained by reducing compound (18b) with a reducing agent. Examples of the reducing agent used can include lithium aluminum hydride and diisobutyl aluminum hydride. Examples of the solvent used in the reaction include, but are not particularly limited to, diethyl ether, tetrahydrofuran, dioxane, toluene, and mixed solvents thereof. The reaction temperature is usually in the range of -78°C to 100°C or the boiling point of the solvent, preferably in the range of -78°C to around room temperature.

Compound (18c) can also be obtained by hydrolyzing the ester group of compound (18b) into a carboxyl group, which is then converted to acid chloride, followed by reduction with a reducing agent. The conditions for the hydrolysis

of the ester differ depending on $R^6$. When $R^6$ is a methyl group, an ethyl group, or the like, compound (18b) can be treated with a base such as sodium hydroxide, potassium hydroxide, lithium hydroxide, or potassium tert-butoxide, or with hydrochloric acid, p-toluenesulfonic acid, or the like to obtain the compound. In this context, examples of the solvent used in the reaction include methanol, ethanol, water, tetrahydrofuran, dioxane, and mixed solvents thereof. An organic solvent that may be mixed with water at an arbitrary ratio is preferred. The reaction temperature is usually in the range of -78°C to 100°C or the boiling point of the solvent, preferably in the range of around room temperature to 100°C. Examples of the reagent for the conversion of the carboxyl group to acid chloride can include thionyl chloride and oxalyl chloride. Examples of the solvent used in the reaction include, but are not particularly limited to, tetrahydrofuran, N,N-dimethylformamide, methylene chloride, toluene, and mixed solvents thereof. The reaction temperature is usually in the range of -78°C to 100°C or the boiling point of the solvent, preferably in the range of 0°C to 100°C. Examples of the reducing agent for the reduction of the acid chloride to an alcohol can include lithium borohydride. Examples of the solvent used in the reaction include, but are not particularly limited to, ether, tetrahydrofuran, dioxane, and mixed solvents thereof. The reaction temperature is usually in the range of -78°C to 100°C or the boiling point of the solvent, preferably in the range of -78°C to 50°C.

Synthesis of compound (12c)

Compound (12c) can be obtained by subjecting compound (18c) to conditions appropriately selected from the reaction conditions described in the method for producing compound (15).

Synthesis of compound (18d)

Compound (18d) can be obtained by hydrolyzing the ester group of compound (18b) into carboxylic acid, followed by reaction with an appropriate amine in the presence of a condensing agent. The conditions for the hydrolysis of the ester are as shown in the method for producing compound (18c). Examples of the condensing agent used can include O-(7-azabenzotriazol-1-yl)-N,N,N',N'-tetramethyluronium hexafluorophosphate (HATU), 4-(4,6-dimethoxy-1,3,5-triazin-2-yl)-4-methylmorpholine (DMT-MM), and 1-(3-dimethylaminopropyl)-3-ethylcarbodiimide (WSC). Examples of the solvent include, but are not particularly limited to, ethanol, tetrahydrofuran, dioxane, acetonitrile, N,N-dimethylformamide, methylene chloride, toluene, and mixed solvents thereof. The reaction temperature is usually in the range of -78°C to 100°C or the boiling point of the solvent, preferably in the range of around room temperature to 100°C.

Alternatively, compound (18d) can also be obtained by converting the carboxy group to acid chloride, followed by reaction with an amine in the presence of a base. The acid chloride can be obtained by using conditions appropriately selected from the reaction conditions described in the method for producing compound (18c). Examples of the base used can include triethylamine and pyridine. Examples of the solvent used in the reaction include, but are not particularly limited to, tetrahydrofuran, acetonitrile, methylene chloride, toluene, and mixed solvents thereof. The reaction temperature is usually in the range of -78°C to 100°C or the boiling point of the solvent, preferably in the range of 0°C to 100°C.

Synthesis of compound (12d)

Compound (12d) can be obtained by subjecting compound (18d) to conditions appropriately selected from the reaction conditions described in the method for producing compound (15).

[Production method 5]

Compound (10) in which an appropriate leaving group is contained or introduced in $R^2$ (compound (10e)) can be converted to compound (10a) or compound (10b) by a method as described below.

[Formula 22]

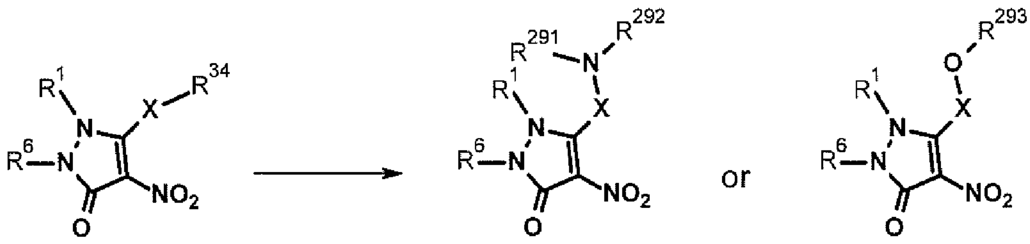

(10e) (10a) or (10b)

wherein $R^{34}$ represents a leaving group such as a halogen atom other than a fluoro group, or a methanesulfonyloxy group, and X, $R^1$, $R^6$, $R^{291}$, $R^{292}$, and $R^{293}$ are as defined above.

Synthesis of compound (10a) and compound (10b)

Compound (10a) or (10b) can be obtained by reacting compound (10e) with an appropriate amine or alcohol in the presence of a base. Examples of the base used can include sodium hydride, triethylamine, and potassium carbonate. Examples of the solvent used in the reaction include, but are not particularly limited to, tetrahydrofuran, acetonitrile, N,N-dimethylformamide, methylene chloride, toluene, and mixed solvents thereof. The reaction temperature is usually in the range of -78°C to 100°C or the boiling point of the solvent, preferably in the range of 0°C to 100°C.

Next, the synthesis of carboxylic acid derivative (24) will be described.

[Formula 23]

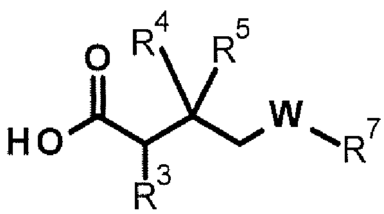

(24)

[Production method 6]

First, the method for producing compound (24a) represented by the general formula (24) wherein W is $W^1$ mentioned above will be described.

[Formula 24]

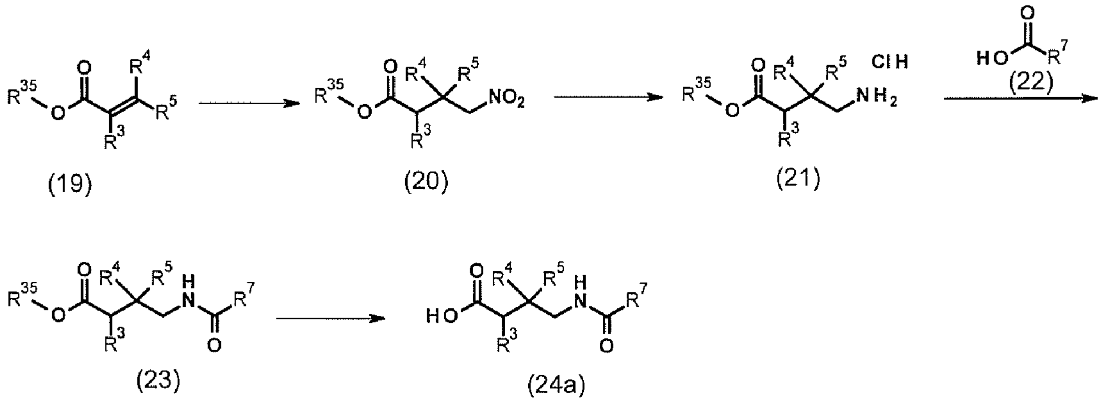

(19) (20) (21) (22) (23) (24a)

wherein $R^3$, $R^4$, $R^5$, and $R^7$ are as defined above, and $R^{35}$ represents a protective group for the carboxy group.

Examples of the protective group for the carboxyl group include a methyl group, an ethyl group, a tert-butyl group, and a benzyl group.

Synthesis of compound (20)

Compound (20) can be obtained by reacting compound (19) with nitromethane in the presence of a base. Compound (19) can be purchased from, for example, Tokyo Chemical Industry Co., Ltd. or can be appropriately synthesized from a commercially available compound for use. Examples of the base used can include 1,8-diazabicyclo(5,4,0)-7-undecene. Examples of the solvent used in the reaction include, but are not particularly limited to, tetrahydrofuran, dioxane, acetonitrile, N,N-dimethylformamide, and mixed solvents thereof. The reaction temperature is usually in the range of -78°C to 150°C or the boiling point of the solvent, preferably in the range of around room temperature to 100°C.

Synthesis of compound (21)

Compound (21) can be obtained by allowing compound (20) to coexist with ammonium chloride in the presence of an iron powder. Examples of the solvent used in the reaction include ethanol, tetrahydrofuran, dioxane, acetic acid, water, and mixed solvents thereof. An organic solvent that may be mixed with water at an arbitrary ratio is preferred. The reaction temperature is usually in the range of -78°C to 150°C or the boiling point of the solvent, preferably in the range of around room temperature to 100°C.

Alternatively, compound (21) can be obtained by adding a reduction catalyst such as palladium carbon to compound (20) dissolved or suspended in a solvent and reducing the compound through reaction under a hydrogen atmosphere. Examples of the solvent used in the reaction include, but are not particularly limited to, methanol, ethanol, tetrahydrofuran, N,N-dimethylformamide, acetic acid, and mixed solvents thereof. The reaction temperature is usually in the range of -78°C to 100°C or the boiling point of the solvent, preferably in the range of around room temperature to 100°C.

When compound (21) needs to be purified, examples of the purification method include a method which involves protecting the amino group with an arbitrary protective group and purifying the compound by silica gel column chromatography, followed by deprotection.

Synthesis of compound (23)

Compound (23) can be obtained by reacting compound (21) with compound (22) in the presence of a condensing agent. Compound (22) can be purchased from, for example, Tokyo Chemical Industry Co., Ltd., or can be appropriately synthesized from a commercially available compound for use. Examples of the condensing agent used can include O-(7-azabenzotriazol-1-yl)-N,N,N',N'-tetramethyluronium hexafluorophosphate (HATU), 4-(4,6-dimethoxy-1,3,5-triazin-2-yl)-4-methylmorpholine (DMT-MM), and 1-(3-dimethylaminopropyl)-3-ethylcarbodiimide (WSC). Examples of the solvent include, but are not particularly limited to, ethanol, tetrahydrofuran, dioxane, acetonitrile, N,N-dimethylformamide, methylene chloride, toluene, and mixed solvents thereof. The reaction temperature is usually in the range of -78°C to 100°C or the boiling point of the solvent, preferably in the range of around room temperature to 100°C.

Alternatively, compound (23) can also be obtained by reacting compound (21) with a compound obtained from compound (22) by the conversion of its carboxyl group to acid chloride in the presence of a base. The reaction conditions

used can be appropriately selected from the reaction conditions of similar reactions described in the methods for producing compound (18c) and (18d).

Synthesis of compound (24a)

This step involves eliminating the protective group $R^{35}$.

Although the deprotection reaction conditions differ depending on the type of $R^{35}$, hydrolysis can be carried out. When $R^{35}$ is a methyl group, an ethyl group, a benzyl group, or the like, compound (24a) can be obtained by treating compound (23) with a base such as sodium hydroxide, potassium hydroxide, lithium hydroxide, or potassium tert-butoxide, or with hydrochloric acid, p-toluenesulfonic acid, or the like. In this context, examples of the solvent used in the reaction include methanol, ethanol, water, tetrahydrofuran, dioxane, and mixed solvents thereof. An organic solvent that may be mixed with water at an arbitrary ratio is preferred. The reaction temperature is usually in the range of -78°C to 100°C or the boiling point of the solvent, preferably in the range of around room temperature to 100°C.

When $R^{35}$ is a tert-butyl group or the like, it is desirable that compound (23) should be treated with trifluoroacetic acid or hydrochloric acid, etc. In this context, examples of the solvent used in the reaction include, but are not particularly limited to, methylene chloride, chloroform, and mixed solvents thereof. The reaction temperature is usually in the range of -78°C to 100°C or the boiling point of the solvent, preferably in the range of -20°C to around 100°C.

When $R^{35}$ is a benzyl group or the like, compound (24a) can be obtained by subjecting compound (23) to conditions appropriately selected from the reaction conditions described in the method for producing compound (15).

[Production method 7]

Next, a method for producing compound (24b) represented by the general formula (24) wherein W is $W^2$ mentioned above will be described.

[Formula 25]

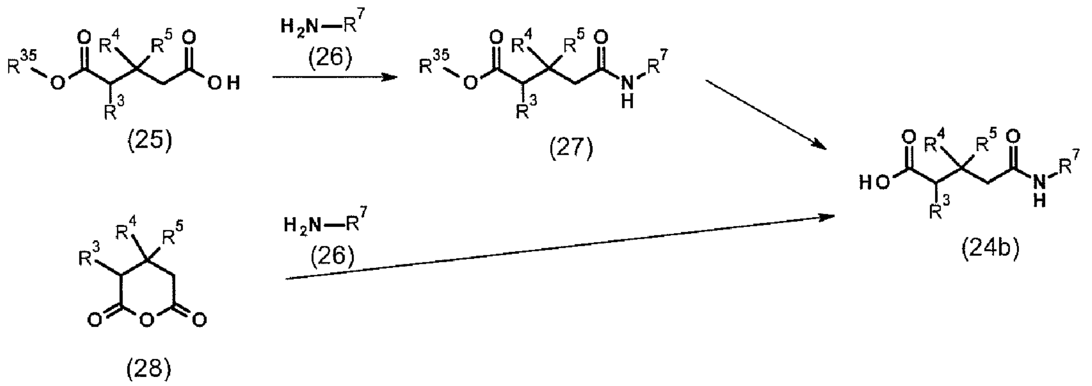


wherein $R^3$, $R^4$, $R^5$, and $R^7$ are as defined above, and $R^{35}$ represents a protective group for the carboxyl group.

Examples of the protective group for the carboxyl group include a methyl group, an ethyl group, a tert-butyl group, and a benzyl group.

Synthesis of compound (27)

Compound (27) can be obtained by reacting compound (25) with compound (26) in the presence of a condensing agent or by reacting compound (26) with a compound obtained from compound (25) by the conversion of its carboxyl group to acid chloride in the presence of a base. Compound (25) can be purchased from Enamine Ltd. or the like, or can be appropriately synthesized for use. Compound (26) can be purchased from Tokyo Chemical Industry Co., Ltd. or the like, or can be appropriately synthesized for use. The reaction conditions used can be appropriately selected from the reaction conditions of similar reactions described in the methods for producing compound (23), (18c), and (18d).

Synthesis of compound (24b) from compound (27)

This step involves eliminating the protective group $R^{35}$.

Compound (24b) can be obtained by subjecting compound (27) to conditions appropriately selected from the

reaction conditions described in the method for producing compound (24a).

Synthesis of compound (24b) from compound (28)

Compound (24b) can also be obtained by reacting commercially available or appropriately synthesized acid anhydride, such as compound (28), with compound (26) in the presence of a base. Examples of the base used can include triethylamine. In this context, examples of the solvent used in the reaction include, but are not particularly limited to, tetrahydrofuran, dioxane, methylene chloride, chloroform, N,N-dimethylformamide, and mixed solvents thereof. The reaction temperature is usually in the range of -78°C to 100°C or the boiling point of the solvent, preferably in the range of around room temperature to 100°C.

[Production method 8]

Next, the method for producing compound (24c) represented by the general formula (24) wherein W is $W^3$ mentioned above will be described.

[Formula 26]

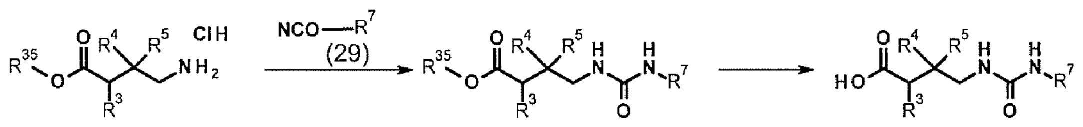

(21) (30) (24c)

wherein $R^3$, $R^4$, $R^5$, and $R^7$ are as defined above, and $R^{35}$ represents a protective group for the carboxyl group.
Examples of the protective group for the carboxyl group include a methyl group, an ethyl group, a tert-butyl group, and a benzyl group.

Synthesis of compound (30)

Compound (30) can be obtained by reacting compound (21) with isocyanate (29). Compound (29) can be purchased from Sigma-Aldrich Corp. or the like, or can be appropriately synthesized from a commercially available compound for use. In this context, examples of the solvent used in the reaction include, but are not particularly limited to, tetrahydrofuran, dioxane, methylene chloride, chloroform, and mixed solvents thereof. The reaction temperature is usually in the range of -78°C to 100°C or the boiling point of the solvent, preferably in the range of around room temperature to 100°C.

Synthesis of compound (24c)

This step involves eliminating the protective group $R^{35}$.
Compound (24c) can be obtained by subjecting compound (30) to conditions appropriately selected from the reaction conditions described in the method for producing compound (24a).

[Production method 9]

Next, synthesis of the compound represented by the general formula (I) using compound (12) and compound (24) will be described.

[Formula 27]

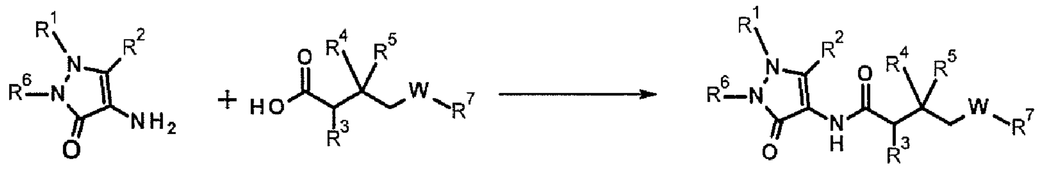

(12) (24) (I)

wherein $R^1$, $R^2$, $R^3$, $R^4$, $R^5$, $R^6$, $R^7$, and W are as defined above.

Synthesis of compound (I)

Compound (I) can be obtained by reacting compound (12) with compound (24) in the presence of a condensing agent. Examples of the condensing agent used can include O-(7-azabenzotriazol-1-yl)-N,N,N',N'-tetramethyluronium hexafluorophosphate (HATU), and 4-(4,6-dimethoxy-1,3,5-triazin-2-yl)-4-methylmorpholine (DMT-MM). Examples of the solvent include, but are not particularly limited to, ethanol, tetrahydrofuran, dioxane, acetonitrile, N,N-dimethylformamide, methylene chloride, toluene, and mixed solvents thereof. The reaction temperature is usually in the range of -78°C to 100°C or the boiling point of the solvent, preferably in the range of around room temperature to 100°C.

The compound of the general formula (I) can also be obtained by condensing compound (12) with site (B) under the conditions described above and then condensing the resulting condensation product with site (C) under suitable conditions appropriately selected from those described in production examples 6 to 8.

[Production method 10]

In the case of a compound of the general formula (I) wherein $R^2$ is represented by the general formula (2), and $R^{29}$ is an amino group substituted by a protective group $R^{31}$ (compound (4a)), the protective group can be deprotected. The deprotected amino group can be further modified with an alkyl group or the like to prepare compound (4b) or (4c).

[Formula 28]

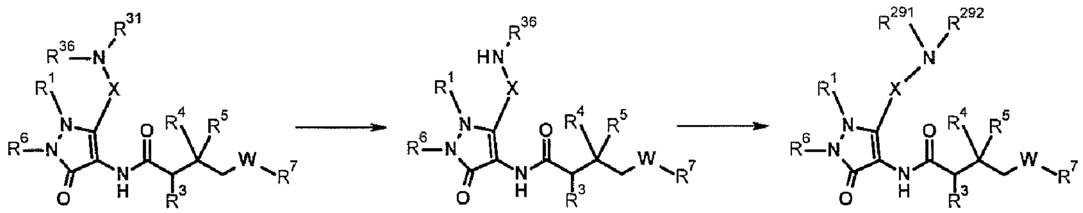

(4a) (4b) (4c)

wherein $R^1$, $R^3$, $R^4$, $R^5$, $R^6$, $R^7$, $R^{291}$, $R^{292}$, W, and X are as defined above, $R^{31}$ represents a protective group for the amino group and is as defined above, and $R^{36}$ represents a hydrogen atom, a $C_3$-$C_6$ cycloalkyl group, a $C_1$-$C_6$ alkyl group optionally having 1 to 3 substituents independently selected from "a halogen atom, a hydroxy group, a cyano group, a $C_1$-$C_6$ alkyl group, and a $C_1$-$C_6$ alkoxy group", or a phenyl-$C_1$-$C_6$ alkyl group optionally having, on the benzene ring, 1 to 3 substituents independently selected from "a halogen atom, a hydroxy group, a cyano group, a $C_1$-$C_6$ alkyl group, and a $C_1$-$C_6$ alkoxy group".

Synthesis of compound (4b)

This step involves deprotecting the protective group $R^{31}$ of compound (4a). The deprotection reaction conditions differ depending on the type of the protective group $R^{31}$. When $R^{31}$ is, for example, a tert-butoxycarbonyl group, this group can be deprotected by treatment with hydrochloric acid or trifluoroacetic acid. In this context, examples of the solvent used include, but are not particularly limited to, dioxane, ethyl acetate, methylene chloride, and mixed solvents thereof. The reaction temperature is usually in the range of -78°C to 100°C or the boiling point of the solvent, preferably in the range of 0°C to 100°C. When $R^{31}$ is, for example, a trifluoroacetyl group, this group can be deprotected by treatment

with a base such as sodium hydroxide or potassium hydroxide. In this context, examples of the solvent used include methanol, ethanol, water, tetrahydrofuran, dioxane, and mixed solvents thereof. An organic solvent that may be mixed with water at an arbitrary ratio is preferred. The reaction temperature is usually in the range of -78°C to 100°C or the boiling point of the solvent, preferably in the range of around room temperature to 100°C. When $R^{31}$ is an aralkyl group such as a benzyl group, this group can be deprotected through catalytic hydrogen reduction reaction in the presence of a palladium catalyst or can be deprotected by treatment with a strong acid such as trifluoroacetic acid. Examples of the solvent used in the catalytic hydrogen reduction reaction can include methanol and ethanol. The reaction temperature is usually in the range of -78°C to 100°C or the boiling point of the solvent, preferably in the range of around room temperature to 100°C. Examples of the solvent used in the strong acid treatment can include methylene chloride, or the solvent is not used. The reaction temperature is usually in the range of -78°C to 100°C or the boiling point of the solvent, preferably in the range of around room temperature to 100°C.

Synthesis of compound (4c)

Compound (4c) can be obtained by reacting compound (4b) with an aldehyde or a ketone in the presence of a reducing agent. Examples of the reducing agent used can include sodium triacetoxyborohydride, sodium cyanoborohydride, and sodium borohydride. Examples of the solvent used include, but are not particularly limited to, methanol, ethanol, water, tetrahydrofuran, dioxane, N,N-dimethylformamide, methylene chloride, acetic acid, and mixed solvents thereof. The reaction temperature is usually in the range of -78°C to 100°C or the boiling point of the solvent, preferably in the range of 0°C to 100°C.

Alternatively, compound (4c) can also be obtained by reacting compound (4b) with an alkylating agent such as alkyl halide or alkyl trifluoromethanesulfonate in the presence of a base. Examples of the base used can include triethylamine, N,N-diisopropylethylamine, and potassium carbonate. Examples of the solvent used include, but are not particularly limited to, tetrahydrofuran, dioxane, N,N-dimethylformamide, methylene chloride, and mixed solvents thereof. The reaction temperature is usually in the range of -78°C to 100°C or the boiling point of the solvent, preferably in the range of 0°C to 100°C.

When $R^{36}$ in compound (4b) is not a hydrogen atom, either $R^{291}$ or $R^{292}$ in compound (4c) is $R^{36}$.

[Production method 11]

A compound represented by the general formula (4) wherein $R^2$ is represented by the general formula (2), and $R^{29}$ is a hydroxy group (compound (4d)) can be converted to compound (4e) by modifying the hydroxy group with an alkyl group or the like.

[Formula 29]

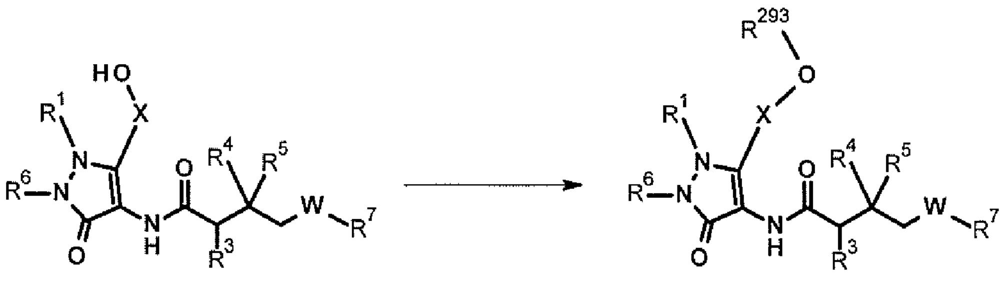

(4d) (4e)

wherein $R^1$, $R^3$, $R^4$, $R^5$, $R^6$, $R^7$, $R^{293}$, W, and X are as defined above.

Synthesis of compound (4e)

Compound (4e) can be obtained by reacting compound (4d) with an alkylating agent in the presence of a base. Examples of the alkylating agent can include alkyl halides and alkyl trifluoromethanesulfonates. Examples of the base used can include triethylamine, N,N-diisopropylethylamine, and potassium carbonate. Examples of the solvent used include, but are not particularly limited to, tetrahydrofuran, dioxane, N,N-dimethylformamide, methylene chloride, and mixed solvents thereof. The reaction temperature is usually in the range of -78°C to 100°C or the boiling point of the

solvent, preferably in the range of 0°C to 100°C.

Alternatively, compound (4e) can be obtained by reacting compound (4d) with $R^{293}$ substituted by a hydroxy group in the presence of phosphine and azodicarboxylic acid ester or azodicarboxylic acid amide. Examples of the phosphine used can include triphenylphosphine and tri-n-butylphosphine. Examples of the azodicarboxylic acid ester or the azodicarboxylic acid amide used can include diethyl azodicarboxylate, diisopropyl azodicarboxylate, and 1,1'-(azodicarbonyl)dipiperidine. Examples of the solvent used in the reaction include, but are not particularly limited to, tetrahydrofuran, dioxane, toluene, and mixed solvents thereof. The reaction temperature is usually in the range of -78°C to 100°C or the boiling point of the solvent, preferably in the range of 0°C to 100°C.

[Production method 12]

A compound represented by the general formula (4) wherein $R^2$ is represented by the general formula (2), and $R^{29}$ is an oxycarbonyl group protected with $R^{35}$ (compound (4f)) can be converted to compound (4g) by deprotection.

[Formula 30]

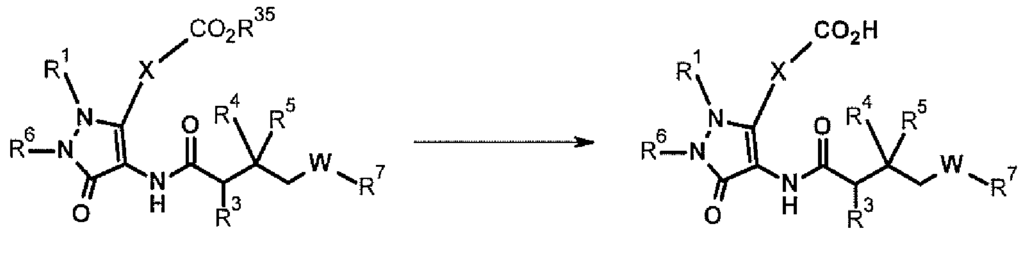

(4f) (4g)

wherein $R^1$, $R^3$, $R^4$, $R^5$, $R^6$, $R^7$, $R^{35}$, W, and X are as defined above.

Synthesis of compound (4g)

Compound (4g) can be obtained by subjecting compound (4f) to conditions appropriately selected from the reaction conditions described in the method for producing compound (24a).

In the present invention, stereoisomers of the compound represented by the general formula (I) can be obtained by using optically active starting compounds, by synthesizing the compound according to the present invention using an asymmetric synthesis or asymmetric induction approach, or by isolating the synthesized compound according to the present invention using a usual optical resolution or isolation method, if desired.

In the present invention, the compound represented by the general formula (I) also encompasses compounds labeled with atomic isotopes or radioisotopes. Such labeled compounds can be produced, for example, by using starting materials labeled with isotopes instead of the starting materials in the production methods of the present invention.

The compound represented by the general formula (I) of the present invention can form a salt through reaction with an acid when having a basic group.

The compound represented by the general formula (I) of the present invention or the salt thereof, when left in air or recrystallized, may incorporate water molecules to form a hydrate.

The compound represented by the general formula (I) of the present invention or the salt thereof, when left in a solvent or recrystallized in a solvent, may absorb certain kinds of solvents to form a solvate.

The compound of the present invention or the pharmacologically acceptable salt thereof can be administered in various forms. Examples of the dosage form can include: tablets, capsules, granules, emulsions, pills, powders, and syrups (solutions) for oral administration; and injections (intravenous, intramuscular, subcutaneous, or intraperitoneal administration), drip infusions, and suppositories (rectal administration) for parenteral administration. These various preparations can be formulated according to routine methods using auxiliary agents that may be usually used in the field of pharmaceutical formulation techniques, such as excipients, binders, disintegrants, lubricants, corrigents, solubilizers, suspending agents, and coating agents, in addition to the active ingredient.

For use as a tablet, examples of carriers that can be used include: excipients such as lactose, saccharose, sodium chloride, glucose, urea, starch, calcium carbonate, kaolin, crystalline cellulose, and silicic acid; binders such as water, ethanol, propanol, simple syrup, glucose solutions, starch solutions, gelatin solutions, carboxymethylcellulose, shellac, methylcellulose, potassium phosphate, and polyvinylpyrrolidone; disintegrants such as dry starch, sodium alginate, agar powder, laminaran powder, sodium bicarbonate, calcium carbonate, polyoxyethylene sorbitan fatty acid ester,

sodium lauryl sulfate, monoglyceride stearate, starch, and lactose; disintegration inhibitors such as saccharose, stearin, cocoa butter, and hydrogenated oil; absorption promoters such as quaternary ammonium salts and sodium lauryl sulfate; moisturizing agents such as glycerin and starch; adsorbents such as starch, lactose, kaolin, bentonite, and colloidal silicic acid; and lubricants such as purified talc, stearate, boric acid powder, and polyethylene glycol. Alternatively, tablets coated in usual manners, for example, sugar coated tablets, gelatin coated tablets, enteric coated tablets, film coated tablets, double layer tablets, and multilayered tablets may be prepared, if necessary.

For use as a pill, examples of carriers that can be used include: excipients such as glucose, lactose, cocoa butter, starch, hydrogenated plant oil, kaolin, and talc; binders such as gum arabic powder, powdered tragacanth, gelatin, and ethanol; and disintegrants such as laminaran and agar.

For use as a suppository, conventional carriers known in the art can be widely used. Examples thereof can include polyethylene glycol, cocoa butter, higher alcohols, esters of higher alcohols, gelatin, and semisynthetic glyceride.

For use as an injection, solutions, emulsions, or suspensions can be used. These solutions, emulsions, or suspensions are preferably sterilized and adjusted to be isotonic to blood. Any solvent that can be used as a medical diluent can be used without particular limitations in the production of these solutions, emulsions, or suspensions. Examples thereof can include water, ethanol, propylene glycol, ethoxylated isostearyl alcohol, polyoxylated isostearyl alcohol, and polyoxyethylene sorbitan fatty acid esters. In this case, each preparation may contain common salt, glucose, or glycerin in an amount sufficient for preparing an isotonic solution. Also, each preparation may contain a usual solubilizer, buffer, soothing agent, and the like.

Each of these preparations may also contain a colorant, a preservative, a fragrance, a flavor, a sweetener, and the like, if necessary, and may further contain an additional pharmaceutical product.

The amount of the active ingredient compound contained in each of these preparations is not particularly limited and is appropriately selected in a wide range. The composition usually contains 0.5 to 70% by weight, preferably 1 to 30% by weight of the compound with respect to the total weight.

The amount of the compound used differs depending on the symptoms, age, etc. of a patient (a warm-blooded animal, particularly, a human). The daily dose for oral administration to an adult human is 2000 mg (preferably 100 mg) as the upper limit and 0.1 mg (preferably 1 mg, more preferably 10 mg) as the lower limit and is desirably administered once to 6 times a day according to the symptoms.

Examples

Hereinafter, the present invention will be described in more detail with reference to Reference Examples, Examples, and Test Examples. However, the scope of the present invention is not intended to be limited by these examples.

Elution in column chromatography in the Reference Examples and Examples was carried out under observation by thin layer chromatography (TLC). In the TLC observation, silica gel 60 $F_{254}$ or silica gel 60 $NH_2F_{254}S$ manufactured by Merck KGaA or NHTLC plate manufactured by Fuji Silysia Chemical Ltd. was used as the TLC plate; a solvent used as an eluting solvent in column chromatography was used as the developing solvent; and a UV detector was adopted in the detection method. Silica gel SK-85 (230 to 400 mesh) manufactured by Merck KGaA or Chromatorex NH (200 to 350 mesh) manufactured by Fuji Silysia Chemical Ltd. was used as the silica gel for the columns. In addition to general column chromatography, an automatic purification apparatus from Shoko Scientific Co., Ltd. (Purif-a2 or Purif-espoir2), an automatic purification apparatus from Yamazen Corp. (YFLC-5404-FC), or an automatic purification apparatus from Biotage Japan Ltd. (HORIZON, SP1, or Isolera) was appropriately used. The eluting solvent used was a solvent specified on a Reference Example or Example basis. The proportion of the eluting solvent described in chromatographic separation and purification is indicated by volume ratio, unless otherwise specified. The abbreviations used in the Reference Examples and Examples are as defined below. mg: milligram, g: gram, μL: microliter, mL: milliliter, L: liter, and MHz: megahertz.

In the Examples below, nuclear magnetic resonance (hereinafter, referred to as $^1$H-NMR: 400 MHz or 500 MHz) spectra were indicated by chemical shift δ values (ppm) determined with tetramethylsilane as the standard. The measurement solvent is shown within parentheses. Splitting patterns were indicated by s = singlet, d = doublet, t = triplet, q = quartet, m = multiplet, and br = broad. The abbreviation "MS" means "mass spectrometry". The ionization method is shown within parentheses.

(Intermediate 1a) 4-[(5-Chloro-2-ethoxybenzoyl)amino]-3,3-dimethylbutanoic acid

[Formula 31]

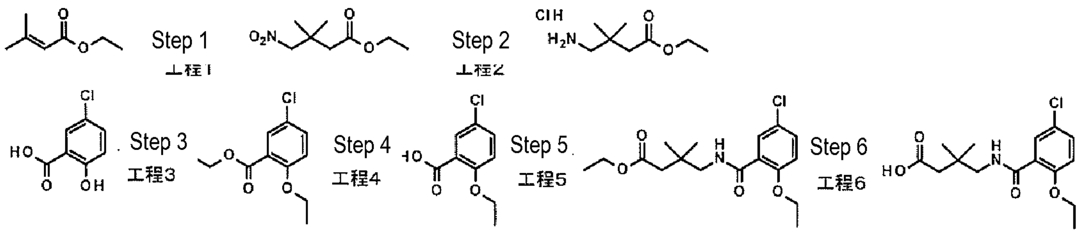


(Step 1) Ethyl 3,3-dimethyl-4-nitrobutanoate

Ethyl 3,3-dimethylacrylate (245 mL, 226 g, 1.76 mol) was dissolved in acetonitrile (1.8 L). To the solution, nitromethane (645 mL, 903 g, 14.8 mol) and 1,8-diazabicyclo(5,4,0)-7-undecene (394 mL, 402 g, 2.64 mol) were added, and the mixture was stirred at 60°C for 5 days. The temperature of the reaction solution was adjusted to room temperature, and the solvent was distilled off under reduced pressure. Then, the residue was neutralized with 2 N hydrochloric acid, followed by extraction with ethyl acetate. The organic layer was washed with saturated saline and then dried over anhydrous magnesium sulfate. After filtration, the filtrate was concentrated, and the obtained crude product was purified by silica gel column chromatography (Biotage Japan Ltd., eluting solvent: hexane/ethyl acetate = 100/0 to 70/30) to obtain the title compound (280 g, yield: 84.1%) as an oil substance.
$^1$H-NMR ($CDCl_3$) $\delta$: 4.54 (2H, s), 4.16 (2H, q, J = 7.2 Hz), 2.45 (2H, s), 1.28 (3H, t, J = 7.2 Hz), 1.17 (6H, s).

(Step 2) Ethyl 3,3-dimethyl-4-aminobutanoate hydrochloride

Ethyl 3,3-dimethyl-4-nitrobutanoate (178 g, 0.941 mmol) synthesized in step 1 was dissolved in ethanol (712 mL). To the solution, a 5% palladium carbon catalyst (M type) (83.0 g) and acetic acid (712 mL) were added, and the mixture was stirred at 70°C for 5 hours under a hydrogen atmosphere of 0.5 MPa. The temperature of the reaction solution was adjusted to room temperature. After filtration, the filtrate was concentrated. The same reaction as above was further carried out for two lots, and the obtained crude product in an amino form was dissolved in toluene (4.10 L). To the solution, di-tert-butyl dicarbonate (926 g, 4.24 mol) was added, and the mixture was stirred at room temperature for 20 hours. The reaction solution was poured into a water/ethyl acetate mixed solution, and the organic layer was washed with a saturated aqueous solution of sodium bicarbonate and saturated saline and then dried over sodium sulfate. After filtration, the filtrate was concentrated, and the obtained crude product in a Boc form was dissolved in ethyl acetate (2.82 L). To the solution, 4 N hydrochloric acid in ethyl acetate (2.82 L) was added, and the mixture was stirred at room temperature for 24 hours. The reaction solution was concentrated, and then, the residue was suspended in diisopropyl ether. Then, the solid was collected by filtration, washed with diisopropyl ether, and then dried to obtain the title compound (290 g, yield: 52.7%) as a solid.
$^1$H-NMR (DMSO-$D_6$) $\delta$: 8.16 (3H, s), 4.07 (2H, q, J = 7.2 Hz), 2.79 (2H, s), 2.37 (2H, s), 1.19 (3H, t, J = 7.2 Hz), 1.02 (6H, s).

(Step 3) Ethyl 5-chloro-2-ethoxybenzoate

5-Chlorosalicylic acid (50.0 g, 290 mmol) was dissolved in N,N-dimethylformamide (250 mL). To the solution, potassium carbonate (120 g, 869 mmol) and ethyl iodide (69.9 mL, 136 g, 869 mmol) were added, and the mixture was stirred at room temperature for 15 hours. Water was added to the reaction solution, followed by extraction with ethyl acetate. The organic layer was washed with saturated saline and then dried over anhydrous magnesium sulfate. After filtration, the filtrate was concentrated, and then, the obtained crude product was purified by silica gel column chromatography (Biotage Japan Ltd., eluting solvent: hexane/ethyl acetate = 95/5 to 70/30) to obtain the title compound (58.6 g, yield: 88.3%) as a solid.
$^1$H-NMR ($CDCl_3$) $\delta$: 7.73 (1H, d, J= 2.4 Hz), 7.37 (1H, dd, J = 8.8, 2.4 Hz), 6.88 (1H, d, J = 8.8 Hz), 4.35 (2H, q, J = 7.1 Hz), 4.08 (2H, q, J = 7.1 Hz), 1.44 (3H, t, J = 7.1 Hz), 1.37 (3H, t, J = 7.1 Hz).

(Step 4) 5-Chloro-2-ethoxybenzoic acid

Ethyl 5-chloro-2-ethoxybenzoate (58.6 g, 256 mmol) synthesized in step 3 was dissolved in ethanol (300 mL). To the solution, water (50 mL) and a 5 N aqueous sodium hydroxide solution (100 mL) were added, and the mixture was stirred at room temperature for 20 hours. Ethanol in the reaction solution was distilled off under reduced pressure,

and then, the residue was neutralized by the addition of 5 N hydrochloric acid under ice cooling, followed by extraction with ethyl acetate. The organic layer was washed with saturated saline and then dried over anhydrous magnesium sulfate. After filtration, the filtrate was concentrated to obtain the title compound (51.4 g, yield: 100%) as a solid.
$^1$H-NMR ($CDCl_3$) $\delta$: 10.88 (1H, s), 8.17 (1H, t, J = 2.4 Hz), 7.51 (1H, dd, J = 8.8, 2.4 Hz), 7. 00 (1H, d, J = 8.8 Hz), 4.33 (2H, q, J = 7.0 Hz), 1.58 (3H, t, J = 7.0 Hz).

(Step 5) Ethyl 4-[(5-chloro-2-ethoxybenzoyl)amino]-3,3-dimethylbutanoate

5-Chloro-2-ethoxybenzoic acid (21.8 g, 109 mmol) synthesized in step 4 and ethyl 3,3-dimethyl-4-aminobutanoate hydrochloride (21.5 g, 110 mmol) synthesized in step 2 were dissolved in ethanol (300 mL) and N,N-diisopropylethylamine (19.2 mL, 14.2 g, 110 mmol). To the solution, 4-(4,6-dimethoxy-1,3,5-triazin-2-yl)-4-methylmorpholinium chloride n-hydrate (DMT-MM) (38.6 g, 131 mmol) was added, and the mixture was stirred at room temperature for 24 hours. The solvent was distilled off under reduced pressure, and water and a saturated aqueous solution of sodium bicarbonate were added to the residue, followed by extraction with ethyl acetate. The organic layer was washed with saturated saline and then dried over anhydrous magnesium sulfate. After filtration, the filtrate was concentrated, and then, the obtained crude product was purified by silica gel column chromatography (Biotage Japan Ltd., eluting solvent: hexane/ethyl acetate = 90/10 to 50/50) to obtain the title compound (36.7 g, yield: 98.5%) as a solid.
$^1$H-NMR ($CDCl_3$) $\delta$: 8.19 (1H, d, J= 2.4 Hz), 8.16-8.14 (1H, br m), 7.36 (1H, dd, J = 8.8, 2.4 Hz), 6.90 (1H, d, J = 8.8 Hz), 4.20 (2H, q, J= 7.1 Hz), 4.13 (2H, q, J = 7.1 Hz), 3.43 (2H, d, J = 6.3 Hz), 2.28 (2H, s), 1.56 (2H, s), 1.52 (3H, t, J= 7.1 Hz), 1.26 (3H, t, J = 7.1 Hz), 1.08 (6H, s).

(Step 6) 4-[(5-Chloro-2-ethoxybenzoyl)amino]-3,3-dimethylbutanoic acid (intermediate 1a)

Ethyl 4-[(5-chloro-2-ethoxybenzoyl)amino]-3,3-dimethylbutanoate (36.7 g, 107 mmol) synthesized in step 5 was dissolved in ethanol (300 mL). To the solution, a 2 N aqueous sodium hydroxide solution (150 mL) was added, and the mixture was stirred at room temperature for 24 hours. Ethanol was distilled off under reduced pressure, and the residue was neutralized by the addition of 2 N hydrochloric acid, followed by extraction with ethyl acetate. The organic layer was washed with saturated saline and then dried over anhydrous magnesium sulfate. After filtration, the filtrate was concentrated to obtain the title compound (intermediate 1a) (32.5 g, yield: 96.9%) as a solid.
$^1$H-NMR ($CDCl_3$) $\delta$: 8.56-8.54 (1H, br m), 8.21 (1H, d, J = 2.4 Hz), 7.43 (1H, dd, J = 8.8, 2.4 Hz), 6.94 (1H, d, J = 8.8 Hz), 4.23 (2H, q, J = 7.0 Hz), 3.42 (2H, d, J = 6.8 Hz), 2.28 (2H, s), 1.53 (3H, t, J = 7.0 Hz), 1.11 (6H, s).
The intermediates of Table 1 were synthesized from the corresponding starting materials in the same way as above.

[Table 1]

| | | |
|---|---|---|
| Intermediate 1 b | 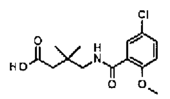 | $^1$H-NMR ($CDCl_3$) $\delta$: 8.38 (1H, br s), 8.19 (1H, d, J = 2.6 Hz), 7.45 (1H, dd, J 9.2, 2.6 Hz), 6.96 (1H, d, J = 9.2 Hz), 4.00 (3H, s), 3.42 (2H, d, J = 6.9 Hz), 2.28 (2H, s), 1.11 (6H, s). |
| Intermediate 1 c | 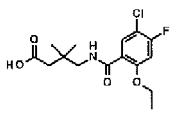 | $^1$H-NMR ($CDCl_3$) $\delta$: 8.39-8.37 (1H, br m), 8.30 (1H, d, J = 8.8 Hz), 6.81 (1H, d, J = 10.3 Hz), 4.21 (2H, q, J = 7.0 Hz), 3.41 (2H, d, J = 6.3 Hz), 2.27 (2H, s), 1.55 (3H, t, J = 7.0 Hz), 1.10 (6H, s). |
| Intermediate 1 d | 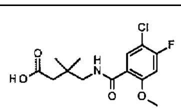 | $^1$H-NMR ($CDCl_3$) $\delta$: 8.29 (1H, dd, J = 8.8, 1,4 Hz), 8.22 (1H, brs), 6.83 (1H, dd, J = 10.4, 1.4 Hz), 4.00 (3H, s), 3.42 (2H, d, J = 6.8 Hz), 2.28 (2H, s), 1.10 (6H, s). |
| Intermediate 1 e | 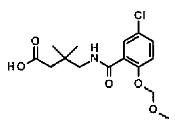 | $^1$H-NMR ($CDCl_3$) $\delta$: 8.34-8.32 (1H, br m), 8.18 (1H, d, J = 2.9 Hz), 7.41 (1H, dd, J = 8.8, 2.9 Hz), 7.17 (1H,d, J = 8.8 Hz), 5.34 (2H, s), 3.54 (3H, s), 3.44 (2H, d, J = 6.6 Hz), 2.29 (2H, s), 1.09 (6H, s). |
| Intermediate 1 f | 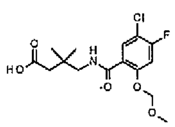 | $^1$H-NMR ($CDCl_3$) $\delta$: 8.29 (1H, d, J = 8.3Hz), 8.24-8.23 (1H, br m), 7.10 (1H, d, J = 10.3 Hz), 5.35 (2H, s), 3.54 (3H, s), 3.44 (2H, d, J = 6.8 Hz), 2.30 (2H, s), 1.11 (6H, s). |

(continued)

| | | |
|---|---|---|
| Intermediate 1 g | 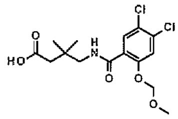 | $^1$H-NMR ($CDCl_3$) $\delta$: 8.28 (1H, s), 8.23-8.22 (1H, br m), 7.37 (1H, s), 5.34 (2H, s), 3.53 (3H, s), 3.43 (2H, d, J = 6.3 Hz), 2.29 (2H, s), 1.11 (6H, s). |
| Intermediate 1 h | 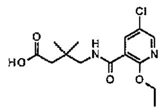 | $^1$H-NMR ($CDCl_3$) $\delta$: 8.50 (1H, d, J = 2.9 Hz), 8.49-8.45 (1H, br m), 8.23 (1H,d, J = 2.9 Hz), 4.57 (2H, q, J = 7.0 Hz), 3.44 (2H, d, J = 6.3 Hz), 2.28 (2H, s), 1.48 (3H, t, J = 7.0 Hz), 1.12 (6H, s). |
| Intermediate 1 i | 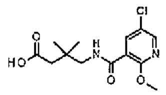 | $^1$H-NMR ($CDCl_3$) $\delta$: 8.49 (1H, d, J = 2.9 Hz), 8.38 (1H, br s), 8.24 (1H, d, J = 2.9 Hz), 4.12 (3H, s), 3.44 (2H, d, J = 6.9 Hz), 2.29 (2H, s), 1.11 (6H, s). |
| Intermediate 1 j | 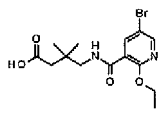 | $^1$H-NMR ($CDCl_3$) $\delta$: 8.62 (1H, d, J = 2.4 Hz), 8.47-8.45 (1H, br m), 8.32 (1H, d, J = 2.4 Hz), 4.56 (2H, q, J = 7.2 Hz), 3.44 (2H, d, J = 6.8 Hz), 2.28 (2H, s), 1.48 (3H, t, J = 7.2 Hz), 1.11 (6H, s). |
| Intermediate 1 k | 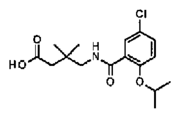 | $^1$H-NMR ($CDCl_3$) $\delta$: 8.66 (1H,br s), 8.22 (1H,d, J = 2.9 Hz), 7.42 (1H, dd, J = 8.8, 2.9 Hz), 6.95 (1H, d, J = 8.8 Hz), 4.82-4.75 (1H, m), 3.41 (2H, d, J = 6.8 Hz), 2.27 (2H, s), 1.45 (6H, d, J = 6.3 Hz), 1.11 (6H, s). |

(Intermediate 11) 4-{[5-Chloro-2-(cyclopropyl)benzoyl]amino}-3,3-dimethylbutanoic acid

[Formula 32]

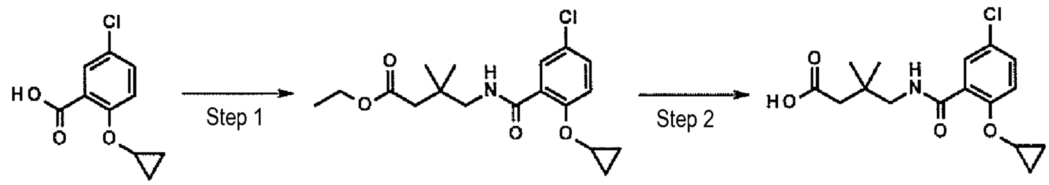


(Step 1) Ethyl 4-{[5-chloro-2-(cyclopropyl)benzoyl]amino}-3,3-dimethylbutanoate

To a solution of 5-chloro-2-(cyclopropyl)benzoic acid (0.106 g, 0.500 mmol) synthesized according to the method described in WO2009/67613 in methylene chloride (10 mL), oxalyl chloride (0.087 mL, 0.127 g, 1.00 mmol) and a catalytic amount of N,N-dimethylformamide were added, and the mixture was stirred at room temperature for 2.5 hours. The reaction solution was concentrated under reduced pressure, then methylene chloride (10 mL), ethyl 3,3-dimethyl-4-aminobutanoate hydrochloride (0.127 g, 0.550 mmol) obtained in step 2 of intermediate 1a, and triethylamine (0.209 mL, 0.152 g, 1.50 mmol) were added to the residue, and the mixture was stirred at room temperature for 1 hour. The reaction solution was concentrated under reduced pressure, and then, the obtained residue was purified by silica gel column chromatography (Yamazen Corp., eluting solvent: hexane/ethyl acetate = 74/26 to 63/37) to obtain the title compound (0.168 g, yield: 95.0%) as an oil substance.
$^1$H-NMR ($CDCl_3$) $\delta$: 8.16 (1H, d, J= 2.9 Hz), 7.93 (1H, s), 7.39 (1H, dd, J= 8.6, 2.9 Hz), 7.32 (1H, d, J= 8.6 Hz), 4.15 (2H, q, J = 7.2 Hz), 3.88-3.84 (1H, m), 3.39 (2H, d, J= 6.3 Hz), 2.26 (2H, s), 1.27 (3H, t, J= 7.2 Hz), 1.06 (6H, s), 0.91-0.90 (4H, m).
MS (API) m/z : 354 [$(M+H)^+$].

(Step 2) 4-{[5-Chloro-2-(cyclopropyl)benzoyl]amino}-3,3-dimethylbutanoic acid

To a solution of ethyl 4-{[5-chloro-2-(cyclopropyl)benzoyl]amino}-3,3-dimethylbutanoate (0.165 g, 0.466 mmol)

synthesized in step 1 in tetrahydrofuran (5.0 mL), methanol (1.4 mL) and a 1 N aqueous sodium hydroxide solution (1.40 mL, 1.40 mmol) were added, and the mixture was stirred overnight at room temperature. The reaction solution was concentrated under reduced pressure, and then, 1 N hydrochloric acid was added to the residue, followed by extraction with ethyl acetate three times. The organic layer was washed with saturated saline and then dried over anhydrous sodium sulfate. After filtration, the filtrate was concentrated under reduced pressure to obtain the title compound (0.151 g, yield: 99.4%) as a solid.
$^1$H-NMR ($CDCl_3$) δ: 8.22 (1H, s), 8.17 (1H, d, J = 2.9 Hz), 7.44 (1H, dd, J = 8.6, 2.9 Hz), 7.35 (1H, d, J = 8.6 Hz), 3.92-3.89 (1H, m), 3.40 (2H, d, J = 6.9 Hz), 2.26 (2H, s), 1.09 (6H, s), 0.98-0.91 (2H, m), 0.90-0.85 (2H, m).
MS (API) m/z : 326 [(M+H)$^+$].

(Intermediate 1m) 4-[(5-Chloro-4-fluoro-2-isopropoxybenzoyl)amino]-3-hydroxy-3-methylbutanoic acid

[Formula 33]

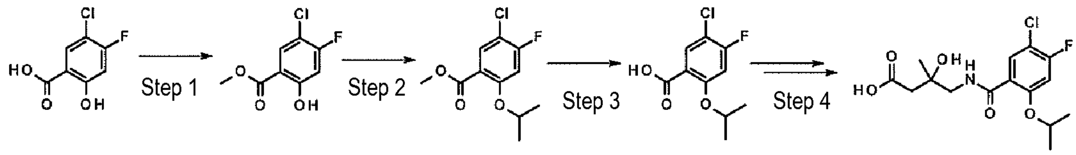


(Step 1) Methyl 5-chloro-4-fluoro-2-hydroxybenzoate

To a solution of 5-chloro-4-fluoro-2-hydroxybenzoic acid (4.98 g, 26.1 mmol) synthesized according to the method described in US2006/69093 in methanol (50 mL), concentrated sulfuric acid (2.0 mL) was added, and the mixture was stirred overnight under heating to reflux. The reaction solution was allowed to cool to room temperature and then concentrated under reduced pressure, and a saturated aqueous solution of sodium bicarbonate was added to the residue, followed by extraction with ethyl acetate three times. The organic layer was washed with saturated saline and then dried over anhydrous sodium sulfate. After filtration, the filtrate was concentrated under reduced pressure, and then, the obtained residue was purified by silica gel column chromatography (Yamazen Corp., eluting solvent: hexane/chloroform = 99/1 to 80/20) to obtain the title compound (4.63 g, yield: 86.6%) as a solid.
$^1$H-NMR ($CDCl_3$) δ: 10.90 (1H, d, J = 1.7 Hz), 7.90 (1H, d, J= 8.6 Hz), 6.78 (1H, d, J = 10.3 Hz), 3.96 (3H, s).

(Step 2) Methyl 5-chloro-4-fluoro-2-isopropoxybenzoate

To methyl 5-chloro-4-fluoro-2-hydroxybenzoate (2.30 g, 11.2 mmol) synthesized in step 1, potassium carbonate (4.66 g, 33.7 mmol), acetone (50 mL), isopropyl iodide (5.59 mL, 9.55 g, 56.2 mmol) were added, and the mixture was stirred overnight under heating to reflux. The reaction solution was allowed to cool to room temperature. After filtration, the filtrate was concentrated under reduced pressure, and then, the obtained residue was purified by silica gel column chromatography (Yamazen Corp., eluting solvent: hexane/ethyl acetate = 100/0 to 82/18) to obtain the title compound (3.02 g, quantitative) as an oil substance.
$^1$H-NMR ($CDCl_3$) δ: 7.87 (1H, d, J = 9.2 Hz), 6.77 (1H, d, J= 10.9 Hz), 4.55-4.48 (1H, m), 3.87 (3H, s), 1.39 (6H, d, J = 6.3 Hz).
MS (API) m/z : 247 [(M+H)$^+$].

(Step 3) 5-Chloro-4-fluoro-2-isopropoxybenzoic acid

To a solution of methyl 5-chloro-4-fluoro-2-isopropoxybenzoate (2.77 g, 11.2 mmol) synthesized in step 2 in tetrahydrofuran (70 mL), methanol (33 mL) and 1 N hydrochloric acid (33.7 mL, 33.7 mmol) were added, and the mixture was stirred at room temperature for 3.5 hours. The reaction solution was concentrated under reduced pressure, and then, 1 N hydrochloric acid was added to the residue. The deposited solid was suspended in water, collected by filtration, and dried to obtain the title compound (2.58 g, yield: 98.6%) as a solid.
$^1$H-NMR ($CDCl_3$) δ: 8.13 (1H, d, J = 2.6 Hz), 7.53 (1H, dd, J = 8.6, 2.6 Hz), 7.38 (1H, d, J = 8.6 Hz), 4.01-3.97 (1H, m), 1.01-0.94 (4H, m).
MS (API) m/z : 233 [(M+H)$^+$].

(Step 4) 4-[(5-Chloro-4-fluoro-2-isopropoxybenzoyl)amino]-3-hydroxy-3-methylbutanoic acid

The title compound was obtained as an oil substance by using 5-chloro-4-fluoro-2-isopropoxybenzoic acid synthesized in step 3 instead of 5-chloro-2-(cyclopropyl)benzoic acid and ethyl 4-amino-3-hydroxy-3-methylbutanoate hydrochloride synthesized according to the method described in J. Org. Chem. 1985, 50, 3627-3631 instead of ethyl 3,3-dimethyl-4-aminobutanoate hydrochloride in step 1 in the synthesis of intermediate 11 and subsequently performing the same reaction as in the synthesis of intermediate 11 up to step 2.
$^1$H-NMR ($CDCl_3$) δ: 8.41 (1H, br t, J = 5.4 Hz), 8.28 (1H, d, J = 9.2 Hz), 6.78 (1H, d, J = 10.9 Hz), 4.71-4.63 (1H, m), 4.28 (1H, s), 4.21-4.14 (2H, m), 3.57 (1H, dd, J = 13.7, 6.3 Hz), 3.50 (1H, dd, J = 13.7, 5.2 Hz), 2.61 (1H, d, J = 16.0 Hz), 2.49 (1H, d, J = 16.0 Hz), 1.45 (6H, d, J = 5.7 Hz), 1.30 (3H, s), 1.27 (3H, t, J = 7.2 Hz).
MS (ESI) m/z : 376 [$(M+H)^+$].

(Intermediate 1n) 4-[(5-Chloro-2-ethoxybenzoyl)amino]-2-fluoro-3,3-dimethylbutanoic acid

[Formula 34]

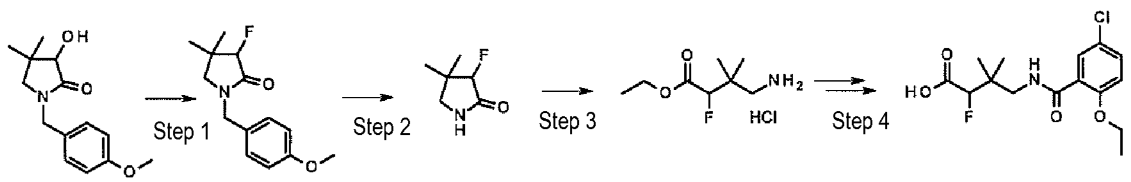


(Step 1) 3-Fluoro-1-[(4-methoxyphenyl)methyl]-4,4-dimethylpyrrolidin-2-one

A solution of 3-hydroxy-1-[(4-methoxyphenyl)methyl]-4,4-dimethylpyrrolidin-2-one (500 mg, 2.00 mmol) synthesized by the method described in Tetrahedron Asymmetry 2010, 21, 2124-2135 and pyridine (0.480 ml, 475 mg, 6.00 mmol) in methylene chloride (10 ml) was cooled in ice. Trifluoromethanesulfonic anhydride (0.400 ml, 688 mg, 2.44 mmol) was added dropwise thereto, and the mixture was stirred at the same temperature as above for 30 minutes. The reaction solution was diluted with methylene chloride. The organic layer was washed with a saturated aqueous solution of sodium bicarbonate, a saturated aqueous solution of copper sulfate, and saturated saline in this order and then dried over anhydrous sodium sulfate. After filtration, the filtrate was concentrated, and the obtained residue was dissolved in tetrahydrofuran (16 ml). The solution was cooled in ice. A solution of 1 N tetra-n-butyl ammonium fluoride in tetrahydrofuran (4 ml) was added thereto, and the reaction solution was brought back to room temperature and stirred for 1 hour. The solvent was distilled off under reduced pressure, and then, the residue was diluted with ethyl acetate. The organic layer was washed with saturated saline (twice) and dried over anhydrous sodium sulfate. After filtration, the filtrate was concentrated, and the obtained crude product was purified by silica gel column chromatography (eluting solvent: hexane/ethyl acetate = 75/25) to obtain the title compound (435 mg, yield: 86.5%) as an oil substance.
$^1$H-NMR ($CDCl_3$) δ: 7.16 (2H, d, J = 8.6 Hz), 6.87 (2H, d, J = 8.6 Hz), 4.64 (1H, d, J = 53 Hz), 4.47 (1H, d, J = 4.5 Hz), 4.33 (1H, d, J = 4.5 Hz), 3.80 (3H, s), 2.96 (1H, dd, J = 10, 1.8 Hz), 2.90 (1H, d, J = 10 Hz), 1.15 (3H, s), 1.04 (3H, s).

(Step 2) 3-Fluoro-4,4-dimethylpyrrolidin-2-one

A solution of 3-fluoro-1-[(4-methoxyphenyl)methyl]-4,4-dimethylpyrrolidin-2-one (1.20 g, 4.77 mmol) synthesized in step 1 in acetonitrile (24 ml) was cooled in ice. A solution of ammonium hexanitratocerate(IV) (5.23 g, 9.54 mmol) in water (5 ml) was added dropwise thereto, and the reaction solution was brought back to room temperature and stirred for 3 hours. Potassium carbonate (2.5 g) was added to the reaction solution, and the mixture was stirred for 10 minutes and then filtered through celite to remove insoluble matter. The obtained filtrate was concentrated, then subjected to azeotropy with ethanol several times, and dissolved in chloroform, followed by drying over anhydrous sodium sulfate. After filtration, the filtrate was concentrated, and the obtained crude product was purified by silica gel column chromatography (eluting solvent: hexane/ethyl acetate = 34/66) to obtain the title compound (395 mg, yield: 63.1%) as a solid.
$^1$H-NMR ($CDCl_3$) δ: 7.14 (1H, br), 4.61 (1H, d, J = 52 Hz), 3.10-3.17 (2H, m), 3.14 (3H, s), 3.12 (3H, s).

(Step 3) Ethyl 4-amino-2-fluoro-3,3-dimethylbutanoate hydrochloride

3-Fluoro-4,4-dimethylpyrrolidin-2-one (350 mg, 2.67 mmol) synthesized in step 2 in 6 N hydrochloric acid (10 ml) was heated to reflux for 16 hours. After concentration, the residue was subjected to azeotropy with ethanol several times, and the obtained residue was dissolved in ethanol (10 ml). To the solution, acetyl chloride (0.65 ml, 9.11 mmol) was added at room temperature, and the mixture was heated to reflux for 2 hours. The reaction solution was brought back to room temperature and concentrated to obtain the title compound (570 mg, quantitative) as a solid.
$^1$H-NMR (DMSO-$D_6$) δ: 8.23 (3H, brs), 5.10 (1H, dd, J = 48, 1.8 Hz), 4.28-4.19 (2H, m), 2.83 (2H, q, J = 13 Hz), 1.26-1.22 (3H, m), 1.04 (3H, s), 1.01 (3H, s).

(Step 4) 4-[(5-Chloro-2-ethoxybenzoyl)amino]-2-fluoro-3,3-dimethylbutanoic acid

The title compound was obtained as a solid by using ethyl 4-amino-2-fluoro-3,3-dimethylbutanoate hydrochloride synthesized in step 3 instead of ethyl 3,3-dimethyl-4-aminobutanoate in step 5 in the synthesis of intermediate 1a and subsequently performing the same reaction as in steps 5 and 6 in the synthesis of intermediate 1a.
$^1$H-NMR ($CDCl_3$) δ: 8.72 (1H, t, J = 5.8 Hz), 8.19 (1H, d, J= 3.0 Hz), 7.44 (1H, dd, J = 8.8, 2.7 Hz), 6.95 (1H, d, J = 9.1 Hz), 4.67 (1H, d, J = 48.0 Hz), 4.24 (2H, q, J = 6.9 Hz), 3.65 (1H, dd, J = 14.6, 7.3 Hz), 3.26 (1H, ddd, J = 15.0, 6.2, 4.1 Hz), 1.55 (3H, t, J = 7.0 Hz), 1.17 (3H, d, J = 1.8 Hz), 1.14 (3H, s).
MS (ESI) m/z : 332 [$(M+H)^+$].

(Intermediate 2a) 5-[(5-Chloro-2-ethoxy-3-pyridyl)amino]-3,3-dimethyl-5-oxopentanoic acid

[Formula 35]

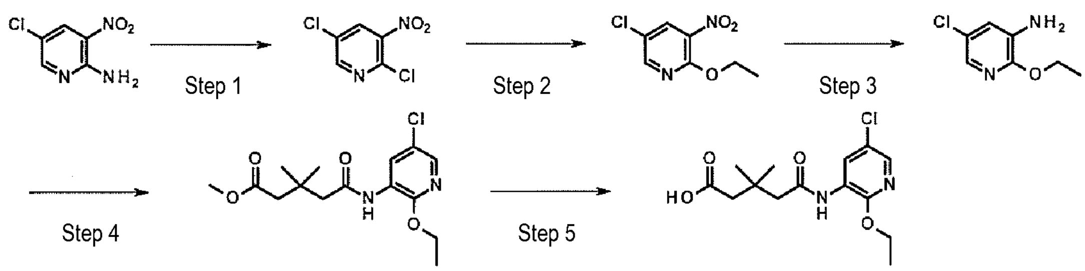


(Step 1) 2,5-Dichloro-3-nitropyridine

5-Chloro-3-nitropyridin-2-amine (10.0 g, 57.6 mmol) was dissolved in concentrated hydrochloric acid (100 mL), and the solution was stirred at -10°C. A solution of sodium nitrite (9.94 g, 144 mmol) dissolved in water (20 mL) was added dropwise thereto over 30 minutes. After the completion of the addition, the mixture was stirred at 0°C for 1 hour. The pH of the reaction solution was adjusted to 9 by the addition of a 1 N aqueous sodium hydroxide solution, followed by extraction with ethyl acetate. Insoluble matter was removed by filtration, and the organic layer was washed with saturated saline and then dried over anhydrous magnesium sulfate. After filtration, the solvent in the filtrate was distilled off under reduced pressure to obtain the title compound (6.50 g, yield: 58.5%) as a solid.
$^1$H-NMR ($CDCl_3$) δ: 8.60 (1H, d, J = 2.9 Hz), 8.24 (1H, d, J = 2.6 Hz).

(Step 2) 5-Chloro-2-ethoxy-3-nitropyridine

2,5-Dichloro-3-nitropyridine (6.00 g, 31.1 mmol) synthesized in step 1 was dissolved in ethanol (30 mL). To the solution, a 21% solution of sodium ethoxide in ethanol (20 mL) was added, and the mixture was stirred at 70°C for 5 hours. The reaction solution was brought back to room temperature. Then, the solvent in the reaction solution was distilled off under reduced pressure, and water was added to the residue, followed by extraction with ethyl acetate. The organic layer was washed with saturated saline and then dried over anhydrous magnesium sulfate. After filtration, the filtrate was concentrated under reduced pressure, and the obtained crude product was purified by silica gel column chromatography (Biotage Japan Ltd., eluting solvent: hexane/ethyl acetate = 100/0 to 85/15) to obtain the title compound (3.39 g, 53.8%) as a solid.

$^1$H-NMR ($CDCl_3$) δ: 8.33 (1H, d, J = 2.3 Hz), 8.24 (1H, d, J = 2.3 Hz), 4.55 (2H, q, J = 7.1 Hz), 1.45 (3H, t, J = 7.1 Hz).

(Step 3) 5-Chloro-2-ethoxypyridin-3-amine

5-Chloro-2-ethoxy-3-nitropyridine (3.39 g, 16.7 mmol) synthesized in step 2 was dissolved in ethanol (30 mL). To the solution, 5% platinum carbon sulfide (0.40 g) was added, and the mixture was stirred at room temperature for 5 hours under a hydrogen atmosphere. After filtration of the reaction solution, the filtrate was concentrated, and then, the obtained crude product was purified by silica gel column chromatography (Biotage Japan Ltd., eluting solvent: hexane/ethyl acetate = 95/5 to 75/25) to obtain the title compound (2.66 g, yield: 92.3%) as a solid.
$^1$H-NMR ($CDCl_3$) δ: 7.47 (1H, d, J = 2.3 Hz), 6.85 (1H, d, J = 2.3 Hz), 4.37 (2H, q, J = 7.1 Hz), 3.87 (2H, s), 1.40 (3H, t, J = 7.1 Hz).

(Step 4) Methyl 5-[(5-chloro-2-ethoxy-3-pyridyl)amino]-3,3-dimethyl-5-oxopentanoate

5-Methoxy-3,3-dimethyl-5-oxopentanoic acid (1.92 g, 11.0 mmol) was dissolved in methylene chloride (30 mL). To the solution, a catalytic amount of N,N-dimethylformamide was added, and the mixture was stirred at room temperature. Oxalyl chloride (1.86 mL, 2.79 g, 22.0 mmol) was added dropwise thereto. After the completion of the addition, the mixture was stirred at room temperature for 30 minutes. The solvent was distilled off under reduced pressure, and the residue was dried and then dissolved in methylene chloride (15 mL). To the solution, a solution of 5-chloro-2-ethoxypyridin-3-amine (1.73 g, 10.0 mmol) synthesized in step 3 and triethylamine (5.58 mL, 4.05 g, 40.0 mmol) in methylene chloride (15 mL) was added, and the mixture was stirred at room temperature for 4 hours. Water was added thereto, followed by extraction with methylene chloride. The organic layer was dried over anhydrous magnesium sulfate. After filtration, the filtrate was concentrated, and the obtained crude product was purified by silica gel column chromatography (Biotage Japan Ltd., eluting solvent: hexane/ethyl acetate = 95/5 to 65/35) to obtain the title compound (3.18 g, yield: 96.7%) as a solid.
$^1$H-NMR ($CDCl_3$) δ: 8.75 (1H, s), 8.68 (1H, d, J = 2.3 Hz), 7.76 (1H, d, J = 2.3 Hz), 4.39 (2H, q, J = 7.1 Hz), 3.73 (3H, s), 2.51 (2H, s), 2.42 (2H, s), 1.41 (3H, t, J = 7.1 Hz), 1.13 (6H, s).

(Step 5) 5-[(5-Chloro-2-ethoxy-3-pyridyl)amino]-3,3-dimethyl-5-oxopentanoic acid (intermediate 2a)

Methyl 5-[(5-chloro-2-ethoxy-3-pyridyl)amino]-3,3-dimethyl-5-oxopentanoate (3.18 g, 9.67 mmol) synthesized in step 4 was dissolved in ethanol (40 mL). To the solution, a 2 N aqueous sodium hydroxide solution (20 mL) was added, and the mixture was stirred at room temperature for 3 days. Ethanol was distilled off under reduced pressure, and the residue was neutralized by the addition of 1 N hydrochloric acid, followed by extraction with ethyl acetate. The organic layer was washed with saturated saline and then dried over anhydrous magnesium sulfate. After filtration, the filtrate was concentrated to obtain the title compound (intermediate 2a) (2.85 g, yield: 93.4%) as a solid.
$^1$H-NMR ($CDCl_3$) δ: 8.65 (1H, d, J = 2.3 Hz), 8.25 (1H, s), 7.81 (1H, d, J = 2.3 Hz), 4.42 (2H, q, J = 7.1 Hz), 2.53 (2H, s), 2.49 (2H, s), 1.40 (3H, t, J= 7.1 Hz), 1.19 (6H, s).
The intermediates of Table 2 were synthesized from the corresponding starting materials in the same way as above.

[Table 2]

| | | |
|---|---|---|
| Intermediate 2 b | 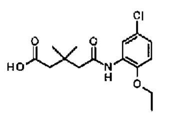 | $^1$H-NMR ($CDCl_3$) δ: 8.37 (1H, d, J = 2.6 Hz), 8.18 (1H, s), 7.04 (1H, dd, J = 8.9, 2.6 Hz), 6.79 (1H, d, J = 8.9 Hz), 4.09 (2H, q, J = 6.9 Hz), 2.51 (2H, s), 2.50 (2H, s), 1.44 (3H, t, J = 6.9 Hz), 1.19 (6H, s). |
| Intermediate 2 c | 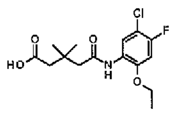 | $^1$H-NMR ($CDCl_3$) δ: 8.40 (1H, d, J = 7.9 Hz), 8,10 (1 H, s), 6.70 (1H, d, J = 10.4 Hz), 4.08 (2H, q, J = 6.9 Hz), 2.50 (2H, s), 2.49 (2H, s), 1.46 (3H, t, J = 6.9 Hz), 1.19 (6H, s). |
| Intermediate 2 d | 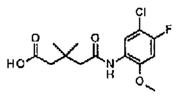 | $^1$H-NMR ($CDCl_3$) δ: 8.44 (1H, d, J = 8.0Hz), 8.11 (1H, s), 6.71 (1H, d, J = 10.3 Hz), 3.87 (3H, s), 2.50 (4H, s), 1.19 (6H, s). |

(continued)

| | | |
|---|---|---|
| Intermediate 2 e | 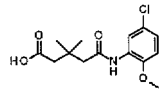 | $^1$H-NMR ($CDCl_3$) δ: 8.40 (1H, d, J =2.3 Hz), 8.17 (1H, s), 7.06 (1H, dd, J = 8.6, 2.3 Hz), 6.81 (1H, d, J = 8.6 Hz), 3.88 (3H, s), 2.50 (4H, s), 1.19 (6H, s). |
| Intermediate 2 f | 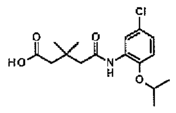 | $^1$H-NMR ($CDCl_3$) δ: 8.37 (1H,d, J = 2.9 Hz), 8.14 (1H, s), 7.04 (1H, dd, J = 9.2, 2.9 Hz), 6.81 (1H, d, J = 9.2 Hz), 4.59-4.57 (1H, m), 2.50 (2H, s), 2.49 (2H, s), 1.36 (6H, d, J = 6.3 Hz), 1.19 (6H, s). |
| Intermediate 2 g | 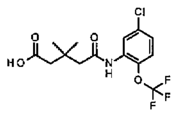 | $^1$H-NMR ($CDCl_3$) δ: 8.63 (1H, br s), 8.56 (1H, d, J = 2.7 Hz), 7.19-7.17 (1H, m), 7.07 (1H, dd, J = 8.8, 2.7 Hz), 2.54 (2H, s), 2.46 (2H, s), 1.19 (6H, s). |

(Intermediate 2h) 2-{1-[2-(5-Chloro-2-ethoxyanilino)-2-oxoethyl]cyclopentyl}acetic acid

[Formula 36]

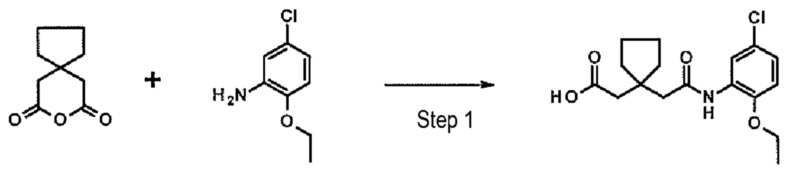


(Step 1) 2-{1-[2-(5-Chloro-2-ethoxyanilino)-2-oxoethyl]cyclopentyl}acetic acid

1,1-Cyclopentanediacetic anhydride (2.50 g, 14.9 mmol) and 5-chloro-2-ethoxyaniline (3.06 g, 17.8 mmol) were dissolved in toluene (20 mL). To the solution, triethylamine (4.94 mL, 3.61 g, 35.7 mmol) was added, and the mixture was stirred at 90°C for 4 hours. The temperature of the reaction solution was adjusted to room temperature, and 5 N hydrochloric acid was added thereto, followed by extraction with ethyl acetate. The organic layer was washed with saturated saline and then dried over anhydrous magnesium sulfate. After filtration, the filtrate was concentrated to obtain the title compound (intermediate 2h) (4.79 g, yield: 94.8%) as a solid.
$^1$H-NMR ($CDCl_3$) δ: 8.38 (1H, d, J = 2.4 Hz), 8.20 (1H, s), 7.03 (1H, dd, J = 8.8, 2.4 Hz), 6.79 (1H, d, J = 8.8 Hz), 4.09 (2H, q, J = 6.8 Hz), 2.58 (2H, s), 2.54 (2H, s), 1.76-1.74 (4H, m), 1.68-1.64 (4H, m), 1.44 (3H, t, J = 6.8 Hz).
Intermediates 2i and 2j were synthesized from the corresponding starting materials in the same way as above and are shown in Table 3.

[Table 3]

| | | |
|---|---|---|
| Intermediate 2 i | 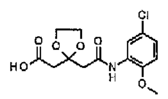 | $^1$H-NMR ($CDCl_3$) δ: 8.58 (1H, s), 7.43 (1H, d, J = 1.4 Hz), 7.01 (1H, dd, J = 8.6, 1.4 Hz), 6.78 (1H, d, J = 8.6 Hz), 4.10 (4H, s), 3.88 (3H, s), 3.05 (2H, s), 2.86 (2H, s). |
| Intermediate 2 j | 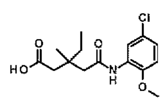 | $^1$H-NMR ($CDCl_3$) δ: 8.41 (1H, d, J = 2.3 Hz), 8.39 (1H, br s), 7.03 (1H, dd, J = 8.7, 2.3 Hz), 6.79 (1H, d, J = 8.7 Hz), 3.86 (3H, s), 2.54-2.48 (4H, m), 1.51 (2H, q, J = 7.5 Hz), 1.11 (3H, s), 0.96 (3H, t, J = 7.5 Hz). |

(Intermediate 2k) 2-{1-[2-(5-Chloro-2-methoxyanilino)-2-oxoethyl]cyclobutyl}acetic acid

[Formula 37]

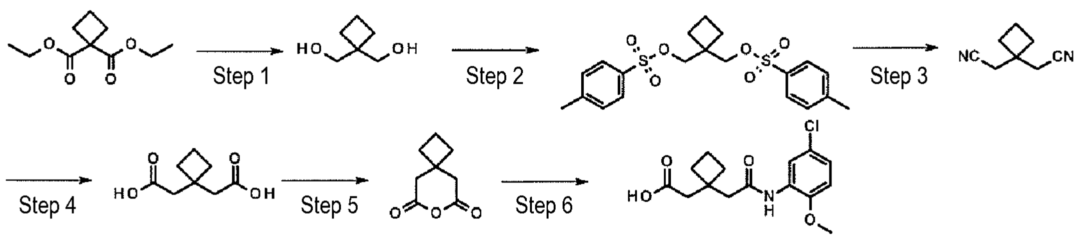


(Step 1) [1-(Hydroxymethyl)cyclobutyl]methanol

Lithium aluminum hydride (2.06 g, 49.4 mmol) was suspended in diethyl ether (200 mL). To the suspension, a solution of diethyl cyclobutane-1,1-dicarboxylate (5.00 g, 25.0 mmol) in diethyl ether (20 mL) was gradually added at 0°C. After the completion of the addition, the mixture was stirred at room temperature for 30 minutes and then heated to reflux for 2 hours. The reaction mixture was cooled to 0°C, then a saturated aqueous solution of sodium sulfate (2.0 mL) was added thereto, and the mixture was vigorously stirred for 15 minutes. The resulting solid was collected by filtration, and this solid was washed with 1 N hydrochloric acid. The washes were subjected to extraction with ethyl acetate. The organic layer was concentrated under reduced pressure, and the obtained residue was purified by silica gel column chromatography (Shoko Scientific Co., Ltd., eluting solvent: hexane/ethyl acetate = 50/50 to 0/100) to obtain the title compound (1.07 g, yield: 36.9%) as an oil substance.
$^1$H-NMR ($CDCl_3$) δ: 3.75 (4H, s), 2.50 (2H, br s), 1.98-1.91 (2H, m), 1.80 (4H, t, J = 7.7 Hz).

(Step 2) [1-(p-Tolylsulfonyloxymethyl)cyclobutyl]methyl-4-methylbenzenesulfonate

[1-(Hydroxymethyl)cyclobutyl]methanol (1.07 g, 9.21 mmol) synthesized in step 1 was dissolved in pyridine (20 mL). To the solution, p-toluenesulfonyl chloride (3.07 g, 16.1 mmol) was added at 0°C, and the mixture was stirred for 45 minutes and then further stirred at room temperature for 21.5 hours. p-Toluenesulfonyl chloride (1.00 g, 5.24 mmol) was added thereto, and the mixture was further stirred at room temperature for 1.5 hours. The reaction mixture was poured into ice water, and the mixture was neutralized by the addition of concentrated hydrochloric acid, followed by extraction with ethyl acetate. The organic layer was washed with 1 N hydrochloric acid and saturated saline and dried over anhydrous sodium sulfate. The solvent was distilled off under reduced pressure, and the obtained residue was purified by silica gel column chromatography (Shoko Scientific Co., Ltd., eluting solvent: hexane/ethyl acetate = 95/5 to 50/50) to obtain the title compound (2.59 g, yield: 66.2%) as a solid.
$^1$H-NMR ($CDCl_3$) δ: 7.74 (4H, d, J= 8.0 Hz), 7.35 (4H, d, J = 8.0 Hz), 3.95 (4H, s), 2.46 (6H, s), 1.88-1.82 (2H, m), 1.80-1.77 (4H, m).

(Step 3) 2-[1-(Cyanomethyl)cyclobutyl]acetonitrile

[1-(p-Tolylsulfonyloxymethyl)cyclobutyl]methyl-4-methylbenzenesulfonate (2.59 g, 6.10 mmol) synthesized in step 2 was dissolved in dimethyl sulfoxide (20 mL). To the solution, potassium cyanide (1.19 g, 18.3 mmol) was added, and the mixture was stirred at 90°C for 3 hours. The reaction mixture was cooled to room temperature, and water was added thereto, followed by extraction with diethyl ether. The organic layer was washed with water and saturated saline and dried over anhydrous sodium sulfate. The solvent was distilled off under reduced pressure to obtain the title compound (0.789 g, yield: 97.5%) as an oil substance.
$^1$H-NMR ($CDCl_3$) δ: 2.66 (4H, s), 2.13-2.10 (4H, m), 2.07-2.02 (2H, m).

(Step 4) 2-[1-(Carboxymethyl)cyclobutyl]acetic acid

2-[1-(Cyanomethyl)cyclobutyl]acetonitrile (0.798 g, 0.595 mmol) synthesized in step 3 was suspended in water (20 mL). To the suspension, potassium hydroxide (1.96 g, 29.7 mmol) was added, and the mixture was stirred at 110°C for 12 hours. The reaction mixture was cooled to room temperature, and concentrated hydrochloric acid was added thereto. The resulting solid was collected by filtration and dried to obtain the title compound (0.454 mg, yield: 44.3%) as a solid.
$^1$H-NMR (DMSO-$D_6$) δ: 12.01 (2H, br s), 2.54 (4H, s), 1.93-1.90 (4H, m), 1.87-1.82 (2H, m).

(Step 5) 7-Oxaspiro[3.5]nonane-6,8-dione

2-[1-(Carboxymethyl)cyclobutyl]acetic acid (0.454 g, 2.61 mmol) synthesized in step 4 was dissolved in methylene chloride (10 mL). To the solution, trifluoroacetic anhydride (2.50 mL, 3.73 g, 17.8 mmol) was added, and the reaction mixture was heated to reflux for 5.5 hours. The reaction solution was cooled to room temperature, and then, the solvent was concentrated under reduced pressure. Then, toluene was added to the residue, and the residual acid was subjected to azeotropy. The obtained residue was further dried under reduced pressure to obtain the title compound (0.381 g, yield: 94.7%) as an oil substance.
$^1$H-NMR ($CDCl_3$) δ: 2.83 (4H, s), 2.00-1.98 (6H, m).

(Step 6) 2-{1-[2-(5-Chloro-2-methoxyanilino)-2-oxo-ethyl]cyclobutyl}acetic acid

In a sealed tube, 7-oxaspiro[3.5]nonane-6,8-dione (50.0 mg, 0.324 mmol) synthesized in step 5 and 5-chloro-2-methoxyaniline (56.2 mg, 0.356 mmol) were dissolved in methylene chloride (1.0 mL). To the solution, triethylamine (0.497 mL, 36.0 mg, 0.356 mmol) and 4-(N,N-dimethylamino)pyridine (1.98 mg, 0.016 mmol) were added, and the mixture was stirred at 50°C for 18 hours. After cooling to room temperature, the reaction mixture was diluted with ethyl acetate. This organic layer was washed with 1 N hydrochloric acid and saturated saline and dried over anhydrous sodium sulfate. The solvent was distilled off under reduced pressure, and the obtained residue was purified by silica gel column chromatography (Shoko Scientific Co., Ltd., eluting solvent: hexane/ethyl acetate = 70/30 to 0/100) to obtain the title compound (74.0 mg, yield: 72.7%) as a solid.
$^1$H-NMR ($CDCl_3$) δ: 8.41 (1H, d, J = 2.8 Hz), 8.13 (1H, br s), 7.02 (1H, dd, J = 8.7, 2.8 Hz), 6.78 (1H, d, J = 8.7 Hz), 3.86 (3H, s), 2.76 (2H, s), 2.75 (2H, s), 2.11-1.99 (6H, m).

The following intermediate 21 was synthesized from the corresponding starting materials in the same way as above and is shown in Table 4.

[Table 4]

| | | |
|---|---|---|
| Intermediate 2 I | 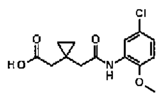 | $^1$H-NMR ($CDCl_3$) δ: 8.45 (1H, d, J = 2.6 Hz), 8.11 (1H, s), 7.02 (1H, dd, J = 8.9, 2.6 Hz), 6.79 (1H, d, J = 8.9 Hz), 3.87 (3H, s), 2.51 (2H, s), 2.47 (2H, s), 0.68 (4H, s). |

(Intermediates 2m and 2n) 3-Acetoxy-5-(5-chloro-2-ethoxyanilino)-3-methyl-5-oxopentanoic acid (intermediate 2m) and 5-(5-chloro-2-ethoxyanilino)-3-hydroxy-3-methyl-5-oxopentanoic acid (intermediate 2n)

[Formula 38]

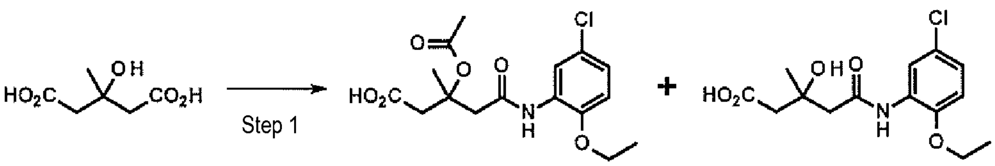


2m 2n

(Step 1) 3-Acetoxy-5-(5-chloro-2-ethoxyanilino)-3-methyl-5-oxopentanoic acid (intermediate 2m) and 5-(5-chloro-2-ethoxyanilino)-3-hydroxy-3-methyl-5-oxopentanoic acid (intermediate 2n)

To 3-hydroxy-3-methylpentanedioic acid (507 mg, 3.13 mmol), acetic anhydride (0.450 mL, 487 mg, 4.77 mmol) was added, and the mixture was stirred at 100°C for 100 minutes. The reaction mixture was cooled to room temperature and then concentrated under reduced pressure. To the obtained residue, methylene chloride was added, and the organic layer was washed with a 5% aqueous sodium bicarbonate solution once, dried over anhydrous sodium sulfate, and then concentrated under reduced pressure to obtain a solid (241 mg). To a solution of this solid (122 mg) in methylene chloride (3.0 mL), 5-chloro-2-ethoxyaniline (236 mg, 1.38 mmol), N,N-dimethyl-4-aminopyridine (8.30 mg, 0.0679 mmol), and N,N-diisopropylethylamine (0.180 mL, 134 mg, 1.04 mmol) were added in this order, and the mixture was stirred at 50°C for 14 hours. The reaction mixture was cooled to room temperature and then concentrated under reduced pressure, and

the obtained residue was purified by reverse-phase HPLC (Gilson, Inc., water (0.10% formic acid)/acetonitrile (0.10% formic acid)) to obtain the title compound (compound 2m) (62.5 mg, yield: 11.0%) as an oil substance.
MS (ESI) m/z: 358 [$(M+H)^+$].

Also, the title compound (compound 2n) (21.6 mg, yield: 4.30%) was obtained as an oil substance.
MS (ESI) m/z: 316 [$(M+H)^+$].

(Intermediate 2o) 5-(5-Chloro-2-methoxyanilino)-3-methoxy-3-methyl-5-oxopentanoic acid

[Formula 39]

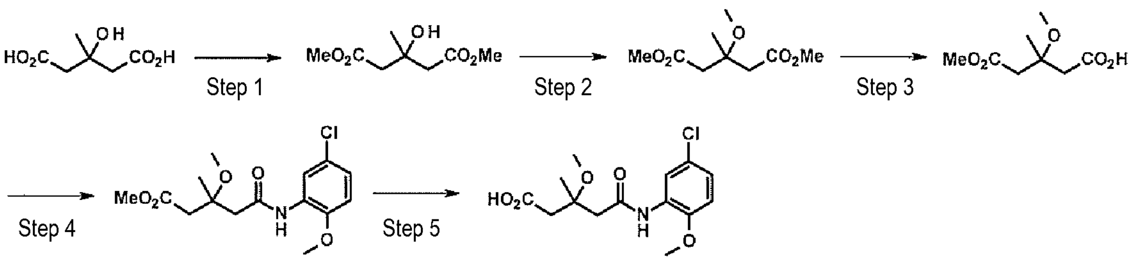


(Step 1) Dimethyl 3-hydroxy-3-methylpentanedioate

A solution of 3-hydroxy-3-methylpentanedioic acid (1.03 g, 6.35 mmol) in methanol (51 mL) was cooled to 0°C. Thionyl chloride (1.10 mL, 1.82 g, 15.3 mmol) was added dropwise thereto over 3 minutes, and the mixture was stirred for 80 minutes. The temperature of the reaction mixture was raised to room temperature, and the reaction mixture was concentrated under reduced pressure. To the obtained residue, water (30 mL) was added, followed by extraction with methylene chloride three times. The organic layers were combined, dried over anhydrous sodium sulfate, and concentrated under reduced pressure to obtain the title compound (1.07 g, yield: 88.7%) as an oil substance.
$^1$H-NMR ($CDCl_3$) δ: 4.07 (1H, br s), 3.71 (6H, s), 2.71 (2H, d, J = 15.5 Hz), 2.63 (2H, d, J = 15.5 Hz), 1.36 (3H, s).

(Step 2) Dimethyl 3-methoxy-3-methylpentanedioate

To a solution of dimethyl 3-hydroxy-3-methylpentanedioate (1.07 g, 5.63 mmol) synthesized in step 1 in N,N-dimethylformamide (32 mL), silver(I) oxide (5.25 g, 22.7 mmol) and methyl iodide (10.5 mL, 23.9 g, 168 mmol) were added, and the mixture was stirred at 100°C for 17 hours. The reaction mixture was cooled to room temperature, filtered by suction, and washed with ethyl acetate and methanol. Volatile substances were distilled off from the mother liquor under reduced pressure. To the obtained residue, water (80 mL) was added, followed by extraction with ethyl acetate. The organic layer was washed with water twice and saturated saline once and dried over anhydrous sodium sulfate. The solvent was distilled off, and the obtained residue was purified by silica gel column chromatography (Biotage Japan Ltd., eluting solvent: hexane/ethyl acetate = 92/8 to 34/66) to obtain the title compound (0.181 g, yield: 15.7%) as an oil substance.
$^1$H-NMR ($CDCl_3$) δ: 3.69 (6H, s), 3.26 (3H, s), 2.78 (2H, d, J = 14.3 Hz), 2.74 (2H, d, J = 14.3 Hz), 1.40 (3H, s).

(Step 3) 3,5-Dimethoxy-3-methyl-5-oxopentanoic acid

A solution of dimethyl 3-methoxy-3-methylpentanedioate (179 mg, 0.877 mmol) synthesized in step 2 in methanol (1.8 mL) was cooled to 0°C. A 1 N aqueous sodium hydroxide solution (0.890 mL, 0.890 mmol) was added thereto, and the mixture was stirred for 10 minutes. The temperature of the reaction mixture was raised to room temperature, and the reaction mixture was stirred for 15 hours. Methanol was distilled off from the reaction mixture under reduced pressure. To the obtained residue, water (8.0 mL) was added, and the mixture was washed with diethyl ether once. The pH of the aqueous layer was adjusted to approximately 3 by the addition of a 1 N aqueous hydrochloric acid solution (0.890 mL, 0.890 mmol), followed by extraction with methylene chloride three times. The organic layers were combined, dried over anhydrous sodium sulfate, and concentrated under reduced pressure to obtain the title compound (110 mg, yield: 65.9%) as an oil substance.
$^1$H-NMR ($CDCl_3$) δ: 3.71 (3H, s), 3.33 (3H, s), 2.86 (1H, d, J = 14.9 Hz), 2.77 (1H, d, J = 11.5 Hz), 2.74 (1H, d, J = 12.0 Hz), 2.67 (1H, d, J = 14.3 Hz), 1.42 (3H, s).

(Step 4) Methyl 5-(5-chloro-2-methoxyanilino)-3-methoxy-3-methyl-5-oxopentanoate

To a solution of 3,5-dimethoxy-3-methyl-5-oxopentanoic acid (108 mg, 0.568 mmol) synthesized in step 3 in N,N-dimethylformamide (2.7 mL), 0-(7-azabenzotriazol-1-yl)-N,N,N',N'-tetramethyluronium hexafluorophosphate (HATU) (287 mg, 0.755 mmol) was added, and the mixture was stirred at room temperature for 10 minutes. Subsequently, 5-chloro-2-methoxyaniline (115 mg, 0.730 mmol) and N,N-diisopropylethylamine (0.160 mL, 119 mg, 0.921 mmol) were added thereto, and the mixture was stirred at 50°C for 5.5 hours. After cooling to room temperature, a saturated aqueous solution of sodium bicarbonate (7.0 mL) and water (10 mL) were added to the reaction mixture, followed by extraction with ethyl acetate. The organic layer was washed with a saturated aqueous solution of sodium bicarbonate/water (1:2) twice and saturated saline once and dried over anhydrous sodium sulfate. The solvent was distilled off, and the obtained residue was purified by silica gel column chromatography (Biotage Japan Ltd., eluting solvent: hexane/ethyl acetate = 90/10 to 20/80) to obtain the title compound (135 mg, yield: 72.0%) as an oil substance.
$^1$H-NMR ($CDCl_3$) δ: 9.17 (1H, br s), 8.44 (1H, d, J = 2.9 Hz), 6.98 (1H, dd, J = 8.6, 2.3 Hz), 6.77 (1H, d, J = 8.6 Hz), 3.86 (3H, s), 3.71 (3H, s), 3.37 (3H, s), 2.87 (1H, d, J= 14.3 Hz), 2.73 (1H, d, J = 14.3 Hz), 2.69 (1H, d, J = 14.3 Hz), 2.60 (1H, d, J = 14.3 Hz), 1.41 (3H, s).

(Step 5) 5-(5-Chloro-2-methoxyanilino)-3-methoxy-3-methyl-5-oxopentanoic acid

To a solution of methyl 5-(5-chloro-2-methoxyanilino)-3-methoxy-3-methyl-5-oxopentanoate (134 mg, 0.406 mmol) synthesized in step 4 in tetrahydrofuran (2.0 mL) and methanol (2.0 mL), a 1 N aqueous sodium hydroxide solution (1.03 mL, 1.03 mmol) was added, and the mixture was stirred at room temperature for 63 hours. The pH of the reaction mixture was adjusted to approximately 2 by the addition of a 1 N aqueous hydrochloric acid solution (1.13 mL, 1.13 mmol) and water (10 mL), followed by extraction with ethyl acetate three times. The organic layers were combined and dried over anhydrous sodium sulfate. The solvent was distilled off, and the obtained residue was purified by silica gel column chromatography (Biotage Japan Ltd., eluting solvent: ethyl acetate/methanol → chloroform/methanol = 100/0 to 75/25 → 100/0 to 85/15) to obtain the title compound (39.3 mg, yield: 30.5%) as an oil substance. $^1$H-NMR ($CDCl_3$) δ: 8.80 (1H, br s), 8.40-8.35 (1H, m), 7.03-6.97 (1H, m), 6.79-6.75 (1H, m), 3.87 (3H, s), 3.33 (3H, s), 2.89-2.82 (1H, m), 2.76-2.68 (3H, m), 1.41 (3H, s) .
MS (ESI) m/z: 316 [$(M+H)^+$].

(Intermediate 3a) 4-[(5-Chloro-2-methylbenzofuran-7-carbonyl)amino]-3,3-dimethylbutanoic acid

[Formula 40]

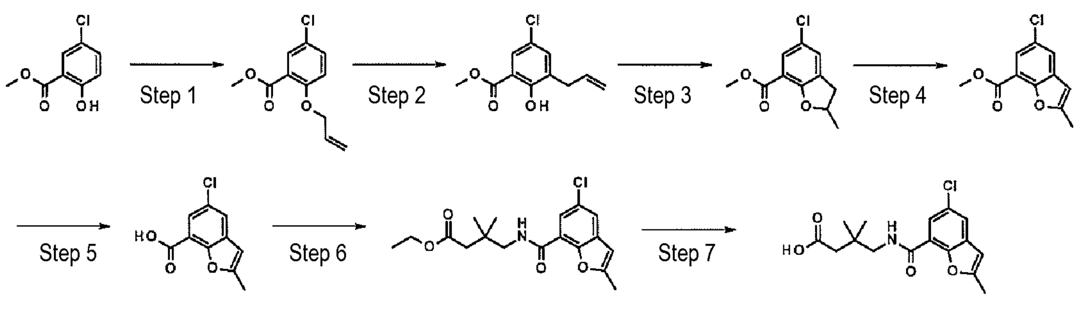


(Step 1) Methyl 2-allyloxy-5-chlorobenzoate

Methyl 5-chlorosalicylate (11.2 g, 60.0 mmol) was dissolved in N,N-dimethylformamide (50 mL). To the solution, potassium carbonate (8.29 g, 60.0 mmol) and allyl bromide (5.08 mL, 7.26 g, 60.0 mmol) were added, and the mixture was stirred at 60°C for 4 hours. The reaction solution was brought back to room temperature, and water was added thereto, followed by extraction with ethyl acetate. The organic layer was washed with saturated saline and then dried over anhydrous magnesium sulfate. After filtration, the filtrate was concentrated, and the obtained crude product was purified by silica gel column chromatography (Biotage Japan Ltd., eluting solvent: hexane/ethyl acetate = 100/0 to 70/30) to obtain the title compound (12.9 g, yield: 94.6%) as a solid.
$^1$H-NMR ($CDCl_3$) δ: 7.78 (1H, d, J = 2.9 Hz), 7.39 (1H, dd, J = 9.2, 2.9 Hz), 6.90 (1H, d, J = 9.2 Hz), 6.07-6.02 (1H, m), 5.50 (1H, d, J = 17.2 Hz), 5.31 (1H, d, J = 10.3 Hz), 4.61-4.61 (2H, m), 3.90 (3H, s).

(Step 2) Methyl 3-allyl-5-chloro-2-hydroxybenzoate

Methyl 2-allyloxy-5-chlorobenzoate (12.9 g, 56.8 mmol) synthesized in step 1 was dissolved in N-methyl-2-pyrrolidone (25 mL), and the solution was stirred at 200°C for 7 hours. The reaction solution was brought back to room temperature, and water was added thereto, followed by extraction with ethyl acetate. The organic layer was washed with saturated saline and then dried over anhydrous magnesium sulfate. After filtration, the filtrate was concentrated, and the obtained crude product was purified by silica gel column chromatography (Biotage Japan Ltd., eluting solvent: hexane/ethyl acetate = 100/0 to 70/30) to obtain the title compound (11.0 g, yield: 85.3%) as an oil substance.
$^1$H-NMR ($CDCl_3$) $\delta$: 10.98 (1H, s), 7.70 (1H, d, J = 2.9 Hz), 7.29 (1H, d, J = 2.9 Hz), 5.99-5.96 (1H, m), 5.13-5.09 (2H, m), 3.95 (3H, s), 3.41-3.40 (2H, m).

(Step 3) Methyl 5-chloro-2-methyl-2,3-dihydrobenzofuran-7-carboxylate

Methyl 3-allyl-5-chloro-2-hydroxybenzoate (11.0 g, 48.4 mmol) synthesized in step 2 was dissolved in methylene chloride (100 mL), and the solution was stirred at 0°C. Zirconium tetrachloride (13.5 g, 58.1 mmol) was added thereto in small portions. After the completion of the addition, the mixture was stirred at room temperature for 18 hours. Water was added to the reaction solution under ice cooling, followed by extraction with ethyl acetate. The organic layer was washed with saturated saline and then dried over anhydrous magnesium sulfate. After filtration, the filtrate was concentrated, and the obtained crude product was purified by silica gel column chromatography (Biotage Japan Ltd., eluting solvent: hexane/ethyl acetate = 95/5 to 70/30) to obtain the title compound (5.80 g, yield: 52.9%) as a solid.
$^1$H-NMR ($CDCl_3$) $\delta$: 7.69-7.68 (1H, m), 7.26-7.26 (1H, m), 5.11-5.09 (1H, m), 3.90 (3H, s), 3.35-3.30 (1H, m), 2.83-2.80 (1H, m), 1.52 (3H, d, J = 5.9 Hz).

(Step 4) Methyl 5-chloro-2-methylbenzofuran-7-carboxylate

Methyl 5-chloro-2-methyl-2,3-dihydrobenzofuran-7-carboxylate (5.22 g, 23.0 mmol) synthesized in step 3 was dissolved in carbon tetrachloride (50 mL). To the solution, N-bromosuccinimide (4.31 g, 24.2 mmol) and 2,2'-azobis(isobutyronitrile) (1.13 g, 6.90 mmol) were added, and the mixture was heated to reflux for 6 hours. The reaction solution was brought back to room temperature, and the deposit was removed by filtration. The filtrate was subjected to extraction with methylene chloride, and the organic layer was washed with water, a saturated aqueous solution of sodium bicarbonate, and saturated saline in this order and then dried over anhydrous magnesium sulfate. After filtration, the filtrate was concentrated, and the obtained crude product was purified by silica gel column chromatography (Biotage Japan Ltd., eluting solvent: hexane/ethyl acetate = 95/5 to 75/25) to obtain the title compound (3.82 g, yield: 73.9%) as a solid.
$^1$H-NMR ($CDCl_3$) $\delta$: 7.82 (1H, d, J = 2.0 Hz), 7.61 (1H, d, J = 2.0 Hz), 6.39 (1H, d, J = 1.0 Hz), 4.00 (3H, s), 2.53 (3H, d, J = 1.0 Hz).

(Step 5) 5-Chloro-2-methylbenzofuran-7-carboxylic acid

Methyl 5-chloro-2-methylbenzofuran-7-carboxylate (3.82 g, 17.0 mmol) synthesized in step 4 was dissolved in methanol (90 mL). To the solution, a 2 N aqueous sodium hydroxide solution (20 mL) was added, and the mixture was stirred at room temperature for 5 hours. Methanol in the reaction solution was distilled off under reduced pressure, and then, the residue was neutralized by the addition of 2 N hydrochloric acid under ice cooling, followed by extraction with ethyl acetate. The organic layer was washed with saturated saline and then dried over anhydrous magnesium sulfate. After filtration, the filtrate was concentrated to obtain the title compound (3.57 g, yield: 99.7%) as a solid.
$^1$H-NMR ($CDCl_3$) $\delta$: 7.90 (1H, d, J= 2.4 Hz), 7.68 (1H, d, J = 2.4 Hz), 6.43 (1H, s), 2.55 (3H, s).

(Step 6) Ethyl 4-[(5-chloro-2-methylbenzofuran-7-carbonyl)amino]-3,3-dimethylbutanoate

5-Chloro-2-methylbenzofuran-7-carboxylic acid (3.85 g, 18.3 mmol) synthesized in step 5 was dissolved in methylene chloride (100 mL). To the solution, a catalytic amount of N,N-dimethylformamide was added, and the mixture was stirred at room temperature. Oxalyl chloride (2.32 mL, 3.48 g, 27.4 mmol) was added dropwise thereto. After the completion of the addition, the mixture was stirred at room temperature for 1 hour. The solvent was distilled off under reduced pressure, and the residue was dried and then dissolved in methylene chloride (100 mL). To the solution, ethyl 3,3-dimethyl-4-aminobutanoate hydrochloride (3.58 g, 18.3 mmol) synthesized in step 2 in the synthesis of intermediate 1a and pyridine (5.51 mL, 5.39 g, 54.9 mmol) were added, and the mixture was stirred at room temperature for 13 hours. Water was added thereto, followed by extraction with methylene chloride. The organic layer was washed with 1 N hydrochloric acid and then dried over anhydrous magnesium sulfate. After filtration, the filtrate was concentrated, and the obtained crude product was purified by silica gel column chromatography (Biotage Japan Ltd., eluting solvent:

hexane/ethyl acetate = 90/10 to 40/60) to obtain the title compound (5.12 g, yield: 79.5%) as a solid.
$^1$H-NMR ($CDCl_3$) δ: 7.97 (1H, d, J = 2.0 Hz), 7.84 (1H, s), 7.56 (1H, d, J = 2.0 Hz), 6.44 (1H, d, J = 1.0 Hz), 4.16 (2H, q, J = 7.1 Hz), 3.50 (2H, d, J = 6.3 Hz), 2.52 (3H, d, J = 1.0 Hz), 2.35 (2H, s), 1.27 (3H, t, J = 7.1 Hz), 1.12 (6H, s).

(Step 7) 4-[(5-Chloro-2-methylbenzofuran-7-carbonyl)amino]-3,3-dimethylbutanoic acid (intermediate 3a)

Ethyl 4-[(5-chloro-2-methylbenzofuran-7-carbonyl)amino]-3,3-dimethylbutanoate (5.12 g, 14.6 mmol) synthesized in step 6 was dissolved in ethanol (50 mL). To the solution, a 2 N aqueous sodium hydroxide solution (20 mL) was added, and the mixture was stirred at room temperature for 15 hours. The solvent in the reaction solution was distilled off under reduced pressure, and the residue was neutralized by the addition of 2 N hydrochloric acid, followed by extraction with ethyl acetate. The organic layer was washed with saturated saline and then dried over anhydrous magnesium sulfate. After filtration, the filtrate was concentrated to obtain the title compound (intermediate 3a) (4.56 g, yield: 96.8%) as a solid.
$^1$H-NMR ($CDCl_3$) δ: 7.98 (1H, d, J = 2.4 Hz), 7.88 (1H, br s), 7.59 (1H, d, J = 2.4 Hz), 6.46 (1H, d, J = 1.0 Hz), 3.53 (2H, d, J = 6.8 Hz), 2.52 (3H, d, J = 1.0 Hz), 2.35 (2H, s), 1.16 (6H, s).
The following intermediate 3b was synthesized through the same reaction as in step 5 and the subsequent steps using methyl 5-chloro-2-methyl-2,3-dihydrobenzofuran-7-carboxylate synthesized in step 3, and is shown in Table 5.

[Table 5]

| | | |
|---|---|---|
| Intermediate 3 b | 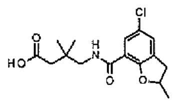 | $^1$H-NMR ($CDCl_3$) δ: 8.07 (1H, t, J = 6.9 Hz), 7.89 (1H, d, J = 2.3 Hz), 7.28-7.28 (1H, m), 5.18-5.16 (1H, m), 3.45-3.37 (3H, m), 2.91-2.88 (1H, m), 2.28-2.25 (2H, m), 1.55 (3H, d, J = 6.3 Hz), 1.10 (3H, s), 1.09 (3H, s). |

(Intermediate 3c) 4-[(5-Chloro-2,2-dimethyl-3H-benzofuran-7-carbonyl)amino]-3,3-dimethylbutanoic acid

[Formula 41]

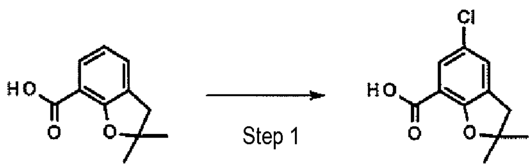


(Step 1) 5-Chloro-2,2-dimethyl-3H-benzofuran-7-carboxylic acid

2,2-Dimethyl-3H-benzofuran-7-carboxylic acid (1.00 g, 5.20 mmol) was dissolved in N,N-dimethylformamide (10 mL). To the solution, N-chlorosuccinimide (0.846 g, 6.24 mmol) was added, and the mixture was stirred at room temperature for 6 hours. Water was added to the reaction solution, followed by extraction with ethyl acetate. The organic layer was washed with saturated saline and then dried over anhydrous magnesium sulfate. After filtration, the filtrate was concentrated to obtain the title compound (1.18 g, quantitative) as a solid.
$^1$H-NMR ($CDCl_3$) δ: 7.80-7.80 (1H, m), 7.32-7.31 (1H, m), 3.09-3.08 (2H, m), 1.59 (6H, s).
The following intermediates 3c to 3e were subsequently synthesized from the corresponding carboxylic acids through the same reaction as in steps 6 and 7 in the synthesis of intermediate 3a and are shown in Table 6.

[Table 6]

| | | |
|---|---|---|
| Intermediate 3 c | 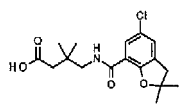 | $^1$H-NMR ($CDCl_3$) δ: 8.10 (1H, t, J = 6.3 Hz), 7.89 (1H, d, J = 2.3 Hz), 7.26-7.26 (1H, m), 3.41 (2H, d, J = 6.3 Hz), 3.08 (2H, s), 2.26 (2H, s), 1.56 (6H, s), 1.10 (6H, s). |

(continued)

| | | |
|---|---|---|
| Intermediate 3 d | 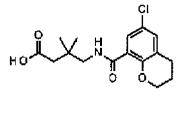 | $^1$H-NMR (DMSO-$D_6$) δ: 12.12 (1H, s), 8.25 (1H, t, J = 6.3 Hz), 7.48 (1H, d, J = 2.3 Hz), 7.28 (1H, d, J = 2.3 Hz), 4.27 (2H, t, J = 5.2 Hz), 3.23 (2H, d, J = 6.3 Hz), 2.80 (2H, t, J = 6.6 Hz), 2.16 (2H, s), 1.97-1.92 (2H, m), 0.97 (6H, s). |
| Intermediate 3 e | 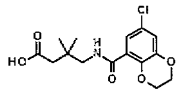 | $^1$H-NMR ($CDCl_3$) δ: 8.13-8.07 (1H, m), 7.73 (1H, d, J = 3.1 Hz), 7.05 (1H, d, J =3.1 Hz), 4.46-4.43 (2H, m), 4.36-4.33 (2H, m), 3.41 (2H, d, J = 6.7 Hz), 2.27 (2H, s), 1.10 (6H, s). |

(Intermediate 3f) 4-[(6-Chloro-2-methylquinoline-8-carbonyl)amino]-3,3-dimethylbutanoic acid

[Formula 42]

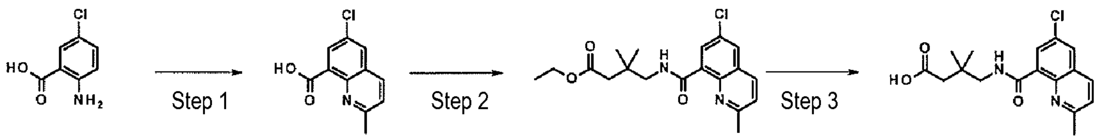


(Step 1) 6-Chloro-2-methylquinoline-8-carboxylic acid

5-Chloroanthranilic acid (3.43 g, 20.0 mmol) was suspended in 6 N hydrochloric acid (40 mL), and the suspension was heated to reflux. Crotonaldehyde (1.99 mL, 1.68 g, 24.0 mmol) was added dropwise thereto over 1 hour. After the completion of the addition, the mixture was heated to reflux for 3 hours. The reaction solution was cooled in ice, and the pH of the reaction solution was adjusted to 3 by the addition of ammonia water, followed by extraction with methylene chloride. The organic layer was washed with water and then dried over anhydrous magnesium sulfate. After filtration, the filtrate was concentrated to obtain the title compound (4.35 g, yield: 98.0%) as a solid.
$^1$H-NMR ($CDCl_3$) δ: 8.68 (1H, d, J = 2.4 Hz), 8.21 (1H, d, J = 8.8 Hz), 8.02 (1H, d, J = 2.4 Hz), 7.50 (1H, d, J = 8.8 Hz), 2.84 (3H, s).

(Step 2) Ethyl 4-[(6-chloro-2-methylquinoline-8-carbonyl)amino]-3,3-dimethylbutanoate

6-Chloro-2-methylquinoline-8-carboxylic acid (2.22 g, 10.0 mmol) synthesized in step 1 was dissolved in N,N-dimethylformamide (40 mL). To the solution, 0-(7-azabenzotriazol-1-yl)-N,N,N',N'-tetramethyluronium hexafluorophosphate (HATU) (5.70 g, 15.0 mmol), N,N-diisopropylethylamine (5.23 mL, 3.88 g, 30.0 mmol), and ethyl 3,3-dimethyl-4-aminobutanoate hydrochloride (1.96 g, 10.0 mmol) synthesized in step 2 of intermediate 1a were added, and the mixture was stirred at room temperature for 5 hours. Water was added to the reaction solution, followed by extraction with ethyl acetate. The organic layer was washed with a saturated aqueous solution of sodium bicarbonate and saturated saline and then dried over anhydrous magnesium sulfate. After filtration, the filtrate was concentrated, and the obtained crude product was purified by silica gel column chromatography (Biotage Japan Ltd., eluting solvent: hexane/ethyl acetate = 90/10 to 50/50) and amino silica gel column chromatography (Biotage Japan Ltd., eluting solvent: hexane/ethyl acetate = 90/10 to 50/50) to obtain the title compound (1.21 g, yield: 33.3%) as a solid.
$^1$H-NMR ($CDCl_3$) δ: 11.55 (1H, br s), 8.78-8.77 (1H, m), 8.08 (1H, d, J = 8.3 Hz), 7.88-7.88 (1H, m), 7.38 (1H, d, J = 8.3 Hz), 4.15 (2H, q, J = 7.1 Hz), 3.58 (2H, d, J = 5.9 Hz), 2.78 (3H, s), 2.39 (2H, s), 1.26 (3H, t, J = 7.1 Hz), 1.18 (6H, s).

(Step 3) 4-[(6-Chloro-2-methylquinoline-8-carbonyl)amino]-3,3-dimethylbutanoic acid

Ethyl 4-[(6-chloro-2-methylquinoline-8-carbonyl)amino]-3,3-dimethylbutanoate (1.20 g, 3.31 mmol) synthesized in step 2 was dissolved in ethanol (12 mL). To the solution, a 2 N aqueous sodium hydroxide solution (4.0 mL) was added, and the mixture was stirred at room temperature for 24 hours. The solvent in the reaction solution was distilled off under reduced pressure. 2 N hydrochloric acid (4.0 mL) was added to the residue, and then, ethyl acetate was added thereto. Insoluble matter was collected by filtration, washed with ethyl acetate, and then dried to obtain the title compound. Also, the organic layer from the filtrate was washed with saturated saline and then dried over anhydrous magnesium

sulfate. After filtration, the filtrate was concentrated, and the residue was combined with the solid preliminarily collected by filtration to obtain the title compound (2.75 g, yield: 99.2%) as a solid.
$^1$H-NMR (DMSO-$D_6$) δ: 12.16 (1H, s), 11.23 (1H, t, J= 6.3 Hz), 8.44-8.43 (2H, m), 8.32 (1H, d, J = 2.4 Hz), 7.64 (1H, d, J= 8.8 Hz), 3.46 (2H, d, J = 6.3 Hz), 2.75 (3H, s), 2.27 (2H, s), 1.09 (6H, s).

(Intermediate 3g) 4-{[5-Chloro-2-(methoxymethyl)benzofuran-7-carbonyl]amino}-3,3-dimethylbutanoic acid

[Formula 43]

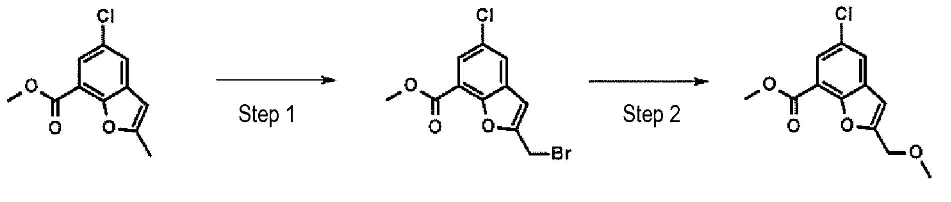


(Step 1) Methyl 2-(bromomethyl)-5-chlorobenzofuran-7- carboxylate

Methyl 2-methyl-5-chlorobenzofuran-7-carboxylate (2.25 g, 10.0 mmol) synthesized in step 4 in the synthesis of intermediate 3a was dissolved in carbon tetrachloride (25 mL). To the solution, N-bromosuccinimide (1.87 g, 10.5 mmol) and 2,2'-azobis(isobutyronitrile) (0.411 g, 2.50 mmol) were added, and the mixture was heated to reflux for 9 hours. The reaction solution was brought back to room temperature and stirred for 72 hours. The deposit was removed by filtration, and the filtrate was concentrated under reduced pressure. Water was added to the residue, followed by extraction with methylene chloride. The organic layer was dried over anhydrous magnesium sulfate. After filtration, the filtrate was concentrated to obtain the title compound (3.56 g, quantitative) as a solid.
$^1$H-NMR ($CDCl_3$) δ: 7.94 (1H, d, J = 2.0 Hz), 7.70 (1H, d, J = 2.0 Hz), 6.78 (1H, s), 4.62 (2H, s), 4.02 (3H, s).

(Step 2) Methyl 5-chloro-2-(methoxymethyl)benzofuran-7-carboxylate

Methyl 2-(bromomethyl)-5-chlorobenzofuran-7-carboxylate (3.56 g, 10.0 mmol) synthesized in step 1 was dissolved in tetrahydrofuran (20 mL), and the solution was stirred at 0°C. A 28% solution of sodium methoxide in methanol (2.23 mL, 2.12 g, 11.0 mmol) was added dropwise thereto. After the completion of the addition, the mixture was stirred at room temperature for 10 minutes. The reaction solution was poured into 1 N hydrochloric acid, followed by extraction with ethyl acetate. The organic layer was washed with saturated saline and then dried over anhydrous magnesium sulfate. After filtration, the filtrate was concentrated, and the obtained crude product was purified by silica gel column chromatography (Biotage Japan Ltd., eluting solvent: hexane/ethyl acetate = 100/0 to 75/25) to obtain the title compound (1.73 g, yield: 67.9%) as a solid.
$^1$H-NMR ($CDCl_3$) δ: 7.90 (1H, d, J = 2.0 Hz), 7.71 (1H, d, J = 2.0 Hz), 6.71 (1H, s), 4.62 (2H, s), 4.00 (3H, s), 3.48 (3H, s).
The following intermediate 3g was subsequently synthesized through the same reaction as in step 5 and the subsequent steps of intermediate 3a and is shown in Table 7.

[Table 7]

| | | |
|---|---|---|
| Intermediate 3 g | 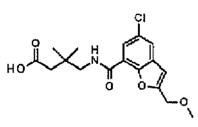 | $^1$H-NMR ($CDCl_3$) δ: 8.07 (1H, d, J = 2.0 Hz), 7.91 (1H, brs), 7.66 (1H, d, J = 2.0 Hz), 6.78 (1H, s), 4.72 (2H, s), 3.62 (2H, d, J = 6.8 Hz), 3.47 (3H, s), 2.32 (2H, s), 1.15 (6H, s). |

(Example 1) 5-Chloro-N-[2,2-dimethyl-4-({1-methyl-5-[1-(methylamino)ethyl]-3-oxo-2-phenyl-2,3-dihydro-1H-pyrazol-4-yl}amino)-4-oxobutyl]-2-ethoxybenzamide

[Formula 44]

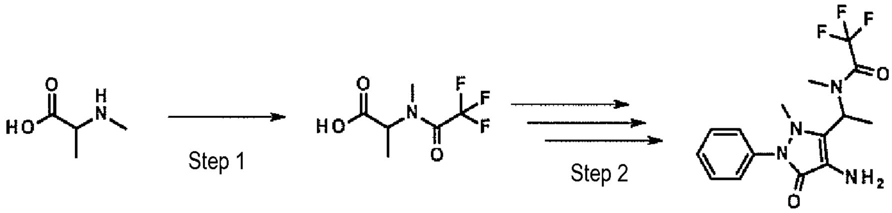


(Step 1) N-Methyl-N-trifluoroacetylalanine

To a solution of N-methylalanine (5.00 g, 48.5 mmol) in methanol (50 mL), 1,1,3,3-tetramethylguanidine (8.50 mL, 7.82 g, 67.9 mmol) and ethyl trifluoroacetate (7.53 mL, 8.96 g, 63.0 mmol) were added, and the mixture was stirred at room temperature for 14 hours. The reaction solution was concentrated under reduced pressure, then water was added to the residue, and the mixture was rendered acidic with concentrated hydrochloric acid, followed by extraction with ethyl acetate three times. The organic layer was washed with saturated saline and then dried over anhydrous sodium sulfate. After filtration, the filtrate was concentrated under reduced pressure to obtain the title compound (7.76 g, yield: 80.4%) as an oil substance.
$^1$H-NMR ($CDCl_3$) $\delta$: 8.66 (1H, br s), 5.06 (1H, q, J = 7.3 Hz), 3.14 (3H, s), 1.54 (3H, d, J = 7.3 Hz).

(Step 2) N-[1-(4-Amino-2-methyl-5-oxo-1-phenyl-2,3-dihydro-1H-pyrazol-3-yl)ethyl]-2,2,2-trifluoro-N-methylacetamide

The title compound was obtained as a solid by using N-methyl-N-trifluoroacetylalanine synthesized in step 1 instead of 2-methoxypropanoic acid in step 1 of Example 122 and subsequently performing the same reaction as in Example 122 up to step 5.
$^1$H-NMR ($CDCl_3$) $\delta$: 7.46-7.44 (4H, m), 7.30-7.25 (1H, m), 5.77 (1H, q, J = 7.3 Hz), 3.40 (2H, s), 3.08 (3H, s), 2.78 (3H, s), 1.72 (3H, d, J= 7.3 Hz).
MS (APCI) m/z : 343 [(M+H)$^+$].

The following compounds of Examples 1 and 2 were subsequently synthesized in the same way as in steps 7 and 8 of Example 66 and are shown in Table 8.

[Table 8]

| Example No. | Structure | Intermediate used | Instrumental analysis data |
|---|---|---|---|
| 1 | 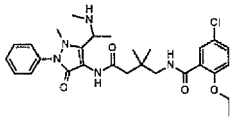 | 1 a | $^1$H-NMR ($CDCl_3$) $\delta$: 9.15 (1H, s), 8.38 (1H, t, J = 6.1 Hz), 8.18 (1H, d, J = 3.0 Hz), 7.46-7.36 (5H, m), 7.30-7.23 (1H, m), 6.91 (1H, d, J = 8.5 Hz), 4.20 (2H, q, J = 7.1 Hz), 3.88 (1H, q, J = 6.9 Hz), 3.67-3.56 (2H, m), 3.27 (3H, s), 2.48 (3H, s), 2.27 (2H, s), 1.70 (1H, brs), 1.54-1.50 (6H, m), 1.14 (6H, s).<br>MS (APCI) m/z : 542 [(M+H)$^+$]. |
| 2 | 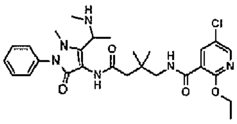 | 1 h | $^1$H-NMR ($CDCl_3$) $\delta$: 8.82 (1H, s), 8.48-8.40 (2H, m), 8.19 (1H, d, J = 2.4 Hz), 7.49-7.39 (4H, m), 7.30-7.25 (1H, m), 4.55 (2H, q, J = 7.1 Hz), 3.88 (1H, q, J = 6.9 Hz), 3.67-3.54 (2H, m), 3.28 (3H, s), 2.47 (3H, s), 2.26 (2H, s), 1.70 (1H, br s), 1.52-1.46 (6H, m), 1.14 (3H, s), 1.14 (3H, s).<br>MS (APCI) m/z : 543 [(M+H)$^+$]. |

(Example 3) 5-Chloro-N-{4-[(5-{[(2,4-dimethoxyphenylmethyl)-methylamino]methyl}-1-methyl-3-oxo-2-phenyl-2,3-dihydro-1H-pyrazol-4-yl)amino]-2,2-dimethyl-4-oxobutyl}-2-methoxybenzamide

[Formula 45]

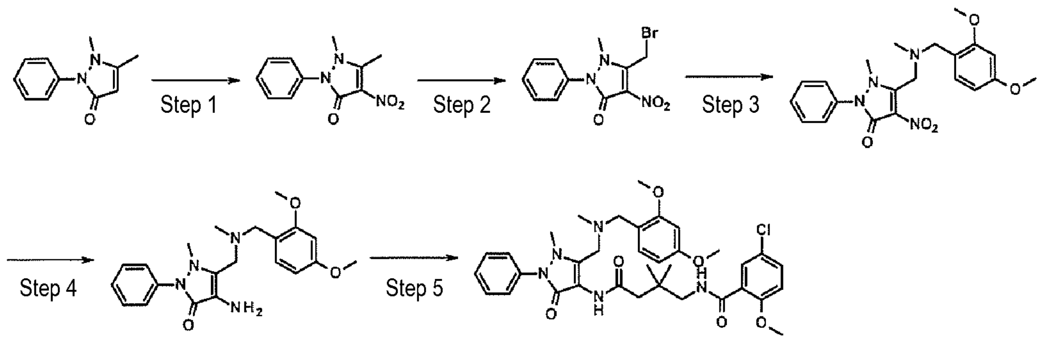


(Step 1) 4-Nitroantipyrine

To antipyrine (2.00 g, 10.6 mmol), 70% nitric acid (10 mL) was added, and the mixture was stirred at 70°C for 1.5 hours. The reaction solution was allowed to cool to room temperature, and ice water was added to the reaction solution. The deposited solid was collected by filtration, and the solid was washed with a small amount of chloroform and then dried to obtain the title compound (1.63 g, 65.8%) as a solid.
$^1$H-NMR (DMSO-$D_6$) δ: 7.60-7.51 (3H, m), 7.42-7.39 (2H, m), 3.69 (3H, s), 2.70 (3H, s).
MS (API) m/z : 234 [$(M+H)^+$].

(Step 2) 5-(Bromomethyl)-1-methyl-4-nitro-2-phenyl-2,3-dihydro-1H-pyrazol-3-one

To a suspension of 4-nitroantipyrine (1.11 g, 4.76 mmol) synthesized in step 1 in methylene chloride (25 mL), 1,1'-azobis(cyclohexane-1-carbonitrile) (0.0174 g, 0.0714 mmol) was added, and N-bromosuccinimide (0.850 mg, 4.76 mmol) was added over 1 hour. After stirring at room temperature for 16 hours, methylene chloride (25 mL) was further added thereto for a homogeneous reaction system, and then, the mixture was stirred at room temperature for 24 hours. N-Bromosuccinimide (0.420 g, 2.38 mmol) was further added thereto, and the mixture was stirred at room temperature for 24 hours. The reaction solution was purified by silica gel column chromatography (Yamazen Corp., eluting solvent: methanol/ethyl acetate = 1/99 to 5/95) to obtain the title compound (1.20 g, yield: 80.8%) as a solid.
$^1$H-NMR (DMSO-$D_6$) δ: 7.63-7.56 (3H, m), 7.48-7.45 (2H, m), 5.08 (2H, s), 3.48 (3H, s).
MS (API) m/z : 312 [$(M+H)^+$].

(Step 3) 5-{[(2,4-Dimethoxyphenyl)methylamino]methyl}-1-methyl-4-nitro-2-phenyl-2,3-dihydro-1H-pyrazol-3-one

To a solution of 5-(bromomethyl)-1-methyl-4-nitro-2-phenyl-2,3-dihydro-1H-pyrazol-3-one (468 mg, 1.50 mmol) synthesized in step 2 in methylene chloride (10 mL), triethylamine (0.314 mL, 228 mg, 2.25 mmol) was added, then a solution of (2,4-dimethoxybenzyl)methylamine (326 mg, 1.80 mmol) in methylene chloride (5 mL) was added, and the mixture was stirred at room temperature for 30 minutes. The reaction solution was concentrated under reduced pressure, and then, the obtained residue was purified by silica gel column chromatography (Yamazen Corp., eluting solvent: ethyl acetate) to obtain the title compound (637 mg, quantitative) as a solid.
$^1$H-NMR ($CDCl_3$) δ: 7.53-7.45 (3H, m), 7.27-7.25 (2H, m), 7.13 (1H, d, J = 9.2 Hz), 6.45-6.41 (2H, m), 4.08 (2H, s), 3.79 (3H, s), 3.76 (3H, s), 3.60 (2H, s), 3.38 (3H, s), 2.37 (3H, s).
MS (API) m/z : 413 [$(M+H)^+$].

(Step 4) 4-Amino-5-{[(2,4-dimethoxybenzyl)methylamino]methyl}-1-methyl-2-phenyl-2,3-dihydro-1H-pyrazol-3-one

To a solution of 5-{[(2,4-dimethoxybenzyl)methylamino]methyl}-1-methyl-4-nitro-2-phenyl-2,3-dihydro-1H-pyrazol-3-one (619 mg, 1.50 mmol) synthesized in step 3 in methanol (20 mL), 5% platinum carbon sulfide (150 mg) was added, and the mixture was stirred for 4 hours under a hydrogen atmosphere. After purging with nitrogen, the reaction solution was filtered, and the filtrate was concentrated under reduced pressure. The obtained residue was purified by silica gel column chromatography (Yamazen Corp., eluting solvent: methanol/chloroform = 1/99 to 5/95) to obtain the title compound (381 mg, yield: 66.4%) as a solid.
$^1$H-NMR ($CDCl_3$) δ: 7.50-7.40 (4H, m), 7.24-7.16 (2H, m), 6.48-6.44 (2H, m), 3.81 (6H, s), 3.62 (2H, s), 3.56 (2H, s), 3.45 (2H, s), 2.80 (3H, s), 2.26 (3H, s).

MS (API) m/z : 383 [$(M+H)^+$].

(Step 5) 5-Chloro-N-{4-[(5-{[(2,4-dimethoxybenzyl)methylamino]methyl}-1-methyl-3-oxo-2-phenyl-2,3-dihydro-1H-pyrazol-4-yl)amino]-2,2-dimethyl-4-oxobutyl}-2-methoxybenzamide

To a solution of 4-amino-5-{[(2,4-dimethoxybenzyl)methylamino]methyl}-1-methyl-2-phenyl-2,3-dihydro-1H-pyrazol-3-one (375 mg, 0.980 mmol) synthesized in step 4 in N,N-dimethylformamide (10 mL), intermediate 1b (293 mg, 0.980 mmol), O-(7-azabenzotriazol-1-yl)-N,N,N',N'-tetramethyluronium hexafluorophosphate (HATU) (447 mg, 1.18 mmol), and N,N-diisopropylethylamine (0.250 mL, 190 mg, 1.47 mmol) were added, and the mixture was stirred at room temperature for 15 hours. The reaction solution was concentrated under reduced pressure, and then, a saturated aqueous solution of sodium bicarbonate was added to the residue, followed by extraction with chloroform three times. The organic layer was dried over anhydrous sodium sulfate. After filtration, the filtrate was concentrated under reduced pressure, and then, the obtained residue was purified by silica gel column chromatography (Yamazen Corp., eluting solvent: methanol/chloroform = 1/99 to 9/91) and further purified by high-performance liquid chromatography (NOMURA Develosil Combi, acetonitrile/water with 0.1% formic acid solution) to obtain the title compound (410 mg, yield: 63.0%) as a solid.
$^1$H-NMR ($CDCl_3$) δ: 8.50 (1H, s), 8.33 (1H, t, J = 6.8 Hz), 8.17 (1H, d, J= 2.9 Hz), 7.47-7.37 (5H, m), 7.29-7.25 (1H, m), 7.19-7.17 (1H, m), 6.91 (1H, d, J = 9.2 Hz), 6.46-6.44 (2H, m), 3.96 (3H, s), 3.80 (3H, s), 3.78 (3H, s), 3.55-3.53 (6H, m), 3.18 (3H, s), 2.27 (5H, m), 1.12 (6H, s).
MS (API) m/z : 664 [$(M+H)^+$].

(Example 4) 5-Chloro-N-(2,2-dimethyl-4-{[1-methyl-5-(methylaminomethyl)-3-oxo-2-phenyl-2,3-dihydro-1H-pyrazol-4-yl]amino}-4-oxobutyl)-2-methoxybenzamide

[Formula 46]

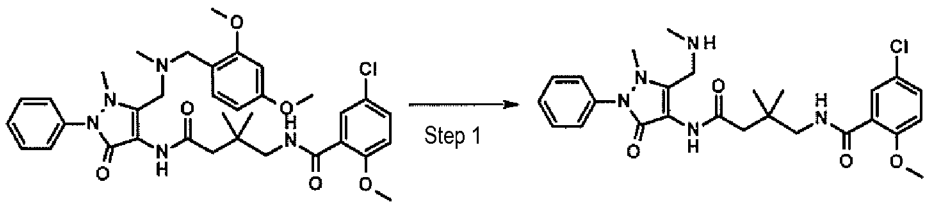


(Step 1) 5-Chloro-N-(2,2-dimethyl-4-{[1-methyl-5-(methylaminomethyl)-3-oxo-2-phenyl-2,3-dihydro-1H-pyrazol-4-yl]amino}-4-oxobutyl)-2-methoxybenzamide

To a solution of 5-chloro-N-{4-[(5-{[(2,4-dimethoxybenzyl)methylamino]methyl}-1-methyl-3-oxo-2-phenyl-2,3-dihydro-1H-pyrazol-4-yl)amino]-2,2-dimethyl-4-oxobutyl}-2-methoxybenzamide (233 mg, 0.351 mmol) synthesized in step 5 of Example 3 in methylene chloride (2.0 mL), trifluoroacetic acid (4.0 mL) and one drop of water were added, and the mixture was stirred at 60°C for 16.5 hours. The reaction solution was allowed to cool to room temperature and then concentrated under reduced pressure, and a saturated aqueous solution of sodium bicarbonate was added to the residue, followed by extraction with chloroform three times. The organic layer was dried over anhydrous sodium sulfate. After filtration, the filtrate was concentrated under reduced pressure, and then, the obtained residue was purified by silica gel column chromatography (Yamazen Corp., eluting solvent: methanol/chloroform = 1/99 to 5/95). After concentration under reduced pressure, the residue was solidified by the addition of ethyl acetate. Ethyl acetate was distilled off under reduced pressure, and then, the solid was suspended in diethyl ether, collected by filtration, and dried to obtain the title compound (136 mg, yield: 75.3%) as a solid.
$^1$H-NMR ($CDCl_3$) δ: 8.95 (1H, s), 8.30 (1H, t, J = 6.3 Hz), 8.19 (1H, d, J= 2.9 Hz), 7.47-7.38 (5H, m), 7.30-7.28 (1H, m), 6.91 (1H, d, J = 9.2 Hz), 3.96 (3H, s), 3.65 (2H, s), 3.56 (2H, d, J = 6.3 Hz), 3.21 (3H, s), 2.50 (3H, s), 2.29 (2H, s), 1.11 (6H, s).
MS (API) m/z : 514[$(M+H)^+$].

The following compounds of Examples 5 to 9 were subsequently synthesized through the same reaction as in step 3 of Example 3 using the corresponding amines instead of (2,4-dimethoxybenzyl)methylamine in step 3 of Example 3, and are shown in Table 9.

[Table 9]

| Example No. | Structure | Intermediate used | Instrumental analysis data |
|---|---|---|---|
| 5 | 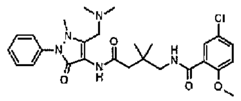 | 1 b | $^1$H-NMR ($CDCl_3$) δ: 8.66 (1H, s), 8.31 (1H, t, J = 6.9 Hz), 8.17 (1H, d, J = 2.9 Hz), 7.47-7.38 (5H, m), 7.30-7.27 (1H, m), 6.91 (1H, d, J = 9.2 Hz), 3.97 (3H, s), 3.55 (2H, d, J = 6.9 Hz), 3.47 (2H, s), 3.24 (3H, s), 2.31 (6H, s), 2.28 (2H, s), 1.12 (6H, s).<br>MS (API) m/z : 528 [$(M+H)^+$]. |
| 6 | 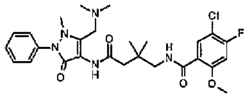 | 1 d | $^1$H-NMR ($CDCl_3$) δ: 8.48 (1H, s), 8.27 (1H, d, J = 8.6 Hz), 8.21 (1H, t, J = 6.9 Hz), 7.47-7.41 (4H, m), 7.31-7.27 (1H, m), 6.79 (1H, d, J = 10.9 Hz), 3.97 (3H, s), 3.54 (2H, d, J = 6.9 Hz), 3.47 (2H, s), 3.24 (3H, s), 2.31-2.28 (8H, m), 1.12 (6H, s).<br>MS (API) m/z : 546 [$(M+H)^+$]. |
| 7 | 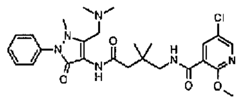 | 1 i | $^1$H-NMR ($CDCl_3$) δ: 8.49-8.48 (2H, m), 8.41 (1H, t, J = 6.3 Hz), 8.20 (1H, d, J = 2.9 Hz), 7.47-7.42 (4H, m), 7.30-7.27 (1H, m), 4.09 (3H, s), 3.54 (2H, d, J = 6.3 Hz), 3.47 (2H, s), 3.24 (3H, s), 2.30-2.28 (8H, m), 1.12 (6H, s).<br>MS (API) m/z : 529 [$(M+H)^+$]. |
| 8 | 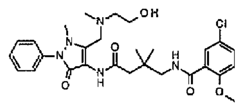 | 1 b | $^1$H-NMR ($CDCl_3$) δ: 8.94 (1H, s), 8.26 (1H, t, J = 6.6 Hz), 8.17 (1H, d, J = 2.9 Hz), 7.47-7.38 (5H, m), 7.31-7.26 (1H, m), 6.92 (1H, d, J = 8.6 Hz), 3.97 (3H, s), 3.69-3.65 (4H, m), 3.56 (2H, d, J = 6.6 Hz), 3.20 (3H, s), 2.67-2.62 (3H, m), 2.38 (3H, s), 2.28 (2H, s), 1.12 (6H, s).<br>MS (API) m/z : 558 [$(M+H)^+$], |
| 9 | 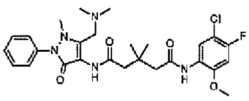 | 2 d | $^1$H-NMR ($CDCl_3$) δ: 8.42-8.36 (3H, m), 7.46-7.40 (4H, m), 7.30-7.27 (1H, m), 6.67 (1H, d, J = 9.7 Hz), 3.83 (3H, s), 3.48 (2H, s), 3.25 (3H, s), 2.67 (2H, s), 2.49 (2H, s), 2.31 (6H, s), 1.17 (6H, s).<br>MS (API) m/z : 546 [$(M+H)^+$]. |

(Example 10) tert-Butyl N-{2-[4({4-[(5-chloro-2-ethoxybenzoyl)amino]-3,3-dimethylbutanoyl}amino)-2-methyl-5-oxo-1-phenyl-2,3-dihydro-1H-pyrazol-3-yl]ethyl}carbamate

[Formula 47]

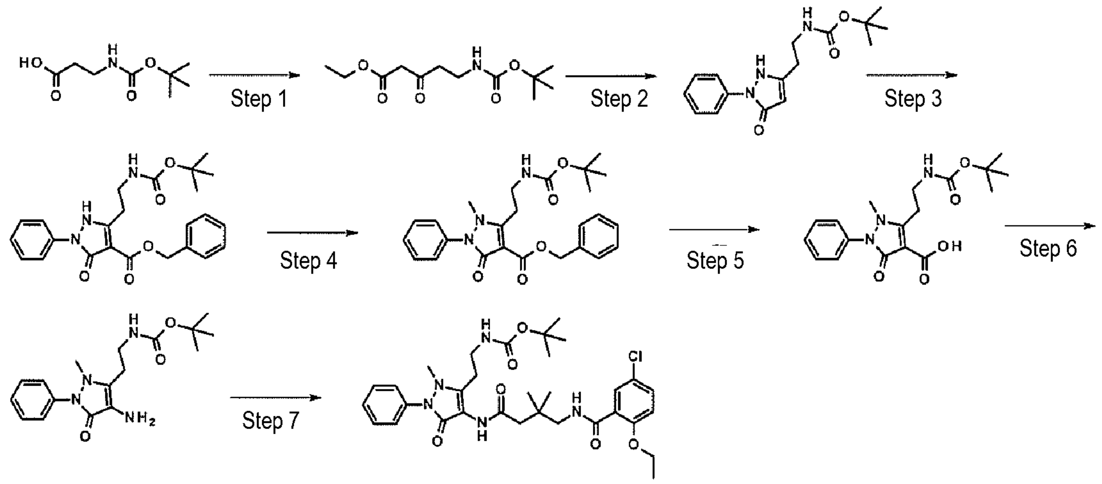


(Step 1) Ethyl 5-(tert-butoxycarbonylamino)-3-oxopentanoate

Potassium monoethyl malonate (9.90 g, 58.1 mmol) was suspended in acetonitrile (200 mL). To the suspension, magnesium chloride (5.54 g, 58.1 mmol) and triethylamine (8.10 mL, 5.88 g, 58.1 mmol) were added, and the mixture was stirred at room temperature for 2 hours. An active ester solution prepared by dissolving 3-(tert-butoxycarbonylamino)propionic acid (10.0 g, 52.9 mmol) in acetonitrile (200 mL), adding 1,1'-carbonyldiimidazole (9.43 g, 58.1 mmol) to the solution, and stirring the mixture at room temperature for 3 hours was added thereto. After the completion of the addition, the mixture was heated to reflux for 4 hours. After cooling to room temperature, the reaction mixture was neutralized by the addition of 1 N hydrochloric acid, followed by extraction with ethyl acetate. The organic layer was washed with water and saturated saline and then dried over anhydrous sodium sulfate, and the solvent was distilled off under reduced pressure. The obtained residue was purified by silica gel chromatography (Shoko Scientific Co., Ltd., eluting solvent: hexane/ethyl acetate = 95/5 to 50/50) to obtain the title compound (13.7 g, yield: 99.0%) as an oil substance.
$^1$H-NMR ($CDCl_3$) δ: 4.99 (1H, br s), 4.20 (2H, q, J = 7.0 Hz), 3.38 (2H, q, J= 5.6 Hz), 2.78 (2H, t, J = 5.6 Hz), 1.43 (9H, s), 1.29 (3H, t, J= 7.0 Hz).
MS (APCI) m/z : 160 [(M-Boc+H)$^+$].

(Step 2) tert-Butyl N-[2-(3-oxo-2-phenyl-1H-pyrazol-5-yl)ethyl]carbamate

Ethyl 5-(tert-butoxycarbonylamino)-3-oxopentanoate (13.7 g, 52.9 mmol) synthesized in step 1 was dissolved in toluene (200 mL). To the solution, phenylhydrazine (5.91 mL, 6.49 g, 58.2 mmol) was added, and the mixture was heated to reflux for 7.2 hours. The reaction solution was cooled to room temperature, left overnight, and then heated to reflux again for 5.8 hours. After cooling to room temperature, the solvent was distilled off under reduced pressure, and the obtained residue was purified by silica gel column chromatography (Shoko Scientific Co., Ltd., eluting solvent: hexane/ethyl acetate = 95/5 to 25/75). The obtained solid was suspended in diethyl ether, collected by filtration, and further dried under reduced pressure to obtain the title compound (14.1 g, yield: 87.7%) as a solid.
$^1$H-NMR (DMSO-$D_6$) δ: 11.51 (1H, br s), 7.72-7.70 (2H, m), 7.43-7.40 (2H, m), 7.23-7.20 (1H, m), 6.88 (1H, t, J= 5.4 Hz), 5.41 (1H, s), 3.19-3.15 (2H, m), 2.57 (2H, t, J = 7.6 Hz), 1.38 (9H, s).
MS (APCI) m/z : 304 [(M+H)$^+$].

(Step 3) Benzyl 5-[2-(tert-butoxycarbonylamino)ethyl]-3-oxo-2-phenyl-1H-pyrazole-4-carboxylate

tert-Butyl N-[2-(3-oxo-2-phenyl-1H-pyrazol-5-yl)ethyl]carbamate (10.6 g, 35.1 mmol) synthesized in step 2 was dissolved in 1,4-dioxane (360 mL). To the solution, calcium hydroxide (5.72 g, 77.2 mmol) was added. The reaction mixture was stirred at 50°C for 0.8 hours, then benzyl chloroformate (5.26 mL, 6.28 g, 36.8 mmol) was added thereto under ice cooling, and the mixture was stirred at 90°C for 3 hours. After cooling to room temperature, the reaction mixture was rendered acidic by the addition of water and 5 N hydrochloric acid, followed by extraction with ethyl acetate. The organic layer was washed with saturated saline and dried over anhydrous sodium sulfate. The solvent was distilled off under reduced pressure, and the obtained residue was purified by silica gel column chromatography (Yamazen Corp.,

eluting solvent: hexane/ethyl acetate = 57/43 to 0/100) to obtain the title compound (14.5 g, yield: 94.6%) as an oil substance.
$^1$H-NMR (DMSO-$D_6$) δ: 7.75-7.70 (2H, m), 7.47-7.44 (4H, m), 7.40-7.36 (2H, m), 7.34-7.28 (3H, m), 6.81 (1H, t, J= 5.2 Hz), 5.28 (2H, s), 3.27-3.22 (4H, m), 2.84 (2H, t, J = 7.0 Hz), 1.35 (9H, s).
MS (APCI) m/z : 438 [$(M+H)^+$].

(Step 4) Benzyl 5-[2-(tert-butoxycarbonylamino)ethyl]-1-methyl-3-oxo-2-phenyl-1H-pyrazole-4-carboxylate

tert-Butyl N-[2-(3-oxo-2-phenyl-1H-pyrazol-5-yl)ethyl]carbamate (5.00 g, 11.4 mmol) obtained in step 3 was dissolved in methylene chloride (60 mL). To the solution, potassium acetate (1.68 g, 14.7 mmol) and methyl trifluoromethanesulfonate (1.68 mL, 2.44 g, 14.9 mmol) were added, and the mixture was stirred at room temperature for 1.5 hours. A saturated aqueous solution of sodium bicarbonate was added to the reaction mixture, followed by extraction with methylene chloride. The organic layer was dried over anhydrous sodium sulfate. The solvent was distilled off under reduced pressure, and the obtained residue was purified by silica gel column chromatography (Yamazen Corp., eluting solvent: hexane/ethyl acetate = 57/43 to 0/100) to obtain the title compound (2.95 g, yield: 57.2%) as a solid.
$^1$H-NMR ($CDCl_3$) δ: 7.52-7.47 (4H, m), 7.42-7.38 (1H, m), 7.35-7.25 (5H, br m), 5.34 (2H, s), 4.98 (1H, t, J= 6.5 Hz), 3.46 (2H, q, J = 6.5 Hz), 3.39 (3H, s), 3.26 (2H, t, J= 6.5 Hz), 1.41 (9H, s).
MS (APCI) m/z : 452 [$(M+H)^+$].

(Step 5) 5-[2-(tert-Butoxycarbonylamino)ethyl]-1-methyl-3-oxo-2-phenyl-1H-pyrazole-4-carboxylic acid

Benzyl 5-[2-(tert-butoxycarbonylamino)ethyl]-1-methyl-3-oxo-2-phenyl-1H-pyrazole-4-carboxylate (3.21 g, 7.11 mmol) synthesized in step 4 was dissolved in ethanol (70 mL). A 10% palladium carbon catalyst (M type) (2.0 g) was suspended in the solution, and the mixture was stirred at room temperature for 1 hour under a hydrogen atmosphere. The reaction system was purged with nitrogen. After filtration, the filtrate was concentrated under reduced pressure to obtain the title compound (2.48 g, yield: 96.5%) as a solid.
$^1$H-NMR ($CDCl_3$) δ: 7.58-7.50 (3H, m), 7.37-7.35 (2H, m), 5.09 (1H, br s), 3.55-3.50 (2H, m), 3.47 (3H, br s), 3.33 (2H, t, J = 5.8 Hz), 1.40 (9H, s).
MS (APCI) m/z : 362 [$(M+H)^+$].

(Step 6) tert-Butyl N-[2-(4-amino-2-methyl-5-oxo-1-phenyl-2,3-dihydro-1H-pyrazol-3-yl)ethyl]carbamate

5-[2-(tert-Butoxycarbonylamino)ethyl]-1-methyl-3-oxo-2-phenyl-1H-pyrazole-4-carboxylic acid (2.48 g, 6.86 mmol) obtained in step 5 was dissolved in N,N-dimethylformamide (50 mL). To the solution, triethylamine (1.24 mL, 0.90 g, 8.92 mmol) and diphenylphosphoric acid azide (1.63 mL, 2.08 g, 7.55 mmol) were added, and the mixture was stirred at room temperature for 1.8 hours. The reaction mixture was added over 1.5 hours to a N,N-dimethylformamide/water (35 mL/35 mL) mixed solvent heated to 100°C, and the mixture was further stirred at the same temperature as above for 1 hour. After cooling to room temperature, saturated sodium bicarbonate was added to the reaction mixture, and the mixture was concentrated under reduced pressure. To the obtained residue, water was added, followed by extraction with ethyl acetate. The combined organic layer was washed with saturated saline and then dried over anhydrous sodium sulfate. The solvent was distilled off under reduced pressure, and the obtained residue was purified by silica gel chromatography (Yamazen Corp., eluting solvent: hexane/ethyl acetate → ethyl acetate/methanol = 85/15 to 0/100 → 100/0 to 90/10) to obtain the title compound (1.17 g, yield: 51.3%) as a solid.
$^1$H-NMR ($CDCl_3$) δ: 7.48-7.42 (4H, m), 7.26-7.23 (1H, m), 5.15 (1H, br s), 3.42-3.38 (2H, m), 3.09 (2H, br s), 2.85 (3H, s), 2.77 (2H, t, J = 6.8 Hz), 1.45 (9H, s).
MS (APCI) m/z : 333 [$(M+H)^+$].

(Step 7) tert-Butyl N-{2-[4({4-[(5-chloro-2-ethoxybenzoyl)amino]-3,3-dimethylbutanoyl}amino)-2-methyl-5-oxo-1-phenyl-2,3-dihydro-1H-pyrazol-3-yl]ethyl}carbamate

tert-Butyl N-[2-(4-amino-2-methyl-5-oxo-1-phenyl-2,3-dihydro-1H-pyrazol-3-yl)ethyl]carbamate (0.320 g, 0.960 mmol) synthesized in step 6 was dissolved in ethanol (5.0 mL). To the solution, intermediate 1a (0.362 g, 1.16 mmol) and 4-(4,6-dimethoxy-1,3,5-triazin-2-yl)-4-methylmorpholinium chloride n-hydrate (DMT-MM) (0.346 g, 1.25 mmol) were added, and the mixture was stirred at room temperature for 15.5 hours. The solvent was distilled off under reduced pressure. To the obtained residue, ethyl acetate was added, and the organic layer was washed with a saturated aqueous solution of sodium bicarbonate and saturated saline and dried over anhydrous sodium sulfate. The solvent was distilled off under reduced pressure, and the obtained residue was suspended by the addition of ethyl acetate. The solid was collected by filtration and dried to obtain the title compound (0.401 g, yield: 66.0%) as a solid.

$^1$H-NMR ($CDCl_3$) δ: 9.24 (1H, s), 8.39 (1H, t, J = 6.7 Hz), 8.21 (1H, d, J = 2.4 Hz), 7.44-7.43 (4H, m), 7.39 (1H, dd, J = 8.5, 2.4 Hz), 7.29-7.25 (1H, m), 6.92 (1H, d, J = 8.5 Hz), 5.77 (1H, t, J = 5.5 Hz), 4.21 (2H, q, J = 6.8 Hz), 3.61 (2H, d, J = 6.7 Hz), 3.54-3.49 (2H, m), 3.14 (3H, s), 2.90 (2H, t, J = 7.0 Hz), 2.27 (2H, s), 1.53 (3H, t, J = 6.9 Hz), 1.43 (9H, s), 1.12 (6H, s).
MS (APCI) m/z : 628 [$(M+H)^+$].

(Example 11) 5-Chloro-N-[4-({5-[2-(dimethylamino)ethyl]-1-methyl-3-oxo-2-phenyl-2,3-dihydro-1H-pyrazol-4-yl}amino)-2,2-dimethyl-4-oxobutyl]-2-ethoxybenzamide

[Formula 49]

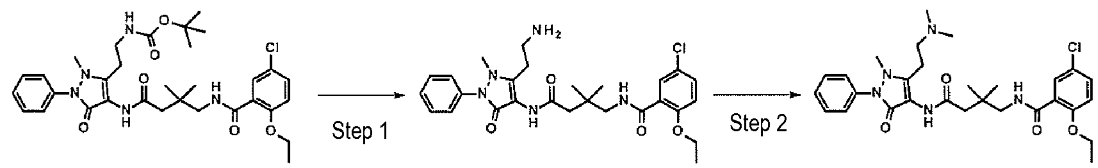


(Step 1) N-(4-{[5-(2-Aminoethyl)-1-methyl-3-oxo-2-phenyl-2,3-dihydro-1H-pyrazol-4-yl]amino}-2,2-dimethyl-4-oxobutyl)-5-chloro-2-ethoxybenzamide

tert-Butyl N-{2-[4({4-[(5-chloro-2-ethoxybenzoyl)amino]-3,3-dimethylbutanoyl}amino)-2-methyl-5-oxo-1-phenyl-2,3-dihydro-1H-pyrazol-3-yl]ethyl}carbamate (1.04 g, 1.66 mmol) synthesized in Example 10 was dissolved in a solution of 4 N hydrochloric acid in 1,4-dioxane (20 mL) and ethanol (4 mL), and the solution was stirred at room temperature for 1 hour. The solvent was distilled off under reduced pressure, and a saturated aqueous solution of sodium bicarbonate was added to the residue, followed by extraction with methylene chloride. The organic layer was washed with saturated saline and then dried over anhydrous sodium sulfate. After filtration, the filtrate was concentrated to obtain the title compound (0.930 g, quantitative) as a solid.
$^1$H-NMR ($CDCl_3$) δ: 9.25 (1H, s), 8.39 (1H, t, J = 6.6 Hz), 8.19 (1H, d, J = 2.7 Hz), 7.45-7.43 (4H, m), 7.39 (1H, dd, J= 8.6, 2.7 Hz), 7.28-7.26 (1H, m), 6.92 (1H, d, J = 8.6 Hz), 4.20 (2H, q, J= 7.0 Hz), 3.60 (2H, d, J = 6.8 Hz), 3.11 (3H, s), 3.09 (2H, t, J= 6.8 Hz), 2.84 (2H, t, J = 6.8 Hz), 2.27 (2H, s), 1.53 (3H, t, J= 7.0 Hz), 1.13 (6H, s) .

(Step 2) 5-Chloro-N-[4-({5-[2-(dimethylamino)ethyl]-1-methyl-3-oxo-2-phenyl-2,3-dihydro-1H-pyrazol-4-yl}amino)-2,2-dimethyl-4-oxobutyl]-2-ethoxybenzamide

N-(4-{[5-(2-Aminoethyl)-1-methyl-3-oxo-2-phenyl-2,3-dihydro-1H-pyrazol-4-yl]amino}-2,2-dimethyl-4-oxobutyl)-5-chloro-2-ethoxybenzamide (1.38 g, 2.61 mmol) synthesized in step 1 was dissolved in methanol (25 mL). To the solution, a 37% aqueous formaldehyde solution (0.980 mL, 13.1 mmol), acetic acid (0.180 mL, 3.13 mmol), and sodium cyanoborohydride (0.860 g, 13.1 mmol) were added, and the mixture was stirred at room temperature for 1.8 hours. A saturated aqueous solution of sodium bicarbonate was added to the reaction mixture, followed by extraction with methylene chloride. The organic layer was washed with saturated saline and dried over anhydrous sodium sulfate. The solvent was distilled off under reduced pressure, and the obtained residue was purified by silica gel column chromatography (Yamazen Corp., eluting solvent: methylene chloride/methanol = 100/0 to 88/12). The solvent was distilled off under reduced pressure. To the obtained residue, diethyl ether was added, and insoluble matter was collected by filtration and dried under reduced pressure to obtain the title compound (1.00 g, yield: 68.9%) as a solid.
1H-NMR ($CDCl_3$) δ: 9.14 (1H, s), 8.38 (1H, t, J = 6.4 Hz), 8.21 (1H, d, J= 2.4 Hz), 7.44-7.43 (4H, m), 7.38 (1H, dd, J = 9.2, 2.4 Hz), 6.91 (1H, d, J = 9.2 Hz), 4.20 (2H, q, J = 7.0Hz), 3.59 (2H, d, J = 6.4 Hz), 3.10 (3H, s), 2.84 (2H, t, J = 7.9 Hz), 2.69 (2H, t, J = 7.9 Hz), 2.33 (6H, s), 2.27 (2H, s), 1.69 (2H, br s), 1.52 (3H, t, J = 7.0 Hz), 1.13 (6H, s).
MS (APCI) m/z : 556 [$(M+H)^+$].
The following compounds of Examples 12 to 25 were synthesized in the same way as above and are shown in Table 10.

[Table 10-1]

| Example No. | Structure | Intermediate used | Instrumental analysis data |
|---|---|---|---|
| 1 2 | 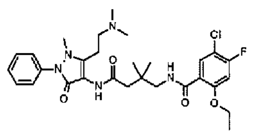 | 1 c | $^1$H-NMR ($CDCl_3$) δ: 9.00 (1H, s), 8.31 (1H, d, J = 9.2 Hz), 8.24 (1H, t, J = 7.3 Hz), 7.44-7.43 (4H, m), 7.28-7.25 (1H, m), 6.78 (1H, d, J = 10.4 Hz), 4.18 (2H, q, J = 7.0 Hz), 3.58 (2H, d, J = 7.3 Hz), 3.10 (3H, s), 2.85 (2H, t, J = 7.9 Hz), 2.68 (2H, t, J = 7.9 Hz), 2.33 (6H, s), 2.26 (2H, s), 1.64 (2H, br s), 1.54 (3H, t, J = 7.0 Hz), 1.12 (6H, s).<br>MS (APCI) m/z: 574 [(M+H)$^+$]. |
| 1 3 | 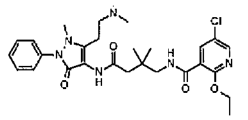 | 1 h | $^1$H-NMR ($CDCl_3$) δ: 8.81 (1H, br s), 8.50 (1H, d, J = 2.4 Hz), 8.41 (1H, t, J = 6.7 Hz), 8.19 (1H, d, J = 2.4 Hz), 7.47-7.42 (4H, m), 7.29-7.25 (1H, m), 4.55 (2H, q, J = 7.1 Hz), 3.59 (2H, d, J = 6.7 Hz), 3.10 (3H, s), 2.85 (2H, t, J = 7.6 Hz), 2.68 (2H, t, J = 7.6 Hz), 2.32 (6H, s), 2.27 (2H, s), 1.65 (2H, s), 1.48 (3H, t, J = 7.0 Hz), 1.13 (6H, s).<br>MS (APCI) m/z : 557 [(M+H)$^+$]. |
| 14 | 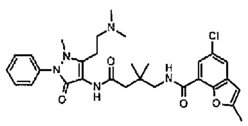 | 3 a | $^1$H-NMR ($CDCl_3$) δ: 8.65 (1H, br s), 7.97 (1H, d, J = 1.8 Hz), 7.92 (1H, t, J = 6.7 Hz), 7.56 (1H, d, J = 1.8 Hz), 7.47-7.41 (4H, m), 7.29-7.25 (1H, m), 6.43 (1H, br s), 3.67 (2H, d, J = 6.7 Hz), 3.11 (3H, s), 2.85 (2H, t, J = 7.6 Hz), 2.68 (2H, t, J = 7.6 Hz), 2.51 (3H, s), 2.33 (2H, s), 2.32 (6H, s), 1.17 (6H, s).<br>MS (APCI) m/z: 566 [(M+H)$^+$]. |
| 1 5 | 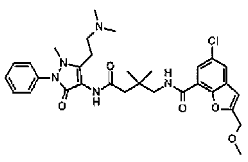 | 3 g | $^1$H-NMR ($CDCl_3$) δ: 8.51 (1H, s), 8.05 (1H, d, J = 2.0 Hz), 7.92 (1H, t, J = 6.3 Hz), 7.65 (1H, d, J = 2.0 Hz), 7.47-7.40 (4H, m), 7.29-7.28 (1H, m), 6.74 (1H, s), 4.59 (2H, s), 3.65 (2H, d, J = 6.3 Hz), 3.43 (3H, s), 3.10 (3H, s), 2.86-2.83 (2H, m), 2.68-2.65 (2H, m), 2.35 (2H, s), 2.31 (6H, s), 1.18 (6H, s).<br>MS (ESI) m/z: 596 [(M+H)$^+$]. |
| 1 6 | 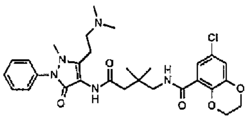 | 3 e | $^1$H-NMR ($CDCl_3$) δ: 8.08-8.02 (1H, m), 7.71 (1H, d, J = 3.1 Hz), 7.47-7.38 (4H, m), 7.30-7.24 (2H, m), 7.00 (1H, d, J = 3.1 Hz), 4.44-4.40 (2H, m), 4.33-4.29 (2H, m), 3.55 (2H, d, J = 6.7 Hz), 3.10 (3H, s), 2.86-2.81 (2H, m), 2.69-2.64 (2H, m), 2.32 (6H, s), 2.28 (2H, s), 1.12 (6H, s).<br>MS (ESI+APCI) m/z : 570 [(M+H)$^+$]. |
| 1 7 | 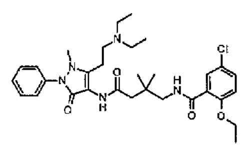 | 1 a | $^1$H-NMR ($CDCl_3$) δ: 9.10 (1H, s), 8.38 (1H, t, J = 6.7 Hz), 8.20 (1H, d, J = 2.7 Hz), 7.46-7.42 (4H, m), 7.38 (1H, dd, J = 8.9, 2.7 Hz), 7.28-7.24 (1H, m), 6.91 (1H, d, J = 9.2 Hz), 4.20 (2H, q, J = 7.0 Hz), 3.59 (2H, d, J = 7.3 Hz), 3.12 (3H, s), 2.87-2.78 (4H, m), 2.62 (4H, q, J = 7.0 Hz), 2.27 (2H, s), 1.52 (3H, t, J = 7.0 Hz), 1.13 (6H, s), 1.07 (6H, t, J = 7.0 Hz). |

[Table 10-2]

| | | | |
|---|---|---|---|
| | | | MS (APCI) m/z : 584 [(M+H)$^+$]. |

(continued)

| | | | |
|---|---|---|---|
| 18 | 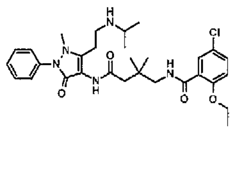 | 1a | $^1$H-NMR ($CDCl_3$) δ: 9.21 (1H, s), 8.38 (1H, t, J = 6.7 Hz), 8.20 (1H, d, J = 3.1 Hz), 7.44-7.37 (5H, m), 7.29-7.24 (1H, m), 6.91 (1H, d, J = 8.5 Hz), 4.20 (2H, q, J = 6.7 Hz), 3.60 (2H, d, J = 7.3 Hz), 3.11 (3H, s), 2.99-2.96 (2H, m), 2.90-2.84 (3H, m), 2.26 (2H, s), 1.52 (3H, t, J = 6.7 Hz), 1.13 (6H, s), 1.09 (6H, d, J = 6.1 Hz).<br>MS (APCI) m/z : 570 [$(M+H)^+$]. |
| 19 | 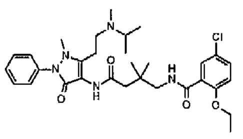 | 1a | $^1$H-NMR ($CDCl_3$) δ: 9.07 (1H, s), 8.39 (1H, t, J = 6.1 Hz), 8.22-8.20 (1H, m), 7.47-7.37 (5H, m), 7.30-7.25 (1H, m), 6.92 (1H, d, J = 9.2 Hz), 4.21 (2H, q, J = 7.0 Hz), 3.59 (2H, d, J = 6.7 Hz), 3.12 (3H, s), 2.94-2.75 (5H, m), 2.30-2.28 (5H, m), 1.53 (3H, t, J = 7.0 Hz), 1.14 (6H, s), 1.04 (6H, d, J = 6.1 Hz).<br>MS (APCI) m/z : 584 [$(M+H)^+$]. |
| 20 | 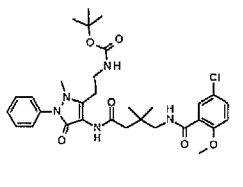 | 1b | $^1$H-NMR ($CDCl_3$) δ: 8.92 (1H, s), 8.23 (1H, t, J = 6.3 Hz), 8.16 (1H, d, J = 2.4 Hz), 7.41-7.40 (4H, m), 7.38 (1H, dd, J = 8.8, 2.4 Hz), 7.26-7.23 (1H, m), 6.91 (1H, d, J = 8.8 Hz), 5.69 (1H, t, J = 5.1 Hz), 3.95 (3H, s), 3.57 (2H, d, J = 6.3 Hz), 3.50-3.46 (2H, m), 3.11 (3H, s), 2.87 (2H, t, J = 6.8 Hz), 2.25 (2H, s), 1.40 (9H, s), 1.09 (6H, s).<br>MS (APCI) m/z : 614 [$(M+H)^+$]. |
| 21 | 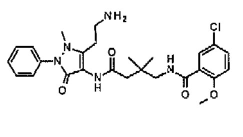 | 1b | $^1$H-NMR (DMSO-$D_6$) δ: 9.33 (1H, s), 8.43 (1H, t, J = 6.3 Hz), 8.17 (3H, br s), 7.69 (1H, d, J = 2.9 Hz), 7.53-7.50 (3H, m), 7.43-7.41 (2H, m), 7.35-7.32 (1H, m), 7.19 (1H, d, J = 8.8 Hz), 3.88 (3H, s), 3.30 (2H, d, J = 6.3 Hz), 3.15-3.10 (2H, m), 2.94-2.91 (2H, m), 2.26 (2H, s), 1.03 (6H, s).<br>MS (APCI) m/z : 514 [$(M+H)^+$]. |
| 22 | 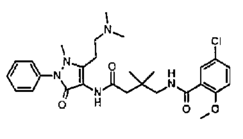 | 1b | $^1$H-NMR ($CDCl_3$) δ: 8.87 (1H, s), 8.28 (1H, t, J = 6.4 Hz), 8.18 (1H, d, J = 2.4 Hz), 7.46-7.42 (4H, m), 7.40 (1 H, dd, J = 9.1, 2.4 Hz), 7.29-7.25 (1H, m), 6.92 (1H, d, J = 9.1 Hz), 3.97 (3H, s), 3.57 (2H, d, J = 6.4 Hz), 3.12 (3H, s), 2.93-2.90 (2H, br m), 2.82-2.77 (2H, br m), 2.40 (6H, br s), 2.28 (2H, s), 1.11 (6H, s).<br>MS (APCI) m/z : 542 [$(M+H)^+$]. |
| 23 | 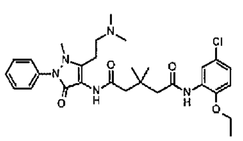 | 2b | $^1$H-NMR ($CDCl_3$) δ: 8.64 (1H, s), 8.44 (1H, s), 8.36 (1H, d, J = 2.4 Hz), 7.46-7.38 (4H, m), 7.30-7.25 (1H, m), 6.98 (1H, dd, J = 8.5, 2.4 Hz), 6.75 (1H, d, J = 8.5 Hz), 4.05 (2H, q, J = 7.0 Hz), 3.10 (3H, s), 2.87-2.83 (2H, m), 2.70-2.64 (4H, m), 2.49 (2H, s), 2.32 (6H, s), 1.40 (3H, t, J = 7.0 Hz), 1.17 (6H, s).<br>MS (APCI) m/z : 556 [$(M+H)^+$]. |

[Table 10-3]

| | | | |
|---|---|---|---|
| 24 | 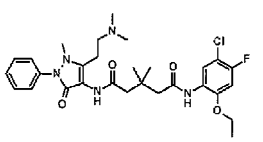 | 2c | $^1$H-NMR ($CDCl_3$) δ: 8.51 (2H, d, J = 3.1 Hz), 8.36 (1H, d, J = 7.9 Hz), 7.46-7.38 (4H, m), 7.30-7.27 (1H, m), 6.65 (1H, d, J = 10.4 Hz), 4.02 (2H, q, J = 7.0 Hz), 3.11 (3H, s), 2.85 (2H, t, J = 7.6 Hz), 2.67-2.63 (4H, m), 2.48 (2H, s), 2.31 (6H, s), 1.40 (3H, t, J = 7.0 Hz), 1.17 (6H, s).<br>MS (APCI) m/z : 574 [$(M+H)^+$]. |
| 25 | 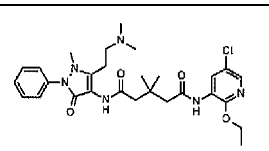 | 2a | $^1$H-NMR ($CDCl_3$) δ: 8.76 (1H, s), 8.62 (1H, s), 8.34 (1H, s), 7.77 (1H, t, J = 2.1 Hz), 7.47-7.26 (5H, m), 4.37 (2H, q, J = 7.0 Hz), 3.11 (3H, s), 2.86 (2H, t, J = 7.3 Hz), 2.68-2.63 (4H, m), 2.47 (2H, s), 2.31 (6H, s), 1.35 (3H, t, J = 7.0 Hz), 1.17 (6H, s).<br>MS (APCI) m/z : 557 [$(M+H)^+$]. |

(Example 26) 5-Chloro-N-[2,2-dimethyl-4-({1-methyl-3-oxo-2-phenyl-5-[2-(2,2,2-trifluoroethylamino)ethyl]pyrazol-4-yl}amino)-4-oxobutyl]-2-ethoxybenzamide

[Formula 50]

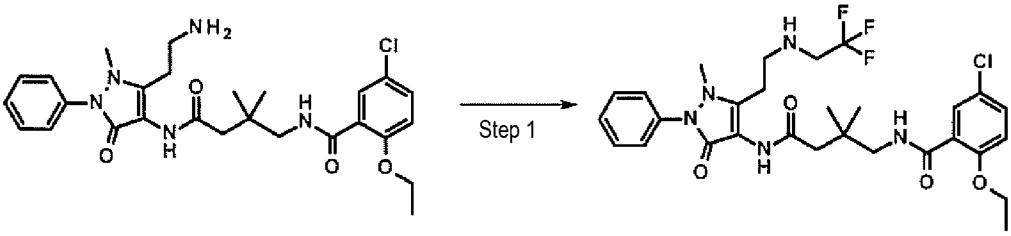


(Step 1) 5-Chloro-N-[2,2-dimethyl-4-({1-methyl-3-oxo-2-phenyl-5-[2-(2,2,2-trifluoroethylamino)ethyl]pyrazol-4-yl}amino)-4-oxobutyl]-2-ethoxybenzamide

To a solution of N-(4-{[5-(2-aminoethyl)-1-methyl-3-oxo-2-phenyl-2,3-dihydro-1H-pyrazol-4-yl]amino}-2,2-dimethyl-4-oxobutyl)-5-chloro-2-ethoxybenzamide (360 mg, 0.682 mmol) synthesized in step 1 of Example 11 in tetrahydrofuran (10 mL), N,N-diisopropylethylamine (0.197 mL, 150 mg, 1.16 mmol) and 2,2,2-trifluoroethyl trifluoromethanesulfonate (0.147 mL, 237 mg, 1.02 mmol) were added, and the mixture was stirred at room temperature for 8.5 hours. Then, N,N-diisopropylethylamine (0.197 mL, 150 mg, 1.16 mmol) and 2,2,2-trifluoroethyl trifluoromethanesulfonate (0.147 mL, 237 mg, 1.02 mmol) were further added thereto, and the mixture was stirred at room temperature for 16 hours. A saturated aqueous solution of sodium bicarbonate was added to the reaction solution, followed by extraction with ethyl acetate three times. The organic layer was washed with saturated saline and then dried over anhydrous sodium sulfate. After filtration, the filtrate was concentrated under reduced pressure, and then, the obtained residue was purified by silica gel column chromatography (Yamazen Corp., eluting solvent: methanol/ethyl acetate = 1/99 to 10/90). After concentration under reduced pressure, the residue was solidified by the addition of ethyl acetate and diethyl ether. The organic solvent was concentrated under reduced pressure, and then, the solid was suspended in diethyl ether, collected by filtration, and dried to obtain the title compound (347 mg, yield: 83.4%) as a solid.
$^1$H-NMR ($CDCl_3$) $\delta$: 9.27 (1H, s), 8.38 (1H, t, J = 6.7 Hz), 8.19 (1H, d, J= 2.4 Hz), 7.47-7.37 (5H, m), 7.30-7.25 (1H, m), 6.92 (1H, d, J = 9.2 Hz), 4.20 (2H, q, J = 7. 0 Hz), 3.60 (2H, d, J = 6.7 Hz), 3.24 (2H, q, J = 9.4 Hz), 3.13-3.06 (5H, m), 2.87 (2H, t, J = 7.0 Hz), 2.27 (2H, s), 1.78 (1H, s), 1.52 (3H, t, J= 7.0 Hz), 1.12 (6H, s).
MS (APCI) m/z : 610 [$(M+H)^+$].

The following compound of Example 27 was synthesized in the same way as above and is shown in Table 11.

[Table 11]

| Example No. | Structure | Intermediate used | Instrumental analysis data |
|---|---|---|---|
| 2 7 | 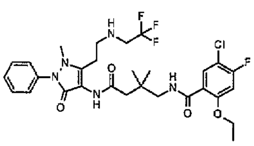 | 1 c | $^1$H-NMR ($CDCl_3$) $\delta$: 9.15 (1H, s), 8.29-8.21 (2H, m), 7.46-7.40 (4H, m), 7.31-7.22 (1H, m), 6.79 (1H, d, J = 10.4 Hz), 4.18 (2H, q, J = 7.0 Hz), 3.59 (2H, d, J = 6.7 Hz), 3.24 (2H, q, J = 9.4 Hz), 3.13-3.06 (5H, m), 2.87 (2H, t, J = 6.7 Hz), 2.26 (2H, s), 1.76 (1H, s), 1.54 (3H, t, J = 7.0 Hz), 1.11 (6H, s).<br>MS (APCI) m/z : 628 [$(M+H)^+$]. |

(Example 28) 5-Chloro-N-[4-({5-[2-(dimethylamino)ethyl]1-methyl-3-oxo-2-phenyl-2,3-dihydro-1H-pyrazol-4-yl}amino)-2,2-dimethyl-4-oxobutyl]-2-(methoxymethoxy)benzamide

[Formula 50]

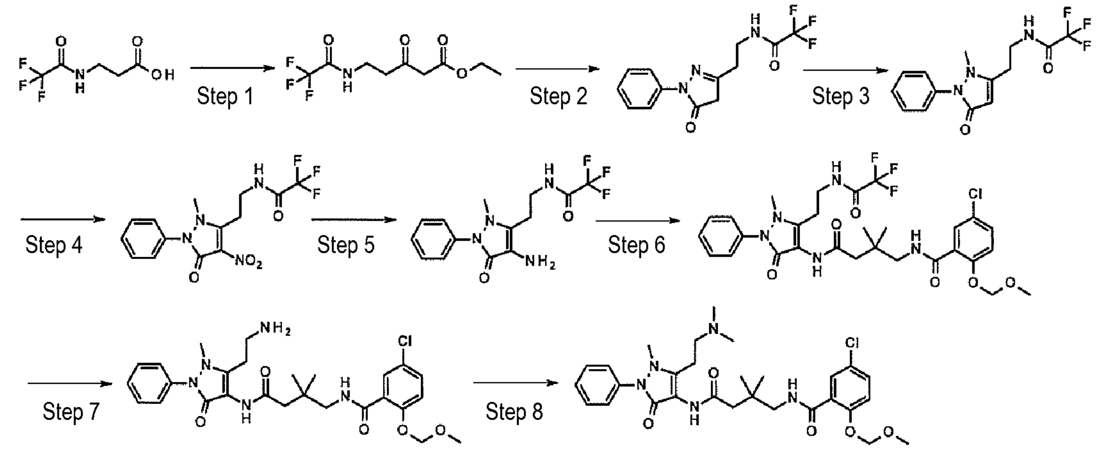


(Step 1) Ethyl 3-oxo-5-[(2,2,2-trifluoroacetyl)amino]pentanoate

To a solution of N-trifluoroacetyl-β-alanine (10.0 g, 54.0 mmol) synthesized according to the method described in Journal of the Chemical Society, Perkin Transactions 1, 1985, 1355-1362 in tetrahydrofuran (250 mL), 1,1'-carbonyldiimidazole (9.64 g, 59.4 mmol) was added under ice cooling, and the mixture was stirred at room temperature for 1 hour. In another reaction vessel, potassium monoethyl malonate (10.1 g, 59.4 mmol) and magnesium chloride (6.17 g, 64.8 mmol) were suspended in tetrahydrofuran (250 mL). To the suspension, triethylamine (9.04 mL, 6.56 g, 64.8 mmol) was added under ice cooling, and the mixture was stirred at room temperature for 1 hour. Then, the reaction solution described above was added dropwise thereto over 1 hour. The reaction solution was stirred at room temperature for 62 hours and then cooled in ice. Water was added thereto, and then, the mixture was rendered acidic with concentrated hydrochloric acid. After extraction with ethyl acetate three times, the organic layer was washed with a saturated aqueous solution of sodium bicarbonate and saturated saline in this order and then dried over anhydrous sodium sulfate. After filtration, the filtrate was concentrated under reduced pressure to obtain the title compound (12.2 g, yield: 88.1%) as an oil substance.
$^1$H-NMR ($CDCl_3$) δ: 6.99 (1H, s), 4.21 (2H, q, J = 7.2 Hz), 3.63 (2H, q, J= 5.7 Hz), 3.48 (2H, s), 2.89 (2H, t, J= 5.7 Hz), 1.29 (3H, t, J = 7.2 Hz).
MS (APCI) m/z : 256[$(M+H)^+$].

(Step 2) 2,2,2-Trifluoro-N-[2-(5-oxo-1-phenyl-4H-pyrazol-3-yl)ethyl]acetamide

To a solution of ethyl 3-oxo-5-[(2,2,2-trifluoroacetyl)amino]pentanoate (12.1 g, 47.4 mmol) synthesized in step 1 in toluene (250 mL), phenylhydrazine (4.76 mL, 5.23 g, 48.4 mmol) was added, and the mixture was stirred for 2 hours under heating to reflux while generated water was removed. The reaction solution was allowed to cool to room temperature and then cooled in ice, and the deposited solid was collected by filtration and dried to obtain the title compound (9.94 g, yield: 70.1%) as a solid.
$^1$H-NMR (DMSO-$D_6$) δ: 11.57 (1H, s), 9.53 (1H, s), 7.70 (2H, d, J = 7. Hz), 7.42 (2H, t, J = 7.3 Hz), 7.22 (1H, t, J = 7.3 Hz), 5.42 (1H, s), 3.45 (2H, q, J = 6.7 Hz), 2.70 (2H, t, J = 7.3 Hz).
MS (APCI) m/z : 300[$(M+H)^+$].

(Step 3) 2,2,2-Trifluoro-N-[2-(2-methyl-5-oxo-1-phenyl-2,3-dihydro-1H-pyrazol-3-yl)ethyl]acetamide

To a solution of 2,2,2-trifluoro-N-[2-(5-oxo-1-phenyl-4H-pyrazol-3-yl)ethyl]acetamide (2.00 g, 6.68 mmol) synthesized in step 2 in N,N-dimethylformamide (2.0 mL), methyl iodide (2.08 mL, 4.74 g, 33.4 mmol) was added, and the mixture was stirred at 100°C for 5 hours in a sealed tube. The reaction solution was allowed to cool to room temperature, and then, a saturated aqueous solution of sodium bicarbonate and saturated saline were added to the reaction solution, followed by extraction with ethyl acetate three times. The organic layer was dried over anhydrous sodium sulfate. After filtration, the filtrate was concentrated under reduced pressure, and then, the obtained residue was solidified by the addition of ethyl acetate. The solid was collected by filtration and dried to obtain the title compound (1.41 g, yield: 67.3%) as a solid.
$^1$H-NMR ($CDCl_3$) δ: 8.85 (1H, s), 7.43-7.48 (2H, m), 7.35-7.31 (3H, m), 5.31 (1H, s), 3.47 (2H, q, J = 6.1 Hz), 3.04 (3H,

s), 2.79 (2H, t, J= 6.1 Hz).
MS (APCI) m/z : 314[(M+H)$^+$].

(Step 4) 2,2,2-Trifluoro-N-[2-(2-methyl-4-nitro-5-oxo-1-phenyl-2,3-dihydro-1H-pyrazol-3-yl)ethyl]acetamide

To a solution of 2,2,2-trifluoro-N-[2-(2-methyl-5-oxo-1-phenyl-2,3-dihydro-1H-pyrazol-3-yl)ethyl]acetamide (1.72 g, 5.49 mmol) synthesized in step 3 in trifluoroacetic acid (17 mL), concentrated nitric acid (1.06 mL, 1.48 g, 16.5 mmol) was added, and the mixture was stirred at room temperature for 0.75 hours. The reaction solution was poured into ice water, followed by extraction with methylene chloride three times. The organic layer was washed with a saturated aqueous solution of sodium bicarbonate and then dried over anhydrous sodium sulfate. After filtration, the filtrate was concentrated under reduced pressure, and then, the obtained solid was suspended in diethyl ether. The solid was collected by filtration and dried to obtain the title compound (1.70 g, yield: 86.4%) as a solid.
$^1$H-NMR ($CDCl_3$) δ: 9.35 (1H, t, J= 6.1 Hz), 7.64-7.55 (3H, m), 7.48-7.46 (2H, m), 3.83 (2H, q, J = 6.1 Hz), 3.48 (2H, t, J = 6.1 Hz), 3.35 (3H, s).
MS (APCI) m/z : 359[(M+H)$^+$].

(Step 5) N-[2-(4-Amino-2-methyl-5-oxo-1-phenyl-2,3-dihydro-1H-pyrazol-3-yl)ethyl]-2,2,2-trifluoroacetamide

To a suspension of 2,2,2-trifluoro-N-[2-(2-methyl-4-nitro-5-oxo-1-phenyl-2,3-dihydro-1H-pyrazol-3-yl)ethyl]acetamide (1.60 g, 4.47 mmol) synthesized in step 4 in ethanol (22 mL), a 10% palladium carbon catalyst (0.500 g) was added, and the mixture was stirred at 50°C for 4 hours under a hydrogen atmosphere. The reaction solution was allowed to cool to room temperature, and then, the reaction system was purged with nitrogen. After filtration of the reaction solution, the filtrate was concentrated under reduced pressure to obtain the title compound (1.46 g, yield: 99.6%) as a solid.
$^1$H-NMR ($CDCl_3$) δ: 8.11 (1H, s), 7.47-7.42 (4H, m), 7.30-7.26 (1H, m), 3.65 (2H, q, J = 6.1 Hz), 3.03 (2H, s), 2.87-2.82 (5H, m).
MS (APCI) m/z : 329[(M+H)$^+$].

(Step 6) 5-Chloro-N-{2,2-dimethyl-4-[(1-methyl-3-oxo-2-phenyl-5-{2-[(2,2,2-trifluoroacetyl)amino]ethyl}pyrazol-4-yl)amino]-4-oxobutyl}-2-(methoxymethoxy)benzamide

To a solution of N-[2-(4-amino-2-methyl-5-oxo-1-phenyl-2,3-dihydro-1H-pyrazol-3-yl)ethyl]-2,2,2-trifluoroacetamide (328 mg, 1.00 mmol) synthesized in step 5 in ethanol (10 mL), intermediate 1e (346 mg, 1.05 mmol) and 4-(4,6-dimethoxy-1,3,5-triazin-2-yl)-4-methylmorpholinium chloride n-hydrate (DMT-MM) (494 mg, 1.30 mmol) were added, and the mixture was stirred at room temperature for 15 hours. The reaction solution was concentrated under reduced pressure, and then, a saturated aqueous solution of ammonium chloride was added to the residue, followed by extraction with methylene chloride three times. The organic layer was washed with a saturated aqueous solution of sodium bicarbonate and then dried over anhydrous sodium sulfate. After filtration, the filtrate was concentrated under reduced pressure, and then, the obtained residue was purified by silica gel column chromatography (Yamazen Corp., eluting solvent: methanol/ethyl acetate = 1/99 to 10/90) to obtain the title compound (633 mg, yield: 98.9%) as a solid.
$^1$H-NMR ($CDCl_3$) δ: 9.65 (1H, s), 9.28 (1H, s), 8.19-8.16 (2H, m), 7.48-7.38 (5H, m), 7.31-7.28 (1H, m), 7.16 (1H, d, J = 8.5 Hz), 5.33 (2H, s), 3.74 (2H, q, J = 6.1 Hz), 3.64 (2H, d, J= 6.7 Hz), 3.51 (3H, s), 3.10 (3H, s), 3.00 (2H, t, J = 6.7 Hz), 2.29 (2H, s), 1.08 (6H, s).
MS (APCI) m/z : 640[(M+H)$^+$].

(Step 7) N-(4-{[5-(2-Aminoethyl)-1-methyl-3-oxo-2-phenyl-2,3-dihydro-1H-pyrazol-4-yl]amino}-2,2-dimethyl-4-oxobutyl)-5-chloro-2-(methoxymethoxy)benzamide

To a solution of 5-chloro-N-{2,2-dimethyl-4-[(1-methyl-3-oxo-2-phenyl-5-{2-[(2,2,2-trifluoroacetyl)amino]ethyl}pyrazol-4-yl)amino]-4-oxobutyl}-2-(methoxymethoxy)benzamide (625 mg, 0.976 mmol) synthesized in step 6 in tetrahydrofuran (6.0 mL), methanol (3.0 mL) and a 1 N aqueous sodium hydroxide solution (2.93 mL, 2.93 mmol) were added, and the mixture was stirred at room temperature for 15.5 hours. After concentration under reduced pressure, water was added to the residue, followed by extraction with methylene chloride three times. The organic layer was dried over anhydrous sodium sulfate. After filtration, the filtrate was concentrated under reduced pressure, and then, the obtained solid was suspended in diethyl ether, collected by filtration, and dried to obtain the title compound (430 mg, yield: 80.9%) as a solid.
$^1$H-NMR ($CDCl_3$) δ: 8.81 (1H, s), 8.29 (1H, t, J = 6.4 Hz), 8.15 (1H, d, J= 2.4 Hz), 7.47-7.35 (5H, m), 7.30-7.26 (1H, m), 7.15 (1H, d, J = 8.5 Hz), 5.33 (2H, s), 3.58 (2H, d, J = 7.3 Hz), 3.57 (2H, s), 3.51 (3H, s), 3.11-3.06 (5H, m), 2.83 (2H,

t, J = 7.0 Hz), 2.28 (2H, s), 1.13 (6H, s). MS (APCI) m/z : 544[(M+H)+].

(Step 8) 5-Chloro-N-[4-({5-[2-(dimethylamino)ethyl]1-methyl-3-oxo-2-phenyl-2,3-dihydro-1H-pyrazol-4-yl}amino)-2,2-dimethyl-4-oxobutyl]-2-(methoxymethoxy)benzamide

To a solution of N-(4-{[5-(2-aminoethyl)-1-methyl-3-oxo-2-phenyl-2,3-dihydro-1H-pyrazol-4-yl]amino}-2,2-dimethyl-4-oxobutyl)-5-chloro-2-(methoxymethoxy)benzamide (425 mg, 0.781 mmol) synthesized in step 7 in methylene chloride (10 mL), a 37% aqueous formaldehyde solution (0.352 mL, 380 mg, 4.69 mmol) was added, and the mixture was stirred at room temperature for 20 minutes. Then, sodium triacetoxyborohydride (993 mg, 4.69 mmmol) and acetic acid (0.268 mL, 281 mg, 4.69 mmol) were added thereto, and the mixture was stirred at room temperature for 1 hour. A saturated aqueous solution of sodium bicarbonate was added to the reaction solution, followed by extraction with methylene chloride three times. The organic layer was dried over anhydrous sodium sulfate. After filtration, the filtrate was concentrated under reduced pressure, and then, the obtained residue was purified by amino silica gel column chromatography (Yamazen Corp., eluting solvent: methanol/ethyl acetate = 1/99 to 15/85). After concentration under reduced pressure, the residue was solidified by the addition of diethyl ether. The solid was collected by filtration and dried to obtain the title compound (408 mg, yield: 91.3%) as a solid.
$^1$H-NMR ($CDCl_3$) δ: 8.69 (1H, s), 8.33 (1H, t, J = 6.7 Hz), 8.16 (1H, d, J= 3.1 Hz), 7.47-7.35 (5H, m), 7.30-7.26 (1H, m), 7.15 (1H, d, J = 9.2 Hz), 5.33 (2H, s), 3.57 (2H, d, J = 6.7 Hz), 3.51 (3H, s), 3.10 (3H, s), 2.86-2.82 (2H, m), 2.68-2.64 (2H, m), 2.31 (6H, s), 2.28 (2H, s), 1.12 (6H, s).
MS (APCI) m/z : 572[(M+H)+].

The following compounds of Examples 29 to 31 were synthesized in the same way as above and are shown in Table 12.

[Table 12]

| Example No. | Structure | Intermediate used | Instrumental analysis data |
|---|---|---|---|
| 29 | 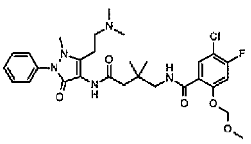 | 1 f | $^1$H-NMR ($CDCl_3$) δ: 8.54 (1H, s), 8.31-8.26 (2H, m), 7.47-7.40 (4H, m), 7.31-7.27 (1H, m), 7.07 (1H, d, J = 10.4 Hz), 5.33 (2H, s), 3.56-3.51 (5H, m), 3.11 (3H, s), 2.86-2.82 (2H, m), 2.68-2.64 (2H, m), 2.31 (6H, s), 2.27 (2H, s), 1.12 (6H, s).<br>MS (APCI) m/z : 590 [(M+H)+]. |
| 3 0 | 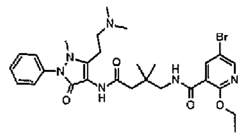 | 1 j | $^1$H-NMR ($CDCl_3$) δ: 8.83 (1H, s), 8.63 (1H, d, J = 2.4 Hz), 8.40 (1H, br t, J = 6.4 Hz), 8.28 (1H, d, J = 2.4 Hz), 7.48-7.40 (4H, m), 7.31-7.24 (1H, m), 4.54 (2H, q, J = 6.9 Hz), 3.59 (2H, d, J = 6.7 Hz), 3.10 (3H, s), 2.89-2.81 (2H, m), 2.71-2.64 (2H, m), 2.32 (6H, s), 2.26 (2H, s), 1.48 (3H, t, J = 7.0 Hz), 1.13 (6H, s).<br>MS (ESI) m/z: 601 [(M+H)+]. |
| 3 1 | 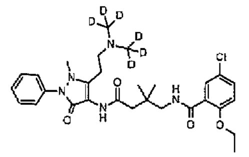 | 1 a | $^1$H-NMR ($CDCl_3$) δ: 9.13 (1H, s), 8.38 (1H, br t, J = 6.4 Hz), 8.21 (1H, d, J = 2.4 Hz), 7.46-7.41 (4H, m), 7.38 (1 H, dd, J = 8.8, 2.7 Hz), 7.29-7.22 (1H, m), 6.91 (1H, d, J = 9.1 Hz), 4.20 (2H, q, J = 6.9 Hz), 3.59 (2H, d, J = 6.7 Hz), 3.10 (3H, s), 2.87-2.80 (2H, m), 2.72-2.65 (2H, m), 2.27 (2H, s), 1.52 (3H, t, J = 7.0 Hz), 1.13 (6H, 5).<br>MS (ESI) m/z: 562 [(M+H)+]. |
| 32 | 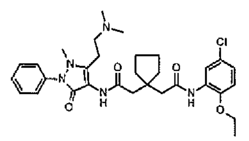 | 2 h | $^1$H-NMR ($CDCl_3$) δ: 8.54 (1H, s), 8.53 (1H, s), 8.33 (1H, d, J = 2.4 Hz), 7.45-7.39 (4H, m), 7.29-7.27 (1H, m), 6.98 (1H, dd, J = 8.8, 2.4 Hz), 6.74 (1H, d, J = 8.8 Hz), 4.04 (2H, q, J = 7.0 Hz), 3.10 (3H, s), 2.86-2.83 (2H, m), 2.75 (2H, s), 2.66-2.64 (2H, m), 2.53 (2H, s), 2.31 (6H, s), 1.71-1.66 (8H, m), 1.39 (3H, t, J = 7.0 Hz).<br>MS (ESI) m/z: 582 [(M+H)-]. |

(Example 33) 5-Chloro-N-[2,2-dimethyl-4-({1-methyl-5-[2-(methylamino)ethyl]-3-oxo-2-phenyl-2,3-dihydro-1H-pyrazol-4-yl}amino)-4-oxobutyl]-2-ethoxybenzamide

[Formula 51]

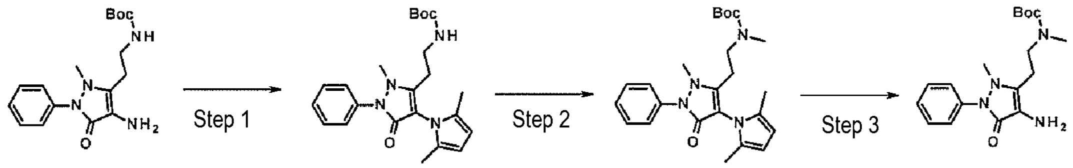


(Step 1) tert-Butyl N-{2-[4-(2,5-dimethylpyrrol-1-yl)-2-methyl-5-oxo-1-phenyl-2,3-dihydro-1H-pyrazol-3-yl]ethyl}carbamate

tert-Butyl N-[2-(4-amino-2-methyl-5-oxo-1-phenyl-2,3-dihydro-1H-pyrazol-3-yl)ethyl]carbamate (0.800 g, 2.41 mmol) synthesized in step 6 of Example 10 was suspended in toluene (20 ml). To the suspension, hexane-2,5-dione (0.330 g, 2.89 mmol) and tosylic acid monohydrate (0.0137 g, 0.0722 mmol) were added, and the mixture was stirred at 70°C for 2 hours. The reaction solution was cooled to room temperature and then separated into aqueous and organic layers by the addition of a saturated aqueous solution of sodium bicarbonate. The organic layer was washed with saturated saline and dried over anhydrous sodium sulfate. The solvent was distilled off under reduced pressure, and the obtained residue was purified by silica gel column chromatography (Shoko Scientific Co., Ltd., eluting solvent: ethyl acetate/hexane = 20/80 to 100/0) to obtain the title compound (1.09 g, quantitative) as a solid.
$^1$H-NMR (CDCl3) $\delta$: 7.48-7.42 (4H, m), 7.32-7.28 (1H, m), 5.85 (2H, s), 4.62 (1H, br s), 3.26-3.21 (5H, m), 2.67 (2H, t, J = 7.1 Hz), 2.05 (6H, s), 1.40 (9H, s).
MS (APCI) m/z : 411 [(M+H)$^+$].

(Step 2) tert-Butyl N-{2-[4-(2,5-dimethylpyrrol-1-yl)-2-methyl-5-oxo-1-phenyl-2,3-dihydro-1H-pyrazol-3-yl]ethyl}-N-methylcarbamate

tert-Butyl N-{2-[4-(2,5-dimethylpyrrol-1-yl)-2-methyl-5-oxo-1-phenyl-2,3-dihydro-1H-pyrazol-3-yl]ethyl}carbamate (1.00 g, 2.44 mmol) synthesized in step 1 was dissolved in tetrahydrofuran (20 ml). To the solution, sodium hydride (60% oil) (0.127 g, 3.17 mmol) was added under water cooling, and the mixture was stirred for 30 minutes. Methyl iodide (0.591 ml, 1.35 g, 9.50 mmol) was added to the reaction solution, and the mixture was stirred at 60°C for 8 hours. The reaction solution was cooled to room temperature, and then, a saturated aqueous solution of ammonium chloride was added thereto, followed by extraction with ethyl acetate. The extract was washed with saturated saline in this order and dried over anhydrous sodium sulfate. The solvent was distilled off under reduced pressure, and the obtained residue was purified by silica gel column chromatography (Shoko Scientific Co., Ltd., eluting solvent: ethyl acetate/methylene chloride = 0/100 to 20/80) to obtain the title compound (0.453 g, yield: 44.0%) as a solid.
$^1$H-NMR (CDCl3) $\delta$: 7.48-7.43 (4H, m), 7.30 (1H, br s), 5.85 (2H, s), 3.42-3.14 (5H, m), 2.83-2.62 (5H, m), 2.05 (6H, s), 1.41 (9H, s).
MS (APCI) m/z : 425 [(M+H)$^+$].

(Step 3) tert-Butyl N-[2-(4-amino-2-methyl-5-oxo-1-phenyl-2,3-dihydro-1H-pyrazol-3-yl)ethyl]-N-methylcarbamate

tert-Butyl N-{2-[4-(2,5-dimethylpyrrol-1-yl)-2-methyl-5-oxo-1-phenyl-2,3-dihydro-1H-pyrazol-3-yl]ethyl}-N-methylcarbamate (0.450 g, 1.06 mmol) synthesized in step 2 was dissolved in ethanol (8.0 ml). To the solution, hydroxylamine hydrochloride (1.47 g, 21.2 mmol), triethylamine (1.47 mL, 1.07 g, 10.6 mmol), and water (2.0 ml) were added in this order, and the mixture was stirred under heating at 100°C for 10 hours in a sealed steel tube. The reaction solution was cooled to room temperature and then concentrated under reduced pressure, and a saturated aqueous solution of sodium bicarbonate was added to the residue, followed by extraction with ethyl acetate. The extract was washed with saturated saline and dried over anhydrous sodium sulfate. The solvent was distilled off under reduced pressure, and the obtained residue was purified by silica gel column chromatography (Shoko Scientific Co., Ltd., eluting solvent: methanol/methylene chloride = 0/100 to 10/90) to obtain the title compound (0.256 g, yield: 70.0%) as a solid.
$^1$H-NMR (CDCl3) $\delta$: 7.46-7.38 (4H, m), 7.23-7.20 (1H, m), 3.45 (2H, t, J = 7.2 Hz), 3.05-2.66 (8H, m), 1.45 (9H, s). MS (APCI) m/z : 347 [(M+H)$^+$].

The following compounds of Examples 33 to 35 were subsequently synthesized in the same way as in step 7 of Example 10 and steps 1 and 2 of Example 11 and are shown in Table 13.

[Table 13]

| Example No. | Structure | Intermediate used | Instrumental analysis data |
|---|---|---|---|
| 3 3 | 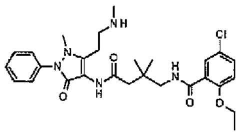 | 1 a | $^1$H-NMR ($CDCl_3$) δ: 9.16 (1H, s), 8.38 (1H, t, J = 6.7 Hz), 8.21-8.19 (1H, m), 7.47-7.37 (5H, m), 7.29-7.25 (1H, m), 6.91 (1H, d, J = 8.5 Hz), 4.20 (2H, q, J = 7.0 Hz), 3.60 (2H, d, J = 6.7 Hz), 3.11 (3H, s), 2.97-2.86 (4H, m), 2.49 (3H, s), 2.27 (2H, s), 1.52 (3H, t, J = 7.0 Hz), 1.13 (6H, s). MS (APCI) m/z : 542 [(M+H)$^+$]. |
| 3 4 | 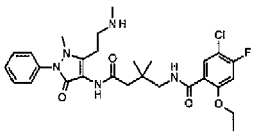 | 1 c | $^1$H-NMR ($CDCl_3$) δ: 9.03 (1H, s), 8.30 (1H, d, J = 9.2 Hz), 8.23 (1H, t, J = 6.7 Hz), 7.47-7.40 (4H, m), 7.29-7.25 (1H, m), 6.78 (1H, d, J = 10.4 Hz), 4.18 (2H, q, J = 7.0 Hz), 3.59 (2H, d, J = 6.7 Hz), 3.11 (3H, s), 2.97-2.85 (4H, m), 2.49 (3H, s), 2.26 (2H, s), 1.54 (3H, t, J = 7.0 Hz), 1.12 (6H, s). MS (APCI) m/z : 560 [(M+H)$^+$]. |
| 3 5 | 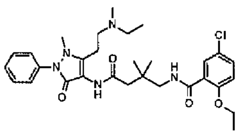 | 1 a | $^1$H-NMR ($CDCl_3$) δ: 9.08 (1H, s), 8.37 (1H, t, J = 6.7 Hz), 8.20 (1H, d, J = 3.1 Hz), 7.46-7.36 (5H, m), 7.30-7.22 (1H, m), 6.91 (1H, d, J = 9.2 Hz), 4.20 (2H, q, J = 7.0 Hz), 3.59 (2H, d, J = 6.7 Hz), 3.10 (3H, s), 2.86-2.74 (4H, m), 2.52 (2H, q, J = 7.0 Hz), 2.32 (3H, s), 2.27 (2H, s), 1.52 (3H, t, J = 7.0 Hz), 1.13-1.07 (9H, m). MS (APCI) m/z : 570 [(M+H)$^+$]. |

(Example 36) 5-Chloro-2-ethoxy-N-{4-[(5-{2-[2-fluoroethyl(methyl)amino]ethyl}-1-methyl-3-oxo-2-phenyl-2,3-dihydro-1H-pyrazol-4-yl)amino]-2,2-dimethyl-4-oxobutyl}benzamide

[Formula 52]

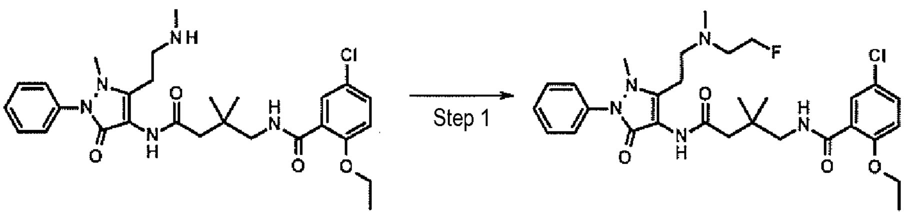


(Step 1) 5-Chloro-2-ethoxy-N-{4-[(5-{2-[2-fluoroethyl(methyl)amino]ethyl}-1-methyl-3-oxo-2-phenyl-2,3-dihydro-1H-pyrazol-4-yl)amino]-2,2-dimethyl-4-oxobutyl}benzamide

To 5-chloro-N-[2,2-dimethyl-4-({1-methyl-5-[2-(methylamino)ethyl]-3-oxo-2-phenyl-2,3-dihydro-1H-pyrazol-4-yl}amino)-4-oxobutyl]-2-ethoxybenzamide (100 mg, 0.184 mmol) synthesized in Example 33, isopropanol (1.0 mL), 1,4-dioxane (1.0 mL), 1-fluoro-2-iodoethane (48.1 mg, 0.277 mmol), and potassium carbonate (38.3 mg, 0.277 mmol) were added, and the mixture was stirred at 100°C for 12 hours in a sealed tube. The reaction solution was allowed to cool to room temperature and then filtered. The filtrate was concentrated under reduced pressure, and the obtained residue was purified by silica gel column chromatography (Biotage Japan Ltd., eluting solvent: methanol/ethyl acetate = 1/99 to 12/88). After concentration under reduced pressure, the residue was solidified by the addition of ethyl acetate and diethyl ether. The organic solvent was distilled off under reduced pressure, and then, the solid was suspended in diethyl ether, collected by filtration, and dried to obtain the title compound (26.0 mg, yield: 24.0%) as a solid.
$^1$H-NMR ($CDCl_3$) δ: 9.09 (1H, s), 8.37 (1H, t, J = 6.7 Hz), 8.21 (1H, d, J= 3.1 Hz), 7.46-7.37 (5H, m), 7.30-7.22 (1H, m),

6.91 (1H, d, J = 9.2 Hz), 4.62 (1H, t, J = 4.9 Hz), 4.50 (1H, t, J = 4.9 Hz), 4.20 (2H, q, J = 7.0 Hz), 3.59 (2H, d, J = 6.7 Hz), 3.10 (3H, s), 2.89-2.76 (6H, m), 2.42 (3H, s), 2.27 (2H, s), 1.52 (3H, t, J = 7.0 Hz), 1.13 (6H, s).
MS (APCI) m/z :588[ $(M+H)^+$].

The following compound of Example 37 was synthesized in the same way as above and is shown in Table 14.

[Table 14]

| Example No. | Structure | Intermediate used | Instrumental analysis data |
|---|---|---|---|
| 3 7 | 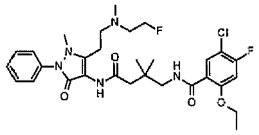 | 1 c | $^1$H-NMR ($CDCl_3$) δ: 9.03 (1H, s), 8.27-8.19 (2H, m), 7.45-7.36 (4H, m), 7.29-7.25 (1H, m), 6.77 (1H, d, J = 10.4 Hz), 4.63 (1H, t, J = 4.6 Hz), 4.51 (1H, t, J = 4.6 Hz), 4.18 (2H, q, J = 7.0 Hz), 3.57 (2H, d, J = 6.7 Hz), 3.11 (3H, s), 2.88-2.78 (6H, m), 2.43 (3H, s), 2.29 (2H, s), 1.54 (3H, t, J = 7.0 Hz), 1.11 (6H, s).<br>MS (APCI) m/z : 606 [$(M+H)^+$]. |

The following compounds of Examples 38 and 39 were synthesized in the same way as in Example 27 and are shown in Table 15.

[Table 15]

| Example No. | Structure | Intermediate used | Instrumental analysis data |
|---|---|---|---|
| 38 | 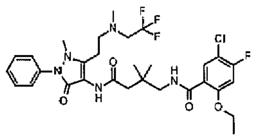 | 1 c: | $^1$H-NMR ($CDCl_3$) δ: 9.04 (1H, s), 8.30 (1H, d, J = 9.2 Hz), 8.22 (1H, t, J = 6.7 Hz), 7.47-7.40 (4H, m), 7.31-7.23 (1H, m), 6.78 (1H, d, J = 11.0 Hz), 4.18 (2H, q, J = 7.0 Hz), 3.58 (2H, d, J = 6.7 Hz), 3.14-3.07 (5H, m), 2.99 (2H, t, J = 7.6 Hz), 2.84 (2H, t, J = 7.6 Hz), 2.53 (3H, s), 2.27 (2H, s), 1.54 (3H, t, J = 7.0 Hz), 1.11 (6H, s).<br>MS (APCI) m/z :642[$(M+H)^+$]. |
| 39 | 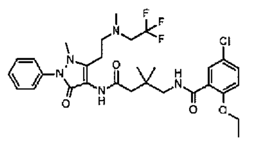 | 1 a | $^1$H-NMR ($CDCl_3$) δ: 9.23 (1H, s), 8.38 (1H, t, J = 6.7 Hz), 8.21 (1H, d, J = 2.4 Hz), 7.46-7.37 (5H, m), 7.28-7.24 (1H, m), 6.91 (1H, d, J = 8.5 Hz), 4.20 (2H, q, J = 6.7 Hz), 3.59 (2H, d, J = 6.7 Hz), 3.15-3.05 (5H, m), 2.99 (2H, t, J = 7.6 Hz), 2.84 (2H, t, J = 7.6 Hz), 2.54 (3H, s), 2.27 (2H, s), 1.52 (3H, t, J = 6.7 Hz), 1.12 (6H, s).<br>MS (APCI) m/z :624 [$(M+H)^+$]. |

(Example 40) 5-Chloro-N-[4-({5-[2-(dimethylamino)ethyl]-1-ethyl-3-oxo-2-phenyl-2,3-dihydro-1H-pyrazol-4-yl}amino)-2,2-dimethyl-4-oxobutyl]-2-ethoxybenzamide

[Formula 58]

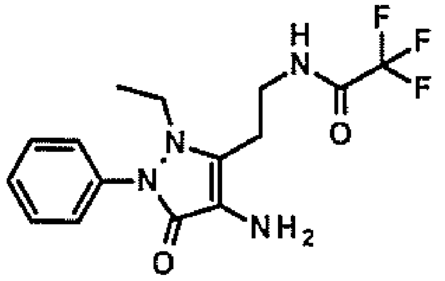

(Step 1) N-[2-(4-Amino-2-ethyl-5-oxo-1-phenyl-2,3-dihydro-1H-pyrazol-3-yl)ethyl]-2,2,2-trifluoroacetamide

The title compound was obtained as a solid by using ethyl iodide instead of methyl iodide in step 3 of Example 28 and subsequently performing the same reaction as in Example 28 up to step 5.
$^1$H-NMR ($CDCl_3$) $\delta$: 8.26 (1H, s), 7.48-7.41 (4H, m), 7.31-7.28 (1H, m), 3.63-3.62 (2H, m), 3.44 (2H, q, J = 7.0 Hz), 3.05 (2H, br s), 2.79 (2H, t, J = 6.7 Hz), 0.71 (3H, t, J = 7.0 Hz).

The following compounds of Examples 40 to 42 were subsequently synthesized in the same way as in steps 6 to 8 of Example 28 and are shown in Table 16.

[Table 16]

| Example No. | Structure | Intermediate used | Instrumental analysis data |
|---|---|---|---|
| 4 0 | 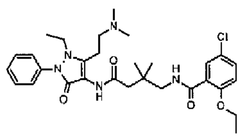 | 1 a | $^1$H-NMR ($CDCl_3$) $\delta$: 8.99 (1H, s), 8.37 (1H, t, J = 6.7 Hz), 8.22 (1H, d, J = 2.4 Hz), 7.46-7.36 (5H, m), 7.28-7.24 (1H, m), 6.91 (1H, d, J = 8.5 Hz), 4.20 (2H, q, J = 7.0 Hz), 3.64-3.58 (4H, m), 2.83-2.69 (4H, m), 2.33 (6H, s), 2.27 (2H, s), 1.52 (3H, t, J = 7.0 Hz), 1.12 (6H, s), 0.89 (3H, t, J = 7.0 Hz).<br>MS (APCI) m/z :570 [(M+H)$^-$]. |
| 4 1 | 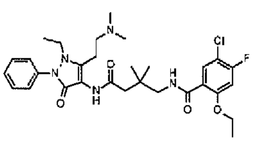 | 1 c | $^1$H-NMR ($CDCl_3$) $\delta$: 8.80 (1H, s), 8.31 (1H, d, J = 9.2 Hz), 8.23 (1H, t, J = 6.4 Hz), 7.48-7.41 (4H, m), 7.31-7.22 (1H, m), 6.78 (1H, d, J = 10.4 Hz), 4.18 (2H, q, J = 7.0 Hz), 3.64-3.56 (4H, m), 2.83-2.79 (2H, m), 2.71-2.68 (2H, m), 2.32 (6H, s), 2.26 (2H, s), 1.53 (3H, t, J = 7.0 Hz), 1.12 (6H, s), 0.88 (3H, t, J = 7.0 Hz).<br>MS (APCI) m/z :588 [(M+H)$^+$]. |
| 4 2 | 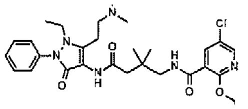 | 1 h | $^1$H-NMR ($CDCl_3$) $\delta$: 8.64 (1H, s), 8.51 (1H, d, J = 2.4 Hz), 8.41 (1H, t, J = 6.7 Hz), 8.19 (1H, d, J = 2.4 Hz), 7.45-7.40 (4H, m), 7.30-7.23 (1H, m), 4.54 (2H, q, J = 7.0 Hz), 3.64-3.57 (4H, m), 2.83-2.79 (2H, m), 2.71-2.67 (2H, m), 2.32 (6H, s), 2.27 (2H, s), 1.47 (3H, t, J = 7.0 Hz), 1.12 (6H, s), 0.88 (3H, t, J = 7.0 Hz).<br>MS (APCI) m/z :571 [(M+H)$^+$]. |

(Example 43) 5-Chloro-N-[4-({3-[2-(dimethylamino)ethyl]-5-oxo-1-phenyl-2-(trideuteriomethyl)pyrazol-4-yl}amino)-2,2-dimethyl-4-oxobutyl]-2-ethoxybenzamide

[Formula 59]

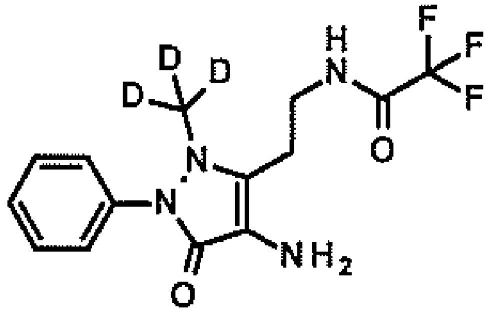

(Step 1) N-{2-[4-Amino-5-oxo-1-phenyl-2-(trideuteriomethyl)pyrazol-3-yl]ethyl}-2,2,2-trifluoroacetamide

The title compound was obtained as a solid by using methyl iodide-d3 instead of methyl iodide in step 3 of Example 28 and subsequently performing the same reaction as in Example 28 up to step 5.
$^1$H-NMR ($CDCl_3$) δ: 8.10 (1H, br s), 7.49-7.41 (4H, m), 7.31-7.27 (1H, m), 3.66 (2H, q, J= 6.3 Hz), 3.02 (2H, br s), 2.84 (2H, t, J = 6.4 Hz) .
MS m/z: 332 [(M+H)$^+$].

The following compounds of Examples 43 and 44 were subsequently synthesized in the same way as in steps 7 to 9 of Example 66 and are shown in Table 17.

[Table 17]

| Example No. | Structure | Intermediate used | Instrumental analysis data |
|---|---|---|---|
| 4 3 | 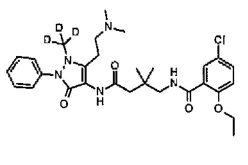 | 1 a | $^1$H-NMR ($CDCl_3$) δ: 9.12 (1H, s), 8.38 (1H, br t, J = 6.4 Hz), 8.21 (1H, d, J = 1.8 Hz), 7.48-7.35 (5H, m), 7.29-7.22 (1H, m), 6.91 (1H, d, J = 8.5 Hz), 4.20 (2H, q, J = 6.9 Hz), 3.59 (2H, d, J = 6.7 Hz), 2.88-2.80 (2H, m), 2.72-2.65 (2H, m), 2.33 (6H, s), 2.27 (2H, s), 1.52 (3H, t, J = 6.7 Hz), 1.13 (6H, s).<br>MS (ESI) m/z: 559 [(M+H)$^+$]. |
| 44 | 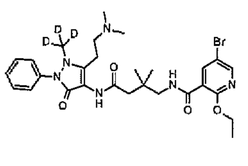 | 1 j | $^1$H-NMR ($CDCl_3$) δ: 8.81 (1H, s), 8.63 (1H, d, *J* = 3.1 Hz), 8.39 (1H, br t, *J* = 6.4 Hz), 8.28 (1H, d, *J* = 2.4 Hz), 7.48-7.40 (4H, m), 7.30-7.24 (1H, m), 4.54 (2H, q, *J* = 7.1 Hz), 3.59 (2H, d, *J* = 6.7 Hz), 2.88-2.81 (2H, m), 2.71-2.63 (2H, m), 2.32 (6H, s), 2.26 (2H, s), 1.48 (3H, t, *J* = 7.0 Hz), 1.13 (6H, s).<br>MS (ESI) m/z: 604 [(M+H)$^+$]. |

(Example 45) 5-Chloro-N-[4-({3-[2-(dimethylamino)ethyl]-1-(2-fluorophenyl)-2-methyl-5-oxopyrazol-4-yl}amino)-2,2-dimethyl-4-oxobutyl]-2-ethoxybenzamide

[Formula 60]

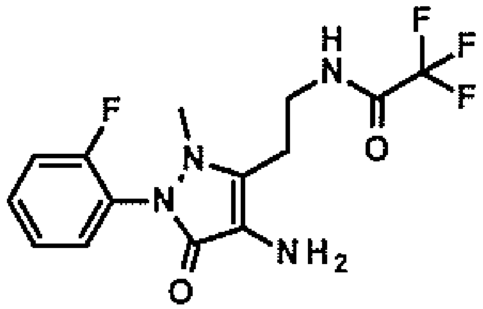

(Step 1) N-{2-[4-Amino-1-(2-fluorophenyl)-2-methyl-5-oxopyrazol-3-yl]ethyl}-2,2,2-trifluoroacetamide

The title compound was obtained as a solid by using 2-fluorophenylhydrazine hydrochloride instead of phenylhydrazine in step 2 of Example 28 and subsequently performing the same reaction as in Example 28 up to step 5. $^1$H-NMR (DMSO-$d_6$) δ: 9.53 (1H, t, J = 5.1 Hz), 7.47-7.43 (1H, m), 7.40-7.37 (1H, m), 7.33-7.29 (2H, m), 3.91 (2H, s), 3.43 (2H, dt, J = 5.1, 6.8 Hz), 3.32 (3H, s), 2.78 (2H, t, J = 6.8 Hz).

The following compounds of Examples 45 and 46 were subsequently synthesized in the same way as in steps 6 to 8 of Example 28 and are shown in Table 18.

[Table 18]

| Example No. | Structure | Intermediate used | Instrumental analysis data |
|---|---|---|---|
| 4 5 | 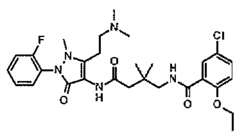 | 1 a | $^1$H-NMR ($CDCl_3$) δ: 8.89 (1H, br s), 8.37 (1H, t, J = 6.4 Hz), 8.20 (1H, d, J = 3.1 Hz), 7.39-7.35 (3H, m), 7.25-7.20 (2H, m), 6.91 (1H, d, J = 9.2 Hz), 4.20 (2H, q, J = 6.9 Hz), 3.58 (2H, d, J = 6.4 Hz), 3.10 (3H, s), 2.84 (2H, t, J = 7.6 Hz), 2.67 (2H, t, J = 7.6 Hz), 2.32 (6H, s), 2.28 (2H, s), 1.52 (3H, t, J = 7.0 Hz), 1.13 (6H, s).<br>MS (APCI) m/z : 574 [(M+H)+]. |
| 4 6 | 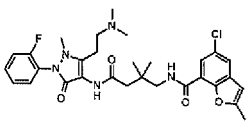 | 3 a | $^1$H-NMR ($CDCl_3$) δ: 8.40 (1H, br s), 7.98-7.95 (2H, br m), 7.56 (1H, d, J = 2.4 Hz), 7.41-7.35 (2H, m), 7.25-7.20 (2H, m), 6.43 (1H, br s), 3.65 (2H, d, J = 6.7 Hz), 3.11 (3H, s), 2.84 (2H, t, J = 7.6 Hz), 2.65 (2H, t, J = 7.6 Hz), 2.51 (3H, s), 2.34 (2H, s), 2.30 (6H, s), 1.18 (6H, s).<br>MS (APCI) m/z : 584 [$(M+H)^+$]. |

(Example 47) 5-Chloro-N-(4-{[1-(6-chloro-2-pyridyl)-3-(2-dimethylamino)ethyl]-2-methyl-5-oxopyrazol-4-yl}amino-2,2-dimethyl-4-oxobutyl)-2-ethoxybenzamide

[Formula 62]

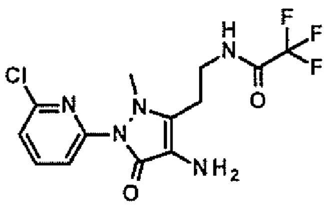

(Step 1) N-{2-[4-Amino-1-(6-chloro-2-pyridyl)-2-methyl-5-oxopyrazol-3-yl]ethyl}-2,2,2-trifluoroacetamide

The title compound was obtained as a solid by using (6-chloro-2-pyridyl)hydrazine instead of phenylhydrazine in step 2 of Example 28 and subsequently performing the same reaction as in Example 28 up to step 5.
$^1$H-NMR ($CDCl_3$) δ: 8.12 (1H, br s), 8.00 (1H, d, J = 8.0 Hz), 7.75 (1H, t, J = 8.0 Hz), 7.16 (1H, d, J = 8.0 Hz), 3.67 (2H, q, J = 6. Hz), 3.11 (3H, s), 2.99 (2H, br s), 2.86 (2H, t, J = 6.1 Hz) .
The following compound of Example 47 was subsequently synthesized in the same way as in steps 7 to 9 of Example 66 and is shown in Table 19.

[Table 19]

| Example No. | Structure | Intermediate used | Instrumental analysis data |
|---|---|---|---|
| 4 7 | 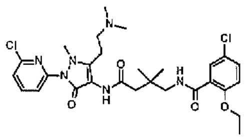 | 1 a | $^1$H-NMR ($CDCl_3$) δ: 9.30 (1H, s), 8.43 (1H, t, J = 6.3 Hz), 8.23 (1H, d, J = 2.9 Hz), 8.05 (1H, d, J = 8.0 Hz), 7.75 (1H, t, J = 8.0 Hz), 7.42 (1H, dd, J = 8.8, 2.9 Hz), 7.18 (1H, d, J = 8.0 Hz), 6.94 (1H, d, J = 8.8 Hz), 4.24 (2H, q, J = 7.0 Hz), 3.61 (2H, d, J = 6.8 Hz), 3.43 (3H, s), 2.89-2.85 (2H, m), 2.77-2.73 (2H, m), 2.36 (6H, s), 2.29 (2H, s), 1.55 (3H, t, J = 7.0 Hz), 1.17 (6H, s).<br>MS (APCI) m/z : 591 [(M+H)$^+$]. |

(Example 48) 5-Chloro-N- [4-({5- [(2R)-2-(dimethylamino)propyl]-1-methyl-3-oxo-2-phenyl-2,3-dihydro-1H-pyrazol-4-yl}amino)-2,2-dimethyl-4-oxobutyl]-2-ethoxybenzamide

[Formula 63]

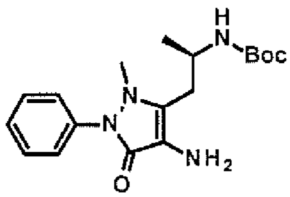

(Step 1) tert-Butyl-N-[(1R)-2-(4-amino-2-methyl-5-oxo-1-phenyl-2,3-dihydro-1H-pyrazol-3-yl)-1-methylethyl]carbamate

The title compound was obtained as a solid by using (3R)-3-(tert-butoxycarbonylamino)butanoic acid instead of 3-(tert-butoxycarbonylamino)propionic acid in step 1 of Example 10 and subsequently performing the same reaction as in Example 10 up to step 6.
$^1$H-NMR ($CDCl_3$) δ: 7.49-7.42 (4H, m), 7.27-7.23 (1H, m), 4.74 (1H, s), 4.00-3.93 (1H, m), 3.11 (2H, s), 2.89-2.82 (4H, m), 2.63 (1H, dd, J = 14.6, 7.3 Hz), 1.45 (9H, s), 1.23 (3H, d, J = 6.7 Hz).
MS (APCI) m/z :347[(M+H)$^+$].
The following compounds of Examples 48 to 50 were subsequently synthesized in the same way as in step 7 of Example 10 and steps 1 and 2 of Example 11 and are shown in Table 20.

[Table 20]

| Example No. | Structure | Intermediate used | Instrumental analysis data |
|---|---|---|---|
| 4 8 | 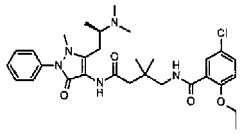 | 1 a | $^1$H-NMR ($CDCl_3$) $\delta$: 8.98 (1H, s), 8.37 (1H, t, $J$ = 6.4 Hz), 8.19 (1H, d, $J$ = 3.1 Hz), 7.46-7.36 (5H, m), 7.28-7.23 (1H, m), 6.91 (1H, d, $J$ = 9.2 Hz), 4.20 (2H, q, $J$ = 7.0 Hz), 3.62-3.52 (2H, m), 3.10 (3H, s), 3.06-2.97 (1H, m), 2.90 (1H, dd, $J$ = 14.0, 4.9 Hz), 2.66 (1H, dd, $J$ = 14.6, 9.2 Hz), 2.33 (6H, s), 2.27 (2H, s), 1.52 (3H, t, $J$ = 7.0 Hz), 1.15-1.10 (9H, m).<br>MS (APCI) m/z :570 $[(M+H)^-]$.<br>$[\alpha]_D^{20}$-1.76 (c = 1.08, MeOH). |
| 4 9 | 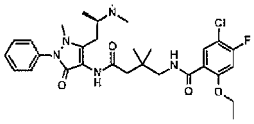 | 1 c | $^1$H-NMR ($CDCl_3$) $\delta$: 8.84 (1H, s), 8.28 (1H, d, J = 9.2 Hz), 8.22 (1H, t, J = 6.7 Hz), 7.46-7.41 (4H, m), 7.29-7.24 (1H, m), 6.78 (1H, d, J = 10.4 Hz), 4.18 (2H, q, J = 7.0 Hz), 3.56 (2H, d, J = 6.7 Hz), 3.10(3H, s), 3.05-3.00 (1H, m), 2.92 (1H, dd, J = 14.6, 4.6 Hz), 2.68 (1H, dd, J = 14.6, 9.2 Hz), 2.34 (6H, s), 2.27 (2H, s), 1.54 (3H, t, J = 7.0 Hz), 1.14-1.10 (9H, m).<br>MS (APCI) m/z :588 $[(M+H)^+]$.<br>$[\alpha]_D^{20}$-1.33 (c= 1.05, MeOH). |
| 5 0 | 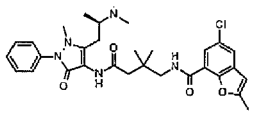 | 3 a | $^1$H-NMR ($CDCl_3$) $\delta$: 8.51 (1H, s), 7.96-7.92 (2H, m), 7.56 (1H, d, J = 1.8 Hz), 7.47-7.40 (4H, m), 7.29-7.25 (1H, m), 6.43 (1H, d, J = 1.2 Hz), 3.65 (2H, d, J = 6.7 Hz), 3.10 (3H, s), 3.03-2.97 (1H, m). 2.91 (1H, dd, J = 14.6, 4.9 Hz), 2.67 (1H, dd, J = 14.6, 9.2 Hz), 2.51 (3H, d, J = 1.2 Hz), 2.33-2.32 (8H, m), 1.18 (3H, s), 1.18 (3H, s), 1.09 (3H, d, J = 6.1 Hz).<br>MS (APCI) m/z :588 $[(M+H)^+]$.<br>$[\alpha]_D^{20}$ -2.05 (c = 1.02, MeOH). |

(Example 51) 5-Chloro-N-[4-({5-[(2S)-2-(dimethylamino)propyl]-1-methyl-3-oxo-2-phenyl-2,3-dihydro-1H-pyrazol-4-yl}amino)-2,2-dimethyl-4-oxobutyl]-2-ethoxybenzamide

[Formula 64]

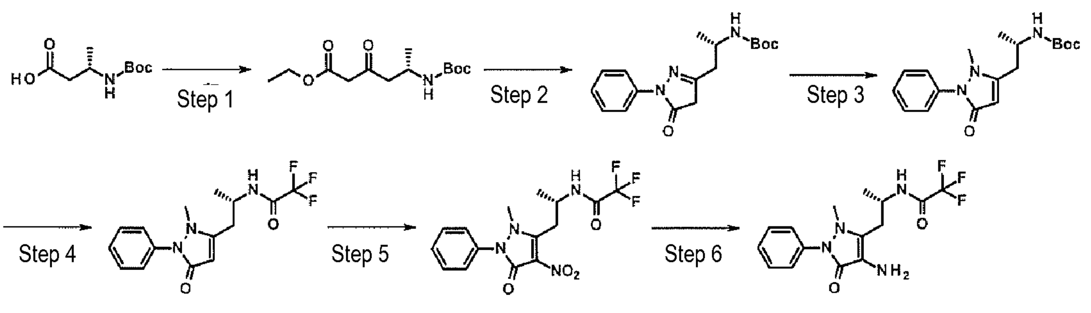


(Step 1) Ethyl (5S)-5-(tert-butoxycarbonylamino)-3-oxohexanoate

(3S)-3-(tert-Butoxycarbonylamino)butanoic acid (25.0 g, 115 mmol) was dissolved in tetrahydrofuran (575 mL). To the solution, 1,1'-carbonyldiimidazole (20.5 g, 127 mmol), potassium monoethyl malonate (21.5 g, 127 mmol), and magnesium chloride (12.1 g, 127 mmol) were added, and the mixture was stirred at room temperature for 16 hours. 1 N hydrochloric acid was added to the reaction solution, followed by extraction with ethyl acetate. Then, the organic layer was washed with 1 N hydrochloric acid, a saturated aqueous solution of sodium bicarbonate, and saturated saline and

dried over anhydrous sodium sulfate. After filtration, the filtrate was concentrated to obtain the title compound (32.5 g, yield: 98.0%) as an oil substance.
1H-NMR (CDCl3) δ: 4.23-4.16 (2H, m), 4.07-4.00 (1H, m), 3.50-3.41 (2H, m), 2.84-2.68 (2H, m), 1.43 (9H, s), 1.31-1.25 (3H, m), 1.23-1.20 (3H, m).

(Step 2) tert-Butyl N-[(1S)-1-methyl-2-(5-oxo-1-phenyl-4H-pyrazol-3-yl)ethyl]carbamate

To a solution of ethyl (5S)-5-(tert-butoxycarbonylamino)-3-oxohexanoate (13.1 g, 48.0 mmol) synthesized in step 1 in toluene (250 mL), phenylhydrazine (4.72 mL, 5.19 g, 48.0 mmol) was added, and the mixture was stirred for 3 hours under heating to reflux while generated water was removed. The reaction solution was allowed to cool to room temperature and then concentrated under reduced pressure, and the obtained residue was purified by silica gel column chromatography (Yamazen Corp., eluting solvent: ethyl acetate/methylene chloride = 9/91 to 30/70). After concentration under reduced pressure, the residue was solidified by the addition of diethyl ether and diisopropyl ether. The solid was collected by filtration and dried to obtain the title compound (10.7 g, yield: 70.3%) as a solid.
$^1$H-NMR ($CDCl_3$) δ: 7.84 (2H, d, J= 7.9 Hz), 7.39 (2H, t, J = 7.9 Hz), 7.18 (1H, t, J = 7.6 Hz), 4.54-4.52 (1H, m), 4.04 (1H, s), 3.73 (1H, d, J = 23.1 Hz), 3.39 (1H, d, J = 23.1 Hz), 2.68-2.56 (2H, m), 1.35 (9H, s), 1.28 (3H, d, J = 6.7 Hz).
MS (APCI) m/z : 318 [$(M+H)^+$].

(Step 3) tert-Butyl N-[(1S)-1-methyl-2-(2-methyl-5-oxo-1-phenyl-2,3-dihydro-1H-pyrazol-3-yl)ethyl]carbamate

To a solution of tert-butyl N-[(1S)-1-methyl-2-(5-oxo-1-phenyl-4H-pyrazol-3-yl)ethyl]carbamate (10.7 g, 33.7 mmol) synthesized in step 2 in methylene chloride (160 mL), N,N-diisopropylethylamine (8.60 mL, 6.54 g, 50.6 mmol) and methyl trifluoromethanesulfonate (5.34 mL, 7.75 g, 47.2 mmol) were added, and the mixture was stirred at room temperature for 14 hours. A saturated aqueous solution of sodium bicarbonate was added to the reaction solution, followed by extraction with methylene chloride three times. The organic layer was dried over anhydrous sodium sulfate. After filtration, the filtrate was concentrated under reduced pressure, and then, the obtained residue was purified by silica gel column chromatography (Yamazen Corp., eluting solvent: methanol/ethyl acetate = 0/100 to 12/88). After concentration under reduced pressure, the residue was solidified by the addition of diethyl ether. The organic solvent was distilled off under reduced pressure, and then, the solid was suspended in diisopropyl ether, collected by filtration, and dried to obtain the title compound (7.00 g, yield: 62.7%) as a solid.
$^1$H-NMR ($CDCl_3$) δ: 7.48-7.44 (2H, m), 7.39-7.36 (2H, m), 7.31-7.27 (1H, m), 5.44 (1H, s), 4.57-4.56 (1H, m), 3.98-3.92 (1H, m), 3.15 (3H, s), 2.89-2.84 (1H, m), 2.65-2.59 (1H, m), 1.45 (9H, s), 1.24 (3H, d, J = 7.3 Hz).
MS (APCI) m/z : 332 [$(M+H)^+$].

(Step 4) 2,2,2-Trifluoro-N-[(1S)-1-methyl-2-(2-methyl-5-oxo-1-phenyl-2,3-dihydro-1H-pyrazol-3-yl)ethyl]acetamide

To tert-butyl N-[(1S)-1-methyl-2-(2-methyl-5-oxo-1-phenyl-2,3-dihydro-1H-pyrazol-3-yl)ethyl]carbamate (6.00 g, 18.1 mmol) synthesized in step 3, ethanol (6.0 mL) and 4 N hydrochloric acid in 1,4-dioxane (60 mL) were added under ice cooling, and the mixture was stirred at room temperature for 1 hour and then concentrated under reduced pressure. To the residue, methylene chloride (90 mL) was added, then triethylamine (12.6 mL, 9.17 g, 90.5 mmol) was added under ice cooling, then trifluoroacetic anhydride (5.04 mL, 7.60 g, 36.2 mmol) was added, and the mixture was stirred for 4.5 hours under ice cooling. To the reaction solution, triethylamine (7.58 mL, 5.50 g, 54.3 mmol) and trifluoroacetic anhydride (5.04 mL, 7.60 g, 36.2 mmol) were further added, and the mixture was stirred for 1 hour under ice cooling. A saturated aqueous solution of sodium bicarbonate was added to the reaction solution, followed by extraction with methylene chloride three times. The organic layer was dried over anhydrous sodium sulfate. After filtration, the filtrate was concentrated under reduced pressure, and then, the obtained residue was purified by amino silica gel column chromatography (Yamazen Corp., eluting solvent: methanol/ethyl acetate = 1/99 to 15/85). After concentration under reduced pressure, the obtained solid was suspended in diisopropyl ether, collected by filtration, and dried to obtain the title compound (5.25 g, yield: 88.6%) as a solid.
$^1$H-NMR ($CDCl_3$) δ: 7.87 (1H, d, J= 7.9 Hz), 7.49-7.45 (2H, m), 7.36-7.30 (3H, m), 5.30 (1H, s), 4.24-4.13 (1H, m), 3.12 (3H, s), 2.85 (1H, dd, J = 14.9, 7.0 Hz), 2.68 (1H, dd, J= 15.2, 6.7 Hz), 1.33 (3H, d, J= 7.3 Hz).
MS (APCI) m/z : 328 [$(M+H)^+$].

(Step 5) 2,2,2-Trifluoro-N-[(1S)-1-methyl-2-(2-methyl-4-nitro-5-oxo-1-phenyl-2,3-dihydro-1H-pyrazol-3-yl)ethyl]acetamide

To a solution of 2,2,2-trifluoro-N-[(1S)-1-methyl-2-(2-methyl-5-oxo-1-phenyl-2,3-dihydro-1H-pyrazol-3-yl)ethyl]acetamide (5.25 g, 16.0 mmol) synthesized in step 4 in trifluoroacetic acid (80 mL), concentrated nitric acid (3.09

mL, 4.33 g, 48.1 mmol) was added, and the mixture was stirred at room temperature for 1 hour. Ice water was added to the reaction solution, followed by extraction with methylene chloride three times. The organic layer was washed with a saturated aqueous solution of sodium bicarbonate and then dried over anhydrous sodium sulfate. After filtration, the filtrate was concentrated under reduced pressure, and then, the obtained residue was solidified by the addition of ethyl acetate and diethyl ether. The solid was collected by filtration and dried to obtain the title compound (4.00 g, yield: 67.0%) as a solid.
$^1$H-NMR ($CDCl_3$) δ: 8.02 (1H, d, J= 8.5 Hz), 7.56-7.50 (3H, m), 7.30-7.27 (2H, m), 4.42-4.35 (1H, m), 3.48-3.36 (5H, m), 1.36 (3H, d, J = 6.7 Hz).
MS (APCI) m/z : 373 [(M+H)$^+$].

(Step 6) N-[(1S)-2-(4-Amino-2-methyl-5-oxo-1-phenyl-2,3-dihydro-1H-pyrazol-3-yl)-1-methylethyl]2,2,2-trifluoroacetamide

To a solution of 2,2,2-trifluoro-N-[(1S)-1-methyl-2-(2-methyl-4-nitro-5-oxo-1-phenyl-2,3-dihydro-1H-pyrazol-3-yl)ethyl]acetamide (4.00 g, 10.7 mmol) synthesized in step 5 in methanol (100 mL), a 10% palladium carbon catalyst (1.50 g) was added, and the mixture was stirred at room temperature for 3.75 hours under a hydrogen atmosphere. After purging with nitrogen, the reaction solution was filtered, and the filtrate was concentrated under reduced pressure. The obtained residue was solidified by the addition of ethyl acetate and diethyl ether. The solid was collected by filtration and dried to obtain the title compound (3.33 g, yield: 90.5%) as a solid.
$^1$H-NMR ($CDCl_3$) δ: 7.68 (1H, d, J= 6.1 Hz), 7.50-7.41 (4H, m), 7.31-7.27 (1H, m), 4.39-4.29 (1H, m), 3.09 (2H, s), 2.89-2.74 (5H, m), 1.33 (3H, d, J= 6.7 Hz).
MS (APCI) m/z :343 [(M+H)$^+$].
The following compounds of Examples 51 to 54 were subsequently synthesized in the same way as in steps 6 to 8 of Example 28 and are shown in Table 21.

[Table 21]

| Example No. | Structure | Intermediate used | Instrumental analysis data |
|---|---|---|---|
| 5 1 | 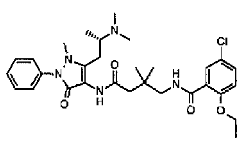 | 1 a | $^1$H-NMR ($CDCl_3$) δ: 8.97 (1H, s), 8.37 (1H, t, J = 6.4 Hz), 8.19 (1H, d, J = 3.1 Hz), 7.46-7.36 (5H, m), 7.29-7.22 (1H, m), 6.91 (1H, d, J = 9.2 Hz), 4.20 (2H, q, J = 7.0 Hz), 3.62-3.52 (2H, m), 3.10 (3H, s), 3.06-2.97 (1 H, m), 2.91 (1 H, dd, J = 14.6, 4.9 Hz), 2.66 (1H, dd, J = 14.6, 9.5 Hz), 2.33 (6H, s), 2.27 (2H, s), 1.52 (3H, t, J = 7.0 Hz), 1.15 (3H, s), 1.14 (3H, s), 1.11 (3H, d, J = 6.7 Hz). MS (APCI) m/z :570 [(M+H)$^+$]. $[\alpha]_D^{20}$ +1.69 (c = 1.00, MeOH). |
| 5 2 | 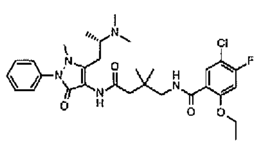 | 1 c | $^1$H-NMR ($CDCl_3$) δ: 8.83 (1H, s), 8.28 (1H, d, J = 8.5 Hz), 8.22 (1H, t, J = 6.7 Hz), 7.46-7.41 (4H, m), 7.28-7.24 (1H, m), 6.78 (1H, d, J = 10.3 Hz), 4.18 (2H, q, J = 7.0 Hz), 3.56 (2H, d, J = 6.7 Hz), 3.10 (3H, s), 3.05-2.96 (1H, m), 2.90 (1H, dd, J = 14.3, 5.2 Hz), 2.66 (1H, dd, J = 14.0, 9.1 Hz), 2.32 (6H, s), 2.26 (2H, s), 1.54 (3H, t, J = 7.0 Hz), 1.14 (3H, s), 1.13 (3H, s), 1.10 (3H, d, J = 6.7 Hz). MS (APCI) m/z :588 [(M+H)$^+$]. $[\alpha]_D^{20}$ +1.66 (c = 1.02, MeOH). |
| 5 3 | 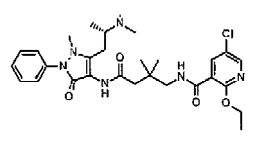 | 1 h | $^1$H-NMR ($CDCl_3$) δ: 8.64 (1H, s), 8.48 (1H, d, J = 3.0 Hz), 8.40 (1H, t, J = 6.7 Hz), 8.19 (1H, d, J = 3.0 Hz), 7.47-7.41 (4H, m), 7.29-7.25 (1H, m), 4.54 (2H, q, J = 7.0 Hz), 3.57 (2H, d, J = 6.7 Hz), 3.10 (3H, s), 3.04-2.89 (2H, m), 2.67 (1 H, dd, J = 14.6, 9.1 Hz), 2.32 (6H, s), 2.27 (2H, s), 1.48 (3H, t, J = 7.0 Hz), 1.14 (6H, s), 1.09 (3H, d, J = 6.7 Hz). MS (APCI) m/z :571 [(M+H)$^+$]. $[\alpha]_D^{20}$ -+2.26 (c = 1.02, MeOH). |

(continued)

| Example No. | Structure | Intermediate used | Instrumental analysis data |
|---|---|---|---|
| 5 4 | 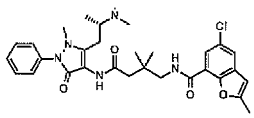 | 3 a | $^1$H-NMR ($CDCl_3$) δ: 8.50 (1H, s), 7.96-7.93 (2H, m), 7.55 (1H, d, J = 1.8 Hz), 7.47-7.40 (4H, m), 7.29-7.25 (1H, m), 6.43 (1H, s), 3.65 (2H, d, J = 6.7 Hz), 3.10 (3H, s), 3.04-2.96 (1H, m), 2.90 (1H, dd, J = 14.3, 4.9 Hz), 2.67 (1H, dd, J = 14.3, 8.9 Hz), 2.51 (3H, s), 2.33-2.31 (8H, m), 1.18 (3H, s), 1.18 (3H, s), 1.09 (3H, d, J = 6.7 Hz).<br>MS (ESI) m/z :580 [(M+H)$^+$].<br>$[\alpha]_D^{20}$ +2.27 (c = 1.02, MeOH). |

(Example 55) N-(4-{[5-(2-Amino-2-methylpropyl)-1-methyl-3-oxo-2-phenyl-2,3-dihydro-1H-pyrazol-4-yl]amino}-2,2-dimethyl-4-oxobutyl)-5-chloro-2-ethoxybenzamide

[Formula 65]

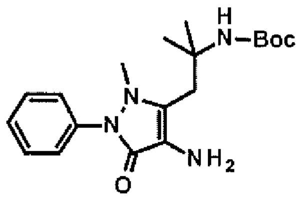

(Step 1) tert-Butyl N-[2-(4-amino-2-methyl-5-oxo-1-phenyl-2,3-dihydro-1H-pyrazol-3-yl)-1,1-dimethylethyl]carbamate

The title compound was obtained as a solid by using ethyl 3-(tert-butoxycarbonylamino)-3-methylbutanoate synthesized in step 1 of Example 59 instead of 3-(tert-butoxycarbonylamino)propionic acid in step 1 of Example 10 and subsequently performing the same reaction as in Example 10 up to step 6.
$^1$H-NMR ($CDCl_3$) δ: 7.51 (2H, d, J = 7.3 Hz), 7.44 (2H, t, J = 7.3 Hz), 7.26-7.22 (1H, m), 4.66 (1H, s), 3.22 (2H, s), 2.97 (2H, s), 2.80 (3H, s), 1.45 (9H, s), 1.43 (6H, s).
MS (ESI) m/z : 361 [(M+H)$^+$].

The following compounds of Examples 55 to 58 were subsequently synthesized in the same way as in step 7 of Example 10 and steps 1 and 2 of Example 11 and are shown in Table 22.

[Table 22]

| Example No. | Structure | Intermediate used | Instrumental analysis data |
|---|---|---|---|
| 5 5 | 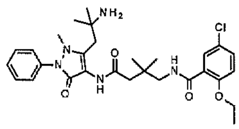 | 1 a | $^1$H-NMR ($CDCl_3$) δ: 9.07 (1H, s), 8.39 (1H, t, J = 6.6 Hz), 8.15 (1H, d, J = 3.1 Hz), 7.47-7.43 (4H, m), 7.39-7.36 (1H, m), 7.29-7.25 (1H, m), 6.91 (1H, d, J = 9.0 Hz), 4.20 (2H, q, J = 7.0 Hz), 3.57 (2H, d, J = 6.6 Hz), 3.12 (3H, s), 2.81 (2H, s), 2.27 (2H, s), 1.53 (3H, t, J = 7.0 Hz), 1.29 (6H, s), 1.16 (6H, s).<br>MS (ESI) m/z : 556 [(M+H)$^+$]. |

(continued)

| Example No. | Structure | Intermediate used | Instrumental analysis data |
|---|---|---|---|
| 5 6 | 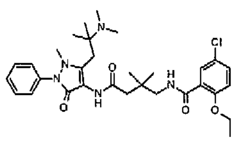 | 1 a | $^1$H-NMR ($CDCl_3$) δ: 8.94 (1H, s), 8.36 (1H, t, J = 6.5 Hz), 8.16 (1H, d, J = 3.1 Hz), 7.46-7.41 (4H, m), 7.39-7.35 (1H, m), 7.28-7.24 (1H, m), 6.90 (1H, d, J = 8.6 Hz), 4.20 (2H, q, J = 7.0 Hz), 3.57 (2H, d, J = 6.5 Hz), 3.09 (3H, s), 2.85 (2H, s), 2.36 (6H, s), 2.27 (2H, s), 1.52 (3H, t, J = 7.0 Hz), 1.20 (6H, s), 1.16 (6H, s).<br>MS (ESI) m/z : 584 [(M+H)$^+$]. |
| 5 7 | 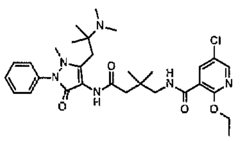 | 1 h | $^1$H-NMR ($CDCl_3$) δ: 8.60 (1H, s), 8.45 (1H, d, J = 2.9 Hz), 8.39 (1H, br s), 8.18 (1H, d, J = 2.9 Hz), 7.46-7.42 (4H, m), 7.28-7.28 (1H, m), 4.54 (2H, q, J = 7.0 Hz), 3.57 (2H, d, J = 6.8 Hz), 3.09 (3H, s), 2.86 (2H, s), 2.35 (6H, s), 2.27 (2H, s), 1.48 (3H, t, J = 7.0 Hz), 1.18 (6H, s), 1.16 (6H, s).<br>MS (ESI) m/z : 585 [(M+H)$^+$]. |
| 5 8 | 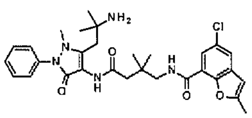 | 3 a | $^1$H-NMR ($CDCl_3$) δ: 8.57 (1H, s), 7.94-7.94 (2H, m), 7.56 (1H, d, J = 2.0 Hz), 7.48-7.43 (4H, m), 7.29-7.28 (1H, m), 6.43 (1H, d, J = 1.2 Hz), 3.66 (2H, d, J = 7.0 Hz), 3.12 (3H, s), 2.81 (2H, s), 2.53 (3H, d, J = 1.2 Hz), 2.33 (2H, s), 1.28 (6H, s), 1.21 (6H, s).<br>MS (ESI) m/z : 566 [(M+H)$^+$]. |

(Example 59) 5-Chloro-N-[2,2-dimethyl-4-({1-methyl-5-[2-methyl-2-(methylamino)propyl]-3-oxo-2-phenyl-2,3-dihydro-1H-pyrazol-4-yl}amino)-4-oxobutyl]-2-ethoxybenzamide

[Formula 66]

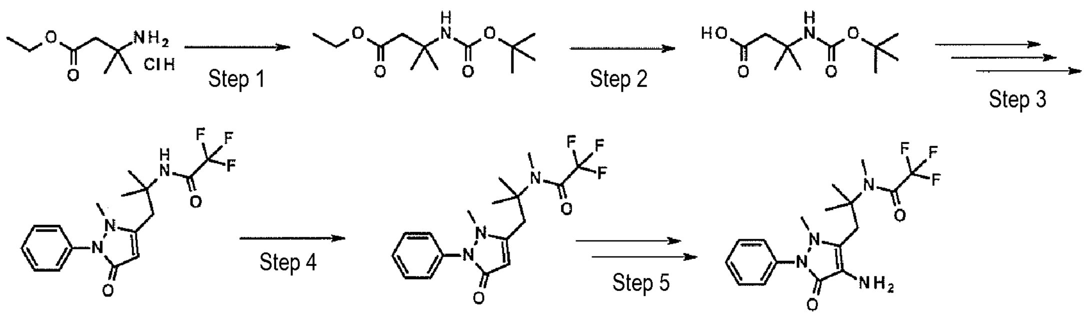


(Step 1) Ethyl 3-(tert-butoxycarbonylamino)-3-methylbutanoate

Ethyl 3-amino-3-methylbutanoate hydrochloride (10.5 g, 57.5 mmol) was dissolved in methylene chloride (100 mL). To the solution, triethylamine (8.19 mL, 5.95 g, 58.8 mmol) and di-tert-butyl dicarbonate (9.41 g, 43.1 mmol) were added, and the mixture was stirred at room temperature for 71.7 hours. Water was added to the reaction mixture, followed by extraction with methylene chloride. The organic layer was washed with water and saturated saline and dried over anhydrous sodium sulfate. The solvent was distilled off under reduced pressure, and the obtained residue was purified by silica gel column chromatography (Biotage Japan Ltd., eluting solvent: hexane/ethyl acetate = 98/2 to 50/50) to obtain the title compound (10.4 g, yield: 73.7%) as an oil substance. $^1$H-NMR ($CDCl_3$) δ: 4.91 (1H, br s), 4.14 (2H, q, J = 7.1 Hz), 2.67 (2H, s), 1.43 (9H, s), 1.38 (6H, s), 1.26 (3H, t, J = 7.1 Hz).

(Step 2) 3-(tert-Butoxycarbonylamino)-3-methylbutanoic acid

Ethyl 3-(tert-butoxycarbonylamino)-3-methylbutanoate (10.4 g, 42.5 mmol) synthesized in step 1 was dissolved in tetrahydrofuran (200 mL). To the solution, a 1 N aqueous sodium hydroxide solution (100 mL) was added, and the mixture was stirred at room temperature for 16 hours. Methanol (50 mL) was added thereto, and the mixture was further stirred for 3 hours. The organic solvent was distilled off under reduced pressure. To the obtained aqueous solution, 1 N hydrochloric acid (120 mL) was added, followed by extraction with methylene chloride. The organic layer was washed with saturated saline and dried over anhydrous sodium sulfate. The solvent was distilled off under reduced pressure to obtain the title compound (10.2 g, yield: 99.0%) as an oil substance.
$^1$H-NMR ($CDCl_3$) δ: 4.97 (1H, br s), 2.73 (2H, br s), 1.45 (9H, s), 1.40 (6H, s).
MS (APCI) m/z : 216 [$(M-H)^-$].

(Step 3) N-[1,1-Dimethyl-2-(2-methyl-5-oxo-1-phenyl-2,3-dihydro-1H-pyrazol-3-yl)ethyl]2,2,2-trifluoroacetamide

The title compound was obtained as a solid by using 3-(tert-butoxycarbonylamino)-3-methylbutanoic acid synthesized in step 2 instead of (3S)-3-(tert-butoxycarbonylamino)butanoic acid in step 1 of Example 51 and subsequently performing the same reaction as in Example 51 up to step 4.
$^1$H-NMR ($CDCl_3$) δ: 7.47-7.43 (2H, m), 7.33-7.28 (3H, m), 7.01 (1H, br s), 5.41 (1H, s), 3.06 (2H, s), 3.04 (3H, s), 1.48 (6H, s).
MS (APCI) m/z : 342 [$(M+H)^+$].

(Step 4) N-[1,1-Dimethyl-2-(2-methyl-5-oxo-1-phenyl-2,3-dihydro-1H-pyrazol-3-yl)ethyl]2,2,2-trifluoro-N-methylacetamide

N-[1,1-Dimethyl-2-(2-methyl-5-oxo-1-phenyl-2,3-dihydro-1H-pyrazol-3-yl)ethyl]2,2,2-trifluoroacetamide (4.24 g, 12.4 mmol) synthesized in step 3 was dissolved in acetonitrile (120 mL). To the solution, potassium carbonate (2.58 g, 18.6 mmol), tetra-n-butyl ammonium bromide (0.40 g, 1.24 mmol), and methyl iodide (7.73 mL, 17.6 g, 124 mmol) were added, and the mixture was stirred at 80°C for 18 hours. After cooling to room temperature, methyl iodide (3.87 mL, 8.82 g, 62.1 mmol) was further added thereto, and the mixture was stirred again at 80°C for 8 hours. After cooling to room temperature, water was added to the reaction mixture, followed by extraction with ethyl acetate. The organic layer was washed with saturated saline and dried over anhydrous sodium sulfate. The solvent was distilled off under reduced pressure, and the obtained residue was purified by silica gel column chromatography (Biotage Japan Ltd., eluting solvent: hexane/10% methanol-ethyl acetate = 50/50 to 40/60) to obtain the title compound (2.56 g, yield: 58.0%) as a solid.
$^1$H-NMR ($CDCl_3$) δ: 7.48-7.44 (2H, m), 7.35-7.28 (3H, m), 5.50 (1H, s), 3.25 (2H, s), 3.05 (3H, s), 3.02 (3H, br s), 1.60 (6H, s).
MS (APCI) m/z : 356 [$(M+H)^+$].

(Step 5) N-[2-(4-Amino-2-methyl-5-oxo-1-phenyl-2,3-dihydro-1H-pyrazol-3-yl)-1,1-dimethylethyl]-2,2,2-trifluoro-N-methylacetamide

The title compound was obtained as a solid by using N-[1,1-dimethyl-2-(2-methyl-5-oxo-1-phenyl-2,3-dihydro-1H-pyrazol-3-yl)ethyl]2,2,2-trifluoro-N-methylacetamide synthesized in step 4 instead of 2,2,2-trifluoro-N-[2-(2-methyl-5-oxo-1-phenyl-2,3-dihydro-1H-pyrazol-3-yl)propyl]acetamide in step 4 of Example 66 and subsequently performing the same reaction as in Example 66 up to step 5.
$^1$H-NMR ($CDCl_3$) δ: 7.48-7.42 (4H, m), 7.27-7.23 (1H, m), 3.19 (4H, br s), 3.00 (3H, s), 2.77 (3H, s), 1.61 (6H, s). MS (APCI) m/z : 371 [$(M+H)^+$].
The following compounds of Examples 59 to 61 were subsequently synthesized in the same way as in steps 6 and 7 of Example 28 and are shown in Table 23.

[Table 23]

| Example No. | Structure | Intermediate used | Instrumental analysis data |
|---|---|---|---|
| 5 9 | 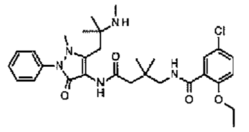 | 1 a | $^1$H-NMR ($CDCl_3$) δ: 9.01 (1H, br s), 8.36 (1H, t, J = 6.7 Hz), 8.16 (1 H, d, J = 2.7 Hz), 7.46-7.42 (4H, m), 7.37 (1 H, dd, J = 8.8, 2.7 Hz), 7.28-7.25 (1H, m), 6.90 (1H, d, J = 8.8 Hz), 4.20 (2H, q, J = 7.1 Hz), 3.58 (2H, d, J = 6.7 Hz), 3.10 (3H, s), 2.82 (2H, s), 2.39 (3H, s), 2.26 (2H, s), 1.52 (4H, t, J = 7.0 Hz), 1.22 (6H, s), 1.16 (6H, s). MS (APCI) m/z : 570 [(M+H)$^+$]. |
| 6 0 | 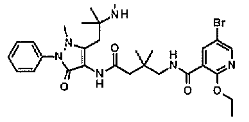 | 1 j | $^1$H-NMR ($CDCl_3$) δ: 8.69 (1H, br s), 8.58 (1H, d, J = 2.4 Hz), 8.38 (1H, t, J = 6.4 Hz), 8.28 (1H, d, J = 2.4 Hz), 7.47-7.41 (4H, m), 7.28-7.25 (1H, m), 4.54 (2H, q, J = 7.1 Hz), 3.58 (2H, d, J = 6.7 Hz), 3.10 (3H, s), 2.84 (2H, s), 2.39 (3H, s), 2.26 (2H, s), 1.48 (3H, t, J = 7.0 Hz), 1.20 (6H, s), 1.16 (6H, s). MS (APCI) m/z : 615 [(M+H)$^+$]. |
| 6 1 | 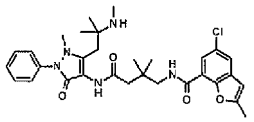 | 3 a | $^1$H-NMR ($CDCl_3$) δ: 8.54 (1H, br s), 7.95-7.92 (2H, m), 7.56 (1H, d, J = 2.4 Hz), 7.47-7.40 (4H, m), 7.29-7.25 (1H, m), 6.43 (1H, s), 3.66 (2H, d, J = 6.7 Hz), 3.10 (3H, s), 2.82 (2H, s), 2.52 (3H, s), 2.39 (3H, s), 2.33 (2H, s), 1.20 (12H, 2s). MS (APCI) m/z : 580 [(M+H)$^+$]. |

(Example 62) 5-Chloro-N-[4-({5-[2-(dimethylamino)ethyl]-1-methyl-3-oxo-2-phenylpyrazolo-4-yl}amino)-2,2-bis(fluoromethyl)-4-oxobutyl]-2-ethoxybenzamide

[Formula 67]

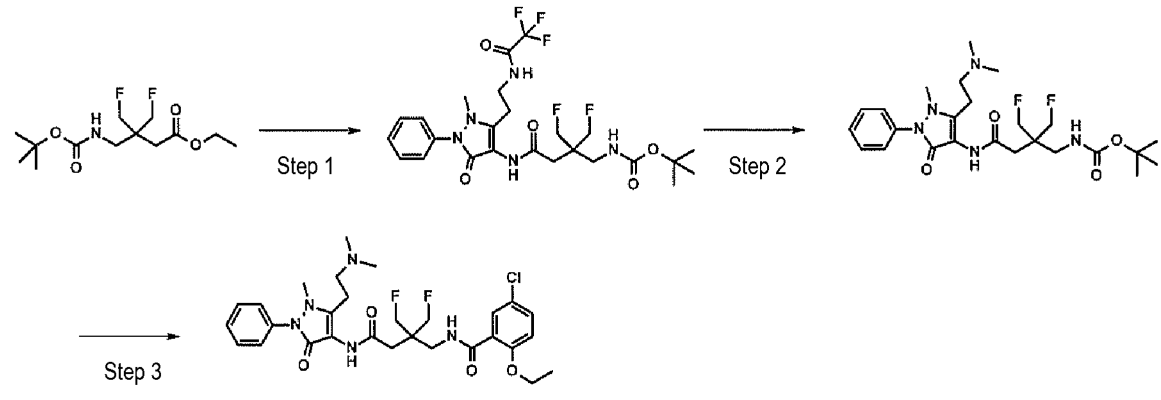


(Step 1) tert-Butyl N-{2,2-bis(fluoromethyl)-4-[(1-methyl-3-oxo-2-phenyl-5-{2-[(2,2,2-trifluoroacetyl)amino]ethyl}pyrazolo-4-yl)amino]-4-oxobutyl}carbamate

Ethyl 3-[(tert-butoxycarbonylamino)methyl]-4-fluoro-3-(fluoromethyl)butanoate (0.295 g, 1.00 mmol) prepared by the method described in J. Med. Chem. 2011, 54, 7030-7054 was dissolved in 1,4-dioxane (8.0 mL). To the solution, a 1 N aqueous sodium hydroxide solution (2.0 mL) was added, and the mixture was stirred for 12 hours. The reaction solution was neutralized by the addition of 1 N hydrochloric acid (2.0 mL), followed by extraction with ethyl acetate. The organic layer was washed with saturated saline and then dried over anhydrous magnesium sulfate. After filtration, the filtrate was concentrated, and the obtained crude carboxylic acid was dissolved in acetonitrile (5.0 mL). To the solution, N-[2-(4-amino-2-methyl-5-oxo-1-phenylpyrazolo-3-yl)ethyl]-2,2,2-trifluoroacetamide (0.328 g, 1.00 mmol) synthesized

in step 5 of Example 28 and 4-(4,6-dimethoxy-1,3,5-triazin-2-yl)-4-methylmorpholine n-hydrate (DMT-MM) (0.651 g, 2.00 mmol) were added, and the mixture was stirred for 18 hours. The reaction solution was neutralized by the addition of 1 N hydrochloric acid (2 mL), followed by extraction with ethyl acetate. The organic layer was washed with saturated saline and then dried over anhydrous magnesium sulfate. After filtration, the filtrate was concentrated, and the obtained crude product was purified by silica gel column chromatography (Shoko Scientific Co., Ltd., eluting solvent: hexane/ethyl acetate = 50/50 to 10/90) to obtain the title compound (0.529 g, yield: 92.0%) as an oil substance.
$^1$H-NMR ($CDCl_3$) δ: 8.74 (1H, br s), 8.48 (1H, br s), 7.52-7.46 (2H, m), 7.39-7.31 (3H, m), 5.02 (1H, br s), 4.55-4.49 (2H, m), 4.43-4.37 (2H, m), 3.75-3.69 (2H, m), 3.47-3.43 (2H, m), 3.12 (3H, s), 3.01-2.95 (2H, m), 2.42 (2H, s), 1.44 (9H, s).

(Step 2) tert-Butyl N-[4-({5-[2-(dimethylamino)ethyl]-1-methyl-3-oxo-2-phenylpyrazolo-4-yl}amino)-2,2-bis(fluoromethyl)-4-oxobutyl]carbamate

tert-Butyl N-{2,2-bis(fluoromethyl)-4-[(1-methyl-3-oxo-2-phenyl-5-{2-[(2,2,2-trifluoroacetyl)amino]ethyl}pyrazolo-4-yl)amino]-4-oxobutyl}carbamate (0.529 g, 0.910 mmol) synthesized in step 1 was dissolved in 1,4-dioxane (10 mL). To the solution, a 1 N aqueous sodium hydroxide solution (4.0 mL) was added, and the mixture was stirred for 12 hours. The reaction solution was neutralized by the addition of 1 N hydrochloric acid (4.0 mL), followed by extraction with ethyl acetate. The organic layer was washed with saturated saline and then dried over anhydrous magnesium sulfate. After filtration, the filtrate was concentrated, and the obtained crude amine form was dissolved in methanol (6.0 mL). To the solution, 37% formaldehyde (0.388 mL, 4.55 mmol) and sodium triacetoxyborohydride (0.960 g, 4.55 mmol) were added, and the mixture was stirred for 3 hours. A saturated aqueous solution of sodium bicarbonate was added to the reaction solution, followed by extraction with ethyl acetate. The organic layer was washed with saturated saline and then dried over anhydrous magnesium sulfate. After filtration, the filtrate was concentrated, and the obtained crude product was purified by silica gel column chromatography (Shoko Scientific Co., Ltd., eluting solvent: methanol/ethyl acetate = 10/90 to 30/70) to obtain the title compound (0.255 g, yield: 55.0%) as an oil substance.
$^1$H-NMR ($CDCl_3$) δ: 8.18 (1H, br s), 7.48-7.39 (4H, m), 7.32-7.27 (1H, m), 5.31-5.26 (1H, m), 4.59-4.52 (2H, m), 4.47-4.42 (2H, m), 3.43-3.37 (2H, m), 3.11 (3H, s), 2.83-2.78 (2H, m), 2.67-2.62 (2H, m), 2.41 (2H, s), 2.32 (6H, s), 1.44 (9H, s).

(Step 3) 5-Chloro-N-[4-({5-[2-(dimethylamino)ethyl]-1-methyl-3-oxo-2-phenylpyrazolo-4-yl}amino)-2,2-bis(fluoromethyl)-4-oxobutyl]-2-ethoxybenzamide

tert-Butyl N-[4-({5-[2-(dimethylamino)ethyl]-1-methyl-3-oxo-2-phenylpyrazolo-4-yl}amino)-2,2-bis(fluoromethyl)-4-oxobutyl]carbamate (0.250 g, 0.490 mmol) synthesized in step 2 was dissolved in a solution of 4 N hydrochloric acid in 1,4-dioxane (5.0 mL), and the solution was stirred at room temperature for 30 minutes. The reaction solution was concentrated, and the obtained crude amine form was suspended in methylene chloride (5.0 mL). To the suspension, triethylamine (0.342 mL, 0.298 g, 2.95 mmol) and 5-chloro-2-ethoxybenzoyl chloride (0.139 g, 0.640 mmol) were added, and the mixture was stirred for 1 hour. Water was added to the reaction solution, followed by extraction with methylene chloride. The organic layer was washed with saturated saline and then dried over anhydrous magnesium sulfate. After filtration, the filtrate was concentrated, and the obtained crude product was purified by silica gel column chromatography (Shoko Scientific Co., Ltd., eluting solvent: methanol/ethyl acetate = 1/99 to 10/90) to obtain the title compound (0.174 g, yield: 60.0%) as a solid.
$^1$H-NMR ($CDCl_3$) δ: 9.04 (1H, s), 8.45-8.40 (1H, m), 8.18 (1H, s), 7.48-7.37 (5H, m), 6.91 (1H, d, J= 9.0 Hz), 4.67-4.57 (2H, m), 4.54-4.46 (2H, m), 4.23-4.16 (2H, m), 3.87-3.83 (2H, m), 3.11 (3H, s), 2.85-2.80 (2H, m), 2.70-2.64 (2H, m), 2.39 (2H, s), 2.33 (6H, s), 1.52 (4H, t, J = 6.8 Hz).
MS (ESI) m/z : 592 [$(M+H)^+$].

(Example 63) 5-Chloro-N-[4-({5-[3-(dimethylamino)propyl]-1-methyl-3-oxo-2-phenyl-2,3-dihydro-1H-pyrazol-4-yl}amino)-2,2-dimethyl-4-oxobutyl]-2-ethoxybenzamide

[Formula 68]

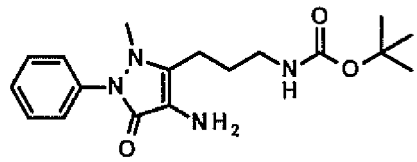

(Step 1) tert-Butyl N-[3-(4-amino-2-methyl-5-oxo-1-phenyl-2,3-dihydro-1H-pyrazol-3-yl)propyl]carbamate

The title compound was obtained as a solid by using 4-(tert-butoxycarbonylamino)butanoic acid instead of 3-(tert-butoxycarbonylamino)propanoic acid in step 1 of Example 10 and subsequently performing the same reaction as in Example 10 up to step 6.
$^1$H-NMR ($CDCl_3$) $\delta$: 7.49-7.41 (4H, m), 7.27-7.21 (1H, m), 4.93 (1H, br s), 3.25-3.17 (2H, m), 3.07 (2H, br s), 2.83 (3H, s), 2.59 (2H, t, J = 7.3 Hz), 1.89-1.80 (2H, m), 1.45 (9H, s).
The following compounds of Examples 63 to 65 were subsequently synthesized in the same way as in step 7 of Example 10 and steps 1 and 2 of Example 11 and are shown in Table 24.

[Table 24]

| Example No. | Structure | Intermediate used | Instrumental analysis data |
|---|---|---|---|
| 6 3 | 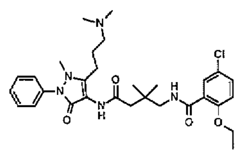 | 1 a | $^1$H-NMR ($CDCl_3$) $\delta$: 9.08 (1H, br s), 8.42-8.35 (1H, m), 8.19 (1H, d, J = 2.4 Hz), 7.47-7.36 (5H, m), 7.29-7.22 (1H, m), 6.91 (1H, d, J = 8.5 Hz), 4.20 (2H, q, J = 6.7 Hz), 3.58 (2H, d, J = 6.7 Hz), 3.10 (3H, s), 2.70 (2H, t, J = 7.3 Hz), 2.36 (2H, t, J = 7.3 Hz), 2.26 (2H, s), 2.25 (6H, s), 1.93-1.84 (2H, m), 1.53 (3H, t, J = 6.7 Hz), 1.14 (6H, s).<br>MS (ESI+APCI) m/z : 570 [(M+H)$^+$]. |
| 6 4 | 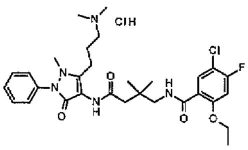 | 1 c | $^1$H-NMR ($CDCl_3$) $\delta$: 12.08 (1H, br s), 8.24-8.17 (2H, m), 7.59-7.52 (2H, m), 7.50-7.43 (3H, m), 6.80 (1H, d, J = 10.4 Hz), 4.19 (2H, q, J = 6.7 Hz), 3.58 (2H, d, J = 6.1 Hz), 3.41 (3H, s), 3.33-3.26 (2H, m), 3.09-3.02 (2H, m), 2.86 (3H, s), 2.85 (3H, s), 2.53-2.50 (2H, m), 2.47-2.38 (2H, m), 1.55 (3H, t, J = 6.7 Hz), 1.14 (6H, s).<br>MS (APCI) m/z : 588 [(M+H)$^+$]. |
| 6 5 | 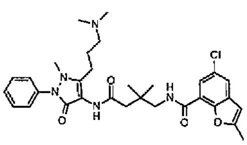 | 3 a | $^1$H-NMR ($CDCl_3$) $\delta$: 8.62 (1H, s), 8.02-7.97 (1H, m), 7.95 (1H, d, J = 2.4 Hz), 7.55 (1H, d, J = 2.4 Hz), 7.47-7.38 (4H, m), 7.31-7.23 (1H, m), 6.42 (1H, s), 3.65 (2H, d, J = 6.7 Hz), 3.09 (3H, s), 2.70 (2H, t, J = 7.3 Hz), 2.52 (3H, s), 2.36-2.31 (4H, m), 2.24 (6H, s), 1.92-1.82 (2H, m), 1.18 (6H, s).<br>MS (ESI+APCI) m/z : 580 [(M+H)$^+$]. |

(Example 66) 5-Chloro-N-[4-({5-[2-(dimethylamino)-1-methylethyl]-1-methyl-3-oxo-2-phenyl-2,3-dihydro-1H-pyrazol-4-yl}amino)-2,2-dimethyl-4-oxobutyl]-2-ethoxybenzamide

[Formula 69]

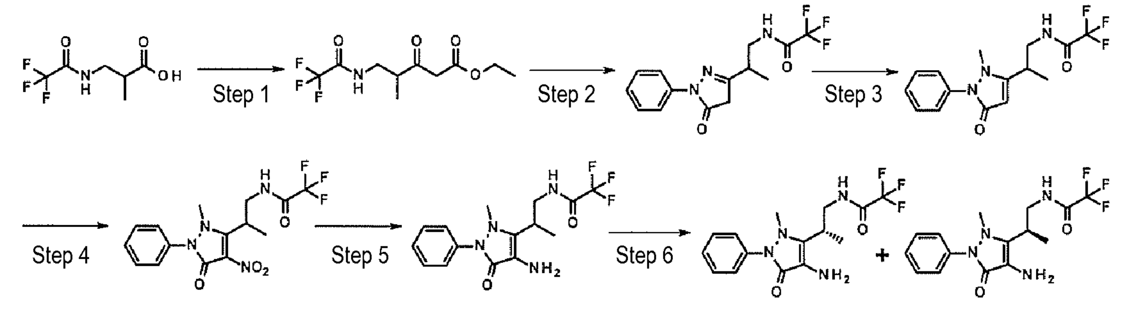


66a 66b 66c

(Step 1) Ethyl 4-methyl-3-oxo-5-[(2,2,2-trifluoroacetyl)amino]pentanoate

To a solution of N-trifluoroacetyl-β-aminoisobutanoic acid (5.00 g, 25.1 mmol) synthesized according to the method described in European Journal of Organic Chemistry, 2012, 29, 5774-5788 in tetrahydrofuran (125 mL), 1,1'-carbonyldiimidazole (4.48 g, 27.6 mmol) was added under ice cooling, and the mixture was stirred at room temperature for 1 hour. In another reaction vessel, potassium monoethyl malonate (4.70 g, 27.6 mmol) and magnesium chloride (2.87 g, 30.1 mmol) were suspended in tetrahydrofuran (125 mL). To the suspension, triethylamine (4.20 mL, 3.05 g, 30.1 mmol) was added under ice cooling, and the mixture was stirred at room temperature for 45 minutes. Then, the reaction solution described above was added dropwise thereto over 1 hour. The reaction solution was stirred at room temperature for 16 hours, then water was added thereto, and the mixture was rendered acidic with concentrated hydrochloric acid. After extraction with ethyl acetate three times, the organic layer was washed with a saturated aqueous solution of sodium bicarbonate and saturated saline in this order and then dried over anhydrous sodium sulfate. After filtration, the filtrate was concentrated under reduced pressure, and then, the obtained residue was purified by silica gel column chromatography (Yamazen Corp., eluting solvent: ethyl acetate/hexane = 17/83 to 38/62) to obtain the title compound (3.94 g, yield: 58.3%) as an oil substance.
$^1$H-NMR ($CDCl_3$) δ: 6.99 (1H, s), 4.24-4.18 (2H, m), 3.60-3.44 (4H, m), 3.07-2.98 (1H, m), 1.30-1.18 (6H, m).
MS (APCI) m/z :270 [$(M+H)^+$].

(Step 2) 2,2,2-Trifluoro-N-[2-(5-oxo-1-phenyl-4H-pyrazol-3-yl)propyl]acetamide

To a solution of ethyl 4-methyl-3-oxo-5-[(2,2,2-trifluoroacetyl)amino]pentanoate (3.94 g, 14.6 mmol) synthesized in step 1 in toluene (70 mL), phenylhydrazine (1.44 mL, 1.58 g, 14.6 mmol) was added, and the mixture was stirred for 4 hours under heating to reflux while generated water was removed. The reaction solution was allowed to cool to room temperature and then concentrated under reduced pressure, and the obtained residue was purified by silica gel column chromatography (Yamazen Corp., eluting solvent: ethyl acetate/methylene chloride = 9/91 to 30/70). After concentration under reduced pressure, the residue was solidified by the addition of diethyl ether. The organic solvent was distilled off under reduced pressure, and then, the solid was suspended in diisopropyl ether, collected by filtration, and dried to obtain the title compound (3.04 g, yield: 66.3%) as a solid.
$^1$H-NMR (DMSO-$D_6$) δ: 11.55 (1H, s), 9.48 (1H, s), 7.72-7.69 (2H, m), 7.45-7.41 (2H, m), 7.24-7.21 (1H, m), 5.43 (1H, s), 3.48-3.27 (2H, m, +H2O), 3.00-2.91 (1H, m), 1.17 (3H, d, J= 6.7 Hz).
MS (APCI) m/z : 314 [$(M+H)^+$].

(Step 3) 2,2,2-Trifluoro-N-[2-(2-methyl-5-oxo-1-phenyl-2,3-dihydro-1H-pyrazol-3-yl)propyl]acetamide

To a solution of 2,2,2-trifluoro-N-[2-(5-oxo-1-phenyl-4H-pyrazol-3-yl)propyl]acetamide (3.04 g, 9.70 mmol) synthesized in step 2 in N,N-dimethylformamide (3.0 mL), methyl iodide (3.02 mL, 6.89 g, 48.5 mmol) was added, and the mixture was stirred at 100°C for 3.5 hours in a sealed tube. The reaction solution was allowed to cool to room temperature, then methyl iodide (1.81 mL, 4.13 g, 29.1 mmol) was further added thereto, and the mixture was further stirred at 100°C for 3.75 hours in a sealed tube. The reaction solution was allowed to cool to room temperature, and then, a saturated aqueous solution of sodium bicarbonate and saturated saline were added to the reaction solution, followed by extraction with ethyl acetate three times. The organic layer was dried over anhydrous sodium sulfate. After filtration, the filtrate was concentrated under reduced pressure, and then, the obtained residue was purified by silica gel column chromatography (Biotage Japan Ltd., eluting solvent: methanol/ethyl acetate = 1/99 to 10/90). After concentration under reduced

pressure, the obtained solid was suspended in diethyl ether, collected by filtration, and dried to obtain the title compound (2.63 g, yield: 82.8%) as a solid.
$^1$H-NMR ($CDCl_3$) δ: 8.99 (1H, s), 7.50-7.46 (2H, m), 7.36-7.33 (3H, m), 5.11 (1H, s), 3.56-3.49 (1H, m), 3.16-3.07 (1H, m), 3.02 (3H, s), 2.69-2.62 (1H, m), 1.14 (3H, d, J = 6.7 Hz).
MS (APCI) m/z : 328 [(M+H)$^+$].

(Step 4) 2,2,2-Trifluoro-N-[2-(2-methyl-4-nitro-5-oxo-1-phenyl-2,3-dihydro-1H-pyrazol-3-yl)propyl]acetamide

To a solution of 2,2,2-trifluoro-N-[2-(2-methyl-5-oxo-1-phenyl-2,3-dihydro-1H-pyrazol-3-yl)propyl]acetamide (2.63 g, 8.04 mmol) synthesized in step 3 in trifluoroacetic acid (40 mL), concentrated nitric acid (1.55 mL, 2.17 g, 24.1 mmol) was added, and the mixture was stirred at room temperature for 1 hour. Ice water was added to the reaction solution, and the deposited solid was collected by filtration and dried to obtain the title compound (2.55 g, yield: 85.2%) as a solid.
$^1$H-NMR (DMSO-$D_6$) δ: 9.77 (1H, t, J= 5.5 Hz), 7.63-7.53 (3H, m), 7.38-7.35 (2H, m), 3.88-3.82 (2H, m), 3.68-3.62 (1H, m), 3.41 (3H, s), 1.43 (3H, d, J= 6.7 Hz).
MS (APCI) m/z : 373 [(M+H)$^+$].

(Step 5) N-[2-(4-Amino-2-methyl-5-oxo-1-phenyl-2,3-dihydro-1H-pyrazol-3-yl)propyl]-2,2,2-trifluoroacetamide (intermediate 66a)

A suspension of 2,2,2-trifluoro-N-[2-(2-methyl-4-nitro-5-oxo-1-phenyl-2,3-dihydro-1H-pyrazol-3-yl)propyl]acetamide (2.55 g, 6.85 mmol) synthesized in step 4 in methanol (300 mL) was heated to 50°C to obtain a homogeneous mixture. Then, a 10% palladium carbon catalyst (1.00 g) was added thereto, and the mixture was stirred at 50°C for 5 hours under the hydrogen atmosphere. The reaction solution was allowed to cool to room temperature, and then, the reaction system was purged with nitrogen. After filtration of the reaction solution, the filtrate was concentrated under reduced pressure. The obtained residue was solidified with ethyl acetate and diethyl ether. The solid was collected by filtration and dried to obtain the title compound (intermediate 66a) (1.85 g, yield: 78.9%) as a solid.
$^1$H-NMR ($CDCl_3$) δ: 8.47 (1H, s), 7.48-7.41 (4H, m), 7.31-7.27 (1H, m), 3.74-3.58 (2H, m), 3.15-3.06 (1H, m), 2.96 (2H, s), 2.87 (3H, s), 1.37 (3H, d, J = 7.3 Hz).
MS (APCI) m/z : 343 [(M+H)$^+$].

(Step 6) N-[(2S)-2-(4-Amino-2-methyl-5-oxo-1-phenyl-2,3-dihydro-1H-pyrazol-3-yl)propyl]-2,2,2-trifluoroacetamide (intermediate 66b) and N-[(2R)-2-(4-amino-2-methyl-5-oxo-1-phenyl-2,3-dihydro-1H-pyrazol-3-yl)propyl]-2,2,2-trifluoroacetamide (intermediate 66c)

N-[2-(4-Amino-2-methyl-5-oxo-1-phenyl-2,3-dihydro-1H-pyrazol-3-yl)propyl]-2,2,2-trifluoroacetamide (intermediate 66a) (5.26 g, 15.4 mmol) synthesized in step 5 was resolved by chiral column chromatography (CHIRALPAK IC, ethanol/hexane = 1/1) to obtain each of N-[(2S)-2-(4-amino-2-methyl-5-oxo-1-phenyl-2,3-dihydro-1H-pyrazol-3-yl)propyl]-2,2,2-trifluoroacetamide (intermediate 66b) (2.48 g, yield: 47.1%, >99% ee) as a solid and N-[(2R)-2-(4-amino-2-methyl-5-oxo-1-phenyl-pyrazol-3-yl)propyl]-2,2,2-trifluoroacetamide (intermediate 66c) (2.58 g, yield: 49.0%, >99% ee) as a solid.
Intermediate 66b
$^1$H-NMR ($CDCl_3$) δ: 8.35 (1H, s), 7.48-7.41 (4H, m), 7.30-7.26 (1H, m), 3.75-3.64 (2H, m), 3.14-3.01 (3H, m), 2.87 (3H, s), 1.38 (3H, d, J = 7.3 Hz).
MS (APCI) m/z :343 [(M+H)$^+$].
$[\alpha]_D^{20}$ + 94.9 (c = 1.01, MeOH).
Intermediate 66b was confirmed to be the (S) form by performing synthesis in the same way as in steps 1 to 6 of Example 51 using (2S)-3-(tert-butoxycarbonylamino)-2-methylpropanoic acid.
Intermediate 66c
$^1$H-NMR ($CDCl_3$) δ: 8.35 (1H, s), 7.47-7.41 (4H, m), 7.30-7.26 (1H, m), 3.75-3.63 (2H, m), 3.14-3.01 (3H, m), 2.87 (3H, s), 1.38 (3H, d, J = 7.3 Hz).
MS (APCI) m/z :343 [(M+H)$^+$].
$[\alpha]_D^{20}$ - 95.0 (c = 1.00, MeOH).
Intermediate 66c was confirmed to be the (R) form by performing synthesis in the same way as in steps 1 to 6 of Example 51 using (2R)-3-(tert-butoxycarbonylamino)-2-methylpropanoic acid.

[Formula 70]

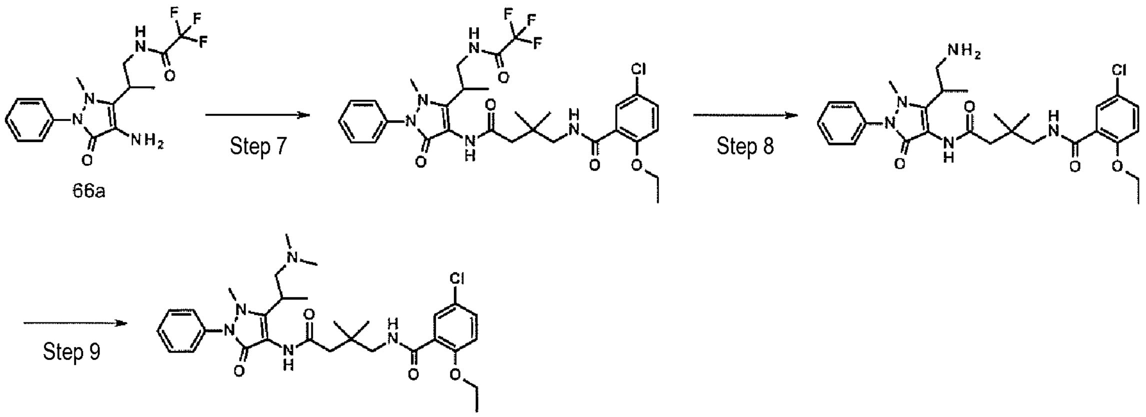


(Step 7) 5-Chloro-N-{2,2-dimethyl-4-[(1-methyl-5-{1-methyl-2-[(2,2,2-trifluoroacetyl)amino]ethyl}-3-oxo-2-phenyl-2,3-dihydro-1H-pyrazol-4-yl)amino]-4-oxobutyl}-2-ethoxybenzamide

Intermediate 1a (1.91 g, 6.10 mmol) was dissolved in N,N-dimethylformamide (30 mL). To the solution, 0-(7-azabenzotriazol-1-yl)-N,N,N',N'-tetramethyluronium hexafluorophosphate (HATU) (2.74 g, 7.20 mmol) and N,N-diisopropylethylamine (1.25 mL, 0.93 g, 7.20 mmol) were added, and the mixture was stirred at room temperature. N-[2-(4-Amino-2-methyl-5-oxo-1-phenyl-2,3-dihydro-1H-pyrazol-3-yl)propyl]-2,2,2-trifluoroacetamide (intermediate 66a) (2.05 g, 6.00 mmol) synthesized in step 5 was added thereto, and the mixture was stirred at room temperature for 2 hours. Water was added to the reaction solution, followed by extraction with ethyl acetate. The organic layer was washed with a saturated aqueous solution of sodium bicarbonate and saturated saline and then dried over anhydrous magnesium sulfate. After filtration, the filtrate was concentrated, and the obtained crude product was purified by amino silica-silica gel column chromatography (Biotage Japan Ltd., eluting solvent: hexane/ethyl acetate → ethyl acetate/methanol = 30/70 to 0/100 → 100/0 to 80/20) to obtain the title compound (3.02 g, yield: 78.9%) as a solid.
$^1$H-NMR ($CDCl_3$) δ: 9.89 (1H, s), 8.98-8.96 (1H, br m), 8.44 (1H, t, J = 6.8 Hz), 8.16 (1H, d, J= 2.9 Hz), 7.47-7.37 (5H, m), 7.29-7.28 (1H, m), 6.93 (1H, d, J = 9.3 Hz), 4.21 (2H, q, J = 7.0 Hz), 3.96-3.93 (1H, m), 3.76-3.71 (1H, m), 3.55-3.40 (3H, m), 3.07 (3H, s), 2.33 (1H, d, J = 12.7 Hz), 2.21 (1H, d, J = 12.7 Hz), 1.53 (3H, t, J = 7.0 Hz), 1.42 (3H, d, J = 7.3 Hz), 1.17 (3H, s), 1.11 (3H, s).

(Step 8) N-(4-{[5-(2-Amino-1-methylethyl)-1-methyl-3-oxo-2-phenyl-2,3-dihydro-1H-pyrazol-4-yl]amino}-2,2-dimethyl-4-oxobutyl)-5-chloro-2-ethoxybenzamide

5-Chloro-N-{2,2-dimethyl-4-[(1-methyl-5-{1-methyl-2-[(2,2,2-trifluoroacetyl)amino]ethyl}-3-oxo-2-phenyl-2,3-dihydro-1H-pyrazol-4-yl)amino]-4-oxobutyl}-2-ethoxybenzamide (3.02 g, 4.73 mmol) synthesized in step 7 was dissolved in ethanol (20 mL). To the solution, a 1 N aqueous sodium hydroxide solution (10 mL) was added, and the mixture was stirred at room temperature for 1 hour. The solvent was distilled off under reduced pressure, and water was added to the obtained residue, followed by extraction with methylene chloride. The organic layer was washed with saturated saline and then dried over anhydrous magnesium sulfate. After filtration, the filtrate was concentrated to obtain the title compound (2.44 g, yield: 95.2%) as a solid.
$^1$H-NMR ($CDCl_3$) δ: 9.33 (1H, s), 8.40 (1H, t, J = 6.7 Hz), 8.16 (1H, d, J = 3.0 Hz), 7.43-7.40 (5H, m), 7.26-7.23 (1H, m), 6.92 (1H, d, J = 9.1 Hz), 4.20 (2H, q, J= 6.9 Hz), 3.76 (1H, dd, J = 14.0, 6.7 Hz), 3.50 (1H, dd, J= 14.0, 6.7 Hz), 3.21-3.15 (1H, m), 3.15 (3H, s), 2.98-2.94 (2H, m), 2.28 (1H, d, J = 12.8 Hz), 2.21 (1H, d, J = 12.8 Hz), 1.52 (3H, t, J = 6.9 Hz), 1.41 (3H, d, J = 6.7 Hz), 1.17 (3H, s), 1.14 (3H, s).

(Step 9) 5-Chloro-N-[4-({5-[2-(dimethylamino)-1-methylethyl]-1-methyl-3-oxo-2-phenyl-2,3-dihydro-1H-pyrazol-4-yl}amino)-2,2-dimethyl-4-oxobutyl]-2-ethoxybenzamide

N-(4-{[5-(2-Amino-1-methylethyl)-1-methyl-3-oxo-2-phenyl-2,3-dihydro-1H-pyrazol-4-yl]amino}-2,2-dimethyl-4-oxobutyl)-5-chloro-2-ethoxybenzamide (2.44 g, 4.50 mmol) synthesized in step 8 was dissolved in methylene chloride (15 mL) and methanol (15 mL). To the solution, sodium triacetoxyborohydride (3.82 g, 18.0 mmol) was added, and the mixture was stirred at room temperature. A 37% aqueous formaldehyde solution (5.0 mL) was added thereto. After the completion of the addition, the mixture was stirred at room temperature for 1 hour. The solvent was distilled off under

reduced pressure, and the obtained residue was neutralized by the addition of a saturated aqueous solution of sodium bicarbonate, followed by extraction with methylene chloride. The organic layer was dried over anhydrous magnesium sulfate. After filtration, the filtrate was concentrated, and the obtained crude product was purified by amino silica gel column chromatography (Biotage Japan Ltd., eluting solvent: hexane/ethyl acetate → ethyl acetate/methanol = 30/70 to 0/100 → 100/0 to 80/20). The solvent was concentrated, and then, the residue was solidified by the addition of ethyl acetate/diethyl ether. The solid was collected by filtration, washed with diethyl ether, and then dried to obtain the title compound (2.02 g, yield: 78.7%) as a solid.
$^1$H-NMR ($CDCl_3$) δ: 9.14 (1H, s), 8.39 (1H, t, J = 6.7 Hz), 8.18 (1H, d, J= 3.1 Hz), 7.45-7.37 (5H, m), 7.27-7.22 (1H, m), 6.91 (1H, d, J = 9.2 Hz), 4.20 (2H, q, J = 7.0 Hz), 3.66 (1H, dd, J = 14.0, 6.7 Hz), 3.55 (1H, dd, J = 14.0, 6.7 Hz), 3.14 (3H, s), 3.09-3.00 (1H, m), 2.75 (1H, dd, J= 12.2, 7.0 Hz), 2.58 (1H, dd, J= 12.2, 7.9 Hz), 2.30 (6H, s), 2.24 (2H, d, J = 3.1 Hz), 1.52 (3H, t, J= 7.0 Hz), 1.42 (3H, d, J = 6.7 Hz), 1.17 (3H, s), 1.15 (3H, s).
MS (APCI) m/z :570 [(M+H)$^+$].

The following compounds of Examples 67 to 75 were synthesized in the same way as above and are shown in Table 25.

[Table 25-1]

| Example No. | Structure | Intermediate used | Instrumental analysis data |
|---|---|---|---|
| 6 7 | 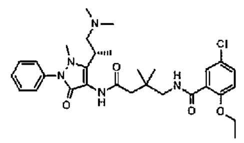 | 1 a<br>6 6 b | $^1$H-NMR ($CDCl_3$) 6: 9.12 (1H, s), 8.39 (1H, t, J = 6.7 Hz), 8.18 (1H, d, J = 3.0 Hz), 7.43-7.37 (5H, m), 7.26-7.22 (1H, m), 6.91 (1H, d, J = 9.1 Hz), 4.20 (2H, q, J = 7.0 Hz), 3.66 (1H, dd, J = 14.3, 6.7 Hz), 3.55 (1H, dd, J = 14.3, 6.7 Hz), 3.13 (3H, s), 3.09-3.00 (1H, m), 2.74 (1H, dd, J = 12.1, 7.3 Hz), 2.58 (1H, dd, J = 12.1, 7.9 Hz), 2.30 (6H, s), 2.24 (2H, d, J = 3.0 Hz), 1.52 (3H, t, J = 7.0 Hz), 1.42 (3H, d, J = 7.3 Hz), 1.17 (3H, s), 1.15 (3H, s).<br>MS (APCI) m/z :570 [(M+H)$^+$].<br>$[\alpha]_D^{20}$ +7.63 (c = 1.05, MeOH). |
| 6 8 | 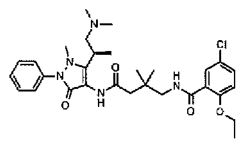 | 1 a<br>6 6 c | $^1$H-NMR ($CDCl_3$) δ: 9.12 (1H, s), 8.39 (1H, t, J = 6.7 Hz), 8.18 (1H, d, J = 3.0 Hz), 7.45-7.36 (5H, m), 7.25-7.22 (1H, m), 6.91 (1H, d, J 9.1 Hz), 4.19 (2H, q, J = 7.0 Hz), 3.66 (1H, dd, J = 14.0, 6.7 Hz), 3.55 (1H, dd, J = 14.0, 6.7 Hz), 3.13 (3H, s), 3.09-3.00 (1H, m), 2.74 (1H, dd, J = 12.1, 7.0 Hz), 2.58 (1H, dd, J = 12.1, 7.9 Hz), 2.30 (6H, s), 2.24 (2H, d, J = 3.0 Hz), 1.52 (3H, t, J = 7.0 Hz), 1.42 (3H, d, J = 7.3 Hz), 1.17 (3H, s), 1.15 (3H, s).<br>MS (APCI) m/z :570 [(M+H)$^+$].<br>$[\alpha]_D^{20}$ -7.51 (c = 1.04, MeOH). |
| 6 9 | 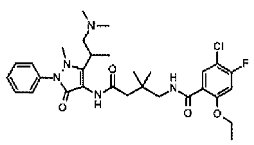 | 1 c<br>6 6 a | $^1$H-NMR ($CDCl_3$) δ: 8.96 (1H, s), 8.28-8.23 (2H, m), 7.43-7.42 (4H, m), 7.27-7.23 (1H, m), 6.78 (1 H, d, J = 10.4 Hz), 4.18 (2H, q, J = 7.0 Hz), 3.67-3.52 (2H, m), 3.14-3.03 (4H, m), 2.75 (1H, dd, J = 12.2, 7.3 Hz), 2.57 (1H, dd, J = 12.2, 7.9 Hz), 2.30 (6H, s), 2.24 (2H, s), 1.53 (3H, t, J = 7.0 Hz), 1.42 (3H, d, J = 6.7 Hz), 1.16 (3H, s), 1.14 (3H, s).<br>MS (APCI) m/z :588 [(M+H)$^+$]. |
| 7 0 | 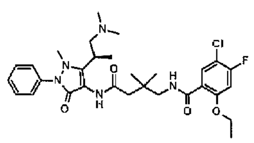 | 1 c<br>6 6 c | $^1$H-NMR ($CDCl_3$) δ: 8.91 (1H, s), 8.27 (1H, d, J = 8.8 Hz), 8.23 (1H, t, J = 6.6 Hz), 7.43-7.42 (4H, m), 7.25-7.24 (1H, m), 6.78 (1H, d, J = 10.7 Hz), 4.18 (2H, q, J = 7.1 Hz), 3.66-3.63 (1H, m), 3.56-3.53 (1H, m), 3.13 (3H, s), 3.05-3.04 (1H, m), 2.75-2.73 (1H, m), 2.58-2.55 (1H, m), 2.30 (6H, s), 2.24 (2H, d, J = 2.4 Hz), 1.53 (3H, t, J = 7.1 Hz), 1.41 (3H, d, J = 7.3 Hz), 1.16 (3H, s).<br>MS (ESI) m/z : 588 [(M+H)$^+$]. |

[Table 25-2]

| | | | |
|---|---|---|---|
| 7 1 | 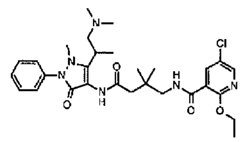 | 1h 66 a | $^1$H-NMR ($CDCl_3$) δ: 8.74 (1H, s), 8.46 (1H, d, J = 2.4 Hz), 8.42 (1H, t, J = 6.7 Hz), 8.19 (1H, d, J = 2.4 Hz), 7.46-7.41 (4H, m), 7.29-7.23 (1H, m), 4.54 (2H, q, J = 7.0 Hz), 3.67-3.55 (2H, m), 3.14 (3H, s), 3.10-3.01 (1H, m), 2.74 (1H, dd, J = 12.2, 7.3 Hz), 2.56 (1H, dd, J = 12.2, 7.9 Hz), 2.29 (6H, s), 2.24 (2H, s), 1.47 (3H, t, J = 7.0 Hz), 1.41 (3H, d, J = 6.7 Hz), 1.17 (3H, s), 1.16 (3H, s).<br>MS (APCI) m/z :571 [$(M+H)^+$]. |
| 7 2 | 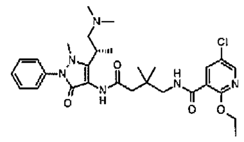 | 1 h 36 b | $^1$H-NMR ($CDCl_3$) δ: 8.72 (1H, s), 8.46-8.39 (2H, m), 8.19 (1H, d, J = 2.4 Hz), 7.45-7.41 (4H, m), 7.27-7.22 (1H, m), 4.54 (2H, q, J = 7.0 Hz), 3.67-3.55 (2H, m), 3.14 (3H, s), 3.09-3.01 (1H, m), 2.73 (1H, dd, J = 12.5, 7.0 Hz), 2.55 (1H, dd, J = 12.5, 7.9 Hz), 2.29 (6H, s), 2.24 (2H, s), 1.47 (3H, t, J = 7.0 Hz), 1.41 (3H, d, J = 6.7 Hz), 1.16 (3H, s), 1.16 (3H, s).<br>MS (APCI) m/z :571 [$(M+H)^+$].<br>$[\alpha]_D^{20}$ +6.66 (c = 1.01, MeOH). |
| 7 3 | 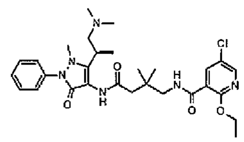 | 1 h 66 c | $^1$H-NMR ($CDCl_3$) δ: 8.71 (1H, s), 8.46-8.39 (2H, m), 8.19 (1H, d, J = 2.4 Hz), 7.45-7.41 (4H, m), 7.27-7.23 (1H, m), 4.54 (2H, q, J = 7.0 Hz), 3.67-3.55 (2H, m), 3.14 (3H, s), 3.10-3.01 (1H, m), 2.73 (1H, dd, J = 12.2, 7.3 Hz), 2.55 (1H, dd, J = 12.2, 7.9 Hz), 2.29 (6H, s), 2.24 (2H, s), 1.47 (3H, t, J = 7.0 Hz), 1.41 (3H, d, J = 7.3 Hz), 1.17 (3H, s), 1.16 (3H, s).<br>MS (APCI) m/z :571 [$(M+H)^+$].<br>$[\alpha]_D^{20}$ -6.95 (c = 1.01, MeOH). |
| 7 4 | 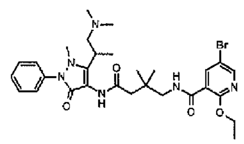 | 1 j 66 a | $^1$H-NMR ($CDCl_3$) δ: 8.73 (1H, s), 8.59 (1H, d, J = 2.4 Hz), 8.39 (1H, t, J = 6.4 Hz), 8.28 (1H, d, J = 3.1 Hz), 7.45-7.41 (4H, m), 7.28-7.23 (1H, m), 4.54 (2H, q, J = 7.0 Hz), 3.67-3.54 (2H, m), 3.14 (3H, s), 3.08-3.01 (1H, m), 2.73 (1H, dd, J = 12.2, 7.3 Hz), 2.56 (1H, dd, J = 12.2, 7.9 Hz), 2.29 (6H, s), 2.24 (2H, s), 1.47 (3H, t, J = 7.0 Hz), 1.41 (3H, d, J = 7.3 Hz), 1.16 (3H, s), 1.15 (3H, s).<br>MS (APCI) m/z :615 [$(M+H)^+$]. |
| 7 5 | 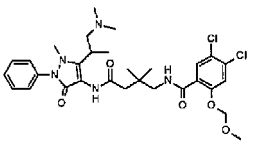 | 1 g 66 a | $^1$H-NMR ($CDCl_3$) δ: 8.35-8.34 (1H, br m), 8.24 (1H, s), 7.45-7.39 (4H, m), 7.34 (1H, s), 7.28-7.28 (1H, m), 5.32 (2H, s), 3.60-3.54 (2H, m), 3.50 (3H, s), 3.13 (3H, s), 3.06-3.03 (1H, m), 2.75-2.73 (1H, m), 2.54-2.51 (1H, m), 2.28 (6H, s), 2.25 (2H, d, J = 2,4 Hz), 1.40 (3H, d, J = 6.8 Hz), 1.15 (3H, s), 1.14 (3H, s).<br>MS (ESI+APCI) m/z: 620 [$(M+H)^+$]. |

(Example 76) 5-Chloro-N-[2,2-dimethyl-4-({1-methyl-5-[1-methyl-2-(methylamino)ethyl]-3-oxo-2-phenyl-2,3-dihydro-1H-pyrazol-4-yl}amino)-4-oxobutyl]-2-ethoxybenzamide

[Formula 71]

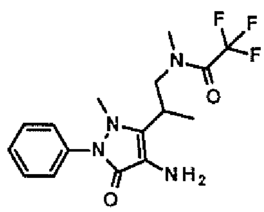

(Step 1) N-[2-(4-Amino-2-methyl-5-oxo-1-phenyl-2,3-dihydro-1H-pyrazol-3-yl)propyl]2,2,2-trifluoro-N-methylacetamide

The title compound was obtained as an oil substance by using 2,2,2-trifluoro-N-[2-(2-methyl-5-oxo-1-phenyl-2,3-dihydro-1H-pyrazol-3-yl)propyl]acetamide synthesized in step 3 of Example 66 instead of N-[1,1-dimethyl-2-(2-methyl-5-oxo-1-phenyl-2,3-dihydro-1H-pyrazol-3-yl)ethyl]2,2,2-trifluoroacetamide in step 4 of Example 59 and subsequently performing the same reaction as in Example 59 up to step 5.
$^1$H-NMR ($CDCl_3$) δ: 7.47-7.42 (4H, m), 7.29-7.22 (1H, m), 3.82 (1H, dd, J = 13.4, 6.7 Hz), 3.68 (1H, dd, J = 13.4, 9.2 Hz), 3.35-3.26 (1H, m), 3.16-3.10 (5H, m), 2.83 (3H, s), 1.38 (3H, d, J = 7.3 Hz).
MS (APCI) m/z : 373 [$(M+H)^+$].

The following compounds of Examples 76 to 78 were subsequently synthesized in the same way as in steps 7 and 8 of Example 66 and are shown in Table 26.

[Table 26]

| Example No. | Structure | Intermediate used | Instrumental analysis data |
|---|---|---|---|
| 7 6 | 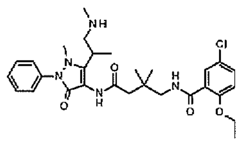 | 1 a | $^1$H-NMR ($CDCl_3$) δ 9.36 (1H, s), 8.40 (1H, t, J = 6.7 Hz), 8.17 (1H, d, J = 3.0 Hz), 7.44-7.38 (5H, m), 7.28-7.22 (1H, m), 6.92 (1H, d, J = 9.1 Hz), 4.20 (2H, q, J = 7.0 Hz), 3.75 (1H, dd, J = 14.6, 7.3 Hz), 3.51 (1H, dd, J = 14.6, 6.7 Hz), 3.15-3.06 (5H, m), 2.82-2.75 (1H, m), 2.46 (3H, s), 2.30-2.19 (2H, m), 1.66 (1H, brs), 1.52 (3H, t, J = 7.0 Hz), 1.42 (3H, d, J = 6.7 Hz), 1.17 (3H, s), 1.15 (3H, s).<br>MS (APCI) m/z :556 [$(M+H)^+$]. |
| 7 7 | 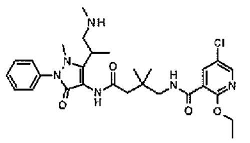 | 1 h | $^1$H-NMR ($CDCl_3$) δ 9.00 (1H, s), 8.46-8.40 (2H, m), 8.20 (1H, d, J = 3.0 Hz), 7.46-7.41 (4H, m), 7.29-7.23 (1H, m), 4.55 (2H, q, J = 7.1 Hz), 3.75 (1H, dd, J = 14.3, 7.3 Hz), 3.52 (1H, dd, J = 14.3, 6.7 Hz), 3.15-3.04 (5H, m), 2.78 (1 H, dd, J = 11.2, 5.8 Hz), 2.45 (3H, s), 2.24 (2H, q, J = 12.6 Hz), 1.64 (1H, br s), 1.48 (3H, t, J = 7.1 Hz), 1.41 (3H, d, J = 7.3 Hz), 1.17 (3H, s), 1.16 (3H, s).<br>MS (APCI) m/z :557 [$(M+H)^+$]. |
| 7 8 | 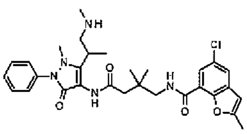 | 3 a | $^1$H-NMR ($CDCl_3$) δ: 8.87 (1H, s), 7.94 (1H, d, J = 2.4 Hz), 7.87 (1H, t, J = 6.7 Hz), 7.57 (1H, d, J = 2.4 Hz), 7.46-7.41 (4H, m), 7.29-7.22 (1H, m), 6.44 (1H, d, J = 1.2 Hz), 3.82 (1H, dd, J = 14.3, 7.0 Hz), 3.61 (1H, dd, J = 14.3, 6.4 Hz), 3.15-3.05 (5H, m), 2.79 (1H, dd, J = 11.0, 5.5 Hz), 2.52 (3H, d, J = 1.2 Hz), 2.45 (3H, s), 2.30 (2H, q, J = 12.8 Hz), 1.42 (3H, d, J = 6.7 Hz), 1.22 (3H, s), 1.20 (3H, s).<br>MS (APCI) m/z :566 [$(M+H)^+$]. |

(Example 79) 5-Chloro-N-[4-({5-[2-(dimethylamino)-1-methoxyethyl]-1-methyl-3-oxo-2-phenyl-2,3-dihydro-1H-pyrazol-4-yl}amino)-2,2-dimethyl-4-oxobutyl]-2-ethoxybenzamide

[Formula 72]

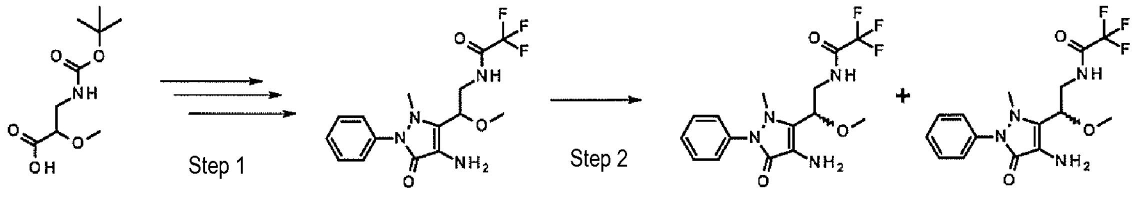


79a (−)−79b (+)−79c

(Step 1) N-[2-(4-Amino-2-methyl-5-oxo-1-phenyl-2,3-dihydro-1H-pyrazol-3-yl)-2-methoxyethyl]-2,2,2-trifluoroacetamide (intermediate 79a)

The title compound (intermediate 79a) was obtained as a solid by using 3-(tert-butoxycarbonylamino)-2-methoxypropanoic acid prepared by the method described in US2007/72934A1 instead of (3S)-3-(tert-butoxycarbonylamino)butanoic acid in step 1 of Example 51 and subsequently performing the same reaction as in Example 51 up to step 6.
$^1$H-NMR ($CDCl_3$) δ: 7.51-7.43 (4H, m), 7.31-7.22 (2H, m), 4.36 (1H, dd, J = 7.9, 4.3 Hz), 3.92-3.86 (1H, m), 3.60-3.46 (6H, m), 2.84 (3H, s).
MS (APCI) m/z : 359 [(M+H)$^+$].

(Step 2) (-)-N-[2-(4-Amino-2-methyl-5-oxo-1-phenyl-2,3-dihydro-1H-pyrazol-3-yl)-2-methoxyethyl]-2,2,2-trifluoroacetamide (intermediate 79b) and (+)-N-[2-(4-amino-2-methyl-5-oxo-1-phenyl-2,3-dihydro-1H-pyrazol-3-yl)-2-methoxyethyl]-2,2,2-trifluoroacetamide (intermediate 79c)

N-[2-(4-Amino-2-methyl-5-oxo-1-phenyl-2,3-dihydro-1H-pyrazol-3-yl)-2-methoxyethyl]-2,2,2-trifluoroacetamide (intermediate 79a) (3.08 g, 8.86 mmol) synthesized in step 1 was resolved by chiral column chromatography (CHIRALPAK IC, ethanol/hexane = 1/1) to obtain each of (-)-N-[2-(4-amino-2-methyl-5-oxo-1-phenyl-2,3-dihydro-1H-pyrazol-3-yl)-2-methoxyethyl]-2,2,2-trifluoroacetamide (intermediate 79b) (1.51 g, yield: 49.0%) as a solid and (+)-N-[2-(4-amino-2-methyl-5-oxo-1-phenyl-2,3-dihydro-1H-pyrazol-3-yl)-2-methoxyethyl]-2,2,2-trifluoroacetamide (intermediate 79c) (1.49 g, yield: 48.4%) as a solid. Intermediate 79b
$^1$H-NMR ($CDCl_3$) δ: 7.49-7.43 (4H, m), 7.31-7.22 (2H, m), 4.36 (1H, dd, J = 7.9, 3.6 Hz), 3.94-3.88 (1H, m), 3.59-3.51 (3H, m), 3.47 (3H, s), 2.85 (3H, s).
MS (ESI) m/z : 359 [(M+H)$^+$].
$[a,]_D^{20}$ -37.4 (c = 1.00, MeOH).
Intermediate 79c
$^1$H-NMR ($CDCl_3$) δ: 7.48-7.43 (4H, m), 7.30-7.23 (2H, m), 4.36 (1H, dd, J = 7.9, 3.6 Hz), 3.94-3.87 (1H, m), 3.59-3.52 (3H, m), 3.47 (3H, s), 2.84 (3H, s).
MS (ESI) m/z : 359 [(M+H)$^+$].
$[\alpha]_D^{20}$ +37.7 (c = 1.01, MeOH).
The following compounds of Examples 79 to 83 were subsequently synthesized in the same way as in steps 7 to 9 of Example 66 and are shown in Table 27.

[Table 27-1]

| Example No. | Structure | Intermediate used | Instrumental analysis data |
|---|---|---|---|
| 7 9 | 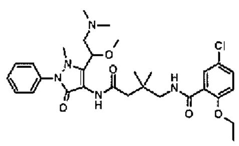 | 1 a<br>7 9 a | $^1$H-NMR ($CDCl_3$) δ 9.22 (1H, s), 8.37 (1H, t, J = 6.7 Hz), 8.20 (1H, d, J = 3.0 Hz), 7.47-7.37 (5H, m), 7.31-7.26 (1H, m), 6.91 (1H, d, J = 8.5 Hz), 4.75 (1H, dd, J = 9.1, 3.0 Hz), 4.20 (2H, q, J = 7.0 Hz), 3.60 (2H, d, J = 6.7 Hz), 3.49 (3H, s), 3.25 (3H, s), 2.94 (1H, dd, J = 13.4, 9.7 Hz), 2.71 (1H, dd, J = 13.4, 3.3 Hz), 2.36 (6H, s), 2.27 (2H, s), 1.52 (3H, t, J = 7.0 Hz), 1.14 (3H, s), 1.12 (3H, s).<br>MS (APCI) m/z :586 [(M+H)$^+$]. |

(continued)

| Example No. | Structure | Intermediate used | Instrumental analysis data |
|---|---|---|---|
| 8 0 | 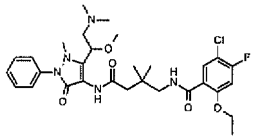 | 1 c<br>7 9 a | $^1$H-NMR ($CDCl_3$) δ: 9.15 (1H, s), 8.31-8.22 (2H, m), 7.48-7.42 (4H, m), 7.31-7.26 (1H, m), 6.79 (1H, d, J = 10.9 Hz), 4.74 (1H, dd, J = 9.1, 3.0 Hz), 4.18 (2H, q, J = 7.0 Hz), 3.64-3.55 (2H, m), 3.48 (3H, s), 3.25 (3H, s), 2.93 (1H, dd, J = 13.4, 9.7 Hz), 2.70 (1H, dd, J = 13.4, 3.3 Hz), 2.36 (6H, s), 2.26 (2H, s), 1.54 (3H, t, J = 7.0 Hz), 1.13 (3H, s), 1.11 (3H, s).<br>MS (APCI) m/z :604 [$(M+H)^+$]. |
| 8 1 | 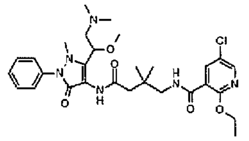 | 1 h<br>7 9 a | $^1$H-NMR ($CDCl_3$) δ: 8.96 (1H, s), 8.49 (1H, d, J = 3.6 Hz), 8.41 (1H, t, J = 6.7 Hz), 8.20-8.19 (1H, m), 7.50-7.39 (4H, m), 7.31-7.27 (1H. m), 4.75 (1H, dd, J = 9.4, 2.7 Hz), 4.55 (2H, q, J = 7.1 Hz), 3.65-3.55 (2H, m), 3.48 (3H, s), 3.26 (3H, s), 2.93 (1H, dd, J = 13.1, 9.7 Hz), 2.70 (1H, dd, J = 13.1, 3.0 Hz), 2.36 (6H, s), 2.26 (2H, s), 1.47 (3H, t, J = 7.1 Hz), 1.14 (3H, s), 1.12 (3H, s).<br>MS (APCI) m/z :587 [$(M+H)^+$]. |
| 8 2 | 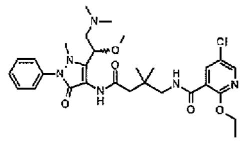 | 1 h<br>7 9 b | $^1$H-NMR ($CDCl_3$) δ 8.91 (1H, s), 8.49 (1H, d, J = 2.4 Hz), 8.40 (1H, t, J = 6.7 Hz), 8.20 (1H, d, J = 2.4 Hz), 7.48-7.41 (4H, m), 7.32-7.27 (1H, m), 4.75 (1H, dd, J = 9.1, 3.0 Hz), 4.55 (2H, q, J = 7.0 Hz), 3.63-3.57 (2H, m), 3.48 (3H, s), 3.25 (3H, s), 2.93 (1H, dd, J = 13.1, 9.4 Hz), 2.71 (1H, dd, J = 13.1, 3.3 Hz), 2.36 (6H, s), 2.26 (2H, s), 1.47 (3H, t, J = 7.0 Hz), 1.14 (3H, s), 1.13 (3H, s).<br>MS (APCI) m/z:587 [$(M+H)^+$].<br>$[\alpha]_D^{20}$-44.2 (c = 1.00, MeOH). |

[Table 27-2]

| | | | |
|---|---|---|---|
| 8<br>3 | 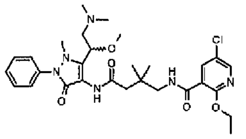 | 1 h<br>7 9 c | $^1$H-NMR ($CDCl_3$) δ 8.90 (1H, s), 8.49 (1H, d, J = 2.4 Hz), 8.40 (1H, t, J = 6.7 Hz), 8.20 (1H, d, J = 2.4 Hz), 7.48-7.41 (4H, m), 7.32-7.27 (1H, m), 4.75 (1H, dd, J = 9.4, 3.3 Hz), 4.55 (2H, q, J = 7.0 Hz), 3.63-3.57 (2H, m), 3.48 (3H, s), 3.25 (3H, s), 2.93 (1H, dd, J = 13.1, 9.4 Hz), 2.71 (1H, dd, J = 13.1, 3.0 Hz), 2.36 (6H, s), 2.26 (2H, s), 1.47 (3H, t, J = 7.0 Hz), 1.14 (3H, s), 1.13 (3H, s).<br>MS (APCI) m/z :587 [$(M+H)^+$].<br>$[\alpha]_D^{20}$ +44.7 (c = 1.08, MeOH). |

(Example 84) 5-Chloro-N-[4-({5-[2-(dimethylamino)-1,1-dimethylethyl]-1-methyl-3-oxo-2-phenyl-2,3-dihydro-1H-pyrazol-4-yl}amino)-2,2-dimethyl-4-oxobutyl]-2-ethoxybenzamide

[Formula 73]

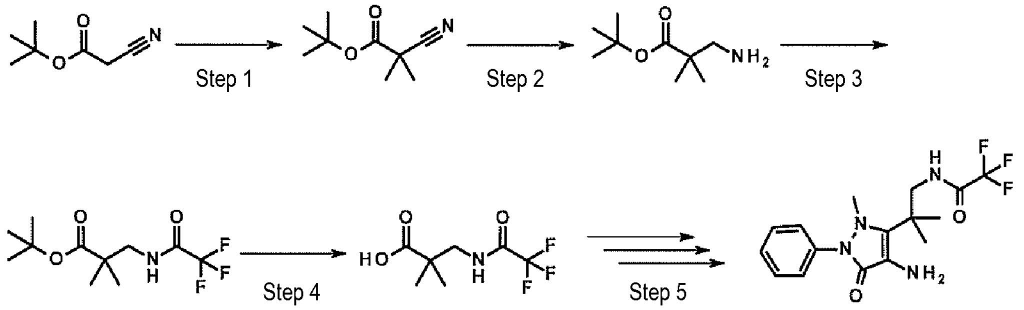


(Step 1) tert-Butyl 2-cyano-2-methylpropanoate

To a solution of tert-butyl cyanoacetate (12.5 g, 88.6 mmol) in N,N-dimethylformamide (400 mL), potassium carbonate (42.8 g, 310 mmol) and methyl iodide (19.3 mL, 44.0 g, 310 mmol) were added under ice cooling, and the mixture was stirred for 1 hour under ice cooling and then stirred at room temperature for 4 hours. The reaction solution was filtered through celite, and water was added to the filtrate, followed by extraction with diethyl ether three times. The organic layer was washed with saturated saline and then dried over anhydrous sodium sulfate. After filtration, the filtrate was concentrated under reduced pressure, and then, the obtained residue was purified by silica gel column chromatography (Yamazen Corp., eluting solvent: ethyl acetate/hexane = 0/100 to 17/83) to obtain the title compound (13.6 g, yield: 90.4%) as an oil substance.
$^1$H-NMR ($CDCl_3$) δ: 1.57 (6H, s), 1.51 (9H, s).

(Step 2) tert-Butyl 3-amino-2,2-dimethylpropanoate

To a solution of tert-butyl 2-cyano-2-methylpropanoate (13.6 g, 80.1 mmol) synthesized in step 1 in ethanol (150 mL), a 4% solution of ammonia in methanol (50 mL) and 5% rhodium on alumina (4.50 g) were added, and the mixture was stirred for 15 hours under a hydrogen atmosphere. Then, 5% rhodium on alumina (4.50 g) was further added thereto, and the mixture was stirred for 10 hours under a hydrogen atmosphere. After purging with nitrogen, the reaction solution was filtered, and the filtrate was concentrated under reduced pressure. Then, the obtained residue was purified by amino silica gel column chromatography (Yamazen Corp., eluting solvent: methanol/ethyl acetate = 1/99 to 10/90) to obtain the title compound (14.8 g, quantitative) as an oil substance.
$^1$H-NMR ($CDCl_3$) δ: 2.70 (2H, s), 1.45 (9H, s), 1.12 (6H, s).

(Step 3) tert-Butyl 2,2-dimethyl-3-[(2,2,2-trifluoroacetyl)amino]propanoate

To a solution of tert-butyl 3-amino-2,2-dimethylpropanoate (13.9 g, 80.1 mmol) synthesized in step 2 in methylene chloride (400 mL), N,N-diisopropylethylamine (27.2 mL, 20.7 g, 160 mmol) and trifluoroacetic anhydride (13.4 mL, 20.2 g, 96.1 mmol) were added under ice cooling, and the mixture was stirred at the same temperature as above for 1 hour. Then, N,N-diisopropylethylamine (6.81 mL, 5.17 g, 40.0 mmol) and trifluoroacetic anhydride (5.57 mL, 8.41 g, 40.0 mmol) were further added thereto, and the mixture was stirred at the same temperature as above for 30 minutes. Water was added to the reaction solution, followed by extraction with methylene chloride three times. The organic layer was washed with 1 N hydrochloric acid, a saturated aqueous solution of sodium bicarbonate, and saturated saline in this order and then dried over anhydrous sodium sulfate. After filtration, the filtrate was concentrated under reduced pressure, and then, the obtained residue was purified by silica gel column chromatography (Yamazen Corp., eluting solvent: ethyl acetate/hexane = 0/100 to 18/82) to obtain the title compound (17.5 g, yield: 80.9%) as an oil substance.
$^1$H-NMR ($CDCl_3$) δ: 7.13 (1H, br s), 3.40 (2H, d, J = 6.1 Hz), 1.46 (9H, s), 1.18 (6H, s).

(Step 4) 2,2-Dimethyl-3-[(2,2,2-trifluoroacetyl)amino]propanoic acid

To a solution of tert-butyl 2,2-dimethyl-3-[(2,2,2-trifluoroacetyl)amino]propanoate (17.5 g, 64.8 mmol) synthesized in step 3 in methylene chloride (200 mL), water (0.1 mL) and trifluoroacetic acid (100 mL) were added under ice cooling, and the mixture was stirred at room temperature for 4 hours. The reaction solution was concentrated under reduced pressure, and then, ethyl acetate was added to the residue. The organic layer was washed with water three times and dried over anhydrous sodium sulfate. After filtration, the filtrate was concentrated under reduced pressure to

obtain the title compound (14.3 g, quantitative) as an oil substance.
$^{1}$H-NMR ($CDCl_3$) δ: 7.78 (1H, br s), 6.97 (1H, br s), 3.50 (2H, d, J = 6.1 Hz), 1.30 (6H, s).

(Step 5) N-[2-(4-Amino-2-methyl-5-oxo-1-phenyl-pyrazol-3-yl)-2-methyl-propyl]-2,2,2-trifluoro-acetamide

The title compound was obtained as a solid by using 2,2-dimethyl-3-[(2,2,2-trifluoroacetyl)amino]propanoic acid synthesized in step 4 instead of N-trifluoroacetyl-β-alanine in step 1 of Example 28 and subsequently performing the same reaction as in Example 28 from step 1 up to step 5.
$^{1}$H-NMR ($CDCl_3$) δ: 8.14 (1H, s), 7.51-7.44 (4H, m), 7.30-7.26 (1H, m), 3.68 (2H, d, J = 6.7 Hz), 3.24 (2H, s), 2.95 (3H, s), 1.43 (6H, s).
MS (APCI) m/z : : 357 [(M+H)$^+$].

The following compound of Example 84 was subsequently synthesized in the same way as in steps 7 to 9 of Example 66 and is shown in Table 28.

[Table 28]

| Example No. | Structure | Intermediate used | Instrumental analysis data |
|---|---|---|---|
| 8 4 | 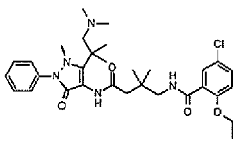 | 1 a | $^{1}$H-NMR ($CDCl_3$) δ: 9.19 (1H, s), 8.38 (1H, t, J = 6.7 Hz), 8.16 (1H, d, J = 2.4 Hz), 7.50-7.36 (5H, m), 7.24-7.21 (1H, m), 6.91 (1H, d, J = 8.5 Hz), 4.19 (2H, q, J = 6.9 Hz), 3.63 (2H, d, J = 6.7 Hz), 3.23 (3H, s), 2.67 (2H, s), 2.35 (6H, s), 2.23 (2H, s), 1.54-1.50 (9H, m), 1.18 (6H, s).<br>MS (APCI) m/z :584 [(M+H)$^+$] |

(Example 85) (+)-/(-)-5-Chloro-N-[2,2-dimethyl-4-({1-methyl-5-[1-(methylamino)ethyl]-3-oxo-2-phenyl-2,3-dihydro-1H-pyrazol-4-yl}amino)-4-oxobutyl]-2-ethoxypyridine-3-carboxamide

[Formula 74]

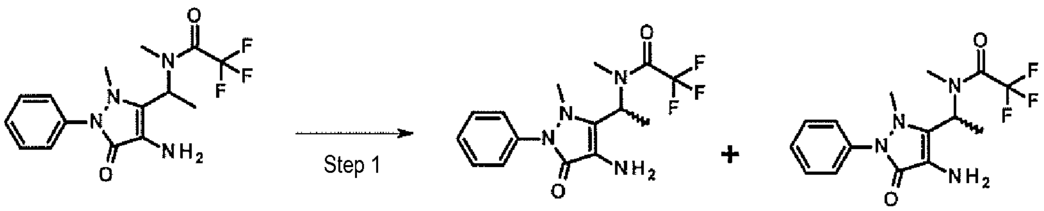


(+)-85a (-)-85b

(Step 1) (+)-N-[1-(4-Amino-2-methyl-5-oxo-1-phenyl-2,3-dihydro-lH-pyrazol-3-yl)ethyl]-2,2,2-trifluoro-N-methylacetamide (intermediate 85a) and (-)-N-[1-(4-amino-2-methyl-5-oxo-1-phenyl-2,3-dihydro-1H-pyrazol-3-yl)ethyl]-2,2,2-trifluoro-N-methylacetamide (intermediate 85b)

N-[1-(4-Amino-2-methyl-5-oxo-1-phenyl-2,3-dihydro-1H-pyrazol-3-yl)ethyl]-2,2,2-trifluoro-N-methylacetamide (5.81 g, 17.0 mmol) synthesized in step 2 of Example 1 was resolved by chiral column chromatography (CHIRALCEL OD-H, ethanol/hexane = 1/1) to obtain each of (+)-N-[1-(4-amino-2-methyl-5-oxo-1-phenyl-2,3-dihydro-1H-pyrazol-3-yl)ethyl]-2,2,2-trifluoro-N-methylacetamide (intermediate 85a) (2.94 g, yield: 50.6%) as a solid and (-)-N-[1-(4-amino-2-methyl-5-oxo-1-phenyl-2,3-dihydro-1H-pyrazol-3-yl)ethyl]-2,2,2-trifluoro-N-methylacetamide (intermediate 85b) (2.71 g, yield: 46.6%) as a solid. Intermediate 85a
$^{1}$H-NMR ($CDCl_3$) δ: 7.48-7.44 (4H, m), 7.30-7.26 (1H, m), 5.77 (1H, q, J = 7.1 Hz), 3.42 (2H, br s), 3.08 (3H, s), 2.78 (3H, s), 1.72 (3H, d, J = 7.1 Hz).
MS (APCI) m/z :343 [(M+H)$^+$].
$[\alpha]_D^{20}$ +211.4 (c = 1.02, MeOH).
Intermediate 85b

$^1$H-NMR ($CDCl_3$) δ: 7.48-7.43 (4H, m), 7.31-7.26 (1H, m), 5.77 (1H, q, J = 7.1 Hz), 3.32 (2H, br s), 3.08 (3H, s), 2.78 (3H, s), 1.72 (3H, d, J= 7.1 Hz).
MS (APCI) m/z :343 [$(M+H)^+$].
[α] $_D^{20}$ -203.1 (c = 1.04, MeOH).

The following compounds of Examples 85 and 86 were subsequently synthesized in the same way as in steps 7 and 8 of Example 66 and are shown in Table 29.

[Table 29]

| Example No. | Structure | Intermediate used | Instrumental analysis data |
|---|---|---|---|
| 8 5 | 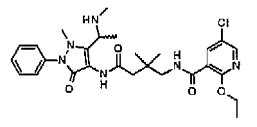 | 1 h<br>8 5 a | $^1$H-NMR ($CDCl_3$) δ 8.78 (1H, brs), 8.47 (1H, d, J = 2.4 Hz), 8.41 (1H, t, J = 6.4 Hz), 8.19 (1H, d, J = 2.4 Hz), 7.47-7.40 (4H, m), 7.29-7.26 (1H, m), 4.55 (2H, q, J = 7.1 Hz), 3.88 (1H, q, J = 6.9 Hz), 3.64 (1H, dd, J = 14.0, 6.4 Hz), 3.57 (1H, dd, J = 14.0, 6.4 Hz), 3.27 (3H, s), 2.47 (3H, s), 2.27 (2H, s), 1.52 (3H, d, J = 6.9 Hz), 1.47 (3H, t, J = 7.1 Hz), 1.14 (6H, s).<br>MS (APCI) m/z :543 [$(M+H)^+$]<br>$[\alpha]_D^{20}$ +23.9 (c = 1.01, MeOH). |
| 8 6 | 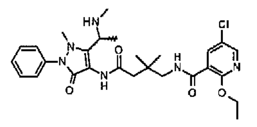 | 1 h<br>8 5 b | $^1$H-NMR ($CDCl_3$) δ: 8.78 (1H, br s), 8.47 (1H, d, J = 2.4 Hz), 8.41 (1H, t, J = 6.7 Hz), 8.19 (1H, d, J = 2.4 Hz), 7.47-7.40 (4H, m), 7.30-7.25 (1H, m), 4.55 (2H, q, J = 7.1 Hz), 3.88 (1H, q, J = 6.9 Hz), 3.64 (1H, dd, J = 14.0, 6.4 Hz), 3.57 (1H, dd, J = 14.0, 6.4 Hz), 3.27 (3H, s), 2.47 (3H, s), 2.27 (2H, s), 1.51 (3H, d, J = 6.9 Hz), 1,47 (3H, t, J = 7.1 Hz), 1.14 (6H, s).<br>MS (APCI) m/z :543 [$(M+H)^+$]<br>$[\alpha]_D^{20}$ -23.6 (c = 1.01, MeOH). |

(Example 87) 5-Chloro-N-[4-({5-[2-(dimethylamino)-1,2-dimethylpropyl]-1-methyl-3-oxo-2-phenyl-2,3-dihydro-1H-pyrazol-4-yl}amino)-2,2-dimethyl-4-oxobutyl]-2-ethoxybenzamide

[Formula 75]

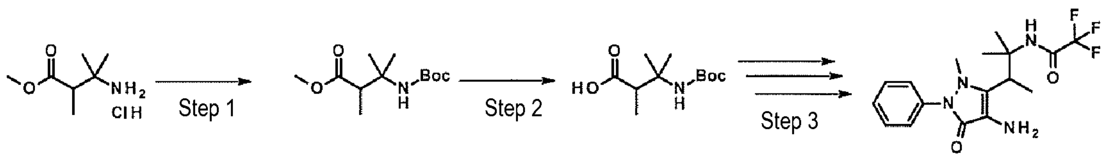


(Step 1) Methyl 3-(tert-butoxycarbonylamino)-2,3-dimethylbutanoate

To a suspension of methyl 3-amino-2,3-dimethylbutanoate hydrochloride (5.00 g, 27.5 mmol) in 1,4-dioxane (75 mL), water (75 mL), sodium carbonate (5.83 g, 55.1 mmol), and di-tert-butyl dicarbonate (7.21 g, 33.0 mmol) were added, and the mixture was stirred at room temperature for 17 hours. The organic solvent was distilled off under reduced pressure, and then, the residue was subjected to extraction with ethyl acetate three times. The organic layer was washed with saturated saline and then dried over anhydrous sodium sulfate. After filtration, the filtrate was concentrated under reduced pressure, and then, the obtained residue was purified by column chromatography (Yamazen Corp., ethyl acetate/hexane = 0/100 to 20/80) to obtain the title compound (5.16 g, yield: 76.4%) as an oil substance.
$^1$H-NMR ($CDCl_3$) δ: 4.86 (1H, s), 3.68 (3H, s), 3.09 (1H, q, J = 7.3 Hz), 1.43 (9H, s), 1.35 (3H, s), 1.32 (3H, s),, 1.14 (3H, d, J= 7.3 Hz).

(Step 2) 3-(tert-Butoxycarbonylamino)-2,3-dimethylbutanoic acid

To a solution of methyl 3-(tert-butoxycarbonylamino)-2,3-dimethylbutanoate (5.16 g, 21.0 mmol) synthesized in step 1 in tetrahydrofuran (130 mL), methanol (63 mL) and a 1 N aqueous sodium hydroxide solution (63.1 mL, 63.1 mmol) were added, and the mixture was stirred at room temperature for 88 hours. The organic solvent was distilled off under reduced pressure, then water was added to the residue, and the mixture was washed with diethyl ether twice. The aqueous layer was rendered acidic by the addition of 1 N hydrochloric acid, followed by extraction with ethyl acetate three times. The organic layer was washed with saturated saline and then dried over anhydrous sodium sulfate. After filtration, the filtrate was concentrated under reduced pressure to obtain the title compound (4.03 g, yield: 82.8%) as a solid.
$^1$H-NMR ($CDCl_3$) δ: 3.17 (1H, q, J= 7.3 Hz), 1.46 (9H, s), 1.37 (3H, s), 1.33 (3H, s), 1.15 (3H, d, J = 7.3 Hz).

(Step 3) N-[2-(4-Amino-2-methyl-5-oxo-1-phenyl-2,3-dihydro-1H-pyrazol-3-yl)-1,1-dimethylpropyl]-2,2,2-trifluoroacetamide

The title compound was obtained as a solid through the same reaction as in Example 51 using 3-(tert-butoxycarbonylamino)-2,3-dimethylbutanoic acid synthesized in step 2.
$^1$H-NMR ($CDCl_3$) δ: 9.01 (1H, s), 7.49-7.40 (4H, m), 7.32-7.27 (1H, m), 2.99-2.83 (6H, m), 1.64 (3H, s), 1.53-1.50 (6H, m).
MS (APCI) m/z : 371 [(M+H)$^+$].
The following compound of Example 87 was subsequently synthesized in the same way as in steps 7 to 9 of Example 66 and is shown in Table 30.

[Table 30]

| Example No. | Structure | Intermediate used | Instrumental analysis data |
|---|---|---|---|
| 8 7 | 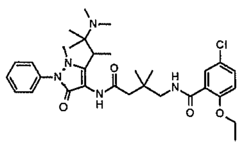 | 1 a | $^1$H-NMR ($CDCl_3$) δ: 9.12 (1H, s), 8.38 (1H, t, J = 6.4 Hz), 8.13 (1H, d, J = 3.0 Hz), 7.45-7.36 (5H, m), 7.26-7.22 (1H, m), 6.90 (1H, d, J = 9.1 Hz), 4.19 (2H, q, J = 7.0 Hz), 3.72 (1H, dd, J = 14.3, 7.3 Hz), 3.46 (1H, dd, J = 14.3, 7.0 Hz), 3.18-3.08 (4H, m), 2.31 (6H, s), 2.26-2.18 (2H, m), 1.52 (3H, t, J = 7.0 Hz), 1.43 (3H, d, J = 7.3 Hz), 1.21-1.11 (12H, m). MS (APCI) m/z :598 [(M+H)$^+$]. |

(Example 88) 5-Chloro-N-[4-({5-[4-(dimethylamino)butyl]-1-methyl-3-oxo-2-phenyl-2,3-dihydro-1H-pyrazol-4-yl}amino)-2,2-dimethyl-4-oxobutyl]-2-ethoxybenzamide

[Formula 76]

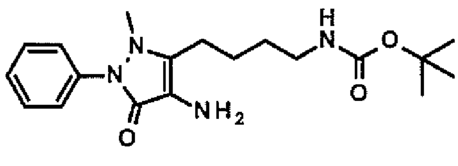

(Step 1) tert-Butyl N-[4-(4-amino-2-methyl-5-oxo-1-phenyl-2,3-dihydro-1H-pyrazol-3-yl)butyl]carbamate

The title compound was obtained as a solid by using 5-(tert-butoxycarbonylamino)pentanoic acid instead of 3-(tert-butoxycarbonylamino)propanoic acid in step 1 of Example 10 and subsequently performing the same reaction as in Example 10 up to step 6.
$^1$H-NMR ($CDCl_3$) δ: 7.50-7.40 (4H, m), 7.28-7.21 (1H, m), 4.62 (1H, br s), 3.26-3.16 (2H, m), 3.07 (2H, br s), 2.83 (3H, s), 2.57 (2H, t, J = 6.7 Hz), 1.74-1.55 (4H, m), 1.45 (9H, s).
The following compounds of Examples 88 to 90 were subsequently synthesized in the same way as in step 7 of Example 10 and steps 1 and 2 of Example 11 and are shown in Table 31.

[Table 31]

| Example No. | Structure | Intermediate used | Instrumental analysis data |
|---|---|---|---|
| 8 8 | 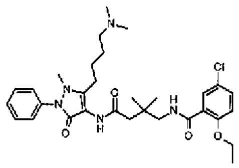 | 1 a | $^1$H-NMR (CDCl$_3$) δ: 9.02 (1H, br s), 8.41-8.35 (1H, m), 8.20 (1H, d, J = 3.1 Hz), 7.47-7.40 (4H, m), 7.38 (1H, dd, J = 8.5, 3.1 Hz), 7.29-7.24 (1H, m), 6.91 (1H, d, J = 8.5 Hz), 4.20 (2H, q, J = 6.7 Hz), 3.58 (2H, d, J = 6.7 Hz), 3.09 (3H, s), 2.68 (2H, t, J = 7.3 Hz), 2.32 (2H, t, J = 7.3 Hz), 2.27 (2H, s), 2.22 (6H, s), 1.78-1.69 (2H, m), 1.64-1.55 (2H, m), 1.52 (3H, t, J = 6.7 Hz), 1.13 (6H, s).<br>MS (ESI+APCI) m/z : 584 [(M+H)$^+$]. |
| 8 9 | 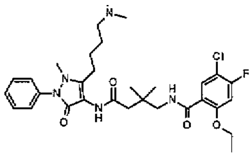 | 1 c | $^1$H-NMR (CDCl$_3$) δ: 8.85 (1H, s), 8.30 (1H, d, J = 8.5 Hz), 8.27-8.21 (1H, m), 7.48-7.40 (4H, m), 7.30-7.22 (1H, m), 6.78 (1H, d, J = 10.4 Hz), 4.18 (2H, q, J = 6.7 Hz), 3.57 (2H, d, J = 6.7 Hz), 3.09 (3H, s), 2.69 (2H, t, J = 7.3 Hz), 2.32 (2H, t, J = 7.3 Hz), 2.27 (2H, s), 2.22 (6H, s), 1.78-1.69 (2H, m), 1.65-1.51 (2H, m), 1.54 (3H, t, J = 6.7 Hz), 1.13 (6H, s).<br>MS (APCI) m/z : 602 [(M+H)$^+$]. |
| 9 0 | 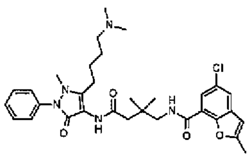 | 3 a | $^1$H-NMR (CDCl$_3$) δ 8.46 (1H, s), 7.96 (1H, d, J = 2.4 Hz), 7.95-7.92 (1H, m), 7.56 (1H, d, J = 2.4 Hz), 7.47-7.39 (4H, m), 7.30-7.25 (1H, m), 6.44-6.42 (1H, m), 3.65 (2H, d, J = 6.7 Hz), 3.09 (3H, s), 2.69 (2H, t, J = 7.9 Hz), 2.51 (3H, s), 2.34 (2H, s), 2.30 (2H, t, J = 7.3 Hz), 2.21 (6H, s), 1.77-1.68 (2H, m), 1.62-1.52 (2H, m), 1.18 (6H, s).<br>MS (ESI+APCI) m/z 594 [(M+H)$^+$]. |

(Example 91) 5-Chloro-N-[4-({5-[2-(dimethylamino)ethyl]-1-methyl-3-oxo-2-phenyl-2,3-dihydro-1H-pyrazol-4-yl}amino)-3-fluoro-2,2-dimethyl-4-oxobutyl]-2-ethoxybenzamide

[Formula 77]

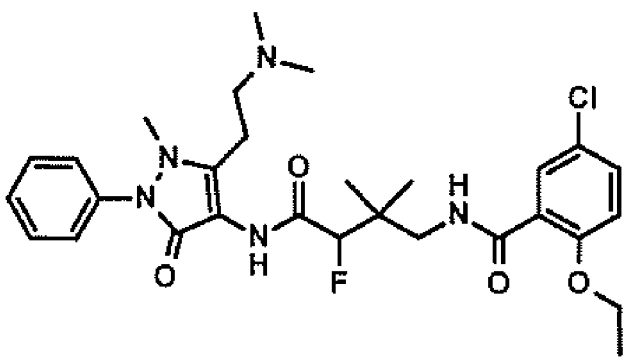

The title compound was synthesized by using intermediate 1n instead of intermediate 1e in step 6 of Example 28 and subsequently performing the same reaction as in Example 28 up to step 8.
$^1$H-NMR (CDCl$_3$) δ: 8.49 (1H, br d, J= 3.6 Hz), 8.28 (1H, br t, J = 6.4 Hz), 8.17 (1H, d, J = 3.0 Hz), 7.49-7.34 (5H, m), 7.32-7.27 (1H, m), 6.89 (1H, d, J = 9.1 Hz), 4.84 (1H, d, J= 48.0 Hz), 4.18 (2H, q, J = 6.9 Hz), 3.79-3.71 (1H, m), 3.50 (1H, dd, J = 14.0, 6.1 Hz), 3.11 (3H, s), 2.82 (2H, t, J = 7.3 Hz), 2.65 (2H, t, J = 7.3 Hz), 2.31 (6H, s), 1.51 (3H, t, J = 7.0 Hz), 1.20 (3H, s), 1.17 (3H, s).

MS (ESI) m/z : 574 [$(M+H)^+$].

(Example 92) 5-Chloro-N-{4-[(1,5-dimethyl-3-oxo-2-phenyl-2,3-dihydro-1H-pyrazol-4-yl)amino]-2,2-dimethyl-4-oxobutyl}-2-ethoxy-4-fluorobenzamide

[Formula 78]

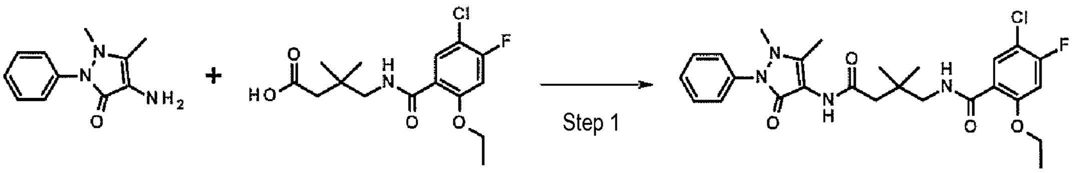


(Step 1) 5-Chloro-N-{4-[(1,5-dimethyl-3-oxo-2-phenyl-2,3-dihydro-1H-pyrazol-4-yl)amino]-2,2-dimethyl-4-oxobutyl}-2-ethoxy-4-fluorobenzamide

4-Aminoantipyrine (0.203 g, 1.00 mmol) and intermediate 1c (0.332 g, 1.00 mmol) were dissolved in ethanol (10 mL). To the solution, 4-(4,6-dimethoxy-1,3,5-triazin-2-yl)-4-methylmorpholinium chloride n-hydrate (DMT-MM) (0.354 g, 1.20 mmol) was added, and the mixture was stirred at room temperature for 5 hours. The solvent in the reaction solution was distilled off under reduced pressure, and water and a saturated aqueous solution of sodium bicarbonate were added to the residue, followed by extraction with methylene chloride. The organic layer was washed with 1 N hydrochloric acid and saturated saline and then dried over anhydrous magnesium sulfate. After filtration, the filtrate was concentrated, and the obtained crude product was purified by amino silica-silica gel column chromatography (Biotage Japan Ltd., eluting solvent: hexane/ethyl acetate → ethyl acetate/methanol = 30/70 to 0/100 → 100/0 to 80/20) to obtain the title compound (0.220 g, yield: 42.6%) as a solid.
$^1$H-NMR ($CDCl_3$) δ: 8.80 (1H, s), 8.31 (1H, d, J = 8.8 Hz), 8.22 (1H, t, J = 6.6 Hz), 7.46-7.41 (4H, m), 7.28-7.27 (1H, m), 6.78 (1H, d, J = 10.7 Hz), 4.18 (2H, t, J = 7.1 Hz), 3.58 (2H, d, J= 6.6 Hz), 3.09 (3H, s), 2.27 (5H, s), 1.54 (3H, t, J = 7.1 Hz), 1.12 (6H, s).
MS (ESI) m/z : 517 [$(M+H)^+$].

(Example 93) 5-Chloro-N-{4-[(1,5-dimethyl-3-oxo-2-phenyl-2,3-dihydro-1H-pyrazol-4-yl)amino-2,2-dimethyl-4-oxobutyl]-2-ethoxybenzamide

[Formula 79]

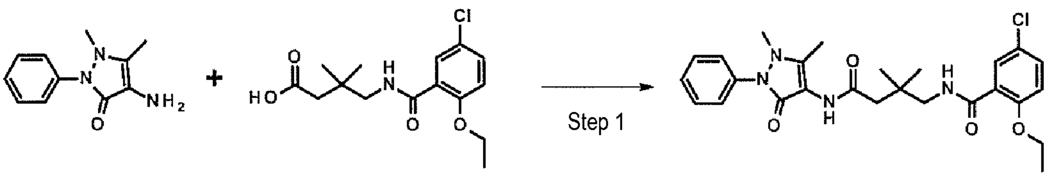


(Step 1) 5-Chloro-N-{4-[(1,5-dimethyl-3-oxo-2-phenyl-2,3-dihydro-1H-pyrazol-4-yl)amino]-2,2-dimethyl-4-oxobutyl}-2-ethoxybenzamide

4-Aminoantipyrine (0.142 g, 0.701 mmol) and intermediate 1a (0.200 g, 0.637 mmol) were dissolved in N,N-dimethylformamide (3.0 mL). To the solution, 0-(7-azabenzotriazol-1-yl)-N,N,N',N'-tetramethyluronium hexafluorophosphate (HATU) (0.315 g, 0.828 mmol) and N,N-diisopropylethylamine (0.167 mL, 0.123 g, 0.959 mmol) were added, and the mixture was stirred at room temperature for 3 hours. Water and a saturated aqueous solution of sodium bicarbonate were added to the reaction solution, followed by extraction with ethyl acetate. The organic layer was washed with water and saturated saline and then dried over anhydrous magnesium sulfate. After filtration, the filtrate was concentrated, and the obtained residue was purified by amino silica-silica gel column chromatography (Shoko Scientific Co., Ltd., eluting solvent: methylene chloride/methanol = 99/1 to 90/10) to obtain the title compound (0.272 g, yield: 85.6%) as a solid.

$^1$H-NMR ($CDCl_3$) δ: 9.01 (1H, br s), 8.39 (1H, t, J = = 6.7 Hz), 8.23 (1H, d, J= 2.7 Hz), 7.48-7.43 (4H, m), 7.39 (1H, dd, J = 8.6, 2.7 Hz), 7.30-7.26 (1H, m), 6.93 (1H, d, J = 8.6 Hz), 4.22 (2H, q, J = 6.9 Hz), 3.61 (2H, d, J = 6.7 Hz), 3.11 (3H, s), 2.30 (2H, s), 2.29 (3H, s), 1.54 (3H, t, J= 6.9 Hz), 1.14 (6H, s).
MS (ESI) m/z : 499 [(M+H)$^+$].

The following compounds of Examples 94 to 107 were synthesized in the same way as in Example 92 or 93 and are shown in Table 32.

[Table 32-1]

| Example No. | Structure | Intermediate used | Instrumental analysis data |
|---|---|---|---|
| 9 4 | 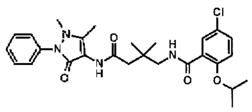 | 1 k | $^1$H-NMR ($CDCl_3$) δ:9.09 (1H, s), 8.44 (1H, t, J = 6.9 Hz), 8.23 (1H, d, J = 2.9 Hz), 7.43-7.42 (4H, m), 7.37 (1H, dd, J = 8.6, 2.9 Hz), 7.27-7.24 (1H, m), 6.92 (1H, d, J = 8.6 Hz), 4.76-4.73 (1H, hp, J = 6.3 Hz), 3.59 (2H, d, J = 6.9 Hz), 3.09 (3H, s), 2.27 (5H, s), 1.44 (6H, d, J = 6.3 Hz), 1.12 (6H, s). |
| 9 5 | 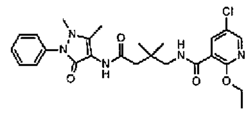 | 1 h | $^1$H-NMR ($CDCl_3$) δ: 8.68 (1H, br s), 8.50 (1H, d, J = 2.9 Hz), 8.41 (1H, t, J = 6.3 Hz), 8.19 (1H, d, J = 2.4 Hz), 7.46-7.41 (4H, m), 7.29-7.25 (1H, m), 4.55 (2H, q, J = 7.2 Hz), 3.57 (2H, d, J = 6.3 Hz), 3.09 (3H, s), 2.27 (5H, s), 1.48 (3H, t, J = 7.1 Hz), 1,12 (6H, s).<br>MS (ESI+APCI) m/z : 500 [(M+H)$^+$]. |
| 9 6 | 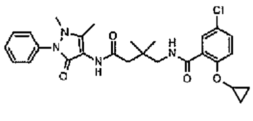 | 1 l | $^1$H-NMR ($CDCl_3$) δ: 8.80 (1H, s), 8.17 (1H, d, J = 2.9 Hz), 8.09 (1H, t, J = 6.9 Hz), 7.46-7.38 (5H, m), 7.32 (1H, d, J = 9.2 Hz), 7.28-7.25 (1H, m), 3.89-3.85 (1H, m), 3.55 (2H, d, J = 6.9 Hz), 3.09 (3H, s), 2.27-2.26 (5H, m), 1.10 (6H, s), 0.91-0.86 (4H, m).<br>MS (API) m/z: 511 [(M+H)$^+$] |
| 9 7 | 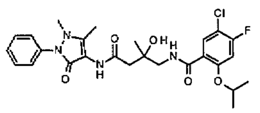 | 1 m | $^1$H-NMR ($CDCl_3$) δ: 8.66 (1H, br s), 8.43 (1H, br t, J = 5.4 Hz), 8.28 (1H, d, J = 9.2 Hz), 7.51-7.45 (2H, m), 7.40-7.31 (3H, m), 6.77 (1H, d, J = 10.3 Hz), 5.16 (1H, s), 4.69-4.62 (1H, m), 3.47 (2H, d, J = 5.7 Hz), 3.11 (3H, s), 2.57 (1H, d, J = 14.9 Hz), 2.43 (1H, d, J = 14.9 Hz), 2.22 (3H, s), 1.44 (6H, d, J = 5.7 Hz), 1.26 (3H, s).<br>MS (ESI) m/z : 533 [(M+H)$^+$]. |
| 9 8 | 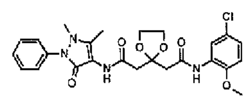 | 2 i | $^1$H-NMR (CDCl3) δ: 8.72 (1H, s), 8.41 (1H, d, J = 2.3 Hz), 7.99 (1H, s), 7.46 (2H, t, J = 7.8 Hz), 7.38 (2H, d, J = 7.8 Hz), 7.30 (1H, t, J = 7.8 Hz), 6.99 (1H, dd, J = 8.8, 2.3 Hz), 6.75 (1H, d, J = 8.8 Hz), 4.27-4.23 (2H, m), 4.11-4.06 (2H, m), 3.83 (3H, s), 3.08 (3H, s), 2.98 (2H, s), 2.89 (2H, s), 2.27 (3H, s).<br>MS (ESI) m/z: 515 [(M+H)$^+$] |

[Table 32-2]

| | | | |
|---|---|---|---|
| 9 9 | 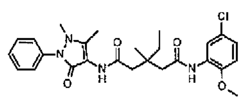 | 2 j | $^1$H-NMR ($CDCl_3$) δ: 8.52 (1H, br s), 8.45 (1H, br s), 8.38 (1H, d, J = 2.6 Hz), 7.45-7.42 (2H, m), 7.40-7.38 (2H, m), 7.28-7.25 (1H, m), 7.00 (1H, dd, J = 8.9, 2.6 Hz), 6.76 (1H, d, J = 8.9 Hz), 3.83 (3H, s), 3.09 (3H, s), 2.68 (2H, dd, J = 17.2, 13.7 Hz), 2.50 (2H, q, J = 13.0 Hz), 2.28 (3H, s), 1.52-1.45 (2H, m), 1.09 (3H, s), 0.96 (3H, t, J = 7.4 Hz).<br>MS (ESI) m/z : 497 [(M+H)$^+$]. |

(continued)

| | | | |
|---|---|---|---|
| 1 0 0 | 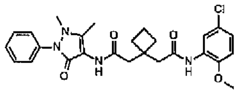 | 2 k | $^1$H-NMR ($CDCl_3$) δ: 8.82 (1H, br s), 8.20 (1H, d, J = 2.3 Hz), 8.05 (1H, br s), 7.46-7.43 (2H, m), 7.39-7.37 (2H, m), 7.30-7.27 (1H, m), 7.01 (1H, dd, J = 8.6, 2.3 Hz), 6.75 (1H, d, J = 8.6 Hz), 3.78 (3H, s), 3.10 (3H, s), 2.87 (2H, s), 2.69 (2H, s), 2.27 (3H, s), 2.13-2.07 (4H, m), 2.04-1.98 (2H, m). MS (ESI) m/z : 495 [(M+H)$^+$]. |
| 1 0 1 | 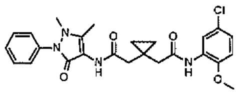 | 2 l | $^1$H-NMR ($CDCl_3$) δ: 8.72 (1H, br s), 8.31 (1H, d, J = 2.9 Hz), 8.08 (1H, br s), 7.47-7.43 (2H, m), 7.40-7.36 (2H, m), 7.32-7.28 (1H, m), 6.99 (1H, dd, J = 8.9, 2.6 Hz), 6.74 (1H, d, J = 8.6 Hz), 3.78 (3H, s), 3.10 (3H, s), 2.55 (2H, s), 2.39 (2H, s), 2.27 (3H, s), 0.69-0.64 (4H, m). MS (ESI) m/z : 483 [(M+H)$^+$]. |
| 1 0 2 | 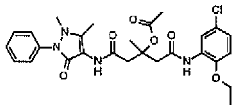 | 2 m | $^1$H-NMR ($CDCl_3$) δ: 8.46 (1 H, br s), 8.41 (1H, br s), 8.39 (1H, d, J = 2.3 Hz), 7.47-7.42 (2H, m), 7.41-7.37 (2H, m), 7.31-7.26 (1H, m), 6.99 (1H, dd, J = 8.6, 2.9 Hz), 6.76 (1H, d, J = 8.6 Hz), 4.07 (2H, q, J = 7.1 Hz), 3.39 (1H, d, J = 13.7 Hz), 3.25-3.19 (2H, m), 3.09 (3H, s), 2.85 (1H, d, J = 13.2 Hz), 2.27 (3H, s), 2.03 (3H, s), 1.75 (3H, s), 1.44 (3H, t, J = 7.2 Hz). MS (ESI) m/z : 543 [(M+H)$^+$]. |
| 1 0 3 | 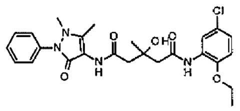 | 2 n | $^1$H-NMR ($CDCl_3$) δ: 9.41 (1H, br s), 8.87 (1H, br s), 8.36 (1H, d, J = 2.3 Hz), 7.52-7.47 (2H, m), 7.40-7.33 (3H, m), 6.96 (1H, dd, J = 8.6, 2.3 Hz), 6.74 (1H, d, J = 9.2 Hz), 5.61 (1H, br s), 4.03 (2H, q, J = 6.9 Hz), 3.13 (3H, s), 2.64 (1H, d, J = 14.9 Hz), 2.59 (1H, d, J = 14.3 Hz), 2.51 (1H, d, J = 8.0 Hz), 2.48 (1H, d, J = 8.0 Hz), 2.22 (3H, s), 1.40 (3H, t, J = 6.9 Hz), 1.31 (3H, s). MS (ESI) m/z : 501 [(M+H)$^+$]. |

[Table 32-3]

| | | | |
|---|---|---|---|
| 1 0 4 | 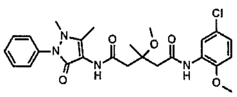 | 2 o | $^1$H-NMR ($CDCl_3$) δ: 8.88 (1H, br s), 8.47 (1H, br s), 8.39 (1H, d, J = 2.3 Hz), 7.49-7.37 (4H, m), 7.31-7.26 (1H, m), 6.98 (1H, dd, J = 8.9, 2.6 Hz), 6.75 (1H, d, J = 8.6 Hz), 3.82 (3H, s), 3.36 (3H, s), 3.09 (3H, s), 2.99 (1H, d, J = 13.7 Hz), 2.79 (1H, d, J = 13.7 Hz), 2.76 (1H, d, J = 12.6 Hz), 2.68 (1H, d, J = 13.7 Hz), 2.27 (3H, s), 1.43 (3H, s). MS (ESI) m/z : 501 [(M+H)$^+$]. |
| 1 0 5 | 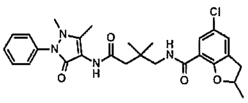 | 3 b | $^1$H-NMR ($CDCl_3$) δ: 8.88 (1H, s), 7.96 (1H, t, J = 6.6 Hz), 7.90 (1H, d, J = 2.3 Hz), 7.44-7.42 (4H, m), 7.28-7.24 (1H, m), 7.23-7.23 (1H, m), 5.16-5.12 (1H, m), 3.67-3.64 (1H, m), 3.50-3.47 (1H, m), 3.38-3.35 (1H, m), 3.08 (3H, s), 2.88-2.85 (1H, m), 2.28-2.24 (5H, m), 1.53 (3H, d, J = 6.3 Hz), 1.11 (3H, s), 1.11 (3H, s). MS (ESI) m/z : 511 [(M+H)$^+$]. |
| 1 0 6 | 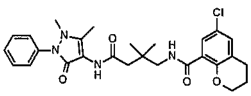 | 3 d | $^1$H-NMR ($CDCl_3$) δ: 8.91 (1H, s), 8.35 (1H, t, J = 6.9 Hz), 7.99 (1H, d, J = 2.9 Hz), 7.46-7.40 (4H, m), 7.28-7.26 (1H, m), 7.12 (1H, d, J = 2.9 Hz), 4.33 (2H, t, J = 5.2 Hz), 3.54 (2H, d, J = 6.9 Hz), 3.09 (3H, s), 2.81 (2H, t, J = 6.6 Hz), 2.27 (2H, s), 2.26 (3H, s), 2.05-2.03 (2H, m), 1.10 (6H, s). MS (ESI) m/z : 511 [(M+H)$^+$]. |
| 1 0 7 | 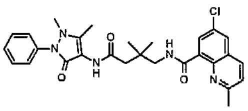 | 3 f | $^1$H-NMR ($CDCl_3$) δ: 11.87 (1H, t, J = 6.3 Hz), 9.31 (1H, s), 8.79 (1H, d, J = 2.4 Hz), 8.09 (1H, d, J = 8.3 Hz), 7.89 (1H, d, J = 2.4 Hz), 7.45-7.41 (4H, m), 7.39 (1H, d, J = 8.3 Hz), 7.26-7.24 (1H, m), 3.75 (2H, d, J = 6.3 Hz), 3.09 (3H, s), 2.77 (3H, s), 2.33 (2H, s), 2.29 (3H, s), 1.22 (6H, s). MS (ESI) m/z : 520 [(M+H)$^+$]. |

(Example 108) 5-Chloro-N-{4-[(1,5-dimethyl-3-oxo-2-phenyl-2,3-dihydro-1H-pyrazol-4-yl)amino]-2,2-dimethyl-4-oxobutyl}-2-(oxetan-3-yloxy)benzamide

[Formula 80]

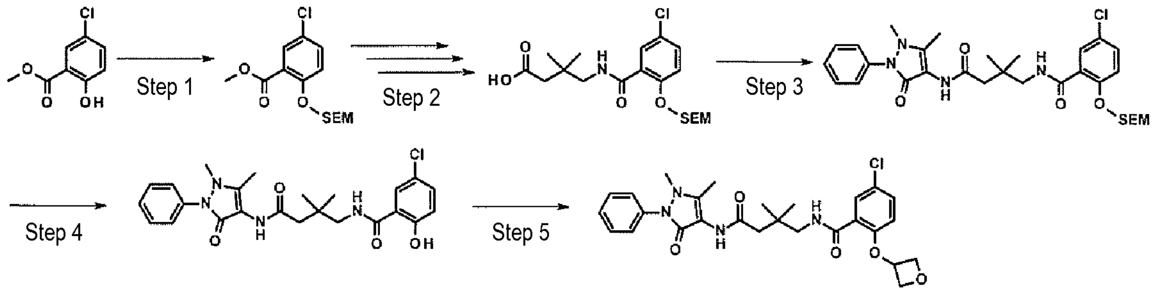


(Step 1) Methyl 5-chloro-2-(2-trimethylsilylethoxymethoxy)benzoate

Methyl 5-chlorosalicylate (0.933 g, 5.00 mmol) was dissolved in methylene chloride (8.0 mL). To the solution, 2-(chloromethoxy)ethyltrimethylsilane (1.77 mL, 1.67 g, 10.0 mmol) was added, and the mixture was stirred at 0°C. N,N-Diisopropylethylamine (2.83 mL, 2.02 g, 20.0 mmol) was gradually added thereto. After the completion of the addition, the mixture was stirred at room temperature for 15 hours. Water was added to the reaction solution, followed by extraction with methylene chloride. The organic layer was dried over anhydrous magnesium sulfate. After filtration, the filtrate was concentrated, and the obtained crude product was purified by silica gel column chromatography (Biotage Japan Ltd., eluting solvent: hexane/ethyl acetate = 100/0 to 85/15) to obtain the title compound (0.747 g, yield: 47.2%) as an oil substance.
$^1$H-NMR ($CDCl_3$) δ: 7.75 (1H, d, J = 2.9 Hz), 7.39 (1H, dd, J = 8.8, 2.9 Hz), 7.19 (1H, d, J = 8.8 Hz), 5.28 (2H, s), 3.89 (3H, s), 3.79 (2H, t, J= 8.3 Hz), 0.95 (2H, t, J = 8.3 Hz), 0.00 (9H, s).

(Step 2) 4-{[5-Chloro-2-(2-trimethylsilylethoxymethoxy)benzoyl]amino}-3,3-dimethylbutanoic acid

The title compound was subsequently obtained as a solid through the same reaction as in steps 4 to 6 in the synthesis of intermediate 1a using methyl 5-chloro-2-(2-trimethylsilylethoxymethoxy)benzoate synthesized in step 1.
$^1$H-NMR ($CDCl_3$) δ: 8.39 (1H, t, J = 6.8 Hz), 8.19-8.19 (1H, m), 7.43-7.41 (1H, m), 7.19 (1H, d, J = 8.8 Hz), 5.39 (2H, s), 3.77 (2H, t, J = 8.3 Hz), 3.43 (2H, d, J= 6. Hz), 2.28 (2H, s), 1.11 (6H, s), 0.97 (2H, t, J = 8.3 Hz), 0.00 (9H, s).

(Step 3) 5-Chloro-N-{4-[(1,5-dimethyl-3-oxo-2-phenyl-2,3-dihydro-1H-pyrazol-4-yl)amino]-2,2-dimethyl-4-oxobutyl}-2-(2-trimethylsilylethoxymethoxy)benzamide

The title compound was obtained as a solid through the same reaction as in step 1 of Example 2 using 4-{[5-chloro-2-(2-trimethylsilylethoxymethoxy)benzoyl]amino}-3,3-dimethylbutanoic acid synthesized in step 2 instead of 4-[(5-chloro-2-ethoxybenzoyl)amino]-3,3-dimethylbutanoic acid.
$^1$H-NMR ($CDCl_3$) δ: 8.60 (1H, s), 8.32 (1H, t, J = 6.6 Hz), 8.19 (1H, d, J = 2.0 Hz), 7.47-7.42 (4H, m), 7.38 (1H, dd, J= 8.8, 2.0 Hz), 7.30-7.28 (1H, m), 7.18 (1H, d, J = 8.8 Hz), 5.39 (2H, s), 3.78 (2H, t, J = 8.1 Hz), 3.58 (2H, d, J= 6.6 Hz), 3.10 (3H, s), 2.29 (2H, s), 2.28 (3H, s), 1.13 (6H, s), 0.96 (2H, t, J = 8.1 Hz), 0.01 (9H, s).

(Step 4) 5-Chloro-N-{4-[(1,5-dimethyl-3-oxo-2-phenyl-2,3-dihydro-1H-pyrazol-4-yl)amino]-2,2-dimethyl-4-oxobutyl}-2-hydroxybenzamide

5-Chloro-N-{4-[(1,5-dimethyl-3-oxo-2-phenyl-2,3-dihydro-1H-pyrazol-4-yl)amino]-2,2-dimethyl-4-oxobutyl}-2-(2-trimethylsilylethoxymethoxy)benzamide (1.26 g, 2.11 mmol) synthesized in step 3 was dissolved in tetrahydrofuran (10 mL). To the solution, tetra-n-butyl ammonium fluoride (1.0 M solution in tetrahydrofuran) (6.0 mL) was added, and the mixture was stirred at room temperature for 3 hours and then stirred at 60°C for 3 hours. The reaction solution was brought back to room temperature, and the solvent was distilled off under reduced pressure. To the obtained residue, water was added, followed by extraction with ethyl acetate. The organic layer was washed with saturated saline and then dried over anhydrous magnesium sulfate. After filtration, the filtrate was concentrated, and the obtained crude

product was purified by silica gel column chromatography (Biotage Japan Ltd., eluting solvent: hexane/ethyl acetate/methylene chloride → ethyl acetate/methanol/methylene chloride = 67/28/5 to 0/95/5 → 95/0/5 to 76/19/5) to obtain the title compound (0.728 g, yield: 73.3%).
$^1$H-NMR ($CDCl_3$) δ: 12.75 (1H, s), 9.01 (1H, br s), 8.64 (1H, s), 7.67 (1H, d, J = 2.4 Hz), 7.46 (2H, t, J = 7.8 Hz), 7.36 (2H, d, J = 7.8 Hz), 7.32 (1H, t, J = 7.8 Hz), 7.28 (1H, dd, J = 8.8, 2.4 Hz), 6.90 (1H, d, J = 8.8 Hz), 3.38 (2H, d, J = 5.4 Hz), 3.12 (3H, s), 2.42 (2H, s), 2.25 (3H, s), 1.05 (6H, s).
MS (ESI) m/z : 471 [$(M+H)^+$].

(Step 5) 5-Chloro-N-{4-[(1,5-dimethyl-3-oxo-2-phenyl-2,3-dihydro-1H-pyrazol-4-yl)amino]-2,2-dimethyl-4-oxobutyl}-2-(oxetan-3-yloxy)benzamide

5-Chloro-N-{4-[(1,5-dimethyl-3-oxo-2-phenyl-2,3-dihydro-1H-pyrazol-4-yl)amino]-2,2-dimethyl-4-oxobutyl}-2-hydroxybenzamide (0.131 g, 0.27 mmol) synthesized in step 4 was dissolved in N,N-dimethylformamide (4.0 mL). To the solution, cesium carbonate (0.132 g, 0.41 mmol) and 3-tosyloxyoxetane (0.124 g, 0.54 mmol) were added, and the mixture was reacted at 65°C for 6 hours. 3-Tosyloxyoxetane (0.100 g, 0.44 mmol) was further added thereto, and the mixture was reacted at 80°C for 20 hours. The reaction solution was brought back to room temperature, and water was added to the reaction solution, followed by extraction with ethyl acetate. The organic layer was washed with saturated saline and then dried over anhydrous magnesium sulfate. After filtration, the filtrate was concentrated, and the obtained crude product was purified by amino silica gel column chromatography (Biotage Japan Ltd., eluting solvent: hexane/ethyl acetate/methylene chloride → ethyl acetate/methanol/methylene chloride = 67/28/5 to 0/95/5 → 95/0/5 to 81/14/5) and concentrated, and the obtained solid was suspended in ethyl acetate, then collected by filtration, and dried to obtain the title compound (0.096 g, yield: 67.4%) as a solid.
$^1$H-NMR ($CDCl_3$) δ: 8.44 (1H, s), 8.21 (1H, d, J = 2.7 Hz), 8.17 (1H, t, J = 6.8 Hz), 7.46-7.42 (4H, m), 7.35-7.33 (1H, m), 7.29-7.28 (1H, m), 6.40 (1H, d, J = 8.8 Hz), 5.35-5.30 (1H, m), 5.02-5.01 (2H, m), 4.85-4.83 (2H, m), 3.58 (2H, d, J = 6.8 Hz), 3.09 (3H, s), 2.30 (2H, s), 2.27 (3H, s), 1.13 (6H, s).
MS (ESI) m/z : 527 [$(M+H)^+$].

(Example 109) 6-Chloro-N-{4-[1,5-dimethyl-3-oxo-2-phenyl-2,3-dihydro-1H-pyrazol-4-yl]amino}-2,2-dimethyl-4-oxobutyl]-2-methyl-3,4-dihydro-2H-1,4-benzoxazine-8-carboxamide

[Formula 81]

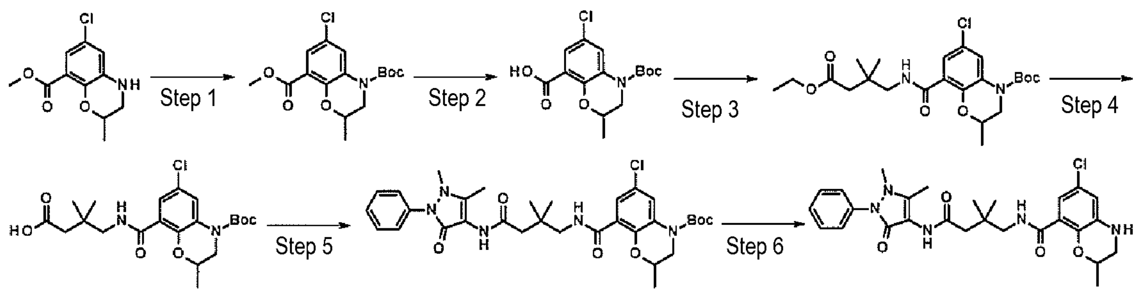


(Step 1) O4-tert-Butyl O8-methyl 6-chloro-2-methyl-2,3-dihydro-1,4-benzoxazine-4,8-dicarboxylate

To a solution of methyl 6-chloro-2-methyl-3,4-dihydro-2H-1,4-benzoxazine-8-carboxylate (0.485 g, 2.01 mmol) synthesized according to the method described in Chem. & Pharm. Bull., 1996, 44, 11, 2051-2060 in tetrahydrofuran (10 mL), di-tert-butyl dicarbonate (0.526 g, 2.41 mmol) and 4-dimethylaminopyridine (0.0245 g, 0.201 mmol) were added, and the mixture was stirred overnight at room temperature. The reaction solution was concentrated under reduced pressure, and then, the obtained residue was purified by silica gel column chromatography (Yamazen Corp., eluting solvent: hexane/ethyl acetate = 95/5 to 74/26) to obtain the title compound (0.421 g, yield: 61.4%) as a solid.
$^1$H-NMR ($CDCl_3$) δ: 7.97 (1H, s), 7.46 (1H, d, J = 3.1 Hz), 4.39-4.32 (1H, m), 4.11 (1H, d, J = 13.4 Hz), 3.88 (3H, s), 3.27 (1H, dd, J = 13.4, 7.9 Hz), 1.54 (9H, s), 1.41 (3H, d, J = 6.1 Hz).
MS (ESI) m/z : 342 [$(M+H)^+$].

(Step 2) 4-tert-Butoxycarbonyl-6-chloro-2-methyl-2,3-dihydro-1,4-benzoxazine-8-carboxylic acid

To a solution of O4-tert-butyl 08-methyl 6-chloro-2-methyl-2,3-dihydro-1,4-benzoxazine-4,8-dicarboxylate (0.415 g, 1.21 mmol) synthesized in step 1 in tetrahydrofuran (8 mL), methanol (3.7 mL) and a 1 N aqueous sodium hydroxide solution (3.64 mL, 3.64 mmol) were added, and the mixture was stirred overnight at room temperature. 1 N hydrochloric acid was added to the reaction solution, followed by extraction with ethyl acetate three times. The organic layer was washed with saturated saline and then dried over anhydrous sodium sulfate. After filtration, the filtrate was concentrated under reduced pressure to obtain the title compound (0.397 g, yield: 95.7%) as a solid.
$^1$H-NMR ($CDCl_3$) $\delta$: 8.05 (1H, s), 7.86 (1H, d, J = 2.4 Hz), 4.59-4.51 (1H, m), 4.26 (1H, dd, J = 13.7, 2.4 Hz), 3.33 (1H, dd, J = 13.7, 8.2 Hz), 1.56 (9H, s), 1.53 (3H, d, J = 6.1 Hz).
MS (ESI) m/z : 328 [$(M+H)^+$].

(Step 3) tert-Butyl 6-chloro-8-[(4-ethoxy-2,2-dimethyl-4-oxobutyl)carbamoyl]-2-methyl-2,3-dihydro-1,4-benzoxazine-4-carboxylate

The title compound was obtained as an oil substance through the same reaction as in step 2 in the synthesis of intermediate 3f using 4-tert-butoxycarbonyl-6-chloro-2-methyl-2,3-dihydro-1,4-benzoxazine-8-carboxylic acid synthesized in step 2 instead of 6-chloro-2-methylquinoline-8-carboxylic acid.
$^1$H-NMR ($CDCl_3$) $\delta$: 7.98 (1H, t, J= 6.1 Hz), 7.89-7.86 (2H, m), 4.48-4.40 (1H, m), 4.23 (1H, dd, J = 13.4, 2.4 Hz), 4.14 (2H, q, J = 7.3 Hz), 3.47-3.34 (2H, m), 3.24 (1H, dd, J = 13.4, 8.5 Hz), 2.27 (2H, s), 1.55 (9H, s), 1.47 (3H, d, J= 6.1 Hz), 1.26 (3H, t, J = 7.3 Hz), 1.07 (6H, s).
MS (ESI) m/z : 469 [$(M+H)^+$].

(Step 4) 4-[(4-tert-Butoxycarbonyl-6-chloro-2-methyl-2,3-dihydro-1,4-benzoxazine-8-carbonyl)amino]-3,3-dimethylbutanoic acid

To a solution of tert-butyl 6-chloro-8-[(4-ethoxy-2,2-dimethyl-4-oxobutyl)carbamoyl]-2-methyl-2,3-dihydro-1,4-benzoxazine-4-carboxylate (0.440 mg, 0.938 mmol) synthesized in step 3 in tetrahydrofuran (6.0 mL), methanol (3.0 mL) and a 1 N aqueous sodium hydroxide solution (2.81 mL, 2.81 mmol) were added, and the mixture was stirred overnight at room temperature. 1 N hydrochloric acid was added to the reaction solution, followed by extraction with ethyl acetate three times. The organic layer was washed with saturated saline and then dried over anhydrous sodium sulfate. After filtration, the filtrate was concentrated under reduced pressure to obtain the title compound (0.420 g, quantitative) as a solid.
$^1$H-NMR (DMSO-$D_6$) $\delta$: 12.10 (1H, s), 8.17 (1H, t, J = 6.1 Hz), 7.86 (1H, s), 7.30 (1H, d, J = 3.1 Hz), 4.42-4.35 (1H, m), 4.12 (1H, dd, J = 13.4, 2.4 Hz), 3.30-3.16 (3H, m), 2.18 (2H, s), 1.49 (9H, s), 1.34 (3H, d, J= 6.1 Hz), 0.99 (6H, s).
MS (ESI) m/z : 441 [$(M+H)^+$].

(Step 5) tert-Butyl 6-chloro-8-({4-[(1,5-dimethyl-3-oxo-2-phenyl-2,3-dihydro-1H-pyrazol-4-yl)amino]-2,2-dimethyl-4-oxobutyl}carbamoyl)-2-methyl-2,3-dihydro-1,4-benzoxazine-4-carboxylate

The title compound was obtained as a solid through the same reaction as in step 1 of Example 93 using 4-[(4-tert-butoxycarbonyl-6-chloro-2-methyl-2,3-dihydro-1,4-benzoxazine-8-carbonyl)amino]-3,3-dimethylbutanoic acid synthesized in step 4 instead of 4-[(5-chloro-2-ethoxybenzoyl)amino]-3,3-dimethylbutanoic acid.
$^1$H-NMR ($CDCl_3$) $\delta$: 8.80 (1H, s), 8.15 (1H, t, J = 6.7 Hz), 7.89 (2H, d, J= 3.1 Hz), 7.44-7.40 (4H, m), 7.29-7.25 (1H, m), 4.47-4.39 (1H, m), 4.24 (1H, dd, J = 13.4, 2.4 Hz), 3.61 (1H, dd, J = 14.0, 6.7 Hz), 3.49 (1H, dd, J = 14.0, 6.7 Hz), 3.21 (1H, dd, J = 14.0, 8.5 Hz), 3.09 (3H, s), 2.30-2.23 (5H, m), 1.54 (9H, s), 1.46 (3H, d, J = 6.7 Hz), 1.10 (6H, s).
MS (ESI) m/z : 626 [$(M+H)^+$].

(Step 6) 6-Chloro-N-{4-[1,5-dimethyl-3-oxo-2-phenyl-2,3-dihydro-1H-pyrazol-4-yl]amino}-2,2-dimethyl-4-oxobutyl]-2-methyl-3,4-dihydro-2H-1,4-benzoxazine-8-carboxamide

To a solution of tert-butyl 6-chloro-8-({4-[(1,5-dimethyl-3-oxo-2-phenyl-2,3-dihydro-1H-pyrazol-4-yl)amino]-2,2-dimethyl-4-oxobutyl}carbamoyl)-2-methyl-2,3-dihydro-1,4-benzoxazine-4-carboxylate (0.589 g, 0.941 mmol) synthesized in step 5 in methylene chloride (5.0 mL), a catalytic amount of water and trifluoroacetic acid (5.0 mL) were added, and the mixture was stirred at room temperature for 1 hour. The reaction solution was concentrated under reduced pressure, and then, a saturated aqueous solution of sodium bicarbonate was added to the residue, followed by extraction with methylene chloride three times. The organic layer was dried over anhydrous sodium sulfate. After filtration, the filtrate was concentrated under reduced pressure, and then, the obtained residue was purified by amino silica gel column

chromatography (Yamazen Corp., eluting solvent: ethyl acetate/methanol = 100/0 to 89/11). After concentration under reduced pressure, the obtained residue was solidified with ethyl acetate. The solid was collected by filtration and dried to obtain the title compound (0.429 g, yield: 86.6%) as a solid.

$^1$H-NMR ($CDCl_3$) δ: 9.21 (1H, s), 8.27 (1H, t, J = 6.4 Hz), 7.46-7.40 (5H, m), 7.28-7.24 (1H, m), 6.67 (1H, d, J = 2.4 Hz), 4.36-4.29 (2H, m), 3.56 (2H, d, J = 7.3 Hz), 3.38-3.33 (1H, m), 3.16-3.11 (1H, m), 3.08 (3H, s), 2.26 (5H, s), 1.43 (3H, d, J = 6.1 Hz), 1.10 (6H, s).

MS (ESI) m/z : 526 [(M+H)$^+$].

(Example 110) 6-Chloro-N-{4-[(1,5-dimethyl-3-oxo-2-phenyl-2,3-dihydro-1H-pyrazol-4-yl)amino]-2,2-dimethyl-4-oxobutyl}-2,4-dimethyl-2,3-dihydro-1,4-benzoxazine-8-carboxamide

[Formula 82]

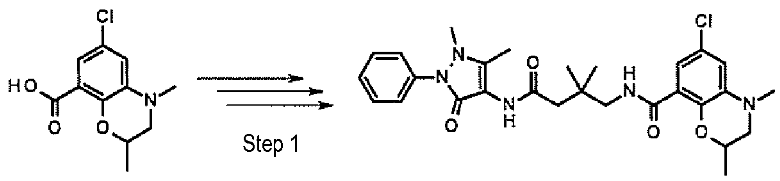


(Step 1) 6-Chloro-N-{4-[(1,5-dimethyl-3-oxo-2-phenyl-2,3-dihydro-1H-pyrazol-4-yl)amino]-2,2-dimethyl-4-oxobutyl}-2,4-dimethyl-2,3-dihydro-1,4-benzoxazine-8-carboxamide

The title compound was subsequently obtained as a solid by synthesis in the same way as in steps 3 to 5 of Example 109 using 6-chloro-2,4-dimethyl-2,3-dihydro-1,4-benzoxazine-8-carboxylic acid synthesized according to the method described in Chem. & Pharm. Bull., 1996, 44, 11, 2051-2060.

$^1$H-NMR ($CDCl_3$) δ: 9.05 (1H, s), 8.24 (1H, t, J = 6.7 Hz), 7.49-7.38 (5H, m), 7.30-7.22 (1H, m), 6.69 (1H, d, J = 2.4 Hz), 4.47-4.40 (1H, m), 3.62-3.50 (2H, m), 3.25 (1H, dd, J= 11.9, 2.7 Hz), 3.13-3.08 (4H, m), 2.90 (3H, s), 2.27-2.26 (5H, m), 1.44 (3H, d, J = 6.1 Hz), 1.10 (6H, d, J = 1.8 Hz).

MS (ESI) m/z : 540 [(M+H)$^+$].

(Example 111) 6-Chloro-N-{4-[(1,5-dimethyl-3-oxo-2-phenyl-2,3-dihydro-1H-pyrazol-4-yl)amino]-2,2-dimethyl-4-oxobutyl}-2-ethyl-1,3-benzoxazole-4-carboxamide

[Formula 83]

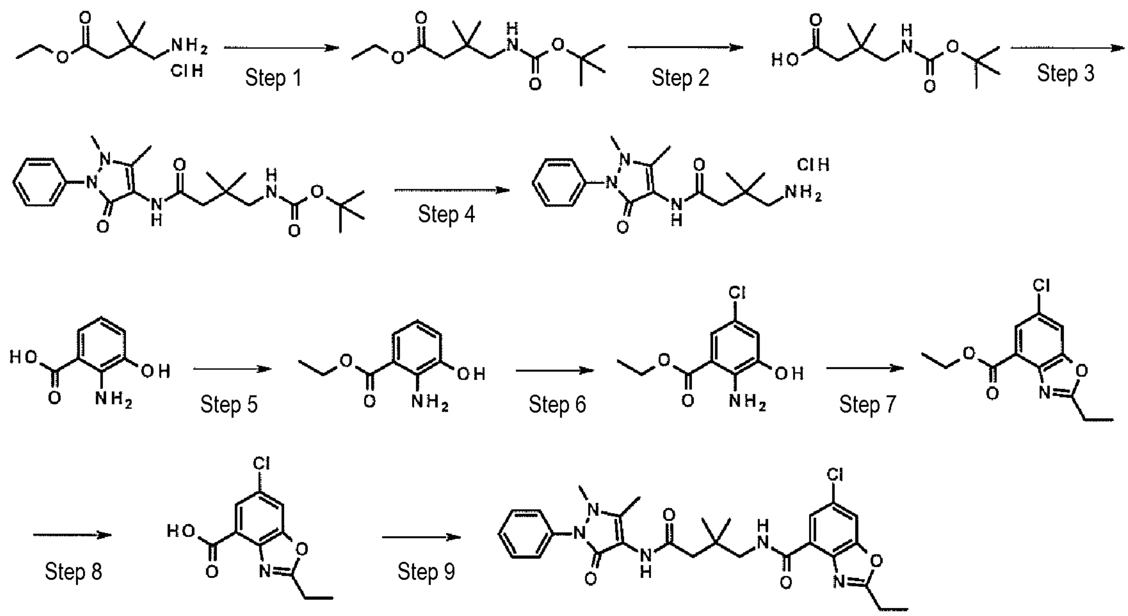


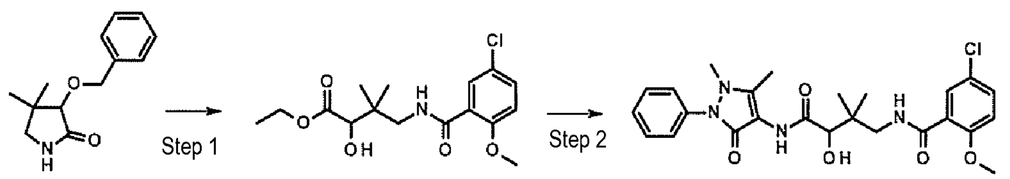


(Step 1) Ethyl 4-(tert-butoxycarbonylamino)-3,3-dimethylbutanoate

Ethyl 4-amino-3,3-dimethylbutanoate hydrochloride (2.50 g, 12.8 mmol) synthesized in step 2 in the synthesis of intermediate 1a was dissolved in tetrahydrofuran (60 ml). To the solution, triethylamine (2.65 ml, 1.93 g, 19.1 mmol) and di-tert-butyl dicarbonate (4.17 g, 19.1 mmol) were added, and the mixture was stirred at room temperature for 2 hours. Water was added to the reaction solution, followed by extraction with ethyl acetate. The organic layer was dried over anhydrous sodium sulfate, and the solvent was distilled off under reduced pressure. The residue was purified by silica gel chromatography (Biotage Japan Ltd., eluting solvent: ethyl acetate/hexane = 2/98-15/85) to obtain the title compound (2.30 g, yield: 69.3%) as an oil substance.
$^1$H-NMR ($CDCl_3$) δ: 4.90 (1H, br s), 4.13 (2H, q, J = 7.2 Hz), 3.04 (2H, d, J = 6.8 Hz), 2.21 (2H, s), 1.44 (9H, s), 1.27 (3H, t, J = 7.2 Hz), 0.99 (6H, s).

(Step 2) 4-(tert-Butoxycarbonylamino)-3,3-dimethylbutanoic acid

Ethyl 4-(tert-butoxycarbonylamino)-3,3-dimethylbutanoate (2.30 g, 8.87 mmol) synthesized in step 1 was dissolved in ethanol (80 ml). To the solution, a 1 N aqueous sodium hydroxide solution (26.6 ml, 26.6 mmol) was added, and the mixture was stirred overnight at room temperature. The reaction solution was concentrated under reduced pressure, and 1 N hydrochloric acid (28.0 ml, 28.0 mmol) was added to the residue, followed by extraction with chloroform. The organic layer was dried over anhydrous sodium sulfate, and the solvent was distilled off under reduced pressure to obtain the title compound (2.20 g, quantitative) as an oil substance. $^1$H-NMR ($CDCl_3$) δ: 4.97 (1H, br s), 3.07 (2H, d, J = 7.1 Hz), 2.25 (2H, s), 1.46 (9H, s), 1.02 (6H, s).
MS (EI) m/z : 231 ($M^+$).

(Step 3) tert-Butyl N-{4-[(1,5-dimethyl-3-oxo-2-phenyl-2,3-dihydro-1H-pyrazol-4-yl)amino]-2,2-dimethyl-4-oxobutyl}carbamate

The title compound was obtained as a solid through the same reaction as in step 1 of Example 93 using 4-(tert-butoxycarbonylamino)-3,3-dimethylbutanoic acid synthesized in step 2 instead of 4-[(5-chloro-2-ethoxybenzoyl)amino]-3,3-dimethylbutanoic acid.

$^1$H-NMR ($CDCl_3$) δ: 8.19 (1H, br s), 7.48-7.38 (4H, m), 7.31-7.27 (1H, m), 5.21 (1H, t, J = 6.4 Hz), 3.13 (2H, d, J = 7.3 Hz), 3.08 (3H, s), 2.24 (3H, s), 2.22 (2H, s), 1.44 (9H, s), 1.02 (6H, s).

(Step 4) 4-Amino-N-(1,5-dimethyl-3-oxo-2-phenyl-2,3-dihydro-1H-pyrazol-4-yl)-3,3-dimethylbutanamide hydrochloride

tert-Butyl N-{4-[(1,5-dimethyl-3-oxo-2-phenyl-2,3-dihydro-1H-pyrazol-4-yl)amino]-2,2-dimethyl-4-oxobutyl}carbamate (9.10 g, 19.3 mmol) synthesized in step 3 was dissolved in 1,4-dioxane (50 mL). To the solution, 4 N hydrochloric acid in 1,4-dioxane (50 mL) was added, and the mixture was stirred at room temperature for 88 hours. The resulting solid was collected by filtration, washed with 1,4-dioxane and ether in this order, and then dried to obtain the title compound (6.81 g, quantitative) as a solid.
$^1$H-NMR (DMSO-$D_6$) δ: 9.34 (1H, s), 8.03 (3H, br s), 7.54-7.48 (2H, m), 7.38-7.30 (3H, m), 3.06 (3H, s), 2.84 (2H, q, J = 5.5 Hz), 2.37 (2H, s), 2.14 (3H, s), 1.05 (6H, s).

(Step 5) Ethyl 2-amino-3-hydroxybenzoate

2-Amino-3-hydroxybenzoic acid (2.95 g, 19.3 mmol) was suspended in ethanol (60 mL). To the suspension, concentrated sulfuric acid (3.0 mL) was added under ice cooling. The reaction mixture was heated to reflux for a total of 30 hours, brought back to room temperature, and then concentrated under reduced pressure. The residue was neutralized by the addition of a saturated aqueous solution of sodium bicarbonate, followed by extraction with ethyl acetate. The organic layer was washed with saturated saline and dried over anhydrous sodium sulfate. After filtration, the filtrate was concentrated, and the obtained residue was purified by silica gel column chromatography (Biotage Japan Ltd., eluting solvent: hexane/ethyl acetate = 95/5 to 50/50) to obtain the title compound (2.36 g, yield: 67.6%) as a solid.
$^1$H-NMR ($CDCl_3$) δ: 7.51 (1H, d, J= 7.9 Hz), 6.82 (1H, d, J = 7.9 Hz), 6.51 (1H, t, J = 7.9 Hz), 5.87 (2H, br s), 4.92 (1H, br s), 4.34 (2H, q, J = 7.3 Hz), 1.38 (3H, t, J= 7.3 Hz).
MS (APCI) m/z : 182 [$(M+H)^+$].

(Step 6) Ethyl 2-amino-5-chloro-3-hydroxybenzoate

Ethyl 2-amino-3-hydroxybenzoate (2.36 g, 13.0 mmol) synthesized in step 5 was dissolved in N,N-dimethylformamide (45 mL). To the solution, N-chlorosuccinimide (1.74 g, 13.0 mmol) was added, and the mixture was stirred at 60°C for 1 hour. After cooling to room temperature, the reaction mixture was diluted with ethyl acetate, and the organic layer was washed with water and saturated saline. The organic layer was dried over anhydrous sodium sulfate. After filtration, the filtrate was concentrated, and the obtained residue was purified by silica gel column chromatography (Biotage Japan Ltd., eluting solvent: hexane/ethyl acetate = 95/5 to 50/50) to obtain the title compound (2.68 g, yield: 95.4%) as a solid.
MS (APCI) m/z: 216 [$(M+H)^+$].

(Step 7) Ethyl 6-chloro-2-ethyl-1,3-benzoxazole-4-carboxylate

Ethyl 2-amino-5-chloro-3-hydroxybenzoate synthesized in step 6 was suspended in trimethylpropionic acid orthoester (4.75 mL, 4.23 g, 24.0 mmol). To the suspension, one drop of concentrated sulfuric acid was added. The reaction mixture was stirred at 110°C for 4.4 hours and cooled to room temperature. The reaction mixture was neutralized by the addition of a saturated aqueous solution of sodium bicarbonate, followed by extraction with methylene chloride. The organic layer was dried over anhydrous sodium sulfate. After filtration, the filtrate was concentrated, and the obtained residue was purified by silica gel column chromatography (Biotage Japan Ltd., eluting solvent: hexane/ethyl acetate = 90/10 to 50/50) to obtain the title compound (1.71 g, yield: 96.5%) as a solid.
$^1$H-NMR ($CDCl_3$) δ: 7.94 (1H, d, J= 1.8 Hz), 7.67 (1H, d, J = 1.8 Hz), 4.48 (2H, q, J = 7.1 Hz), 3.04 (2H, q, J = 7.7 Hz), 1.48-1.43 (6H, m).
MS (APCI) m/z : 254 [$(M+H)^+$].

(Step 8) 6-Chloro-2-ethyl-1,3-benzoxazole-4-carboxylic acid

Ethyl 6-chloro-2-ethyl-1,3-benzoxazole-4-carboxylate (1.71 g, 6.74 mmol) synthesized in step 7 was dissolved in tetrahydrofuran (10 mL) and ethanol (10 mL). To the solution, a 1 N aqueous sodium hydroxide solution (10.0 mL, 10.0 mmol) was added. The reaction mixture was stirred for 23 hours. Then, the organic solvent was distilled off under reduced pressure, and the residue was neutralized by the addition of 1 N hydrochloric acid, followed by extraction with methylene chloride. The extract was dried over anhydrous sodium sulfate. After filtration, the filtrate was concentrated to obtain the title compound (1.51 g, yield: 99.3%) as a solid.

$^1$H-NMR ($CDCl_3$) δ: 8.07 (1H, d, J = 1.8 Hz), 7.74 (1H, d, J= 1.8 Hz), 3.08 (2H, q, J = 7.6 Hz), 1.50 (3H, t, J = 7.6 Hz). MS (APCI) m/z : 226 [$(M+H)^+$].

(Step 9) 6-Chloro-N-{4-[(1,5-dimethyl-3-oxo-2-phenyl-2,3-dihydro-1H-pyrazol-4-yl)amino]-2,2-dimethyl-4-oxobutyl}-2-ethyl-1,3-benzoxazole-4-carboxamide

4-Amino-N-(1,5-dimethyl-3-oxo-2-phenyl-2,3-dihydro-1H-pyrazol-4-yl)-3,3-dimethylbutanamide hydrochloride (0.258 g, 0.731 mmol) synthesized in step 4 and 6-chloro-2-ethyl-1,3-benzoxazole-4-carboxylic acid (0.150 g, 0.664 mmol) synthesized in step 8 were dissolved in N,N-dimethylformamide (5.0 mL). To the solution, 0-(7-azabenzotriazol-1-yl)-N,N,N',N'-tetramethyluronium hexafluorophosphate (HATU) (0.328 g, 0.865 mmol) and N,N-diisopropylethylamine (0.347 mL, 0.258 g, 1.99 mmol) were added, and the mixture was stirred at room temperature for 5.8 hours. A saturated aqueous solution of sodium bicarbonate was added to the reaction mixture, followed by extraction with ethyl acetate. The organic layer was washed with saturated saline and dried over anhydrous sodium sulfate. After filtration, the filtrate was concentrated, and the obtained residue was purified by amino silica-silica gel column chromatography (Biotage Japan Ltd., eluting solvent: hexane/10% methanol-ethyl acetate = 70/30 to 0/100) to obtain the title compound (0.197 g, yield: 56.5%) as a solid.
$^1$H-NMR ($CDCl_3$) δ: 9.29 (1H, t, J= 6.1 Hz), 8.93 (1H, br s), 8.15 (1H, d, J= 1.8 Hz), 7.62 (1H, d, J = 1.8 Hz), 7.43-7.42 (4H, m), 7.27-7.25 (3H, m), 3.68 (2H, d, J = 6.1 Hz), 3.09 (3H, s), 3.01 (2H, q, J = 7.6 Hz), 2.31 (2H, s), 1.46 (3H, t, J = 7.6 Hz), 1.18 (6H, s). MS (APCI) m/z : 524 [$(M+H)^+$].

(Example 112) 5-Chloro-N-[4-(1,5-dimethyl-3-oxo-2-phenyl-2,3-dihydro-1H-pyrazol-4-yl)amino]-3-hydroxy-2,2-dimethyl-4-oxobutyl]-2-methoxybenzamide

[Formula 84]

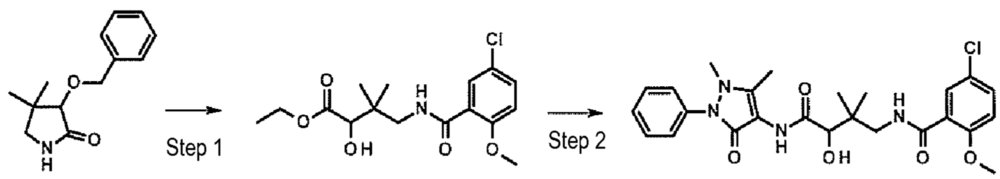


(Step 1) Ethyl 4-[(5-chloro-2-methoxybenzoyl)amino]-2-hydroxy-3,3-dimethylbutanoate

3-Benzyloxy-4,4-dimethylpyrrolidin-2-one (280 mg, 1.27 mmol) synthesized by the method described in J. Org. Chem., 2011, 66, 5859-5865 in 6 N hydrochloric acid (10 ml) was heated to reflux for 15 hours. The mixture was concentrated and then subjected to azeotropy with ethanol several times, and the obtained residue was dissolved in ethanol (10 ml). To the solution, acetyl chloride (0.320 ml, 349 mg, 4.45 mmol) was added at room temperature, and the mixture was heated to reflux for 2 hours. The reaction solution was brought back to room temperature and concentrated, and the obtained residue was dissolved in N,N-dimethylformamide (8.0 ml). To the solution, 5-chloro-2-methoxybenzoic acid (284 mg, 1.52 mmol), triethylamine (0.35 ml, 257 mg, 2.54 mmol), 1-hydroxybenzotriazole (HOBt) (35 mg, 20 mol%), and 1-ethyl-3-(3-dimethylaminopropyl)carbodiimide hydrochloride (EDC·HCl) (292 mg, 1.52 mmol) were added at room temperature, and the mixture was stirred at the same temperature as above for 16 hours. The reaction solution was diluted with ethyl acetate, and the organic layer was washed with water, 1 N hydrochloric acid, a saturated aqueous solution of sodium bicarbonate, and saturated saline in this order and then dried over anhydrous sodium sulfate. After filtration, the filtrate was concentrated, and diethyl ether was added to the obtained residue. The deposit was collected by filtration and dried to obtain the title compound (255 mg, yield: 58.4%) as a solid.
$^1$H-NMR ($CDCl_3$) δ: 8.26 (1H, br), 8.17 (1H, d, J = 2.8 Hz), 7.40 (1H, dd, J = 9.2, 2.8 Hz), 6.92 (1H, d, J = 9.2 Hz), 4.30-4.23 (2H, m), 3.97 (3H, s), 3.69-3.74 (2H, m), 3.18 (1H, dd, J = 13.8, 5.5 Hz), 1.31 (3H, t, J = 7.3 Hz), 1.08 (3H, s), 0.99 (3H, s).

(Step 2) 5-Chloro-N-[4-(1,5-dimethyl-3-oxo-2-phenyl-2,3-dihydro-1H-pyrazol-4-yl)amino]-3-hydroxy-2,2-dimethyl-4-oxobutyl]-2-methoxybenzamide

Ethyl 4-[(5-chloro-2-methoxybenzoyl)amino]-2-hydroxy-3,3-dimethylbutanoate (250 mg, 0.727 mmol) synthesized in step 1 was dissolved in ethanol (5.0 ml). To the solution, a 1 N aqueous sodium hydroxide solution (2.0 ml) was added at room temperature, and the mixture was stirred for 1.5 hours. The reaction solution was cooled in ice, and 1 N hydrochloric acid (4.0 ml) was added thereto. The deposit was subjected to extraction with ethyl acetate, and the organic layer was washed with saturated saline and then dried over anhydrous sodium sulfate. After filtration, the filtrate was concentrated, and the obtained residue was dissolved in N,N-dimethylformamide (5.0 ml). To the solution, 4-aminoantipyrine (229 mg, 1.17 mmol), N,N-diisopropylethylamine (0.255 ml, 194 mg, 1.50 mmol), and O-(7-azabenzotriazol-1-yl)-N,N,N',N'-tetramethyluronium hexafluorophosphate (HATU) (428 mg, 1.17 mmol) were added at room temperature, and the mixture was stirred at room temperature for 19 hours. Water was added to the reaction solution, followed by extraction with ethyl acetate. The organic layer was washed with water and saturated saline in this order and then dried over anhydrous sodium sulfate. After filtration, the filtrate was concentrated, and the obtained crude product was purified by silica gel column chromatography (eluting solvent: methanol/chloroform = 2/98) to obtain the title compound (235 mg, yield: 64.5%) as a solid.
$^1$H-NMR ($CDCl_3$) $\delta$: 8.52 (1H, s), 8.32 (1H, br), 8.16 (1H, d, J = 2.8 Hz), 7.48-7.39 (5H, m), 7.29-7.29 7 (1H, m), 6.94 (1H, d, J = 8.7 Hz), 5.40 (1H, d, J = 4.5 Hz), 3.98 (3H, s), 3.94 (1H, d, J = 4.5 Hz), 3.87 (1H, dd, J = 14.2, 8.2 Hz), 3.09 (3H, s), 3.05 (1H, dd, J = 14.2, 5.9 Hz), 2.25 (3H, s), 1.17 (3H, s), 1.10 (3H, s). MS (APCI) m/z : 501 [(M+H)$^+$].

(Example 113) 5-Chloro-2-ethoxy-4-fluoro-N-(4-{[5-(methoxymethyl)-1-methyl-3-oxo-2-phenyl-2,3-dihydro-1H-pyrazol-4-yl]amino}-2,2-dimethyl-4-oxobutyl)benzamide

[Formula 85]

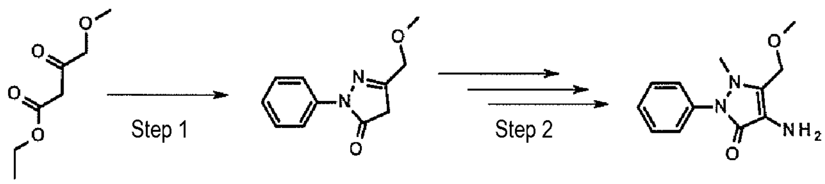


(Step 1) 5-(Methoxymethyl)-2-phenyl-4H-pyrazol-3-one

To a solution of ethyl 4-methoxy-3-oxobutanoate (0.481 g, 3.00 mmol) in acetic acid (4.0 mL), phenylhydrazine (0.295 mL, 0.324 g, 3.00 mmol) was added, and the mixture was stirred at 110°C for 5 hours under a nitrogen atmosphere. The reaction solution was allowed to cool to room temperature and then concentrated under reduced pressure, and water was added to the residue, followed by extraction with ethyl acetate three times. The organic layer was washed with saturated saline and then dried over anhydrous sodium sulfate. After filtration, the filtrate was concentrated under reduced pressure, and then, the obtained residue was purified by silica gel column chromatography (Yamazen Corp., eluting solvent: chloroform/ethyl acetate = 90/10 to 69/31) to obtain the title compound (0.231 g, yield: 37.7%) as a solid.
$^1$H-NMR (DMSO-$D_6$) $\delta$: 11.62 (1H, s), 7.72-7.70 (2H, m), 7.46-7.42 (2H, m), 7.26-7.24 (1H, m), 5.53 (1H, s), 4.26 (2H, s), 3.27 (3H, s).
MS (API) m/z : 205[(M+H)$^+$].

(Step 2) 4-Amino-5-(methoxymethyl)-1-methyl-2-phenyl-2,3-dihydro-1H-pyrazol-3-one

The title compound was obtained as a solid by using 5-(methoxymethyl)-2-phenyl-4H-pyrazol-3-one synthesized in step 1 instead of 2,2,2-trifluoro-N-[2-(5-oxo-1-phenyl-4H-pyrazol-3-yl)propyl]acetamide in step 3 of Example 66 and subsequently performing the same reaction as in Example 66 up to step 5.
$^1$H-NMR ($CDCl_3$) $\delta$: 7.51-7.42 (4H, m), 7.27-7.24 (1H, m), 4.41 (2H, s), 3.46 (3H, s), 3.33 (2H, s), 2.85 (3H, s). MS (API) m/z : 234[(M+H)$^+$].
The following compounds of Examples 113 to 121 were subsequently synthesized in the same way as in Example 92 or 93 and are shown in Table 33.

[Table 33-1]

| Example No. | Structure | Intermediate used | Instrumental analysis data |
|---|---|---|---|
| 1 1 3 | 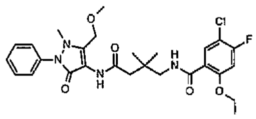 | 1 c | $^1$H-NMR ($CDCl_3$) $\delta$: 9.02 (1H, s), 8.30 (1H, d, J = 8.7 Hz), 8.21 (1H, t, J = 6.4 Hz), 7.46-7.44 (4H, m), 7.31-7.28 (1H, m), 6.78 (1H, d, J = 10.5 Hz), 4.54 (2H, s), 4.18 (2H, q, J = 6.9 Hz), 3.56 (2H, d, J = 6.4 Hz), 3.44 (3H, s), 3.19 (3H, s), 2.28 (2H, s), 1.54 (3H, t, J = 6.9 Hz), 1.11 (6H, s).<br>MS (API) m/z : 547 [(M+H)$^+$]. |
| 1 1 4 | 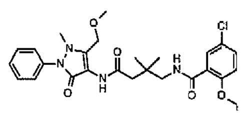 | 1 a | $^1$H-NMR ($CDCl_3$) $\delta$: 9.20 (1H, s), 8.39 (1H, t, J = 6.7 Hz), 8.23 (1H, d, J = 2.7 Hz), 7.48-7.45 (4H, m), 7.40 (1 H, dd, J = 8.8, 2.9 Hz), 7.33-7.30 (1H, m), 6.93 (1H, d, J = 8.6 Hz), 4.56 (2H, s), 4.22 (2H, q, J = 7.0 Hz), 3.60 (2H, d, J = 6.7 Hz), 3.47 (3H, s), 3.21 (3H, s), 2.31 (2H, s), 1.55 (3H, t, J = 7.0 Hz), 1.14 (61H, s).<br>MS (ESI+APCI) m/z : 529 [(M+H)$^+$]. |
| 1 1 5 | 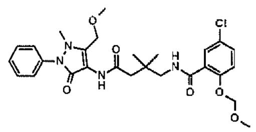 | 1 c | $^1$H-NMR ($CDCl_3$) $\delta$: 8.67 (1H, s), 8.27 (1H, t, J = 6.6 Hz), 8.17 (1H, d, J = 2.9 Hz), 7.48-7.43 (4H, m), 7.36 (1H, dd, J = 8.8, 2.9 Hz), 7.31-7.29 (1H, m), 7.15 (1H, d, J = 8.8 Hz), 5.33 (2H, s), 4.54 (2H, s), 3.56 (2H, d, J = 6.6 Hz), 3.51 (3H, s), 3.44 (3H, s), 3.19 (3H, s), 2.30 (2H, s), 1.12 (6H, s).<br>MS (ESI) m/z : 545 [(M+H)$^+$]. |
| 1 1 6 | 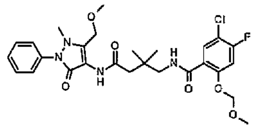 | 1 f | $^1$H-NMR ($CDCl_3$) $\delta$: 8.57 (1H, s), 8.27 (1H, d, J = 8.8 Hz), 8.23 (1H, t, J = 6.6 Hz), 7.48-7.42 (4H, m), 7.32-7.30 (1H, m), 7.06 (1H, d, J = 10.7 Hz), 5.32 (2H, s), 4.53 (2H, s), 3.53 (2H, d, J = 6.8 Hz), 3.51 (3H, s), 3.44 (3H, s), 3.19 (3H, s), 2.29 (2H, s), 1.10 (6H, s).<br>MS (ESI) m/z : 563 [(M+H)$^+$]. |
| 1 1 7 | 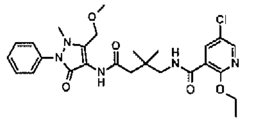 | 1 h | $^1$H-NMR ($CDCl_3$) $\delta$: 8.87 (1H, br s), 8.50 (1H, d, J = 2.3 Hz), 8.40 (1H, t, J = 6.9 Hz), 8.19 (1H, d, J = 2.3 Hz), 7.48-7.43 (4H, m), 7.32-7.28 (1H, m), 4.57-4.53 (4H, m), 3.56 (2H, d, J = 6.9 Hz), 3.44 (3H, s), 3.19 (3H, s), 2.29 (2H, s), 1.48 (3H, t, J = 7.2 Hz), 1.12 (6H, s).<br>MS (ESI) m/z : 530 [(M+H)$^+$]. |
| 1 1 8 | 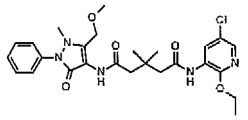 | 2 a | $^1$H-NMR ($CDCl_3$) $\delta$: 8.71 (1H, s), 8.63 (1H, d, J = 2.3 Hz), 8.46 (1H, s), 7.77 (1 H, d, J = 2.3 Hz), 7.48-7.45 (2H, m), 7.42-7.40 (2H, m), 7.33-7.31 (1H, m), 4.55 (2H, s), 4.38 (2H, q, J = 7.1 Hz), 3.44 (3H, s), 3.20 (3H, s), 2.64 (2H, s), 2.47 (2H, s), 1.36 (3H, t, J = 7.1 Hz), 1.15 (6H, s).<br>MS (ESI) m/z : 530 [(M+H)$^+$]. |

[Table 33-2]

| | | | |
|---|---|---|---|
| 1 1 9 | 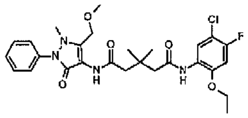 | 2 c | $^1$H-NMR ($CDCl_3$) $\delta$: 8.68 (1H, s), 8.47 (1H, s), 8.38 (1H, d, J = 7.9 Hz), 7.48-7.40 (4H, m), 7.33-7.28 (1H, m), 6.66 (1H, d, J = 10.4 Hz), 4.54 (2H, s), 4.03 (2H, q, J = 7.0 Hz), 3.44 (3H, s), 3.20 (3H, s), 2.64 (2H, s), 2.49 (2H, s), 1.41 (3H, t, J = 7.0 Hz), 1.15 (6H, s).<br>MS (API) m/z : 547 [(M+H)$^+$]. |

(continued)

| | | | |
|---|---|---|---|
| 120 | 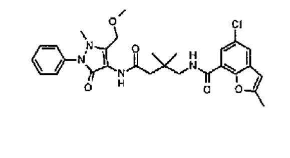 | 3a | $^1$H-NMR ($CDCl_3$) δ: 8.73 (1H, s), 7.97 (1H, d, J = 2.4 Hz), 7.91 (1H, t, J = 6.3 Hz), 7.56 (1H, d, J = 2.4 Hz), 7.48-7.43 (4H, m), 7.31-7.28 (1H, m), 6.43 (1H, s), 4.53 (2H, s), 3.64 (2H, d, J = 6.3 Hz), 3.43 (3H, s), 3.19 (3H, s), 2.51 (3H, s), 2.34 (2H, s), 1.15 (6H, s).<br>MS (ESI) m/z : 539 [(M+H)$^+$]. |
| 121 | 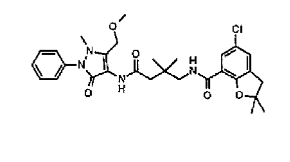 | 3c | $^1$H-NMR ($CDCl_3$) δ: 9.07 (1H, s), 7.98 (1H, t, J = 6.6 Hz), 7.90 (1H, d, J = 2.9 Hz), 7.45-7.44 (4H, m), 7.31-7.27 (1H, m), 7.22-7.21 (1H, m), 4.54 (2H, s), 3.56 (2H, d, J = 6.6 Hz), 3.45 (3H, s), 3.19 (3H, s), 3.06 (2H, s), 2.27 (2H, s), 1.55 (6H, s), 1.10 (6H, s).<br>MS (ESI) m/z: 555 [(M+H)$^+$]. |

(Example 122) 5-Chloro-2-ethoxy-4-fluoro-N-(4-{[5-(1-methoxyethyl)-1-methyl-3-oxo-2-phenyl-2,3-dihydro-1H-pyrazol-4-yl]amino}-2,2-dimethyl-4-oxobutyl)benzamide

[Formula 86]

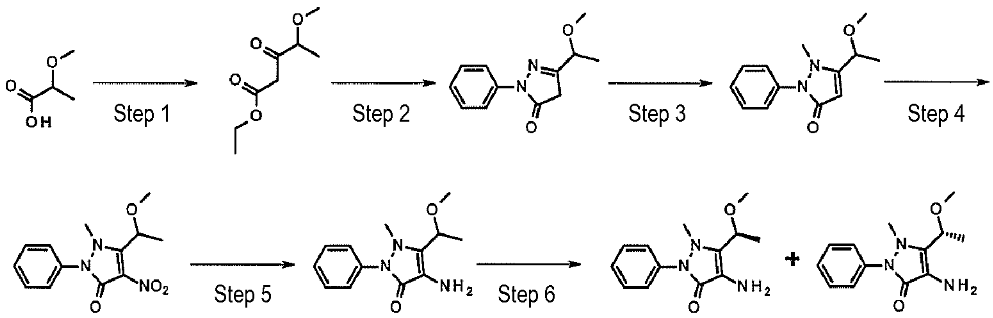


122a (-)-(S)--122b (+)-(R)-122c

(Step 1) Ethyl 4-methoxy-3-oxopentanoate

2-Methoxypropanoic acid (25.0 g, 240 mmol) was dissolved in tetrahydrofuran (400 mL). To the solution, 1,1'-carbonyldiimidazole (46.7 g, 288 mmol) was added, and the mixture was stirred at room temperature for 2 hours. Magnesium chloride (22.9 g, 240 mmol) and potassium monoethyl malonate (61.3 g, 360 mmol) were added to the reaction solution, and the mixture was stirred at room temperature for 6 hours. The solvent in the reaction solution was distilled off under reduced pressure. Ethyl acetate and water were added to the residue, and the mixture was stirred at 0°C. 2 N hydrochloric acid (200 mL) was added thereto, followed by extraction with ethyl acetate. The organic layer was washed with saturated saline and then dried over anhydrous magnesium sulfate. After filtration, the filtrate was concentrated, and the obtained crude product was purified by silica gel column chromatography (Biotage Japan Ltd., eluting solvent: hexane/ethyl acetate = 95/5 to 65/35) to obtain the title compound (37.1 g, yield: 88.8%) as an oil substance.
$^1$H-NMR ($CDCl_3$) δ: 4.20 (2H, q, J = 7.2 Hz), 3.83 (1H, q, J = 6.8 Hz), 3.60 (1H, d, J = 16.1 Hz), 3.54 (1H, d, J = 16.1 Hz), 3.39 (3H, s), 1.33 (3H, d, J = 6.8 Hz), 1.28 (3H, t, J = 7.2 Hz).

(Step 2) 5-(1-Methoxyethyl)-2-phenyl-4H-pyrazol-3-one

Ethyl 4-methoxy-3-oxopentanoate (37.1 g, 213 mmol) synthesized in step 1 was dissolved in anhydrous toluene (250 mL). To the solution, phenylhydrazine (22.0 mL, 24.2 g, 224 mmol) was added, and the mixture was heated to reflux for 18 hours while generated water was removed. The temperature of the reaction solution was adjusted to room temperature. The solvent was distilled off under reduced pressure, and the obtained crude product was purified by silica gel column chromatography (Biotage Japan Ltd., eluting solvent: methylene chloride/methanol = 100/0 to 90/10) to obtain the title compound (42.8 g, yield: 92.0%) as a solid.
$^1$H-NMR ($CDCl_3$) δ: 7.87-7.85 (2H, m), 7.42-7.39 (2H, m), 7.21-7.19 (1H, m), 4.21 (1H, q, J = 6.8 Hz), 3.50 (2H, s), 3.37

(3H, s), 1.45 (3H, d, J = 6.8 Hz).

(Step 3) 5-(1-Methoxyethyl)-1-methyl-2-phenyl-2,3-dihydro-1H-pyrazol-3-one

5-(1-Methoxyethyl)-2-phenyl-4H-pyrazol-3-one (21.8 g, 100 mmol) synthesized in step 2 was dissolved in N,N-dimethylformamide (100 mL). To the solution, methyl iodide (46.7 mL, 107 g, 750 mmol) was added, and the mixture was heated to reflux for 9 hours. The solvent was distilled off under reduced pressure, and water and a saturated aqueous solution of sodium bicarbonate were added to the residue, followed by extraction with ethyl acetate. The organic layer was washed with saturated saline and then dried over anhydrous magnesium sulfate. After filtration, the filtrate was concentrated, and the obtained crude product was purified by amino silica gel column chromatography (Biotage Japan Ltd., eluting solvent: hexane/ethyl acetate = 60/40 to 20/80) and silica gel column chromatography (Biotage Japan Ltd., eluting solvent: hexane/ethyl acetate → ethyl acetate/methanol = 10/90 to 0/100 → 100/0 to 90/10) to obtain the title compound (10.6 g, yield: 45.5%) as a solid.
$^1$H-NMR ($CDCl_3$) δ: 7.48-7.47 (2H, m), 7.39-7.38 (2H, m), 7.33-7.30 (1H, m), 5.55 (1H, s), 4.39 (1H, q, J = 6.3 Hz), 3.39 (3H, s), 3.17 (3H, s), 1.54 (3H, d, J= 6.3 Hz).

(Step 4) 5-(1-Methoxyethyl)-1-methyl-4-nitro-2-phenyl-2,3-dihydro-1H-pyrazol-3-one

5-(1-Methoxyethyl)-1-methyl-2-phenyl-2,3-dihydro-1H-pyrazol-3-one (10.6 g, 45.5 mmol) synthesized in step 3 was dissolved in trifluoroacetic acid (75 mL), and the solution was stirred under water cooling. This solution was stirred while concentrated nitric acid (7.91 mL) was gradually added thereto. After the completion of the addition, the mixture was stirred at room temperature for 2 hours. The reaction solution was poured into ice water, followed by extraction with methylene chloride. The organic layer was washed with a saturated aqueous solution of sodium bicarbonate and then dried over anhydrous magnesium sulfate. After filtration, the filtrate was concentrated under reduced pressure to obtain the title compound (11.2 g, yield: 89.2%) as a solid.
$^1$H-NMR ($CDCl_3$) δ: 7.57-7.51 (3H, m), 7.33-7.31 (2H, m), 5.67 (1H, q, J = 6.8 Hz), 3.62 (3H, s), 3.46 (3H, s), 1.64 (3H, d, J = 6.8 Hz).

(Step 5) 4-Amino-5-(1-methoxyethyl)-1-methyl-2-phenyl-2,3-dihydro-1H-pyrazol-3-one (intermediate 122a)

5-(1-Methoxyethyl)-1-methyl-4-nitro-2-phenyl-2,3-dihydro-1H-pyrazol-3-one (11.2 g, 40.5 mmol) synthesized in step 4 was dissolved in methanol (100 mL). To the solution, a 10% palladium carbon catalyst (AD) (3.13 g) was added, and the mixture was stirred at 50°C for 12 hours under a hydrogen atmosphere. The reaction solution was brought back to room temperature. After filtration, the filtrate was concentrated, and the obtained crude product was purified by amino silica gel column chromatography (Biotage Japan Ltd., eluting solvent: hexane/ethyl acetate = 50/50 to 0/100). The obtained solid was suspended in ethyl acetate, then collected by filtration, washed with ethyl acetate, and dried to obtain the title compound (intermediate 122a) (5.00 g, yield: 49.9%) as a solid.
$^1$H-NMR ($CDCl_3$) δ: 7.51-7.49 (2H, m), 7.45-7.43 (2H, m), 7.25-7.24 (1H, m), 4.33 (1H, q, J = 6.5 Hz), 3.42 (2H, br s), 3.41 (3H, s), 2.83 (3H, s), 1.54 (3H, d, J= 6.5 Hz).

(Step 6) (-)-4-Amino-5-((1S)-1-methoxyethyl)-1-methyl-2-phenyl-2,3-dihydro-1H-pyrazol-3-one (intermediate 122b) and (+)-4-amino-5-((1R)-1-methoxyethyl)-1-methyl-2-phenyl-2,3-dihydro-1H-pyrazol-3-one (intermediate 122c)

4-Amino-5-(1-methoxyethyl)-1-methyl-2-phenyl-2,3-dihydro-1H-pyrazol-3-one (intermediate 122a) (2.48 g, 10.0 mmol) synthesized in step 5 was resolved by chiral column chromatography (CHIRALCEL OD-H, hexane/2-propanol = 80/20) to obtain each of 4-amino-5-((1S)-1-methoxyethyl)-1-methyl-2-phenyl-2,3-dihydro-1H-pyrazol-3-one (intermediate 122b) (1.20 g, yield: 48.4%, >99% ee) as a solid and 4-amino-5-((1R)-1-methoxyethyl)-1-methyl-2-phenyl-2,3-dihydro-1H-pyrazol-3-one (intermediate 122c) (0.604 g, yield: 24.4%, >99% ee) as a solid. Intermediate 122b
$^1$H-NMR ($CDCl_3$) δ: 7.52-7.47 (2H, m), 7.47-7.41 (2H, m), 7.27-7.22 (1H, m), 4.33 (1H, q, J = 6.8 Hz), 3.45-3.36 (5H, m), 2.83 (3H, s), 1.53 (3H, d, J = 6.8 Hz).
$[\alpha]_D^{20}$ -94.2 (c = 1.00, MeOH).
Intermediate 53b was confirmed to be the (S) form by using (2S)-2-methoxypropanoic acid instead of 2-methoxypropanoic acid in step 1 and subsequently performing the same reaction as above up to step 5.
Intermediate 122c
$^1$H-NMR ($CDCl_3$) δ: 7.52-7.47 (2H, m), 7.47-7.41 (2H, m), 7.27-7.22 (1H, m), 4.33 (1H, q, J = 6.8 Hz), 3.45-3.36 (5H, m), 2.82 (3H, s), 1.53 (3H, d, J = 6.8 Hz).
$[\alpha]_D^{20}$ +110.2 (c = 1.00, MeOH).
The following compounds of Examples 122 to 126 were subsequently synthesized in the same way as in

Example 93 and are shown in Table 34.

[Table 34-1]

| Example No. | Structure | Intermediate used | Instrumental analysis data |
|---|---|---|---|
| 1 2 2 | 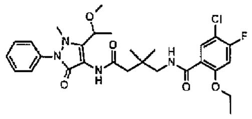 | 1 c<br>1 2 2<br>a | $^1$H-NMR ($CDCl_3$) $\delta$: 9.01 (1H, s), 8.30 (1H, d, J = 8.8 Hz), 8.23 (1H, t, J = 6.8 Hz), 7.45-7.44 (4H, m), 7.30-7.28 (1H, m), 6.78 (1H, d, J = 10.3 Hz), 4.68 (1H, q, J = 6.8 Hz), 4.18 (2H, q, J = 6.8 Hz), 3.68 (1H, dd, J = 14.2, 6.8 Hz), 3.51 (1H, dd, J = 14.2, 6.8 Hz), 3.43 (3H, s), 3.26 (3H, s), 2.28 (1H, d, J = 12.7 Hz), 2.23 (1H, d, J = 12.7 Hz), 1.63 (3H, d, J = 6.8 Hz), 1.54 (3H, t, J = 6.8 Hz), 1.12 (3H, s), 1.11 (3H, s).<br>MS (ESI) m/z : 561 [(M+H)$^+$]. |
| 1 2 3 | 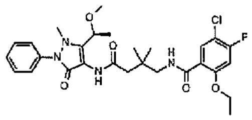 | 1 c<br>1 2 2<br>b | $^1$H-NMR ($CDCl_3$) $\delta$: 9.05 (1H, s), 8.29 (1H, d, J = 9.2 Hz), 8.23 (1H, t, J = 6,8 Hz), 7.49-7.39 (4H, m), 7.32-7.26 (1H, m), 6.78 (1H, d, J = 11.2 Hz), 4.68 (1H, q, J = 6.8 Hz), 4.18 (2H, q, J = 6.8 Hz), 3.67 (1H, dd, J = 14.0, 6.8 Hz), 3.51 (1H, dd, J = 14.0, 6.8 Hz), 3.43 (3H, s), 3.26 (3H, s), 2.28 (1H, d, J = 12.8 Hz), 2.23 (1H, d, J = 12.8 Hz), 1.63 (3H, d, J = 6.8 Hz), 1.53 (3H, t, J = 6.8 Hz), 1.12 (3H, s), 1.10 (3H, s).<br>MS (ESI) m/z : 561 [(M+H)$^+$].<br>$[\alpha]_D^{20}$ -50.5 (c = 1.00, MeOH). |
| 1 2 4 | 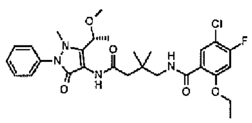 | 1 c<br>1 2 2<br>c | $^1$H-NMR ($CDCl_3$) $\delta$: 9.05 (1H, s), 8.29 (1H, d, J = 8.4 Hz), 8.23 (1H, t, J = 6.8 Hz), 7.48-7.39 (4H, m), 7.32-7.26 (1H, m), 6.78 (1H, d, J = 10.4 Hz), 4.67 (1H, q, J = 6.8 Hz), 4.18 (2H, q, J = 6.8 Hz), 3.67 (1H, dd, J = 14.0, 6.8 Hz), 3.51 (1H, dd, J = 14.0, 6.8 Hz), 3.43 (3H, s), 3.26 (3H, s), 2.28 (1H, d, J = 12.8 Hz), 2.23 (1H, d, J = 12.8 Hz), 1.64 (3H, d, J = 6.8 Hz), 1.53 (3H, t, J = 6.8 Hz), 1.11 (3H, s), 1.10 (3H, s).<br>MS (ESI) m/z : 561 [(M+H)$^+$].<br>$[\alpha]_D^{20}$ +49.1 (c = 1.00, MeOH). |
| 1 2 5 | 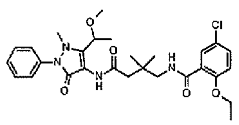 | 1 a<br>1 2 2<br>a | $^1$H-NMR ($CDCl_3$) $\delta$: 9.17 (1H, s), 8.38 (1H, t, J = 6.8 Hz), 8.20 (1H, d, J = 2.4 Hz), 7.46-7.44 (4H, m), 7.39 (1H, dd, J = 8.8, 2.4 Hz), 7.30-7.27 (1H, m), 6.91 (1H, d, J = 8.8 Hz), 4.69 (1H, q, J = 6.8 Hz), 4.20 (2H, q, J = 7.0 Hz), 3.69 (1H, dd, J = 14.2, 6.8 Hz), 3.52 (1H, dd, J = 14.2, 6.8 Hz), 3.44 (3H, s), 3.26 (3H, s), 2.29 (1H, d, J = 12.7 Hz), 2.24 (1H, d, J = 12.7 Hz), 1.63 (3H, d, J = 6.8 Hz), 1.52 (3H, t, J = 7.0 Hz), 1.13 (3H, s), 1.11 (3H, s).<br>MS (ESI) m/z : 543[(M+H)$^+$]. |

[Table 34-2]

| | | | |
|---|---|---|---|
| 126 | 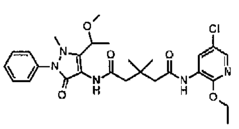 | 2 a<br>122<br>a | $^1$H-NMR ($CDCl_3$) $\delta$: 8.62 (1H, d, J = 2.4 Hz), 8.41 (1H, s), 8.39 (1H, s), 7.78 (1 H, d, J = 2.4 Hz), 7.47-7.45 (2H, 7.40-7.38 (2H, m), 7.32-7.30 (1H, m), 4.67 (1H, q, J = 6.8 Hz), 4.39 (2H, q, J = 7.0 Hz), 3.42 (3H, s), 3.27 (3H, s), 2.80 (1H, d, J = 13.7 Hz), 2.63 (1H, d, J = 13.7 Hz), 2.54 (1H, d, J = 12.7 Hz), 2.42 (1H, d, J = 12.7 Hz), 1.64 (3H, d, J = 6.8 Hz), 1.36 (3H, t, J = 7.0 Hz), 1.17 (3H, s), 1.16 (3H, s).<br>MS (ESI) m/z : 544 [(M+H)$^+$]. |

(Example 127) 5-Chloro-N-{4-[(5-ethyl-1-methyl-3-oxo-2-phenyl-2,3-dihydro-1H-pyrazol-4-yl)amino]-2,2-dimethyl-4-oxobutyl}-2-(methoxymethoxy)benzamide

[Formula 87]

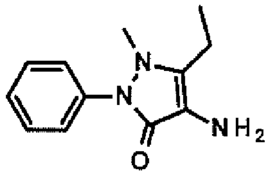

(Step 1) 4-Amino-5-ethyl-1-methyl-2-phenyl-2,3-dihydro-1H-pyrazol-3-one

The title compound was obtained as a solid by using ethyl 3-oxopentanoate instead of ethyl 4-methoxy-3-oxopentanoate in Example 113 and subsequently performing the same reaction as in Example 113 up to step 2. $^1$H-NMR ($CDCl_3$) δ: 7.50-7.47 (2H, m), 7.45-7.42 (2H, m), 7.25-7.22 (1H, m), 2.94 (2H, br s), 2.84 (3H, s), 2.56 (2H, q, J = 7.4 Hz), 1.27 (3H, t, J = 7.4 Hz).

The following compound of Example 127 was subsequently synthesized in the same way as in Example 93 and is shown in Table 35.

[Table 35]

| Example No. | Structure | Intermediate used | Instrumental analysis data |
|---|---|---|---|
| 1 2 7 | 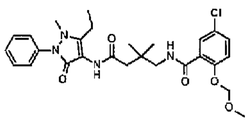 | 1 e | $^1$H-NMR ($CDCl_3$) δ: 8.41 (1H, s), 8.31 (1H, t, J = 6.8 Hz), 8.16 (1H, d, J = 2.9 Hz), 7.46-7.42 (4H, m), 7.36 (1H, dd, J = 8.8, 2.9 Hz), 7.28-7.27 (1H, m), 7.15 (1H, d, J = 8.8 Hz), 5.33 (2H, s), 3.56 (2H, d, J = 6.8 Hz), 3.50 (3H, s), 3.09 (3H, s), 2.69 (2H, q, J = 7.5 Hz), 2.28 (2H, s), 1.30 (3H, t, J = 7.5 Hz), 1.12 (6H, s).<br>MS (ESI) m/z : 529 [(M+H)$^+$]. |

(Example 128) N-(5-Chloro-2-methoxyphenyl)-N'-(5-isopropyl-1-methyl-3-oxo-2-phenyl-2,3-dihydro-1H-pyrazol-4-yl)-3,3-dimethylpentanediamide

[Formula 88]

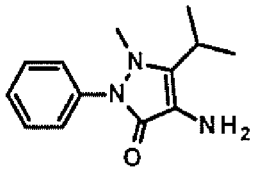

(Step 1) 4-Amino-5-isopropyl-1-methyl-2-phenyl-2,3-dihydro-1H-pyrazol-3-one

The title compound was obtained as a solid by using ethyl 4-methyl-3-oxopentanoate instead of ethyl 4-methoxy-3-oxopentanoate in step 1 of Example 113 and subsequently performing the same reaction as in Example 113 up to step 2. $^1$H-NMR ($CDCl_3$) δ: 7.50-7.41 (4H, m), 7.25-7.21 (1H, m), 3.02 (2H, s), 2.93-2.84 (4H, m), 1.38 (6H, d, J= 6.9 Hz). MS (API) m/z : 232 [(M+H)$^+$].

The following compound of Example 128 was subsequently synthesized in the same way as in Example 93 and is shown in Table 36.

[Table 36]

| Example No. | Structure | Intermediate used | Instrumental analysis data |
|---|---|---|---|
| 1 2 8 | 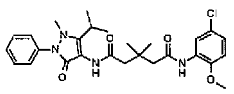 | 2 e | $^1$H-NMR ($CDCl_3$) $\delta$: 8.38-8.34 (3H, m), 7.44-7.37 (4H, m), 7.27-7.24 (1H, m), 7.00 (1H, dd, J = 9.2, 2.6 Hz), 6.76 (1H, d, J = 9.2 Hz), 3.82 (3H, s), 3.13 (3H, s), 3.09-3.03 (1H, m), 2.78 (2H, s), 2.48 (2H, s), 1.42 (6H, d, J = 6.9 Hz), 1.18 (6H, s). MS (API) m/z : 513 [(M+H)$^+$]. |

(Example 129) 5-Chloro-N-[4-({5-[4-(dimethylamino)-4-oxobutyl]-1-methyl-3-oxo-2-phenyl-2,3-dihydro-1H-pyrazol-4-yl}amino)-2,2-dimethyl-4-oxobutyl]-2-ethoxybenzamide

[Formula 89]

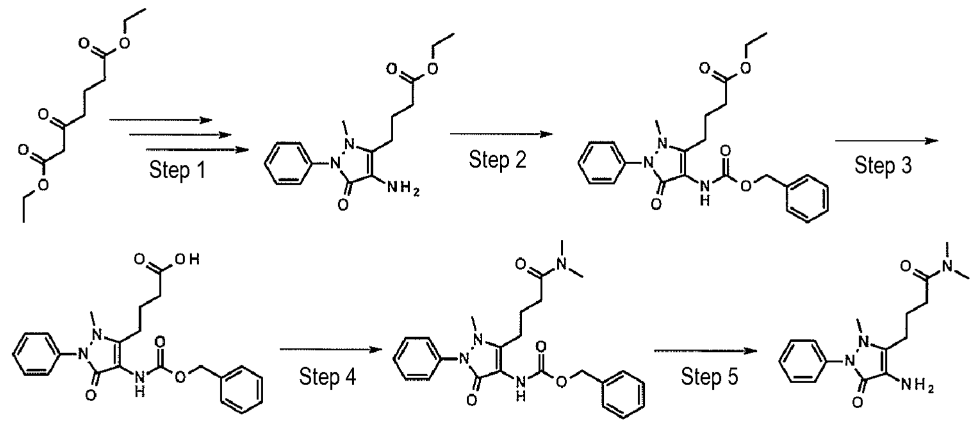


(Step 1) Ethyl 4-(4-amino-2-methyl-5-oxo-1-phenyl-2,3-dihydro-1H-pyrazol-3-yl)butanoate

The title compound was obtained as an oil substance by using diethyl 3-oxopimelate instead of ethyl 4-methoxy-3-oxopentanoate in step 2 of Example 122 and subsequently performing the same reaction as in Example 122 up to step 5. $^1$H-NMR ($CDCl_3$) $\delta$: 7.49-7.42 (4H, m) 7.25-7.22 (1H, m), 4.16 (2H, q, J = 7.1 Hz), 3.07 (2H, br s), 2.85 (3H, s), 2.61 (2H, t, J = 7.6 Hz), 2.43 (2H, t, J = 7.0 Hz), 2.01-1.94 (2H, m), 1.28 (3H, t, J = 7.1 Hz).
MS (APCI) m/z: 304 [(M+H)$^+$].

(Step 2) Ethyl 4-[4-(benzyloxycarbonylamino)-2-methyl-5-oxo-1-phenyl-2,3-dihydro-1H-pyrazol-3-yl]butanoate

Ethyl 4-(4-amino-2-methyl-5-oxo-1-phenyl-2,3-dihydro-1H-pyrazol-3-yl)butanoate (0.298 g, 0.980 mmol) synthesized in step 1 and N,N-diisopropylethylamine (0.342 mL, 0.253 g, 1.96 mmol) were dissolved in methylene chloride (10 mL). To the solution, benzyloxycarbonyl chloride (0.210 mL, 0.251 g, 1.47 mmol) was added, and the mixture was stirred at room temperature for 2 hours. The reaction solution was diluted with water, followed by extraction with chloroform. The combined extract was washed with saturated saline and dried over anhydrous sodium sulfate. Then, the solvent was distilled off under reduced pressure, and the obtained residue was purified by silica gel column chromatography (Biotage Japan Ltd., eluting solvent: hexane/ethyl acetate → ethyl acetate/methanol = 100/0 to 50/50 → 100/0 to 95/5) to obtain the title compound (0.341 g, yield: 79.5%) as a solid. $^1$H-NMR ($CDCl_3$) $\delta$: 7.48-7.28 (10H, m), 6.18 (1H, br s), 5.17 (2H, s), 4.14 (2H, q, J = 7.1 Hz), 3.09 (3H, s), 2.80-2.71 (2H, m), 2.45-2.38 (2H, m), 2.04-1.95 (2H, m), 1.27 (3H, t, J = 7.1 Hz).
MS (APCI) m/z: 438 [(M+H)$^+$].

(Step 3) 4-[4-(Benzylcarbonylamino)-2-methyl-5-oxo-1-phenyl-2,3-dihydro-1H-pyrazol-3-yl]butanoic acid

Ethyl 4-[4-(benzyloxycarbonylamino)-2-methyl-5-oxo-1-phenyl-2,3-dihydro-1H-pyrazol-3-yl]butanoate (0.341 g, 0.779 mmol) synthesized in step 2 was dissolved in tetrahydrofuran (3.0 mL) and methanol (3.0 mL). To the solution, a 1 N aqueous sodium hydroxide solution (2.24 mL) was added, and the mixture was stirred at room temperature for 2 hours. 1 N hydrochloric acid (2.24 mL) was added to the reaction solution, and then, the solvent was distilled off under reduced pressure to obtain 4-[4-(benzylcarbonylamino)-2-methyl-5-oxo-l-phenyl-2,3-dihydro-1H-pyrazol-3-yl]butanoic acid (0.319 g, quantitative) as an oil substance.
$^1$H-NMR (DMSO-$D_6$) δ: 12.17 (1H, s), 8.51 (1H, s), 7.54-7.46 (2H, m), 7.39-7.30 (8H, m), 5.08 (2H, s),3.06 (3H, s), 2.57 (2H, t, J = 7.9 Hz), 2.33 (2H, t, J = 7.0 Hz), 1.85-1.76 (2H, m).
MS (APCI) m/z: 410 [$(M+H)^+$]

(Step 4) Benzyl N-{5-[4-(dimethylamino)-4-oxobutyl]-1-methyl-3-oxo-2-phenyl-2,3-dihydro-1H-pyrazol-4-yl}carbamate

4-[4-(Benzylcarbonylamino)-2-methyl-5-oxo-1-phenyl-2,3-dihydro-1H-pyrazol-3-yl]butanoic acid (0.315 g, 0.768 mmol) synthesized in step 3, dimethylamine (9.5 M aqueous solution) (0.118 mL), 1-ethyl-3-(3-dimethylaminopropyl)carbodiimide hydrochloride (EDC·HCl) (0.323 g, 1.69 mmol), 1-hydroxybenzotriazole hydrate (HOBt·$H_2O$) (0.172 g, 1.12 mmol), and triethylamine (0.470 mL, 0.113 g, 1.12 mmol) were dissolved in N,N-dimethylformamide (10 mL), and the solution was stirred at room temperature for 5 hours. The reaction solution was diluted with water, followed by extraction with ethyl acetate. The combined extract was washed with saturated saline and dried over anhydrous sodium sulfate, and then, the solvent was distilled off under reduced pressure. The obtained residue was purified by silica gel column chromatography (Biotage Japan Ltd., eluting solvent: hexane/ethyl acetate → ethyl acetate/methanol = 100/0 to 50/50 → 100/0 to 92/8) to obtain the title compound (0.258 g, yield: 76.8%) as an oil substance. $^1$H-NMR ($CDCl_3$) δ: 7.48-7.27 (10H, m), 6.21 (1H, br s), 5.16 (2H, s), 3.16 (3H, s), 2.99 (3H, s), 2.96 (3H, s), 2.74 (2H, br s), 2.40 (2H, br s), 2.01 (2H, br s). MS (APCI) m/z: 437 [$(M+H)^+$].

(Step 5) 4-(4-Amino-2-methyl-5-oxo-1-phenyl-2,3-dihydro-1H-pyrazol-3-yl)-N,N-dimethylbutanamide

Benzyl N-{5-[4-(dimethylamino)-4-oxobutyl]-1-methyl-3-oxo-2-phenyl-2,3-dihydro-1H-pyrazol-4-yl}carbamate (0.258 g, 0.590 mmol) synthesized in step 4 was dissolved in methanol (10 mL). To the solution, a 10% palladium carbon catalyst (0.150 g) was added, and the mixture was stirred at room temperature for 4 hours under a hydrogen atmosphere. The reaction solution was filtered through celite, and the solvent in the filtrate was distilled off under reduced pressure to obtain the title compound (0.155 g, yield: 86.9%) as an oil substance.
$^1$H-NMR ($CDCl_3$) δ: 7.49-7.41 (4H, m), 7.25-7.21 (1H, m), 3.21 (2H, br s), 3.01 (3H, s), 2.97 (3H, s), 2.87 (3H, s), 2.63 (2H, t, J = 7.6 Hz), 2.41 (2H, t, J= 6.7 Hz), 2.01-1.94 (2H, m).
MS (APCI) m/z: 303 [$(M+H)^+$].

The following compound of Example 129 was subsequently synthesized in the same way as in Example 93 and is shown in Table 37.

[Table 37]

| Example No. | Structure | Intermediate used | Instrumental analysis data |
|---|---|---|---|
| 1 2 9 | 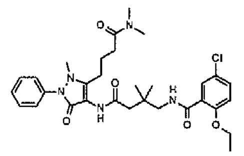 | 1 a | $^1$H-NMR (DMSO-$D_6$) δ: 9.05 (1H, s), 8.31 (1H, t, J = 6.1 Hz), 7.70-7.71 (1H, m), 7.30-7.37 (3H, m), 7.48-7.52 (3H, m), 7.18 (1H, d, J = 9.2 Hz), 4.16 (2H, q, J = 7.0 Hz), 3.30-3.31 (2H, m), 3.09 (3H, s), 2.94 (3H, s), 2.81 (3H, s), 2.55 (2H, t, J = 7.9 Hz), 2.37 (2H, t, J = 7.0 Hz), 2.21 (2H, s), 1.77-1.85 (2H, m), 1.37 (3H, t, J = 7.0 Hz), 1.02 (6H, s). MS (APCI) m/z: 598 [$(M+H)^+$]. |

(Example 130) 5-Chloro-2-ethoxy-N-(4-{[5-(4-hydroxybutyl)-1-methyl-3-oxo-2-phenyl-2,3-dihydro-1H-pyrazol-4-yl]amino}-2,2-dimethyl-4-oxobutyl)benzamide

[Formula 90]

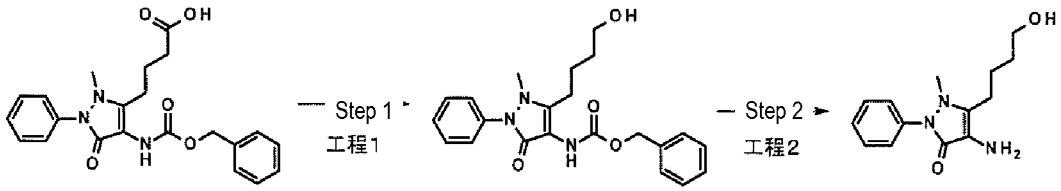


(Step 1) Benzyl N-[5-(hydroxybutyl)-1-methyl-3-oxo-2-phenyl-2,3-dihydro-1H-pyrazol-4-yl]carbamate

4-[4-(Benzylcarbonylamino)-2-methyl-5-oxo-1-phenyl-2,3-dihydro-1H-pyrazol-3-yl]butanoic acid (0.612 g, 1.49 mmol) synthesized in step 3 of Example 129 was suspended in methylene chloride (30 mL). To the suspension, oxalyl chloride (0.260 mL, 0.379 g, 2.99 mmol) was added at room temperature, and the mixture was stirred at room temperature for 1 hour. The solvent was distilled off under reduced pressure, and the obtained residue was dissolved in tetrahydrofuran (20 mL). The solution was added dropwise to a suspension of lithium borohydride (0.0650 g, 2.99 mmol) in tetrahydrofuran (20 mL) at 0°C, and the mixture was stirred at 0°C for 3 hours. 1 N hydrochloric acid was added to the reaction solution, followed by extraction with ethyl acetate. The organic layer was washed with saturated saline and then dried over anhydrous sodium sulfate. After filtration, the filtrate was concentrated to obtain the title compound (0.583 g, yield: 98.9%) as a solid.
$^1$H-NMR ($CDCl_3$) δ: 7.47-7.28 (11H, m), 6.39 (1H, br s), 5.16 (2H, s), 3.70-3.63 (2H, m), 3.06 (3H, s), 2.73-2.65 (2H, m), 1.79-1.65 (4H, m).
MS (APCI) m/z: 396 [$(M+H)^+$].

(Step 2) 4-Amino-5-(4-hydroxybutyl)-1-methyl-2-phenyl-2,3-dihydro-1H-pyrazol-3-one

Benzyl N-[5-(hydroxybutyl)-1-methyl-3-oxo-2-phenyl-2,3-dihydro-1H-pyrazol-4-yl]carbamate (0.583 g, 1.47 mmol) synthesized in step 1 was dissolved in methanol (20 mL). To the solution, a 10% palladium carbon catalyst (0.300 g) was added, and the mixture was stirred at room temperature for 2 hours under a hydrogen atmosphere. The reaction solution was filtered through celite, and the solvent in the filtrate was distilled off under reduced pressure to obtain the title compound (0.363 g, yield: 94.5%) as an oil substance.
$^1$H-NMR ($CDCl_3$) δ: 7.49-7.41 (4H, m), 7.25-7.23 (1H, m), 3.71 (2H, t, J = 6.1 Hz), 2.84 (3H, s), 2.59 (2H, t, J = 7.6 Hz), 2.53 (2H, br s), 1.80-1.73 (2H, m), 1.70-1.63 (2H, m).
MS (APCI) m/z: 262 [$(M+H)^+$].

The following compound of Example 130 was subsequently synthesized in the same way as in Example 93 and is shown in Table 38.

[Table 38]

| Example No. | Structure | Intermediate used | Instrumental analysis data |
|---|---|---|---|
| 1 3 0 | 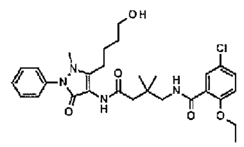 | 1 a | $^1$H-NMR (DMSO-$D_6$) δ: 9.05 (1H, s), 8.31 (1H, t, J = 6.4 Hz), 7.72-7.71 (1H, m), 7.52-7.48 (3H, m), 7.36-7.29 (3H, m), 7.18 (1H, d, J = 9.2 Hz), 4.43 (1H, t, J = 5.2 Hz), 4.16 (2H, q, J = 7.1 Hz), 3.42 (2H, q, J = 5.9 Hz), 3.30 (2H, d, J = 6.1 Hz), 3.05 (3H, s), 2.54 (2H, t, J = 7.6 Hz), 2.22 (2H, s), 1.66-1.58 (2H, m), 1.52-1.45 (2H, m), 1.38 (3H, t, J = 7.1 Hz), 1.02 (6H, s).<br>MS (APCI) m/z: 557 [$(M+H)^+$]. |

(Example 131) Methyl 2-[4-({4-[(5-chloro-2-ethoxybenzoyl)amino]-3,3-dimethylbutanoyl}amino)-2-methyl-5-oxo-1-phenyl-2,3-dihydro-1H-pyrazol-3-yl]acetate

[Formula 91]

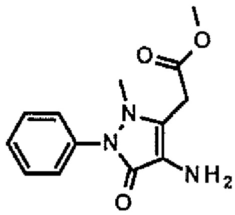

(Step 1) Methyl 2-(4-amino-2-methyl-5-oxo-1-phenyl-2,3-dihydro-1H-pyrazol-3-yl)acetate

The title compound was obtained as a solid by using dimethyl 3-oxoglutarate instead of diethyl 3-oxopimelate in step 1 of Example 129 and subsequently performing the same reaction as in Example 129 up to step 5. $^1$H-NMR ($CDCl_3$) δ: 7.49-7.42 (4H, m), 7.28-7.23 (1H, m), 3.78 (3H, s), 3.56 (2H, s), 3.29 (2H, s), 2.85 (3H, s). MS (API) m/z: 262 [(M+H)$^+$].

The following compound of Example 131 was subsequently synthesized in the same way as in Example 93 and is shown in Table 39.

[Table 39]

| Example No. | Structure | Intermediate used | Instrumental analysis data |
|---|---|---|---|
| 1 3 1 | 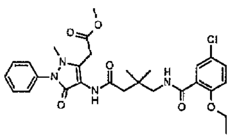 | 1 a | $^1$H-NMR ($CDCl_3$) δ: 9.00 (1H, s), 8.36 (1H, t, J = 6.7 Hz), 8.22 (1H, d, J = 2.4 Hz), 7.48-7.43 (4H, m), 7.37 (1H, dd, J = 9.2, 2.4 Hz), 7.31-7.27 (1H, m), 6.90 (1H, d, J = 9.2 Hz), 4.20 (2H, q, J = 7.0 Hz), 3.87 (2H, s), 3.77 (3H, s), 3.55 (2H, d, J = 6.7 Hz), 3.12 (3H, s), 2.28 (2H, s), 1.52 (3H, t, J = 7.0 Hz), 1.11 (6H, s).<br>MS (API) m/z: 557 [(M+H)$^+$]. |

(Example 132) N-(4-{[5-(2-Amino-2-oxoethyl)-1-methyl-3-oxo-2-phenyl-2,3-dihydro-1H-pyrazol-4-yl]amino}-2,2-dimethyl-4-oxobutyl)-5-chloro-2-ethoxybenzamide

[Formula 92]

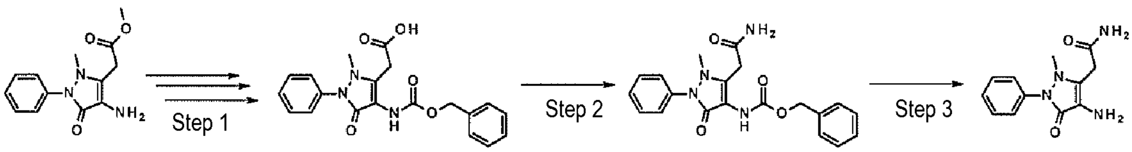


(Step 1) 2-[4-(Benzyloxycarbamoylamino)-2-methyl-5-oxo-1-phenyl-2,3-dihydro-1H-pyrazol-3-yl]acetic acid

The title compound was obtained as a solid by using methyl 2-(4-amino-2-methyl-5-oxo-1-phenyl-2,3-dihydro-1H-pyrazol-3-yl)acetate synthesized in step 1 of Example 131 instead of ethyl 4-(4-amino-2-methyl-5-oxo-1-phenyl-2,3-dihydro-1H-pyrazol-3-yl)butanoate in step 2 of Example 129 and subsequently performing the same reaction as in Example 129 up to step 3.
$^1$H-NMR (DMSO-$D_6$) δ: 7.63-7.57 (3H, m), 7.44-7.43 (2H, m), 4.37 (2H, s), 3.71 (3H, s), 3.48 (3H, s). MS (APCI) m/z: 382 [(M+H)$^+$].

(Step 2) Benzyl N-[5-(2-amino-2-oxoethyl)-1-methyl-3-oxo-2-phenyl-2,3-dihydro-1H-pyrazol-4-yl]carbamate

2-[4-(Benzyloxycarbamoylamino)-2-methyl-5-oxo-1-phenyl-2,3-dihydro-1H-pyrazol-3-yl]acetic acid (1.56 g,

4.09 mmol) synthesized in step 1 was dissolved in N,N-dimethylformamide (20 mL). To the solution, ammonium chloride (0.440 g, 8.18 mmol), 1-ethyl-3-(3-dimethylaminopropyl)carbodiimide hydrochloride (EDC·HCl) (1.18 g, 4.09 mmol), 1-hydroxybenzotriazole hydrate (HOBt·$H_2O$), (0.550 g, 4.09 mmol), and N,N-diisopropylethylamine (2.85 mL, 2.11 g, 16.4 mmol) were added, and the mixture was stirred at room temperature for 5 days. Water and a saturated aqueous solution of sodium bicarbonate were added to the reaction mixture, followed by extraction with ethyl acetate. The organic layer was washed with water and saturated saline and then dried over anhydrous magnesium sulfate. After filtration, the filtrate was concentrated under reduced pressure, and the obtained residue was purified by silica gel column chromatography (Biotage Japan Ltd., eluting solvent: hexane/10% methanol-ethyl acetate = 70/30 to 0/100) to obtain the title compound (1.45 g, yield: 93.2%) as a solid.
$^1$H-NMR ($CDCl_3$) δ: 7.83 (1H, br s), 7.48-7.44 (2H, m), 7.39-7.31 (8H, m), 7.26 (1H, br s), 5.47 (1H, br s), 5.16 (2H, s), 3.57 (2H, s), 3.18 (3H, s).
MS (APCI) m/z: 381 [$(M+H)^+$].

(Step 3) 2-(4-Amino-2-methyl-5-oxo-1-phenyl-2,3-dihydro-1H-pyrazol-3-yl)acetamide

Benzyl N-[5-(2-amino-2-oxoethyl)-1-methyl-3-oxo-2-phenyl-2,3-dihydro-1H-pyrazol-4-yl]carbamate (0.824 g, 2.16 mmol) synthesized in step 2 was dissolved in methanol (30 mL). To the solution, a 10% palladium carbon catalyst (M type) (0.300 g) was added, and the mixture was stirred for 5 hours under a hydrogen atmosphere. The reaction solution was filtered through celite to remove the catalyst. Then, the solvent was distilled off under reduced pressure to obtain the title compound (0.484 g, yield: 90.7%) as a solid.
$^1$H-NMR (DMSO-$D_6$) δ: 7.45-7.49 (3H, m), 7.40-7.42 (2H, m), 7.24-7.26 (1H, m), 7.11 (1H, br s), 3.98-4.00 (2H, m), 3.41 (2H, s), 2.76 (3H, s).
MS (APCI) m/z: 247 [$(M+H)^+$].

The following compound of Example 132 was subsequently synthesized in the same way as in Example 93 and is shown in Table 40.

[Table 40]

| Example No. | Structure | Intermediate used | Instrumental analysis data |
|---|---|---|---|
| 1 3 2 | 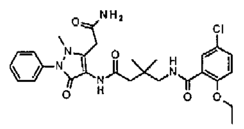 | 1 a | $^1$H-NMR (DMSO-$D_6$) δ: 9.22 (1H, s), 8.30 (1H, t, J = 6.4 Hz), 7.71-7.70 (1H, m), 7.55-7.45 (4H, m), 7.35-7.32 (3H, m), 7.179-7.17 (2H, m), 4.16 (2H, q, J = 7.1 Hz), 3.52 (2H, s), 3.31-3.30 (2H, m), 3.07 (3H, s), 2.25 (2H, s), 1.38 (3H, t, J = 7.1 Hz), 1.02 (6H, s).<br>MS (APCI) m/z: 542 [$(M+H)^+$]. |

(Example 133) 5-Chloro-N-(2,2-dimethyl-4-{[1-methyl-3-oxo-5-(3-oxo-3-pyrrolidin-1-ylpropyl)-2-phenyl-2,3-dihydro-1H-pyrazol-4-yl]amino}-4-oxobutyl)-2-ethoxybenzamide

[Formula 93]

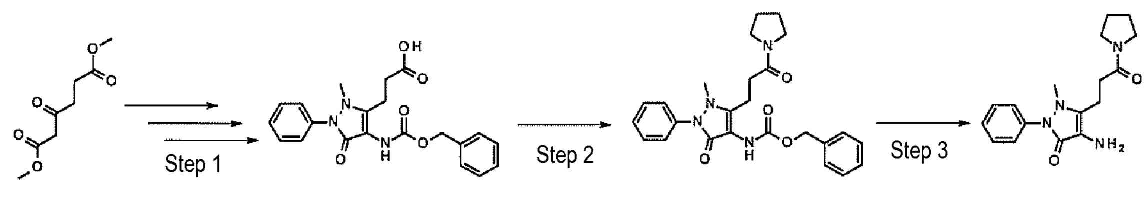


(Step 1) 3-[4-(Benzyloxycarbonylamino)-2-methyl-5-oxo-1-phenyl-2,3-dihydro-1H-pyrazol-3-yl]propanoic acid

The title compound was obtained as a solid by using dimethyl 3-oxoadipate instead of diethyl 3-oxopimelate in step 1 of Example 129 and subsequently performing the same reaction as in Example 129 up to step 3. $^1$H-NMR

($CDCl_3$) δ: 7.45-7.41 (2H, m), 7.36-7.26 (8H, m), 5.13 (2H, s), 3.05 (3H, br s), 2.88 (2H, t, J = 6.4 Hz), 2.64 (2H, t, J = 6.4 Hz).

(Step 2) Benzyl N-[1-methyl-3-oxo-5-(3-oxo-3-pyrrolidin-1-ylpropyl)-2-phenyl-2,3-dihydro-1H-pyrazol-4-yl]carbamate

3-[4-(Benzyloxycarbonylamino)-2-methyl-5-oxo-1-phenyl-2,3-dihydro-1H-pyrazol-3-yl]propanoic acid (0.164 g, 0.410 mmol) synthesized in step 1, pyrrolidine (0.035 mL, 0.029 g, 0.410 mmol), 1-ethyl-3-(3-dimethylaminopropyl)carbodiimide hydrochloride (EDC·HCl) (0.119 g, 0.620 mmol), 1-hydroxybenzotriazole hydrate (HOBt·$H_2O$) (0.064 g, 0.410 mmol), and triethylamine (0.173 mL, 0.125 g, 1.24 mmol) were dissolved in N,N-dimethylformamide (10 mL), and the solution was stirred at room temperature for 6 hours. Water was added to the reaction solution, followed by extraction with ethyl acetate. The organic layer was washed with saturated saline and then dried over anhydrous sodium sulfate. After filtration, the filtrate was concentrated, and the obtained crude product was purified by silica gel column chromatography (Biotage Japan Ltd., eluting solvent: hexane/ethyl acetate → ethyl acetate/methanol = 100/0 to 50/50 → 100/0 to 90/10) to obtain the title compound (0.155 g, yield: 84.3%) as a solid.
$^1$H-NMR ($CDCl_3$) δ: 7.47-7.29 (10H, m), 6.41 (1H, br s), 5.16 (2H, s), 3.47 (2H, t, J = 6.7 Hz), 3.36 (2H, br s), 3.12 (3H, s), 3.02 (2H, t, J = 7.0 Hz), 2.67 (2H, br s), 1.97-1.83 (4H, m).
MS (APCI) m/z: 449 [$(M+H)^+$].

(Step 3) 4-Amino-1-methyl-5-(3-oxo-3-pyrrolidin-1-ylpropyl)-2-phenyl-2,3-dihydro-1H-pyrazol-3-one

Benzyl N-[1-methyl-3-oxo-5-(3-oxo-3-pyrrolidin-1-ylpropyl)-2-phenyl-2,3-dihydro-1H-pyrazol-4-yl]carbamate (0.155 g, 0.350 mmol) synthesized in step 2 was dissolved in methanol (10 mL). To the solution, a 10% palladium carbon catalyst (AD type) (0.150 g) was added, and the mixture was stirred at room temperature for 4 hours under a hydrogen atmosphere. The reaction solution was filtered through celite, and the solvent in the filtrate was distilled off under reduced pressure to obtain the title compound (0.106 g, yield: 96.3%) as an oil substance.
$^1$H-NMR ($CDCl_3$) δ: 7.48-7.38 (4H, m), 7.32-7.27 (1H, m), 4.11 (2H, br s), 3.49-3.43 (4H, m), 3.20-3.13 (1H, m), 3.08-2.99 (4H, m), 2.74-2.65 (2H, m), 2.00-1.93 (2H, m), 1.89-1.82 (2H, m).
MS (APCI) m/z: 315 [$(M+H)^+$].
The following compound of Example 133 was subsequently synthesized in the same way as in Example 93 and is shown in Table 41.

[Table 41]

| Example No. | Structure | Intermediate used | Instrumental analysis data |
|---|---|---|---|
| 1 3 3 | 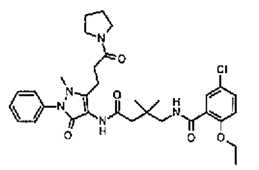 | 1 a | $^1$H-NMR (DMSO-$D_6$) δ: 9.09 (1H, s), 8.31 (1H, t, J = 6.4 Hz), 7.71 (1H, d, J = 2.4 Hz), 7.52-7.48 (3H, m), 7.36-7.30 (3H, m), 7.18 (1H, d, J = 8.5 Hz), 4.16 (2H, q, J = 7.0 Hz), 3.40-3.37 (2H, m), 3.32-3.28 (4H, m), 3.08 (3H, s), 2.78-2.74 (2H, m), 2.63-2.59 (2H, m), 2.22 (2H, s), 1.88-1.81 (2H, m), 1.78-1.72 (2H, m), 1.37 (3H, t, J = 7.0 Hz), 1.01 (6H, s). MS (APCI) m/z: 610 [$(M+H)^+$]. |

(Example 134) 5-Chloro-N-[2,2-dimethyl-4-({1-methyl-5-[(1-methylazetidin-3-yl)oxymethyl]-3-oxo-2-phenyl-2,3-dihydro-1H-pyrazol-4-yl}amino)-4-oxobutyl-2-methoxybenzamide

[Formula 94]

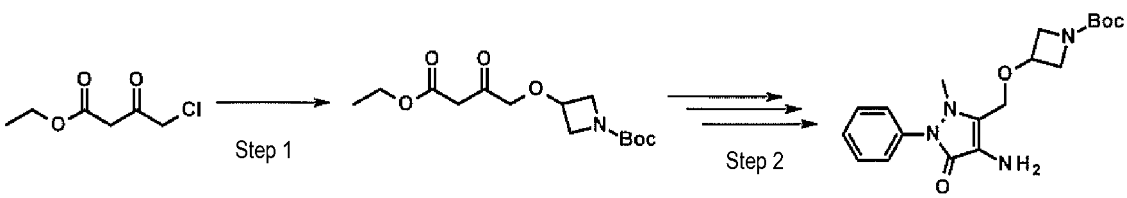

(Step 1) tert-Butyl 3-(4-ethoxy-2,4-dioxobutoxy)azetidine-1-carboxylate

To a solution of tert-butyl 3-hydroxyazetidine-1-carboxylate (0.866 g, 5.00 mmol) in tetrahydrofuran (25.0 mL), sodium hydride (0.480 mg, 11.0 mmol) was added under ice cooling, and the mixture was stirred at room temperature for 30 minutes under a nitrogen atmosphere and then cooled in ice again. Ethyl 4-chloro-3-oxobutanoate (0.680 mL, 0.823 g, 5,00 mmol) was added thereto, and the mixture was stirred overnight at room temperature. 1 N hydrochloric acid was added to the reaction solution, followed by extraction with ethyl acetate three times. The organic layer was washed with saturated saline and then dried over anhydrous sodium sulfate. After filtration, the filtrate was concentrated under reduced pressure, and then, the obtained residue was purified by silica gel column chromatography (Yamazen Corp., eluting solvent: hexane/ethyl acetate = 68/32 to 47/53) to obtain the title compound (1.15 g, yield: 76.3%) as an oil substance.
$^1$H-NMR ($CDCl_3$) δ: 4.30-4.07 (7H, m), 3.90-3.85 (2H, m), 3.52 (2H, s), 1.44 (9H, s), 1.33-1.24 (3H, m).

(Step 2) tert-Butyl 3-[(4-amino-2-methyl-5-oxo-1-phenyl-2,3-dihydro-1H-pyrazol-3-yl)methoxy]azetidine-1-carboxylate

The title compound was obtained as a solid by using tert-butyl 3-(4-ethoxy-2,4-dioxobutoxy)azetidine-1-carboxylate synthesized in step 1 instead of ethyl 5-(tert-butoxycarbonylamino)-3-oxopentanoate in step 2 of Example 10 and subsequently performing synthesis in the same way as in Example 10 from step 2 up to step 6. $^1$H-NMR ($CDCl_3$) δ: 7.50-7.41 (4H, m), 7.31-7.25 (1H, m), 4.41-4.35 (3H, m), 4.16-4.10 (2H, m), 3.92 (2H, dd, J= 9.8, 4.3 Hz), 3.30 (2H, s), 2.85 (3H, s), 1.45 (9H, s). MS (ESI) m/z: 375 [(M+H)$^+$].

The following compound of Example 134 was subsequently synthesized through the same reaction as in step 7 of Example 10 and steps 1 and 2 of Example 11 and is shown in Table 42.

[Table 42]

| Example No. | Structure | Intermediate used | Instrumental analysis data |
|---|---|---|---|
| 1 3 4 | 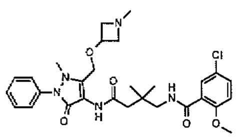 | 1 b | $^1$H-NMR ($CDCl_3$) δ: 8.98 (1H, s), 8.26 (1H, t, J = 6.7 Hz), 8.20 (1H, d, J = 2.4 Hz), 7.48-7.38 (5H, m), 7.32-7.28 (1H, m), 6.93 (1H, d, J = 8.5 Hz), 4.52 (2H, s), 4.25-4.20 (1H, m), 3.97 (3H, s), 3.66-3.62 (2H, m), 3.55 (2H, d, J = 6.7 Hz), 3.20 (3H, s), 3.00-2.96 (2H, m), 2.36 (3H, s), 2.28 (2H, s), 1.11 (6H, s). MS (ESI) m/z: 570 [(M+H)$^+$]. |

(Example 135) 5-Chloro-N-(2,2-dimethyl-4-{[1-methyl-3-oxo-2-phenyl-5-(3-pyridyloxymethyl)pyrazol-4-yl]amino}-4-oxobutyl)-2-ethoxybenzamide

[Formula 95]

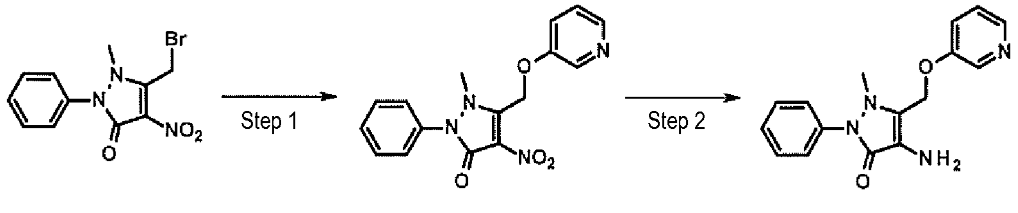


(Step 1) 1-Methyl-4-nitro-2-phenyl-5-(3-pyridyloxymethyl)pyrazol-3-one

To a suspension of sodium hydride (0.096 g, 2.20 mmol) in dimethoxyethane (20 mL), 3-hydroxypyridine (0.228 g, 2.40 mmol) was added under ice cooling, and the mixture was stirred at room temperature for 40 minutes. Then, 5-(bromomethyl)-1-methyl-4-nitro-2-phenyl-2,3-dihydro-1H-pyrazol-3-one (0.624 g, 2.00 mmol) synthesized in step 2 of Example 2 was added thereto, and the mixture was stirred at room temperature for 30 minutes. Water was added to the

reaction solution, followed by extraction with ethyl acetate three times. The organic layer was washed with saturated saline and then dried over anhydrous sodium sulfate. After filtration, the filtrate was concentrated under reduced pressure, and then, the obtained residue was purified by silica gel column chromatography (Yamazen Corp., eluting solvent: ethyl acetate/methanol = 99/1 to 68/32) and amino silica gel column chromatography (Yamazen Corp., eluting solvent: ethyl acetate/methanol = 99/1 to 88/12) to obtain the title compound (0.0840 g, yield: 12.9%) as a solid.
$^1$H-NMR ($CDCl_3$) $\delta$: 8.44 (1H, d, J= 2.7 Hz), 8.37-8.35 (1H, m), 7.58-7.52 (3H, m), 7.43-7.40 (1H, m), 7.33-7.30 (3H, m), 5.77 (2H, s), 3.60 (3H, s).
MS (APCI) m/z: 327 [$(M+H)^+$].

(Step 2) 4-Amino-1-methyl-2-phenyl-5-(3-pyridyloxymethyl)pyrazol-3-one

To a solution of 1-methyl-4-nitro-2-phenyl-5-(3-pyridyloxymethyl)pyrazol-3-one (0.0800 g, 0.245 mmol) synthesized in step 1 in ethanol (5.0 mL), a 10% palladium carbon catalyst (aqueous) (0.080 g) was added, and the mixture was stirred at room temperature for 5.5 hours under a hydrogen atmosphere. After purging with nitrogen, the reaction solution was filtered, and the filtrate was concentrated under reduced pressure. The obtained residue was purified by silica gel column chromatography (Yamazen Corp., eluting solvent: methylene chloride/methanol = 99/1 to 88/12) to obtain the title compound (0.055 g, yield: 75.7%) as a solid.
$^1$H-NMR ($CDCl_3$) $\delta$: 8.43 (1H, d, J = 3.1 Hz), 8.31 (1H, dd, J = 4.9, 1.2 Hz), 7.50-7.28 (7H, m), 5.04 (2H, s), 3.38 (2H, s), 2.93 (3H, s).
MS (API) m/z: 297 [$(M+H)^+$].
The following compound of Example 135 was subsequently synthesized in the same way as in Example 92 and is shown in Table 43.

[Table 43]

| Example No. | Structure | Intermediate used | Instrumental analysis data |
|---|---|---|---|
| 1 3 5 | 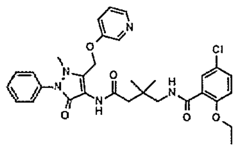 | 1 a | $^1$H-NMR ($CDCl_3$) $\delta$: 9.49 (1H, s), 8.42 (1H, d, J = 2.4 Hz), 8.37 (1H, t, J = 6.8 Hz), 8.28 (1H, d, J = 3.9 Hz), 8.22 (1H, d, J = 2.9 Hz), 7.46-7.34 (6H, m), 7.31-7.24 (2H, m), 6.90 (1H, d, J = 8.8 Hz), 5.24 (2H, s), 4.19 (2H, q, J = 7.0 Hz), 3.57 (2H, d, J = 6.8 Hz), 3.22 (3H, s), 2.31 (2H, s), 1.51 (3H, t, J = 7.0 Hz), 1.11 (6H, s).<br>MS (APCI) m/z: 592 [$(M+H)^+$]. |

(Example 136) N-(5-Chloro-2-methoxyphenyl)-3,3-dimethyl-N'-(1-methyl-3-oxo-2-phenyl-2,3-dihydro-1H-pyrazol-4-yl)pentanediamide

[Formula 96]

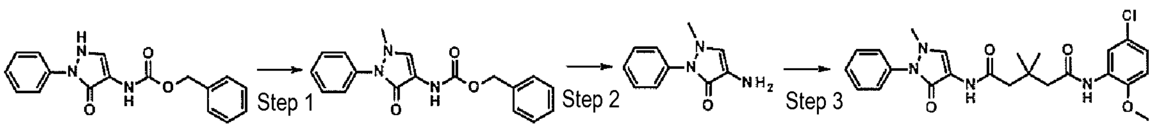


(Step 1) Benzyl N-(1-methyl-3-oxo-2-phenyl-2,3-dihydro-1H-pyrazol-4-yl)carbamate

To a solution of N-(3-oxo-2-phenyl-1H-pyrazol-4-yl)carbamate (309 mg, 1.00 mmol) synthesized by the method described in J. Heterocycl. Chem. 2006, 43, 1205-1215 in N,N-dimethylformamide (3.0 ml), methyl iodide (0.190 ml, 426 mg, 3.0 mmol) was added at room temperature, and the mixture was stirred at 80°C for 3 hours in a sealed tube. The reaction solution was allowed to cool, and then, water was added to the reaction solution, followed by extraction with ethyl acetate. The organic layer was washed with water and saturated saline in this order and then dried over anhydrous sodium sulfate. After filtration, the filtrate was concentrated, and the obtained crude product was purified by silica gel column chromatography (eluting solvent: hexane/ethyl acetate = 34/66) to obtain the title compound (110 mg,

yield: 34.0%) as a solid. $^1$H-NMR ($CDCl_3$) δ: 7.70 (1H, s), 7.48-7.30 (10H, m), 6.86 (1H, brs), 5.21 (2H, s), 2.99 (3H, s).

(Step 2) 4-Amino-1-methyl-2-phenyl-2,3-dihydro-1H-pyrazol-3-one

To a solution of benzyl N-(1-methyl-3-oxo-2-phenyl-2,3-dihydro-1H-pyrazol-4-yl)carbamate (100 mg, 0.309 mmol) synthesized in step 1 in ethanol (5.0 ml), cyclohexene (0.25 ml) and a 5% palladium carbon (50 mg) were added, and the mixture was heated to reflux for 30 minutes. The reaction solution was allowed to cool and then filtered through celite to remove insoluble matter. The filtrate was concentrated, and the obtained crude product was purified by silica gel column chromatography (eluting solvent: ethyl acetate alone) to obtain the title compound (50.0 mg, yield: 85.5%) as a solid. $^1$H-NMR (DMSO-$D_6$) δ: 7.49-7.41 (4H, m), 7.28-7.24 (1H, m), 6.92 (1H, s), 4.11 (2H, brs), 2.75 (3H, s).

(Step 3) N-(5-Chloro-2-methoxyphenyl)-3,3-dimethyl-N'-(1-methyl-3-oxo-2-phenyl-2,3-dihydro-1H-pyrazol-4-yl)pentanediamide

4-Amino-1-methyl-2-phenyl-2,3-dihydro-1H-pyrazol-3-one (50.0 mg, 0.264 mmol) synthesized in step 2 and intermediate 2e (80.0 mg, 0.267 mmol) were dissolved in N,N-dimethylformamide (2.0 ml). To the solution, N,N-diisopropylethylamine (0.0923 ml, 68.5 mg, 0.530 mmol) and O-(7-azabenzotriazol-1-yl)-N,N,N',N'-tetramethyluronium hexafluorophosphate (HATU) (100 mg, 0.264 mmol) were added at room temperature, and the mixture was stirred at room temperature for 16 hours. Water was added to the reaction solution, followed by extraction with ethyl acetate. The organic layer was washed with water and saturated saline in this order and then dried over anhydrous sodium sulfate. After filtration, the filtrate was concentrated, and the obtained crude product was purified by silica gel column chromatography (eluting solvent: methanol/chloroform = 2/98) to obtain the title compound (58.0 mg, yield: 46.1%) as a solid.
$^1$H-NMR (DMSO-$D_6$) δ: 9.83 (1H, s), 9.61 (1H, s), 8.29 (1H, s), 8.16 (1H, d, J = 2.3 Hz), 7.54-7.51 (2H, m), 7.42-7.40 (2H, m), 7.37-7.33 (1H, m), 7.11-7.03 (2H, m), 3.82 (3H, s), 3.03 (3H, s), 2.51-2.47 (4H, m), 1.06 (6H, s).
MS (APCI) m/z : 471 [(M+H)$^+$].

(Example 137) 5-Chloro-2-ethoxy-N-{4-[(1-ethyl-5-methyl-3-oxo-2-phenyl-2,3-dihydro-1H-pyrazol-4-yl)amino]-2,2-dimethyl-4-oxobutyl}benzamide

[Formula 97]

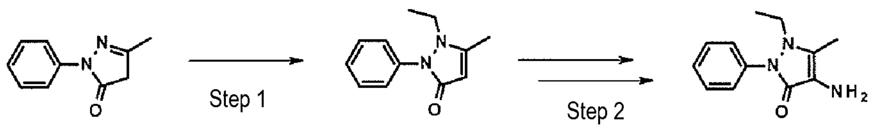


(Step 1) 1-Ethyl-5-methyl-2-phenyl-2,3-dihydro-1H-pyrazol-3-one

To a solution of 5-methyl-2-phenyl-1H-pyrazol-3-one (554 mg, 3.18 mmol) in N,N-dimethylformamide (3.2 mL), ethyl iodide (0.645 mL, 1.25 g, 8.01 mmol) was added, and the mixture was stirred under heating at 100°C for 6 hours in a sealed tube. The reaction mixture was cooled to room temperature and then concentrated under reduced pressure, and a saturated aqueous solution of sodium bicarbonate (15 mL) was added to the obtained residue, followed by extraction with ethyl acetate twice. The organic layers were combined and dried over anhydrous sodium sulfate. The solvent was distilled off, and the obtained residue was purified by silica gel column chromatography (Biotage Japan Ltd., eluting solvent: ethyl acetate/methanol = 100/0 to 82/18) to obtain the title compound (323 mg, yield: 50.3%) as an oil substance.
$^1$H-NMR ($CDCl_3$) δ: 7.48-7.43 (2H, m), 7.41-7.38 (2H, m), 7.31-7.26 (1H, m), 5.44 (1H, s), 3.57 (2H, q, J = 7.1 Hz), 2.24 (3H, s), 0.88 (3H, t, J= 6.9 Hz).
MS (ESI) m/z: 203 [(M+H)$^+$]

(Step 2) 4-Amino-1-ethyl-5-methyl-2-phenyl-2,3-dihydro-1H-pyrazol-3-one

The title compound was obtained as an oil substance by using 1-ethyl-5-methyl-2-phenyl-2,3-dihydro-1H-pyrazol-3-one synthesized in step 1 instead of 5-(1-methoxyethyl)-1-methyl-2-phenyl-2,3-dihydro-1H-pyrazol-3-one in step 4 of Example 122 and subsequently performing the same reaction as in Example 122 up to step 5.

$^1$H-NMR ($CDCl_3$) δ: 7.49-7.41 (4H, m), 7.26-7.22 (1H, m), 3.42 (2H, q, J = 7.1 Hz), 2.91 (2H, br s), 2.14 (3H, s), 0.73 (3H, t, J = 6.9 Hz).
MS (ESI) m/z: 218 [$(M+H)^+$].

The following compound of Example 137 was subsequently synthesized in the same way as in Example 93 and is shown in Table 44.

[Table 44]

| Example No. | Structure | Intermediate used | Instrumental analysis data |
|---|---|---|---|
| 1 3 7 | 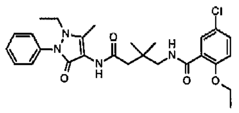 | 1 a | $^1$H-NMR ($CDCl_3$) δ: 8.81 (1H, s), 8.36 (1H, t, J = 6.5 Hz), 8.21 (1H, d, J = 2.7 Hz), 7.45-7.41 (4H, m), 7.37 (1H, dd, J = 8.6, 2.7 Hz), 7.29-7.24 (3H, m), 6.90 (1H, d, J = 8.6 Hz), 4.19 (2H, q, J = 7.0 Hz), 3.62-3.57 (4H, m), 2.28 (2H, s), 2.25 (3H, s), 1.52 (3H, t, J = 7.0 Hz), 1.11 (6H, s), 0.89 (3H, t, J = 7.0 Hz).<br>MS (ESI+APCI) m/z : 513 [$(M+H)^+$]. |

(Example 138) N-(5-Chloro-4-fluoro-2-methoxyphenyl)-3,3-dimethyl-N'-(3-methyl-5-oxo-1-phenyl-2-propylpyrazol-4-yl)pentanediamide

[Formula 98]

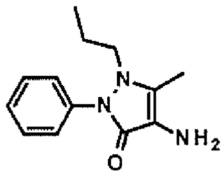

(Step 1) 4-Amino-5-methyl-2-phenyl-1-propylpyrazol-3-one

The title compound was obtained as an oil substance by using propyl iodide instead of ethyl iodide in step 1 of Example 137 and subsequently performing the same reaction as in Example 137 up to step 2.
$^1$H-NMR ($CDCl_3$) δ: 7.46-7.41 (4H, m), 7.27-7.23 (1H, m), 3.34-3.29 (2H, m), 2.87 (2H, br s), 2.15 (3H, s), 1.24-1.16 (2H, m), 0.72 (3H, t, J = 7.4 Hz).
MS (ESI) m/z : 232 [$(M+H)^+$].

The following compound of Example 138 was subsequently synthesized in the same way as in Example 93 and is shown in Table 45.

[Table 45]

| Example No. | Structure | Intermediate used | Instrumental analysis data |
|---|---|---|---|
| 138 | 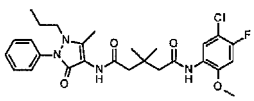 | 2d | $^1$H-NMR ($CDCl_3$) δ: 8.66 (1H, br s), 8.40 (1H, d, J = 8.0 Hz), 8.36 (1H, br s), 7.47-7.42 (2H, m), 7.41-7.36 (2H, m), 7.32-7.27 (1H, m), 6.66 (1H, d, J = 10.3 Hz), 3.80 (3H, s), 3.54-3.49 (2H, m), 2.63 (2H, s), 2.45 (2H, s), 2.28 (3H, s), 1.41-1.32 (2H, m), 1.14 (6H, s), 0.77 (3H, t, J = 7.4 Hz).<br>MS (ESI) m/z : 531 [$(M+H)^+$]. |

(Example 139) 5-Chloro-N-(4-{[1-(2-hydroxyethyl)-5-methyl-3-oxo-2-phenyl-2,3-dihydro-1H-pyrazol-4-yl]amino}-2,2-dimethyl-4-oxobutyl)-2,2-dimethyl-3H-benzofuran-7-carboxamide

[Formula 99]

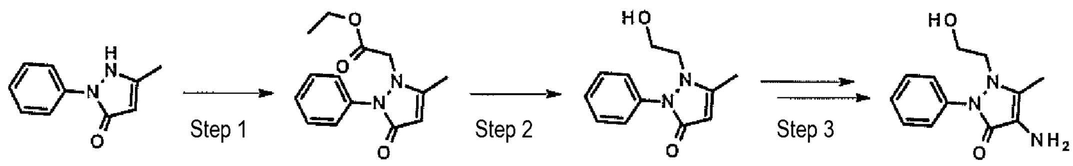


(Step 1) Ethyl 2-(5-methyl-3-oxo-2-phenyl-2,3-dihydro-1H-pyrazol-1-yl)acetate

To a mixture of 5-methyl-2-phenyl-1H-pyrazol-3-one (8.02 g, 46.0 mmol) and potassium iodide (1.52 g, 9.16 mmol), N,N-dimethylformamide (17 mL) and ethyl 2-bromoacetate (10.3 mL, 15.5 g, 92.8 mmol) were added, and the resulting mixture was stirred at 100°C for 2 hours. The reaction mixture was cooled to room temperature, and water (30 mL) was added to the reaction mixture, followed by extraction with ethyl acetate once and methylene chloride/methanol (9:1) once. The organic layers were combined and dried over anhydrous sodium sulfate. The solvent was distilled off, and the obtained residue was purified by silica gel column chromatography (Biotage Japan Ltd., eluting solvent: ethyl acetate/methanol = 100/0 to 88/12) to obtain the title compound (8.17 g, yield: 68.2%) as an oil substance.
$^1$H-NMR ($CDCl_3$) δ: 7.48-7.42 (2H, m), 7.35-7.29 (3H, m), 5.48 (1H, s), 4.20 (2H, s), 4.13 (2H, q, J= 7.2 Hz), 2.25 (3H, s), 1.21 (3H, t, J= 7.0 Hz).
MS (ESI) m/z : 261 [$(M+H)^+$].

(Step 2) 1-(2-Hydroxyethyl)-5-methyl-2-phenyl-2,3-dihydro-1H-pyrazol-3-one

To a solution of ethyl 2-(5-methyl-3-oxo-2-phenyl-2,3-dihydro-1H-pyrazol-1-yl)acetate (8.17 g, 31.4 mmol) synthesized in step 1 in methanol (200 mL), sodium borohydride (12.5 g, 330 mmol) was added in 6 divided portions over 45 minutes, and the mixture was stirred at room temperature for 30 minutes. The reaction mixture was cooled to 0°C, then a 5 N aqueous hydrochloric acid solution (110 mL, 550 mmol) was added thereto, and the mixture was stirred for 30 minutes. Subsequently, the pH of the reaction mixture was adjusted to 7 to 8 by the addition of a 5 N aqueous sodium hydroxide solution (50.0 mL, 250 mmol). Methanol was distilled off from the reaction mixture under reduced pressure, and the obtained residue was subjected to extraction with methylene chloride/methanol (19:1) twice. The organic layers were combined and dried over anhydrous sodium sulfate. The solvent was distilled off, and the obtained residue was purified by silica gel column chromatography (Biotage Japan Ltd., eluting solvent: ethyl acetate/methanol = 100/0 to 80/20) to obtain the title compound (5.41 g, yield: 78.9%) as a solid.
$^1$H-NMR ($CDCl_3$) δ: 7.49-7.43 (2H, m), 7.40-7.35 (2H, m), 7.34-7.28 (1H, m), 5.30 (1H, s), 3.73 (2H, t, J = 4.9 Hz), 3.60-3.53 (2H, m), 3.23 (1H, br s), 2.38 (3H, s). MS (ESI) m/z : 219 [$(M+H)^+$].

(Step 3) 4-Amino-1-(2-hydroxyethyl)-5-methyl-2-phenyl-2,3-dihydro-1H-pyrazol-3-one

The title compound was obtained as a solid by using 1-(2-hydroxyethyl)-5-methyl-2-phenyl-2,3-dihydro-1H-pyrazol-3-one synthesized in step 2 instead of 5-(1-methoxyethyl)-1-methyl-2-phenyl-2,3-dihydro-1H-pyrazol-3-one in step 4 of Example 122 and subsequently performing the same reaction as in Example 122 up to step 5. Aminoantipyrine form NMR
$^1$H-NMR ($CDCl_3$) δ: 7.49-7.40 (4H, m), 7.31-7.26 (1H, m), 3.60 (2H, t, J = 4.9 Hz), 3.50 (2H, t, J = 5.2 Hz), 2.86 (2H, br s), 2.44 (1H, br s), 2.25 (3H, s).
MS (ESI) m/z : 234 [$(M+H)^+$].
The following compound of Example 139 was subsequently synthesized in the same way as in Example 93 and is shown in Table 46.

[Table 46]

| Example No. | Structure | Intermediate used | Instrumental analysis data |
|---|---|---|---|
| 1 3 9 | 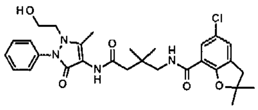 | 3 c | $^1$H-NMR ($CDCl_3$) $\delta$: 9.09 (1H, s), 8.01 (1H, t, J = 6.6 Hz), 7.88 (1H, d, J = 2.3 Hz), 7.45-7.43 (4H, m), 7.28-7.25 (1H, m), 7.22-7.22 (1H, m), 3.74-3.73 (2H, m), 3.63 (1H, t, J = 6.6 Hz), 3.56-3.53 (4H, m), 3.05 (2H, s), 2.35 (3H, s), 2.24 (2H, s), 1.54 (6H, s), 1.10 (6H, s).<br>MS (FAB) m/z : 555 [(M+H)$^+$]. |

(Example 140) N-[4-({1-[2-(Azetidin-1-yl)ethyl]-5-methyl-3-oxo-2-phenyl-2,3-dihydro-1H-pyrazol-4-yl}amino)-2,2-dimethyl-4-oxobutyl]-5-chloro-2-ethoxypyridine-3-carboxamide

[Formula 100]

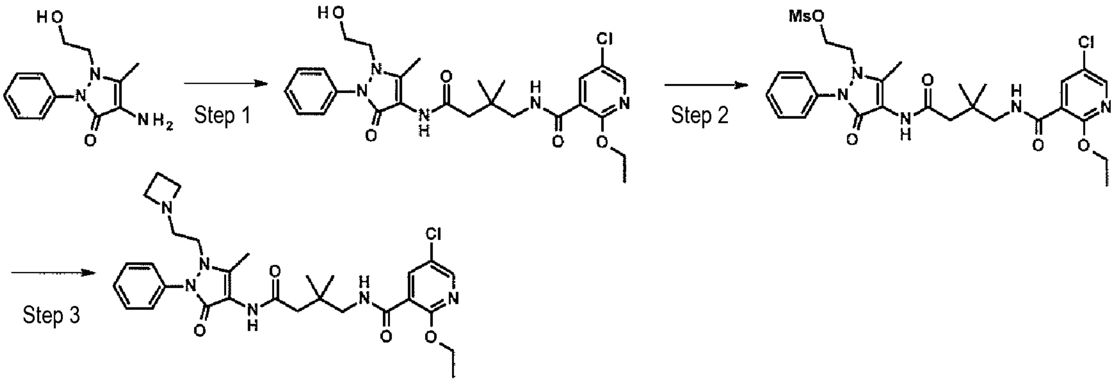


(Step 1) 5-Chloro-2-ethoxy-N-(4-{[1-(2-hydroxyethyl)-5-methyl-3-oxo-2-phenyl-2,3-dihydro-1H-pyrazol-4-yl]amino}-2,2-dimethyl-4-oxobutyl)pyridine-3-carboxamide

The title compound was obtained as a solid through the same reaction as in Example 93 using 4-amino-1-(2-hydroxyethyl)-5-methyl-2-phenyl-2,3-dihydro-1H-pyrazol-3-one synthesized in step 3 of Example 139 and intermediate 1h.
$^1$H-NMR ($CDCl_3$) $\delta$: 8.92 (1H, br s), 8.48 (1H, d, J = 3.1 Hz), 8.43 (1H, t, J= 6.7 Hz), 8.18 (1H, d, J = 2.4 Hz), 7.49-7.41 (4H, m), 7.31-7.27 (1H, m), 4.54 (2H, q, J = 7.1 Hz), 3.76-3.72 (2H, m), 3.60-3.52 (4H, m), 3.39 (1H, t, J = 5.5 Hz), 2.35 (3H, s), 2.26 (2H, s), 1.47 (3H, t, J = 7.3 Hz), 1.11 (6H, s).
MS (ESI) m/z: 530 [(M+H)$^+$].

(Step 2) 2-[4-({4-[(5-Chloro-2-ethoxypyridine-3-carbonyl)amino]-3,3-dimethylbutanoyl}amino)-5-methyl-3-oxo-2-phenyl-2,3-dihydro-1H-pyrazol-1-yl]ethylmethanesulfonate

To a solution of 5-chloro-2-ethoxy-N-(4-{[1-(2-hydroxyethyl)-5-methyl-3-oxo-2-phenyl-2,3-dihydro-1H-pyrazol-4-yl]amino}-2,2-dimethyl-4-oxobutyl)pyridine-3-carboxamide (407 mg, 0.768 mmol) synthesized in step 1 in pyridine (7.0 mL), methanesulfonyl chloride (0.240 mL, 355 mg, 3.10 mmol) was added, and the mixture was stirred at room temperature for 16 hours. The reaction mixture was concentrated under reduced pressure, and the obtained residue was purified by silica gel column chromatography (Biotage Japan Ltd., eluting solvent: ethyl acetate/methanol = 100/0 to 86/14) to obtain the title compound (447 mg, yield: 95.8%) as a solid.
$^1$H-NMR ($CDCl_3$) $\delta$: 8.93 (1H, br s), 8.49 (1H, d, J = 3.1 Hz), 8.41 (1H, t, J= 6.7 Hz), 8.19 (1H, d, J = 3.1 Hz), 7.50-7.40 (4H, m), 7.33-7.27 (1H, m), 4.55 (2H, q, J = 7.1 Hz), 4.10 (2H, t, J= 4.9 Hz), 3.95 (2H, t, J = 4.9 Hz), 3.57 (2H, d, J = 7.3 Hz), 3.08 (3H, s), 2.33 (3H, s), 2.25 (2H, s), 1.48 (3H, t, J = 7.0 Hz), 1.12 (6H, s).
MS (ESI) m/z : 608 [(M+H)$^+$].

(Step 3) N-[4-({1-[2-(Azetidin-1-yl)ethyl]-5-methyl-3-oxo-2-phenyl-2,3-dihydro-1H-pyrazol-4-yl}amino)-2,2-dimethyl-4-oxobutyl]-5-chloro-2-ethoxypyridine-3-carboxamide

To a mixture of 2-[4-({4-[(5-chloro-2-ethoxypyridine-3-carbonyl)amino]-3,3-dimethylbutanoyl}amino)-5-methyl-3-oxo-2-phenyl-2,3-dihydro-1H-pyrazol-1-yl]ethylmethanesulfonate (248 mg, 0.408 mmol) synthesized in step 2, potassium carbonate (297 mg, 2.15 mmol), and potassium iodide (71.2 mg, 0.429 mmol), N,N-dimethylformamide (6.3 mL) and azetidine (0.560 mL, 474 mg, 8.31 mmol) were added, and the resulting mixture was stirred at 80°C for 8 hours. The reaction mixture was cooled to room temperature, and water (10 mL) was added to the reaction mixture, followed by extraction with ethyl acetate once. The organic layer was washed with water twice and saturated saline once and dried over anhydrous sodium sulfate. The solvent was distilled off, and the obtained residue was purified by amino silica gel column chromatography (Biotage Japan Ltd., eluting solvent: ethyl acetate/methanol = 100/0 to 86/14) and silica gel column chromatography (Biotage Japan Ltd., eluting solvent: methylene chloride/methanol = 100/0 to 82/18) in this order to obtain the title compound (163 mg, yield: 70.1%) as a solid.
$^1$H-NMR ($CDCl_3$) δ: 8.63 (1H, br s), 8.51 (1H, d, J = 2.4 Hz), 8.42 (1H, t, J= 6.1 Hz), 8.19 (1H, d, J = 1.8 Hz), 7.49-7.37 (4H, m), 7.31-7.25 (1H, m), 4.54 (2H, q, J = 7.1 Hz), 3.56 (2H, d, J= 6.7 Hz), 3.49 (2H, t, J = 7.3 Hz), 3.03 (4H, t, J = 7.0 Hz), 2.33 (2H, t, J = 7.3 Hz), 2.28 (3H, s), 2.26 (2H, s), 2.02-1.94 (2H, m), 1.47 (3H, t, J = 7.0 Hz), 1.11 (6H, s).
MS (ESI) m/z : 569 [(M+H)$^+$].

The following compounds of Examples 141 and 142 were synthesized through the same reaction as above using the corresponding amines instead of azetidine and are shown in Table 47.

[Table 47]

| Example No. | Structure | Intermediate used |
|---|---|---|
| 141 | 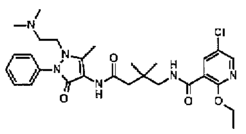 | $^1$H-NMR ($CDCl_3$) δ: 8.64 (1H, br s), 8.53-8.49 (1H, m), 8.42 (1H, t, J = 6.4 Hz), 8.21-8.15 (1H, m), 7.49-7.38 (4H, m), 7.32-7.25 (1H, m), 4.54 (2H, q, J = 6.9 Hz), 3.64 (2H, t, J = 7.3 Hz), 3.55 (2H, d, J = 6.1 Hz), 2.28 (3H, s), 2.26 (2H, s), 2.21 (2H, t, J = 7.3 Hz), 2.08 (6H, s), 1.47 (3H, t, J = 7.0 Hz), 1.10 (6H, s).<br>MS (ESI) 557 [(M+H)$^+$]. |
| 1 4 2 | 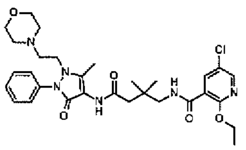 | $^1$H-NMR ($CDCl_3$) δ: 8.62 (1H, br s), 8.50 (1H, d, J = 2.4 Hz), 8.41 (1H, t, J = 6.4 Hz), 8.19 (1H, d, J = 2.4 Hz), 7.48-7.40 (4H, m), 7.31-7.27 (1H, m), 4.54 (2H, q, J = 7.1 Hz), 3.66 (2H, t, J = 6.7 Hz), 3.63-3.58 (4H, m), 3.57 (2H, d, J = 7.3 Hz), 2.31 (3H, s), 2.31-2.24 (8H, m), 1.48 (3H, t, J = 7.0 Hz), 1.12 (6H, s).<br>MS (ESI) m/z : 599 [(M+H)$^+$]. |

(Example 143) N-(5-Chloro-4-fluoro-2-methoxyphenyl)-3,3-dimethyl-N'-(3-methyl-5-oxo-1-phenyl-2-vinylpyrazol-4-yl)pentanediamide

[Formula 101]

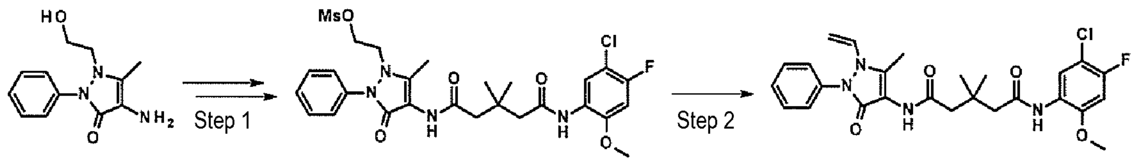


(Step 1) 2-(4-{[5-(5-Chloro-4-fluoro-2-methoxyanilino)-3,3-dimethyl-5-oxopentanoyl]amino}-5-methyl-3-oxo-2-phenyl-2,3-dihydro-1H-pyrazol-1-yl)ethylmethanesulfonate

The title compound was obtained as a solid by using intermediate 2d instead of intermediate 1h in step 1 of Example 140 and subsequently performing the same reaction as in Example 140 up to step 2.
$^1$H-NMR ($CDCl_3$) δ: 8.43 (1H, br s), 8.40 (1H, d, J = 8.0 Hz), 8.25 (1H, br s), 7.49-7.44 (2H, m), 7.41-7.37 (2H, m),

7.33-7.28 (1H, m), 6.69 (1H, d, J= 10.3 Hz), 4.10 (2H, t, J = 4.9 Hz), 3.95 (2H, t, J = 4.9 Hz), 3.84 (3H, s), 3.08 (3H, s), 2.64 (2H, s), 2.48 (2H, s), 2.34 (3H, s), 1.17 (6H, s).
MS (ESI) m/z : 611 [(M+H)$^+$].

(Step 2) N-(5-Chloro-4-fluoro-2-methoxyphenyl)-3,3-dimethyl-N'-(3-methyl-5-oxo-1-phenyl-2-vinylpyrazol-4-yl)pentanediamide

To a mixture of 2-(4-{[5-(5-chloro-4-fluoro-2-methoxyanilino)-3,3-dimethyl-5-oxopentanoyl]amino}-5-methyl-3-oxo-2-phenyl-2,3-dihydro-1H-pyrazol-1-yl)ethylmethanesulfonate (142 mg, 0.232 mmol) synthesized in step 1, potassium carbonate (167 mg, 1.21 mmol), and potassium iodide (42.4 mg, 0.255 mmol), N,N-dimethylformamide (4.5 mL) and triethylamine (0.330 mL, 241 mg, 2.38 mmol) were added, and the resulting mixture was stirred at 80°C for 10 hours. Subsequently, triethylamine (0.650 mL, 474 mg, 4.68 mmol) was further added thereto, and the mixture was stirred at 80°C for 11 hours. Then, triethylamine (0.650 mL, 474 mg, 4.68 mmol) was further added thereto, and the mixture was stirred at the same temperature as above for 9 hours. The reaction mixture was cooled to room temperature, and water (15 mL) was added to the reaction mixture, followed by extraction with ethyl acetate. The organic layer was washed with water twice and saturated saline once and dried over anhydrous sodium sulfate. The solvent was distilled off, and the obtained residue was purified by silica gel column chromatography (Biotage Japan Ltd., eluting solvent: ethyl acetate/methanol = 100/0 to 92/8) and amino silica gel column chromatography (Biotage Japan Ltd., eluting solvent: ethyl acetate/methanol = 100/0 to 95/5) in this order to obtain the title compound (56.3 mg, yield: 47.0%) as a solid.
$^1$H-NMR ($CDCl_3$) δ: 8.52 (1H, br s), 8.40 (1H, d, J = 8.0 Hz), 8.33 (1H, br s), 7.43-7.34 (4H, m), 7.26-7.22 (1H, m), 6.68 (1H, d, J = 10.3 Hz), 6.43 (1H, dd, J = 15.2, 8.9 Hz), 4.68 (1H, dd, J = 8.9, 1.4 Hz), 4.53 (1H, dd, J = 15.2, 1.4 Hz), 3.83 (3H, s), 2.64 (2H, s), 2.49 (2H, s), 2.35 (3H, s), 1.16 (6H, s).
MS (ESI) m/z : 515 [(M+H)$^+$].

(Example 144) N-(5-Chloro-4-fluoro-2-methoxyphenyl)-3,3-dimethyl-N'-(5-methyl-3-oxo-2-phenyl-1H-pyrazol-4-yl)pentanediamide

[Formula 102]

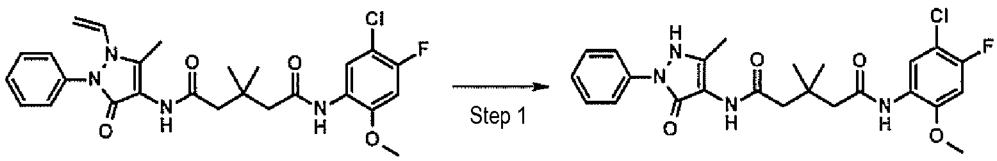


(Step 1) N-(5-Chloro-4-fluoro-2-methoxyphenyl)-3,3-dimethyl-N'-(5-methyl-3-oxo-2-phenyl-1H-pyrazol-4-yl)pentanediamide

To N-(5-chloro-4-fluoro-2-methoxyphenyl)-3,3-dimethyl-N'-(3-methyl-5-oxo-1-phenyl-2-vinylpyrazol-4-yl)pentanediamide (37.7 mg, 0.0732 mmol) synthesized in step 2 of Example 143, 4 N hydrochloric acid in 1,4-dioxane (1.25 mL, 5.00 mmol) and water (1.3 mL) were added, and the mixture was stirred at room temperature for 5 hours. The reaction mixture was concentrated under reduced pressure, and the obtained residue was purified by silica gel column chromatography (Biotage Japan Ltd., eluting solvent: hexane/ethyl acetate = 80/20 to 0/100) to obtain the title compound (25.5 mg, yield: 71.3%) as a solid.
$^1$H-NMR ($CDCl_3$) δ: 11.91 (1H, br s), 9.89 (1H, br s), 8.45 (1H, d, J = 8.0 Hz), 7.86 (1H, br s), 7.77 (2H, d, J = 7.4 Hz), 7.43-7.38 (2H, m), 7.25-7.20 (1H, m), 6.75 (1H, d, J= 9.7 Hz), 3.90 (3H, s), 2.52 (2H, s), 2.46 (2H, s), 2.26 (3H, s), 1.19 (6H, s).
MS (ESI) m/z : 489 [(M+H)$^+$].

(Example 145) 5-Chloro-N-(4-{[1,5-dimethyl-2-(m-toluyl)-3-oxopyrazol-4-yl]amino}-2,2-dimethyl-4-oxobutyl)-2-methoxybenzamide

[Formula 103]

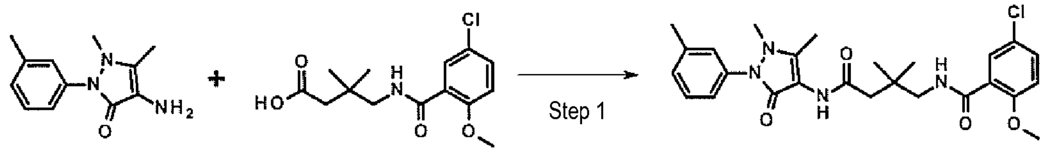


(Step 1)

The title compound was obtained as a solid through the same reaction as in Example 93 using 4-amino-1,5-dimethyl-2-(m-toluyl)pyrazol-3-one instead of 4-aminoantipyrine and intermediate 1b instead of intermediate 1a.
$^1$H-NMR ($CDCl_3$) δ: 8.65 (1H, br s), 8.31 (1H, t, J = 6.0 Hz), 8.18 (1H, d, J= 2.6 Hz), 7.39 (1H, dd, J = 8.9, 2.6 Hz), 7.32 (1H, t, J = 7.7 Hz), 7.16 (1H, d, J = 7.7 Hz), 7.09 (1H, d, J = 7.7 Hz), 6.91 (1H, d, J = 8.9 Hz), 3.97 (3H, s), 3.08 (3H, s), 2.39 (3H, s), 2.28 (2H, s), 2.26 (3H, s).
MS (ESI) m/z : 499 [$(M+H)^+$].

(Example 146) N-(5-Chloro-2-methoxyphenyl)-N'-[1-(3-methoxyphenyl)-2,3-dimethyl-5-oxopyrazol-4-yl]-3,3-dimethylpentanediamide

[Formula 104]

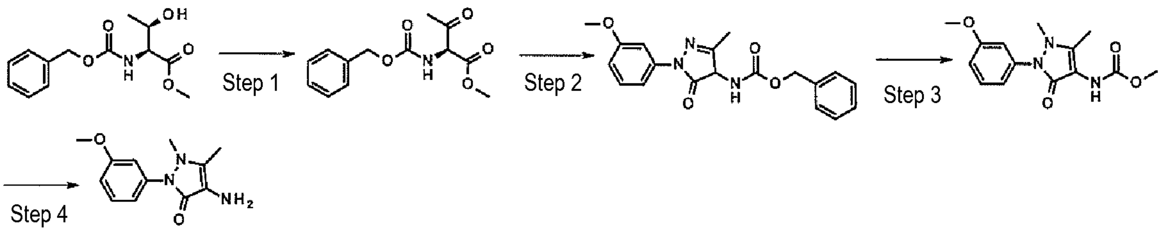


(Step 1) Methyl (2S)-2-(benzyloxycarbonylamino)-3-oxobutanoate

Methyl (2S,3R)-2-(benzyloxycarbonylamino)-3-hydroxybutanoate (10.0 g, 37.4 mmol) was dissolved in methylene chloride (400 ml). To the solution, Dess-Martin reagent (20.6 g, 48.6 mmol) was added, and the mixture was stirred at room temperature for 3 hours. The reaction solution was concentrated under reduced pressure, and the residue was purified by silica gel chromatography (Biotage Japan Ltd., eluting solvent: ethyl acetate/hexane = 5/95 to 50/50) to obtain the title compound (11.4 g, quantitative) as an oil substance. $^1$H-NMR ($CDCl_3$) δ: 7.43-7.32 (5H, m), 6.04-5.93 (1H, m), 5.13 (3H, s), 3.83 (3H, s), 2.42-2.10 (3H, m).

(Step 2) Benzyl N-[2-(3-methoxyphenyl)-5-methyl-3-oxopyrazol-4-yl]carbamate

Methyl (2S)-2-(benzyloxycarbonylamino)-3-oxobutanoate (2.05 g, 7.74 mmol) synthesized in step 1 was dissolved in acetic acid (15 ml). To the solution, 3-methoxyphenylhydrazine hydrochloride (1.24 g, 7.10 mmol) was added, and the mixture was heated to reflux for 4 hours. The reaction solution was cooled to room temperature and concentrated under reduced pressure. The residue was neutralized by the addition of a saturated aqueous solution of sodium bicarbonate, followed by extraction with methylene chloride. The organic layer was dried over sodium. After filtration, the filtrate was concentrated under reduced pressure, and then, the obtained residue was purified by silica gel chromatography (Biotage Japan Ltd., eluting solvent: methanol/methylene chloride = 2/98 to 10/90) to obtain the title compound (0.230 g, yield: 9.17%) as a solid.
$^1$H-NMR ($CDCl_3$) δ: 7.44-7.26 (8H, m), 6.80-6.76 (1H, m), 6.30 (1H, br s), 5.21 (2H, s), 3.83 (3H, s), 2.18 (3H, s). MS (APCI) m/z: 354 [$(M+H)^+$].

(Step 3) Methyl N-[2-(3-methoxyphenyl)-1,5-dimethyl-3-oxopyrazol-4-yl]carbamate

Benzyl N-[2-(3-methoxyphenyl)-5-methyl-3-oxopyrazol-4-yl]carbamate (225 mg, 0.637 mmol) synthesized in step 2 was dissolved in methanol (5.0 ml). To the solution, calcium oxide (71.0 mg, 1.30 mmol) and dimethylsulfuric acid (0.120 ml, 1.30 mmol) were added, and the mixture was heated to reflux for 1 hour. The reaction solution was cooled to room temperature, and insoluble matter was filtered off. Then, the filtrate was concentrated under reduced pressure. The obtained residue was purified by silica gel chromatography (Biotage Japan Ltd., eluting solvent: methanol/methylene chloride = 1/99 to 10/90) to obtain the title compound (135 mg, yield: 72.7%) as a solid.
$^1$H-NMR ($CDCl_3$) $\delta$: 7.35 (1H, t, J = 8.5 Hz), 6.98-6.95 (2H, m), 6.86-6.83 (1H, m), 6.18 (1H, br s), 3.83 (3H, s), 3.75 (3H, s), 3.08 (3H, s), 2.27 (3H, s).
MS (APCI) m/z: 292 [$(M+H)^+$].

(Step 4) 4-Amino-2-(3-methoxyphenyl)-1,5-dimethylpyrazol-3-one

Methyl N-[2-(3-methoxyphenyl)-1,5-dimethyl-3-oxopyrazol-4-yl]carbamate (130 mg, 0.447 mmol) synthesized in step 3 was dissolved in methanol (1.0 ml). To the solution, a 1 N aqueous sodium hydroxide solution (1.34 ml, 1.34 mmol) was added, and the mixture was heated to reflux for 2 days. The reaction solution was cooled to room temperature, then 1 N hydrochloric acid (1.34 ml, 1.34 mmol) was added thereto, and the mixture was concentrated under reduced pressure. The obtained residue was purified (Biotage Japan Ltd., eluting solvent: methanol/methylene chloride = 1/99 to 15/85) to obtain the title compound (64.0 mg, yield: 61.4%) as a solid.
$^1$H-NMR ($CDCl_3$) $\delta$: 7.33 (1H, t, J = 8.2 Hz), 7.07-7.03 (2H, m), 6.81-6.77 (1H, m), 3.84 (3H, s), 2.86 (3H, s), 2.15 (3H, s).
MS (APCI) m/z: 234 [$(M+H)^+$].

The following compound of Example 146 was subsequently synthesized in the same way as in Example 93 and is shown in Table 48.

[Table 48]

| Example No. | Structure | Intermediate used | Instrumental analysis data |
|---|---|---|---|
| 1 4 6 | 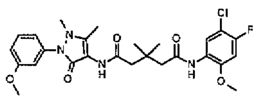 | 2 d | $^1$H-NMR ($CDCl_3$) $\delta$: 8.53 (1H, br s), 8.39-8.37 (2H, m), 7.33 (1H, t, *J* = 8.0 Hz), 6.97-6.95 (2H, m), 6.83 (1H, dd, *J* = 8.3, 2.0 Hz), 6.67 (1H, d, *J* = 10.3 Hz), 3.82 (3H, s), 3.82 (3H, s), 3.11 (3H, s), 2.63 (2H, s), 2.47 (2H, s), 2.28 (3H, s), 1.16 (6H, s). MS (ESI) m/z : 533 [$(M+H)^+$]. |

(Example 147) N-(5-Chloro-2-isopropoxy-phenyl)-N'-[1,5-dimethyl-3-oxo-2-[3-(trifluoromethyl)phenyl]pyrazol-4-yl]-3,3-dimethylpentanediamide

[Formula 105]

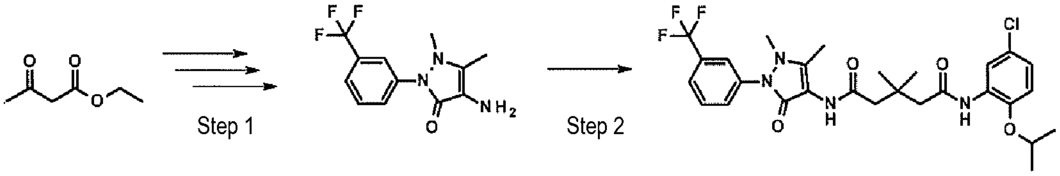


(Step 1) 4-Amino-1,5-dimethyl-2-[3-(trifluoromethyl)phenyl]pyrazol-3-one

The title compound was obtained as a solid by using methyl 3-oxobutanoate instead of ethyl 3-oxo-5-[(2,2,2-trifluoroacetyl)amino]pentanoate and [3-(trifluoromethyl)phenyl]hydrazine instead of phenylhydrazine in step 2 of Example 28 and subsequently performing the same reaction as in Example 28 up to step 5.
$^1$H-NMR ($CDCl_3$) $\delta$: 7.76 (1H, d, J = 8.0 Hz), 7.71 (1H, s), 7.56 (1H, t, J= 8.0 Hz), 7.48 (1H, d, J = 7.4 Hz), 2.85 (3H, s),

2.17 (3H, s).
MS (API) m/z: 272 [$(M+H)^+$].

(Step 2) N-(5-Chloro-2-isopropoxyphenyl)-N'-[1,5-dimethyl-3-oxo-2-[3-(trifluoromethyl)phenyl]pyrazol-4-yl]-3,3-dimethylpentanediamide

A solution of intermediate 2f (25.2 mg, 0.0769 mmol), 1-ethyl-3-(3-dimethylaminopropyl)carbodiimide hydrochloride (EDC·HCl) (25.1 mg, 0.131 mmol), and 1-hydroxybenzotriazole (HOBt) (14.7 mg, 0.109 mmol) in methylene chloride (1.3 mL) was stirred at room temperature for 1 hour. Subsequently, 4-amino-1,5-dimethyl-2-[3-(trifluoromethyl)phenyl]pyrazol-3-one (26.0 mg, 0.0959 mmol) synthesized in step 1 and N,N-diisopropylethylamine (0.0210 mL, 15.6 mg, 0.121 mmol) were added thereto, and the mixture was stirred at room temperature for 210 hours. The reaction mixture was concentrated under reduced pressure, and the obtained residue was purified by reverse-phase HPLC (water (0.10% formic acid)/acetonitrile (0.10% formic acid), Gilson, Inc.) to obtain the title compound (5.75 mg, yield: 12.9%) as a solid.
$^1$H-NMR ($CDCl_3$) δ: 8.74 (1H, br s), 8.39 (1H, d, J = 2.9 Hz), 8.21 (1H, br s), 7.68-7.64 (2H, m), 7.59-7.54 (1H, m), 7.53-7.49 (1H, m), 6.98 (1H, dd, J= 8.6, 2.3 Hz), 6.78 (1H, d, J= 8.6 Hz), 4.58-4.53 (1H, m), 3.09 (3H, s), 2.67 (2H, s), 2.49 (2H, s), 2.29 (3H, s), 1.34 (6H, d, J = 5.7 Hz), 1.17 (6H, s).
MS (ESI) m/z : 581 [$(M+H)^+$].

(Example 148) N-(2,2-Bis(fluoromethyl)-4-{[5-(methoxymethyl)-1-methyl-3-oxo-2-phenyl-2,3-dihydro-1H-pyrazol-4-yl]amino}-4-oxobutyl)-5-chloro-2-ethoxybenzamide

[Formula 106]

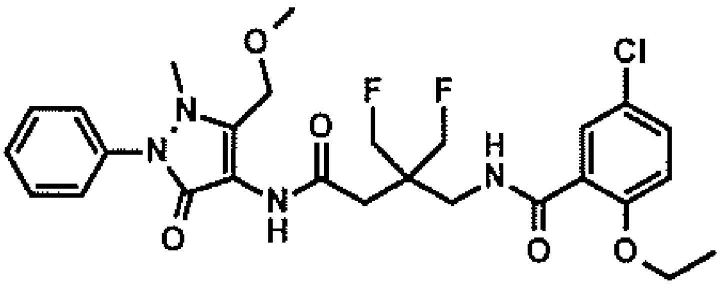

The title compound was obtained as a solid by using 4-amino-5-(methoxymethyl)-1-methyl-2-phenyl-2,3-dihydro-1H-pyrazol-3-one synthesized in step 2 of Example 113 instead of N-[2-(4-amino-2-methyl-5-oxo-1-phenyl-pyrazolo-3-yl)ethyl]-2,2,2-trifluoroacetamide in step 1 of Example 62 and subsequently performing the same reaction as in steps 1 and 3 of Example 62.
$^1$H-NMR ($CDCl_3$) δ: 9.07 (1H, s), 8.41 (1H, s), 8.18 (1H, s), 7.50-7.36 (4H, m), 7.34-7.28 (1H, m), 6.91 (1H, d, J= 8.6 Hz), 5.30 (1H, s), 4.64-4.56 (2H, m), 4.54-4.43 (4H, m), 4.23-4.15 (2H, m), 3.87-3.81 (2H, m), 3.45 (3H, s), 3.20 (3H, s), 2.41 (2H, s), 1.52 (3H, t, J = 6.8 Hz).

(Example 149) 5-Chloro-N-(4-{[1,5-dimethyl-3-oxo-2-(2-pyridyl)pyrazol-4-yl]amino}-2,2-dimethyl-4-oxobutyl)-2-ethoxybenzamide

[Formula 107]

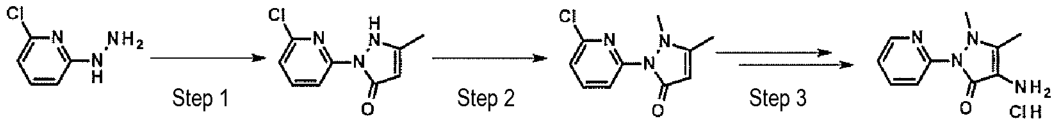

(Step 1) 2-(6-Chloro-2-pyridyl)-5-methyl-4H-pyrazol-3-one

(6-Chloro-2-pyridyl)hydrazine (10.0 g, 69.7 mmol) was dissolved in ethanol (200 ml). To the solution, ethyl acetoacetate (9.24 ml, 73.1 mmol) was added, and the mixture was stirred overnight at room temperature. The reaction solution was concentrated under reduced pressure to obtain an oil substance. This oil substance was dissolved in ethanol (140 ml). To the solution, potassium tert-butoxide (9.39 g, 83.6 mmol) was added, and the mixture was stirred at room temperature for 3 hours. Water was added to the reaction solution, and the mixture was washed with diethyl ether. 5 N hydrochloric acid (18 ml) was added to the aqueous layer, and the resulting precipitate was collected by filtration and dried to obtain the title compound (13.6 g, yield: 93.1%) as a solid.
$^1$H-NMR ($CDCl_3$) $\delta$: 11.27 (1H, s), 7.78-7.72 (2H, m), 7.10 (1H, d, J = 6.8 Hz), 5.42 (1H, s), 2.22 (3H, s). MS (APCI) m/z: 210 [$(M+H)^+$].

(Step 2) 2-(6-Chloro-2-pyridyl)-1,5-dimethyl-4H-pyrazol-3-one

2-(6-Chloro-2-pyridyl)-5-methyl-4H-pyrazol-3-one (0.200 g, 0.954 mmol) synthesized in step 1, methanol (0.386 ml, 0.306 g, 9.54 mmol), and tributylphosphine (0.480 ml, 0.386 g, 1.91 mmol) were dissolved in tetrahydrofuran (12 ml). To the solution, 1,1-(azodicarbonyl)dipiperidine (0.289 g, 1.14 mmol) was added, and the mixture was stirred at room temperature for 2 hours. The reaction solution was separated into aqueous and organic layers by the addition of ethyl acetate and saturated saline. The organic layer was dried over anhydrous sodium sulfate, and the solvent was distilled off under reduced pressure. Diethyl ether was added to the residue, and insoluble matter was removed. Then, the filtrate was concentrated under reduced pressure, and the obtained residue was purified by silica gel chromatography (Shoko Scientific Co., Ltd., eluting solvent: ethyl acetate/hexane = 5/100 to 100/0) to obtain the title compound (0.144 g, yield: 67.5%) as a solid.
$^1$H-NMR ($CDCl_3$) $\delta$: 7.91 (1H, d, J = 7.8 Hz), 7.73 (1H, t, J = 7.8 Hz), 7.14 (1H, d, J = 7.8 Hz), 5.32 (1H, s), 3.33 (3H, s), 2.23 (3H, s).
MS (APCI) m/z: 224 [$(M+H)^+$].

(Step 3) 4-Amino-1,5-dimethyl-2-(2-pyridyl)pyrazol-3-one hydrochloride

The title compound was obtained as a solid by using 2-(6-chloro-2-pyridyl)-1,5-dimethyl-4H-pyrazol-3-one synthesized in step 2 instead of 5-(1-methoxyethyl)-1-methyl-2-phenyl-2,3-dihydro-1H-pyrazol-3-one in step 4 of Example 122 and subsequently performing the same reaction as in Example 122 up to step 5.
$^1$H-NMR (DMSO-D6) $\delta$: 9.77 (2H, s), 8.51 (1H, dq, J = 4.9, 1.5 Hz), 8.00-7.96 (1H, m), 7.73 (1H, d, J = 8.3 Hz), 7.37-7.33 (1H, m), 3.29 (3H, s), 2.37 (3H, s).
MS (APCI) m/z: 205 [$(M+H)^+$].
The following compounds of Examples 149 to 151 were subsequently synthesized in the same way as in Example 93 and are shown in Table 49.

[Table 49]

| Example No. | Structure | Intermediate used | Instrumental analysis data |
|---|---|---|---|
| 1 4 9 | 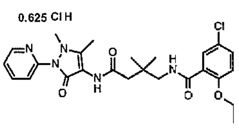 | 1 a | $^1$H-NMR ($CDCl_3$) $\delta$: 9.66 (1H, s), 8.60-8.57 (1H, m), 8.40-8.34 (1H, m), 8.19 (1H, d, J = 3.1 Hz), 8.01-7.90 (2H, m), 7.38 (1H, dd, J =8.5, 3.1 Hz), 7.33-7.29 (1H, m), 6.91 (1H, d, J = 8.5 Hz), 4.21 (2H, q, J = 7.3 Hz), 3.57 (2H, d, J = 6.7 Hz), 3.46 (3H, s), 2.39 (3H, s), 2.38 (2H, s), 1.53 (3H, t, J = 7.3 Hz), 1.15 (6H, s).<br>MS (ESI+APCI) m/z : 500 [$(M+H)^+$]. |
| 1 5 0 | 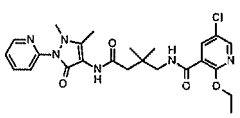 | 1 h | $^1$H-NMR ($CDCl_3$) $\delta$: 8.66 (1H, br s), 8.50-8.48 (2H, m), 8.42 (1H, t, J = 6.9 Hz), 7.98 (1H, d, J = 8.6 Hz), 7.80 (1H, td, J = 8.0, 1.5 Hz), 7.15 (1H, dd, J = 6.9, 5.2 Hz), 4.55 (2H, q, J = 7.2 Hz), 3.58 (2H, d, J = 6.9 Hz), 3.36 (3H, s), 2.28 (3H, s), 2.28 (2H, s), 1.48 (3H, t, J = 7.2 Hz), 1.14 (6H, s).<br>MS (ESI) m/z : 501 [$(M+H)^+$]. |

(continued)

| Example No. | Structure | Intermediate used | Instrumental analysis data |
|---|---|---|---|
| 1 5 1 | 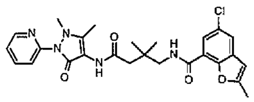 | 3 a | $^1$H-NMR (DMSO-D$_6$) δ: 9.11 (1H, s), 8.50 (1H, d, J = 4.4 Hz), 8.41 (1H, t, J = 6.3 Hz), 7.95-7.93 (1H, m), 7.77-7.76 (2H, m), 7.56 (1H, d, J = 2.0 Hz), 7.30-7.28 (1H, m), 6.68 (1H, s), 3.36 (2H, d, J = 6.3 Hz), 3.27 (3H, s), 2.46 (3H, s), 2.29 (2H, s), 2.13 (3H, s), 1.07 (6H, s).<br>MS (ESI) m/z: 510 [(M+H)$^+$]. |

(Example 152) 5-Chloro-N-(4-{[1,5-dimethyl-2-(6-methyl-2-pyridyl)-3-oxopyrazol-4-yl]amino}-2,2-dimethyl-4-oxobutyl)-2-ethoxybenzamide

[Formula 108]

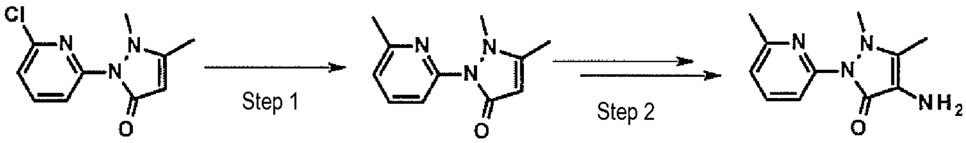


(Step 1) 1,5-Dimethyl-2-(6-methyl-2-pyridyl)pyrazol-3-one

2-(6-Chloro-2-pyridyl)-1,5-dimethylpyrazol-3-one (0.447 g, 2.00 mmol) synthesized in step 2 of Example 149 and trimethylboroxine (50% solution in tetrahydrofuran) (0.753 g, 3.00 mmol) were dissolved in 1,4-dioxane (12 mL). To the solution, water (1.2 mL), potassium carbonate (0.829 g, 6.00 mmol), and tetrakis(triphenylphosphine)palladium (0.231 g, 0.200 mmol) were added, and the mixture was stirred at 110°C for 3 hours. The reaction solution was brought back to room temperature, and water was added thereto, followed by extraction with ethyl acetate. The organic layer was washed with saturated saline and then dried over anhydrous magnesium sulfate. After filtration, the filtrate was concentrated, and the obtained crude product was purified by silica gel column chromatography (Biotage Japan Ltd., eluting solvent: hexane/ethyl acetate → ethyl acetate/methanol = 10/90 to 0/100 → 100/0 to 80/20) to obtain the title compound (0.337 g, yield: 82.9%) as a solid.
$^1$H-NMR (CDCl$_3$) δ: 7.74-7.68 (2H, m), 7.01 (1H, d, J= 7.3 Hz), 5.36 (1H, d, J = 1.0 Hz), 3.33 (3H, s), 2.56 (3H, s), 2.25 (3H, s).

(Step 2) 4-Amino-1,5-dimethyl-2-(6-methyl-2-pyridyl)pyrazol-3-one

The title compound was obtained as a solid by using 1,5-dimethyl-2-(6-methyl-2-pyridyl)pyrazol-3-one synthesized in step 1 instead of 5-(1-methoxyethyl)-1-methyl-2-phenyl-2,3-dihydro-1H-pyrazol-3-one in step 4 of Example 122 and subsequently performing the same reaction as in Example 122 up to step 5.
$^1$H-NMR (CDCl$_3$) δ: 7.86 (1H, d, J = 7.8 Hz), 7.68 (1H, t, J = 7.8 Hz), 6.98 (1H, d, J = 7.8 Hz), 3.10 (3H, s), 2.88 (2H, s), 2.57 (3H, s), 2.18 (3H, s).
The following compounds of Examples 152 to 155 were subsequently synthesized in the same way as in Example 93 and are shown in Table 50.

[Table 50]

| Example No. | Structure | Intermediate used | Instrumental analysis data |
|---|---|---|---|
| 1 5 2 | 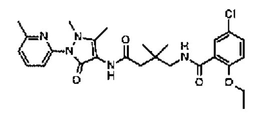 | 1 a | $^1$H-NMR ($CDCl_3$) δ: 8.82 (1H, s), 8.36 (1H, t, J = 6.8 Hz), 8.20 (1H, d, J = 2.9 Hz), 7.78 (1H, d, J = 7.8 Hz), 7.67 (1H, t, J = 7.8 Hz), 7.37 (1H, dd, J = 8.8, 2.9 Hz), 6.99 (1H, d, J = 7.8 Hz), 6.90 (1H, d, J = 8.8 Hz), 4.20 (2H, q, J = 7.0 Hz), 3.58 (2H, d, J = 6.8 Hz), 3.36 (3H, s), 2.56 (3H, s), 2.28 (3H, s), 2.27 (2H, s), 1.52 (3H, t, J = 7.0 Hz), 1.13 (6H, s).<br>MS (ESI) m/z : 514 [(M+H)$^+$]. |
| 1 5 3 | 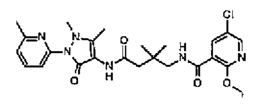 | 1 h | $^1$H-NMR ($CDCl_3$) δ: 8.57 (1H, br s), 8.49 (1H, d, J = 2.4 Hz), 8.42 (1H, t, J = 6.7 Hz), 8.18 (1H, d, J = 2.4 Hz), 7.79-7.74 (1H, m), 7.71-7.64 (1H, m), 7.01 (1H, d, J = 7.3 Hz), 4.54 (2H, q, J = 7.1 Hz), 3.56 (2H, d, J = 7.3 Hz), 3.36 (3H, s), 2.56 (3H, s), 2.28 (3H, s), 2.27 (2H, s), 1.48 (3H, t, J = 7.0 Hz), 1.13 (6H, s).<br>$^1$MS (ESI) m/z: 515 [(M+H)$^+$]. |
| 1 5 4 | 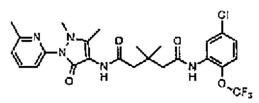 | 2 g | $^1$H-NMR ($CDCl_3$) δ: 9.80 (1H, s), 8.55 (1H, d, J = 2.4 Hz), 7.81 (1H, s), 7.71-7.69 (2H, m), 7.16-7.15 (1H, m), 7.05-7.02 (2H, m), 3.38 (3H, s), 2.57 (3H, s), 2.56 (2H, s), 2.38 (2H, s), 2.29 (3H, s), 1.13 (6H, s).<br>MS (ESI) m/z : 554 [(M+H)$^+$]. |
| 1 5 5 | 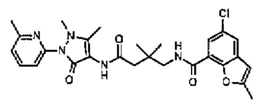 | 3a | $^1$H-NMR ($CDCl_3$) δ: 8.27 (1H, s), 7.97 (1H, d, J = 2.2 Hz), 7.95 (1H, t, J = 6.8 Hz), 7.76 (1H, d, J = 7.8 Hz), 7.68 (1H, t, J = 7.8 Hz), 7.55 (1H, d, J = 2.2 Hz), 7.01 (1H, d, J = 7.8 Hz), 6.43 (1H, d, J = 1.0 Hz), 3.64 (2H, d, J = 6.8 Hz), 3.36 (3H, s), 2.56 (3H, s), 2.51 (3H, s), 2.33 (2H, s), 2.28 (3H, s), 1.17 (6H, s).<br>MS (ESI) m/z : 524 [(M+H)$^+$]. |

(Example 156) 5-Chloro-2-ethoxy-N-(4-{[1-isopropyl-5-methyl-3-oxo-2-(2-pyridyl)pyrazol-4-yl]amino}-2,2-dimethyl-4-oxobutyl)benzamide

[Formula 109]

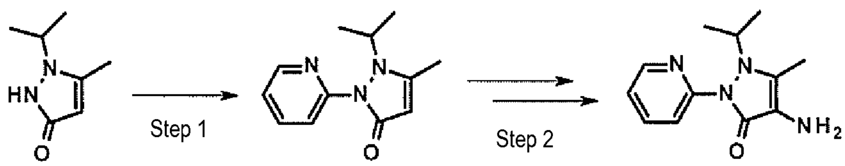


(Step 1) 1-Isopropyl-5-methyl-2-(2-pyridyl)pyrazol-3-one

To a mixture of 2-isopropyl-3-methyl-1H-pyrazol-5-one (1.26 g, 8.99 mmol) synthesized according to the method described in WO2007/10015, copper(I) iodide (0.176 g, 0.924 mmol), and potassium carbonate (2.55 g, 18.5 mmol), N,N-dimethylformamide (10 mL), (1R,2R)-N1,N2-dimethylcyclohexane-1,2-diamine (0.295 mL, 266 g, 1.87 mmol), and 2-bromopyridine (0.980 mL, 1.60 g, 10.1 mmol) were added, and the resulting mixture was irradiated with microwaves at 150°C for 1 hour. Water (30 mL) was added to the reaction mixture, followed by extraction with methylene chloride/methanol (9:1) twice. The organic layers were combined and dried over anhydrous sodium sulfate. The solvent was distilled off, and the obtained residue was purified by silica gel column chromatography (Biotage Japan Ltd., hexane/ethyl

acetate → ethyl acetate/methanol = 90/10 to 0/100 → 100/0 to 85/15) to obtain the title compound (0.0990 g, yield: 5.07%) as an oil substance.
$^1$H-NMR ($CDCl_3$) δ: 8.53-8.47 (1H, m), 7. 85-7.78 (1H, m), 7.75-7.71 (1H, m), 7.20-7.15 (1H, m), 5.39 (1H, s), 4.16-4.05 (1H, m), 2.32 (3H, s), 1.22 (6H, d, J = 6.7 Hz). MS (ESI) m/z : 218 [$(M+H)^+$].

(Step 2) 4-Amino-1-isopropyl-5-methyl-2-(2-pyridyl)pyrazol-3-one

The title compound was obtained as an oil substance by using 1-isopropyl-5-methyl-2-(2-pyridyl)pyrazol-3-one synthesized in step 1 instead of 5-(1-methoxyethyl)-1-methyl-2-phenyl-2,3-dihydro-1H-pyrazol-3-one in step 4 of Example 122 and subsequently performing the same reaction as in Example 149 up to step 4.
$^1$H-NMR ($CDCl_3$) δ: 8.53-8.47 (1H, m), 7. 84-7.75 (2H, m), 7.18-7.12 (1H, m), 3.99-3.88 (1H, m), 2.88 (2H, br s), 2.22 (3H, s), 1.12 (6H, d, J = 6.7 Hz).
MS (ESI) m/z : 233 [$(M+H)^+$].
The following compound of Example 156 was subsequently synthesized in the same way as in Example 93 and is shown in Table 51.

[Table 51]

| Example No. | Structure | Intermediate used | Instrumental analysis data |
|---|---|---|---|
| 1 5 6 | 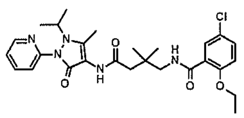 | 1 a | $^1$H-NMR ($CDCl_3$) δ: 8.66 (1H, br s), 8.51-8.48 (1H, m), 8.35 (1H, br t, J = 7.0 Hz), 8.19 (1H, d, J = 3.1 Hz), 7.82-7.76 (2H, m), 7.37 (1H, dd, J = 8.9, 2.7 Hz), 7.19-7.14 (1H, m), 6.90 (1H, d, J = 9.2 Hz), 4.19 (2H, q, J = 6.9 Hz), 4.17-4.08 (1H, m), 3.56 (2H, d, J = 6.7 Hz), 2.29 (3H, s), 2.27 (2H, s), 1.52 (3H, t, J = 7.0 Hz), 1.25 (6H, d, J = 6.7 Hz), 1.12 (6H, s). MS (ESI) m/z : 528 [$(M+H)^+$]. |

(Example 157) 5-Chloro-2-ethoxy-N-(4-{[1-(4-methoxy-2-pyridyl)-2,3-dimethyl-5-oxopyrazol-4-yl]amino}-2,2-dimethyl-4-oxobutyl)benzamide

[Formula 110]

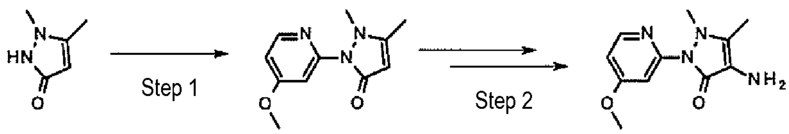


(Step 1) 2-(4-Methoxy-2-pyridyl)-1,5-dimethylpyrazol-3-one

2,3-Dimethyl-1H-pyrazol-5-one (0.500 g, 4.46 mmol), 2-bromo-4-methoxypyridine (1.01 g, 5.35 mmol), copper() iodide (0.0849 g, 0.446 mmol), trans-N,N'-dimethylcyclohexane-1,2-diamine (0.141 mL, 0.127 g, 0.892 mmol), and potassium carbonate (0.945 g, 8.92 mmol) were suspended in 1,4-dioxane (9.0 mL), and the suspension was stirred at 110°C for 4 hours and then at 130°C for 8 hours. The reaction solution was allowed to cool, then N,N-dimethylformamide (9.0 mL) was added to the reaction solution, and the mixture was stirred at 150°C for 6 hours and then at 140°C for 4 hours. The reaction solution was allowed to cool and then filtered through celite to remove a deposit, which was then washed with ethyl acetate. The solvent was distilled off from the filtrate and the washes under reduced pressure. To the obtained residue, toluene was added, and the solvent was distilled off again under reduced pressure. The obtained residue was purified by silica gel column chromatography (Biotage Japan Ltd., eluting solvent: hexane/ethyl acetate → ethyl acetate/methanol = 50/50 to 0/100 → 100/0 to 95/5) to obtain the title compound (0.272 g, yield: 27.9%) as a solid.
$^1$H-NMR ($CDCl_3$) δ: 8.32-8.20 (1H, m), 7.56-7.45 (1H, m), 6.75-6.64 (1H, m), 5.41-5.30 (1H, m), 3.90 (3H, s), 3.33 (3H, s), 2.26 (3H, s).

(Step 2) 4-Amino-2-(4-methoxy-2-pyridyl)-1,5-dimethylpyrazol-3-one

The title compound was obtained as an oil substance by using 2-(4-methoxy-2-pyridyl)-1,5-dimethylpyrazol-3-one synthesized in step 1 of Example 157 instead of 5-(1-methoxyethyl)-1-methyl-2-phenyl-2,3-dihydro-1H-pyrazol-3-one in step 4 of Example 122 and subsequently performing the same reaction as in Example 122 up to step 5.
$^1$H-NMR ($CDCl_3$) δ: 8.29 (1H, d, J = 5.9 Hz), 7.69 (1H, d, J = 2.2 Hz), 6.71 (1H, dd, J = 5.9, 2.2 Hz), 3.94 (3H, s), 3.13 (3H, s), 2.92 (2H, br s), 2.20 (3H, s).

The following compound of Example 157 was subsequently synthesized in the same way as in Example 93 and is shown in Table 52.

[Table 52]

| Example No. | Structure | Intermediate used | Instrumental analysis data |
|---|---|---|---|
| 1 5 7 | 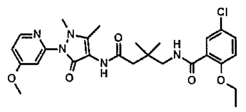 | 1 a | $^1$H-NMR ($CDCl_3$) δ: 9.04 (1H, s), 8.42-8.36 (1H, m), 8.26 (1H, d, J = 6.1 Hz), 8.21 (1H, d, J = 2.4 Hz), 7.57 (1H, d, J = 2.4 Hz), 7.38 (1H, dd, J = 8.5, 2.4 Hz), 6.91 (1H, d, J = 8.5 Hz), 6.69 (1H, dd, J = 6.1, 2.4 Hz), 4.20 (2H, q, J = 7.3 Hz), 3.90 (3H, s), 3.57 (2H, d, J = 6.7 Hz), 3.35 (3H, s), 2.27 (2H, s), 2.27 (3H, s), 1.53 (3H, t, J = 7.3 Hz), 1.14 (6H, s). MS (ESI+APCI) m/z : 530 [(M+H)$^+$]. |

(Example 158) 5-Chloro-N-(2,2-dimethyl-4-{[1-methyl-3-oxo-2-phenyl-5-(3,3,3-trifluoropropyl)pyrazol-4-yl]amino}-4-oxobutyl)-2-ethoxybenzamide

[Formula 111]

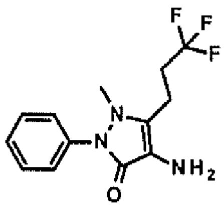

(Step 1) 4-Amino-1-methyl-2-phenyl-5-(3,3,3-trifluoropropyl)pyrazol-3-one

The title compound was obtained as a solid by using 4,4,4-trifluorobutanoic acid instead of 2-methoxypropanoic acid in step 1 of Example 122 and subsequently performing the same reaction as in Example 122 up to step 5.
$^1$H-NMR ($CDCl_3$) δ: 7.47-7.45 (4H, m), 7.28-7.25 (1H, m), 3.03 (2H, br s), 2.84 (3H, s), 2.81-2.78 (2H, m), 2.52-2.43 (2H, m).

The following compound of Example 158 was subsequently synthesized in the same way as in Example 92 and is shown in Table 53.

[Table 53]

| Example No. | Structure | Intermediate used | Instrumental analysis data |
|---|---|---|---|
| 1 5 8 | 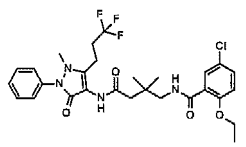 | 1 a | $^1$H-NMR ($CDCl_3$) δ: 9.44 (1H, s), 8.40 (1H, t, J = 6.7 Hz), 8.22 (1H, d, J = 2.7 Hz), 7.46-7.43 (4H, m), 7.39 (1H, dd, J = 8.6, 2.7 Hz), 7.29-7.28 (1H, m), 6.92 (1H, d, J = 8.6 Hz), 4.21 (2H, q, J = 7.0 Hz), 3.60 (2H, d, J = 6.7 Hz), 3.08 (3H, s), 2.93-2.89 (2H, m), 2.66-2.60 (2H, m), 2.28 (2H, s), 1.53 (3H, t, J = 7.0 Hz), 1.11 (6H, s). MS (ESI) m/z : 581 [(M+H)$^+$]. |

(Example 159) 5-Chloro-2-ethoxy-4-fluoro-N-(4-{[5-(methoxymethyl)-1-methyl-3-oxo-2-phenyl-2,3-dihydro-1H-pyrazol-4-yl]amino}-2,2,3-trimethyl-4-oxobutyl)benzamide

[Formula 112]

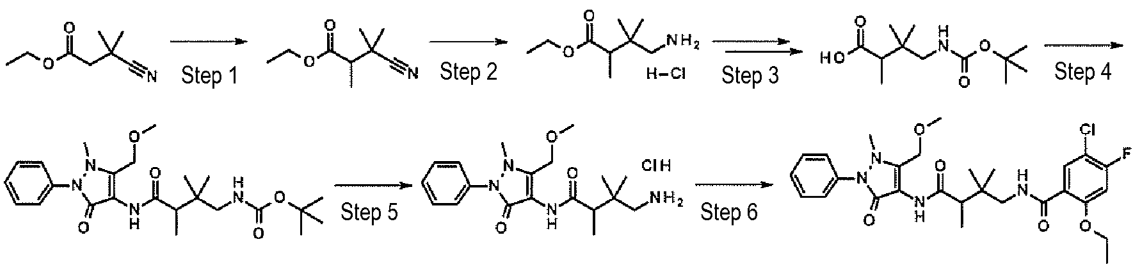


(Step 1) Ethyl 3-cyano-2,3-dimethyl-butanoate

A solution of ethyl 3-cyano-3-methylbutanoate (3.51 g, 22.6 mmol) synthesized according to the method described in J. Chem. Soc., Perkin Trans. 1 1989, 265-278 in tetrahydrofuran (80 mL) was cooled to -78°C. Lithium diisopropylamide (prepared by cooling a solution of N,N-diisopropylamine (3.80 mL, 2.74 g, 27.1 mmol) in tetrahydrofuran (32 mL) to 0°C, then adding thereto n-butyllithium (1.65 M solution in hexane) (15.0 mL, 24.8 mmol), and stirring the mixture for 60 minutes) was added thereto over 3 minutes, and the mixture was stirred for 1.5 hours. Subsequently, a solution of a mixture of methyl iodide (4.20 mL, 9.58 g, 67.0 mmol) and hexamethylphosphoric acid triamide (7.90 mL, 8.13 g, 45.0 mmol) in tetrahydrofuran (10 mL) was added thereto over 1 minute, and the resulting mixture was stirred for 2 hours. A saturated aqueous solution of ammonia chloride (120 mL) and water (30 mL) were added to the reaction mixture, and the temperature of the mixture was raised to room temperature. After extraction with diethyl ether twice, the organic layers were combined and dried over anhydrous sodium sulfate. Then, the solvent was distilled off, and the obtained residue was purified by silica gel column chromatography (Biotage Japan Ltd., eluting solvent: hexane/ethyl acetate = 98/2 to 70/30) to obtain the title compound (3.24 g, yield: 84.7%) as an oil substance. $^1$H-NMR ($CDCl_3$) δ: 4.20 (2H, q, J= 7.1 Hz), 2.53 (1H, q, J = 7.3 Hz), 1.42 (3H, s), 1.41 (3H, s), 1.35 (3H, d, J = 7.3 Hz), 1.29 (3H, t, J= 7.0 Hz).

(Step 2) Ethyl 4-amino-2,3,3-trimethylbutanoate hydrochloride

To a mixture of ethyl 3-cyano-2,3-dimethylbutanoate (3.24 g, 19.1 mmol) synthesized in step 1 and platinum(IV) oxide (0.885 g, 3.90 mmol), ethanol (75 mL) and concentrated hydrochloric acid (1.50 mL, 17.0 mmol) were added, and the resulting mixture was stirred at room temperature for 5 hours under a hydrogen atmosphere. Subsequently, platinum(IV) oxide (0.865 g, 3.81 mmol) and concentrated hydrochloric acid (1.50 mL, 17.0 mmol) were further added thereto, and the mixture was stirred at room temperature for 3 hours under a hydrogen atmosphere. The reaction mixture was filtered and washed with ethanol, and the filtrate was concentrated. The obtained residue was dissolved in 1,4-dioxane (30 mL). To the solution, a solution of 4 N hydrochloric acid in 1,4-dioxane (30.0 mL, 120 mmol) was added, and the mixture was stirred at room temperature for 10 minutes. The reaction mixture was concentrated under reduced pressure, and the obtained solid was filtered and washed with diethyl ether to obtain the title compound (3.70 g, yield: 92.1%) as a solid.
$^1$H-NMR ($CD_3OD$) δ: 4.21-4.12 (2H, m), 3. 02 (1H, d, J = 12.8 Hz), 2.87 (1H, d, J = 13.4 Hz), 2.53 (1H, q, J = 7.3 Hz), 1.27 (3H, t, J= 7.3 Hz), 1.17 (3H, d, J = 7.3 Hz), 1.08 (3H, s), 1.04 (3H, s).

(Step 3) 4-(tert-Butoxycarbonylamino)-2,3,3-trimethylbutanoic acid

The title compound was obtained as a solid by using ethyl 4-amino-2,3,3-trimethylbutanoate hydrochloride synthesized in step 2 instead of ethyl 4-amino-3,3-dimethylbutanoate hydrochloride in step 1 of Example 111 and subsequently performing the same reaction as in Example 111 up to step 2.
$^1$H-NMR ($CDCl_3$) δ: 4.95 (1H, br t, J= 6.4 Hz), 3.19 (1H, dd, J = 14.6, 6.7 Hz), 2.98 (1H, dd, J= 14.3, 7.0 Hz), 2.43 (1H, q, J= 6.9 Hz), 1.46 (9H, s), 1.13 (3H, d, J= 7.3 Hz), 0.96 (3H, s), 0.92 (3H, s).

(Step 4) tert-Butyl N-(4-{[5-(methoxymethyl)-1-methyl-3-oxo-2-phenyl-2,3-dihydro-1H-pyrazol-4-yl]amino}-2,2,3-trimethyl-4-oxobutyl)carbamate

To a solution of 4-(tert-butoxycarbonylamino)-2,3,3-trimethylbutanoic acid (244 mg, 0.995 mmol) synthesized in step 3, 4-amino-5-(methoxymethyl)-1-methyl-2-phenyl-2,3-dihydro-1H-pyrazol-3-one (282 mg, 1.21 mmol) synthesized in step 2 of Example 113, and 0-(7-azabenzotriazol-1-yl)-N,N,N',N'-tetramethyluronium hexafluorophosphate (HATU) (493 mg, 1.30 mmol) in N,N-dimethylformamide (7.2 mL), N,N-diisopropylethylamine (0.260 mL, 193 mg, 1.49 mmol) was added, and the mixture was stirred at 60°C for 9 hours. The reaction mixture was cooled to room temperature, and a saturated aqueous solution of sodium bicarbonate (8.0 mL) and water (16 mL) were added thereto, followed by extraction with ethyl acetate. The organic layer was washed with a saturated aqueous solution of sodium bicarbonate/water (1:2) once, water once, and saturated saline once and dried over anhydrous sodium sulfate. The solvent was distilled off, and the obtained residue was purified by silica gel column chromatography (Biotage Japan Ltd., hexane/ethyl acetate → ethyl acetate/methanol = 70/30 to 0/100 → 100/0 to 92/8) to obtain the title compound (83.0 mg, yield: 18.1%) as a solid.
$^1$H-NMR ($CDCl_3$) δ: 7.96 (1H, br s), 7.49-7.40 (4H, m), 7.34-7.29 (1H, m), 5.24 (1H, br s), 4.54 (2H, s), 3.44 (3H, s), 3.32-3.23 (1H, m), 3.18 (3H, s), 3.06-2.97 (1H, m), 2.41 (1H, q, J= 6.9 Hz), 1.44 (9H, s), 1.17 (3H, d, J= 6.7 Hz), 0.99 (3H, s), 0.97 (3H, s).
MS (ESI) m/z : 461 [$(M+H)^+$].

(Step 5) 4-Amino-N-[5-(methoxymethyl)-1-methyl-3-oxo-2-phenyl-2,3-dihydro-1H-pyrazol-4-yl]-2,3,3-trimethylbutanamide hydrochloride

The title compound was obtained as an oil substance through the same reaction as in step 4 of Example 111 using tert-butyl N-(4-{[5-(methoxymethyl)-1-methyl-3-oxo-2-phenyl-2,3-dihydro-1H-pyrazol-4-yl]amino}-2,2,3-trimethyl-4-oxobutyl)carbamate synthesized in step 4 instead of tert-butyl N-{4-[(1,5-dimethyl-3-oxo-2-phenyl-2,3-dihydro-1H-pyrazol-4-yl)amino]-2,2-dimethyl-4-oxobutyl}carbamate.
MS (APCI) m/z: 361 [$(M+H)^+$].

(Step 6) 5-Chloro-2-ethoxy-4-fluoro-N-(4-{[5-(methoxymethyl)-1-methyl-3-oxo-2-phenyl-2,3-dihydro-1H-pyrazol-4-yl]amino}-2,2,3-trimethyl-4-oxobutyl)benzamide

The title compound was obtained as a solid through the same reaction as in Example 93 using 5-chloro-2-ethoxy-4-fluorobenzoic acid synthesized using ethyl iodide instead of isopropyl iodide in step 2 in the synthesis of intermediate 1m, and 4-amino-N-[5-(methoxymethyl)-1-methyl-3-oxo-2-phenyl-2,3-dihydro-1H-pyrazol-4-yl]-2,3,3-trimethylbutanamide hydrochloride synthesized in step 5.
$^1$H-NMR ($CDCl_3$) δ: 8.94 (1H, br s), 8.31 (1H, d, J = 8.5 Hz), 8.19 (1H, br t, J = 6.4 Hz), 7.48-7.42 (4H, m), 7.32-7.26 (1H, m), 6.78 (1H, d, J = 11.0 Hz), 4.56 (1H, d, J = 14.0 Hz), 4.51 (1H, d, J = 14.0 Hz), 4.18 (2H, q, J = 6.9 Hz), 4.08 (1H, dd, J = 14.0, 7.9 Hz), 3.44 (3H, s), 3.18 (3H, s), 3.12 (1H, dd, J = 14.0, 6.1 Hz), 2.50 (1H, q, J = 6.9 Hz), 1.55 (3H, t, J = 7.0 Hz), 1.16 (3H, d, J = 6.7 Hz), 1.05 (3H, s), 1.02 (3H, s).
MS (ESI) m/z : 561 [$(M+H)^+$].

(Example 160) N-(5-Chloro-2-methoxyphenyl)-N'-(1-cyclohexyl-2,3-dimethyl-5-oxopyrazol-4-yl)-3,3-dimethylpentanediamide

[Formula 113]

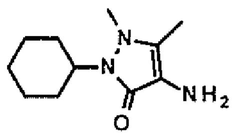

(Step 1) 4-Amino-2-cyclohexyl-1,5-dimethylpyrazol-3-one

The title compound was obtained as an oil substance by using methyl 3-oxobutanoate instead of ethyl 3-oxo-5-[(2,2,2-trifluoroacetyl)amino]pentanoate and cyclohexylhydrazine instead of phenylhydrazine in step 2 of Example 28 and subsequently performing the same reaction as in Example 28 up to step 5.

$^1$H-NMR ($CD_3OD$) δ: 4.22-4.16 (1H, m), 3.68 (3H, s), 2.66 (3H, s), 2.30-2.22 (2H, m), 1.89-1.81 (3H, m), 1.78-1.74 (1H, m), 1.71-1.67 (1H, m), 1.45-1.34 (2H, m), 1.29-1.20 (1H, m).
MS (ESI) m/z : 210 [(M+H)$^+$].

The following compound of Example 160 was subsequently synthesized in the same way as in Example 93 and is shown in Table 54.

[Table 54]

| Example No. | Structure | Intermediate used | Instrumental analysis data |
|---|---|---|---|
| 1 6 0 | 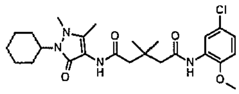 | 2 e | $^1$H-NMR ($CDCl_3$) δ: 8.94 (1H, br s), 8.53 (1H, br s), 8.37 (1H, d, J = 2.3 Hz), 6.98 (1H, dd, J = 8.6, 2.3 Hz), 6.73 (1H, d, J = 8.6 Hz), 4.00 (1H, tt, J = 12.3, 3.4 Hz), 3.21 (3H, s), 2.62 (2H, s), 2.45 (2H, s), 2.15 (3H, s), 2.01-1.94 (2H, m), 1.86-1.82 (4H, m), 1.69-1.66 (1H, m), 1.37-1.29 (2H, m), 1.23-1.17 (1H, m), 1.16 (6H, s). MS (ESI) m/z : 491 [(M+H)$^+$]. |

The produced compound names of Examples will be described below.

[Table 55-1]

| Example | Compound name |
|---|---|
| Example 1 | 5-Chloro-N-[2,2-dimethyl-4-({1-methyl-5-[1-(methylamino)ethyl]-3-oxo-2-phenyl-2,3-dihydro-1H-pyrazol-4-yl}amino)-4-oxobutyl]-2-ethoxybenzamide |
| Example 2 | 5-Chloro-N-[2,2-dimethyl-4-({1-methyl-5-[1-(methylamino)ethyl]-3-oxo-2-phenyl-2,3-dihydro-1H-pyrazol-4-yl}amino)-4-oxobutyl]-2-ethoxypyridine-3-carboxamide |
| Example 3 | 5-Chloro-N-{4-[(5-{[(2,4-dimethoxyphenylmethyl)-methylamino]methyl}-1-methyl-3-oxo-2-phenyl-2,3-dihydro-1H-pyrazol-4-yl)amino]-2,2-dimethyl-4-oxobutyl}-2-methoxybenzamide |
| Example 4 | 5-Chloro-N-(2,2-dimethyl-4-{[1-methyl-5-(methylaminomethyl)-3-oxo-2-phenyl-2,3-dihydro-1H-pyrazol-4-yl]amino}-4-oxobutyl)-2-methoxybenzamide |
| Example 5 | 5-Chloro-N-[4-({5-[(dimethylamino)methyl]-1-methyl-3-oxo-2-phenyl-2,3-dihydro-1H-pyrazol-4-yl}amino)-2,2-dimethyl-4-oxobutyl]-2-methoxybenzamide |
| Example 6 | 5-Chloro-N-[4-({5-[(dimethylamino)methyl]-1-methyl-3-oxo-2-phenyl-2,3-dihydro-1H-pyrazol-4-yl}amino)-2,2-dimethyl-4-oxobutyl]-4-fluoro-2-methoxybenzamide |
| Example 7 | 5-Chloro-N-[4-({5-[(dimethylamino)methyl]-1-methyl-3-oxo-2-phenyl-2,3-dihydro-1H-pyrazol-4-yl}amino)-2,2-dimethyl-4-oxobutyl]-2-methoxypyridine-3-carboxamide |
| Example 8 | 5-Chloro-N-(4-[(5-{[2-hydroxyethyl(methyl)amino]methyl}-1-methyl-3-oxo-2-phenyl-2,3-dihydro-1H-pyrazol-4-yl)amino]-2,2-dimethyl-4-oxobutyl)-2-methoxybenzamide |
| Example 9 | N-(5-Chloro-4-fluoro-2-methoxyphenyl)-N'-{5-[(dimethylamino)methyl]-1-methyl-3-oxo-2-phenyl-2,3-dihydro-1H-pyrazol-4-yl}-3,3-dimethylpentanediamide |
| Example 10 | tert-Butyl N-{2-[4({4-[(5-chloro-2-ethoxybenzoyl)amino]-3,3-dimethylbutanoyl}amino)-2-methyl-5-oxo-1-phenyl-2,3-dihydro-1H-pyrazol-3-yl]ethyl}carbamate |
| Example 11 | 5-Chloro-N-[4-({5-[2-(dimethylamino)ethyl]-1-methyl-3-oxo-2-phenyl-2,3-dihydro-1H-pyrazol-4-yl}amino)-2,2-dimethyl-4-oxobutyl]-2-ethoxybenzamide |
| Example 12 | 5-Chloro-N-[4-({5-[2-(dimethylamino)ethyl]-1-methyl-3-oxo-2-phenyl-2,3-dihydro-1H-pyrazol-4-yl}amino)-2,2-dimethyl-4-oxobutyl]-2-ethoxy-4-fluorobenzamide |
| Example 13 | 5-Chloro-N-[4-({5-[2-(dimethylamino)ethyl]-1-methyl-3-oxo-2-phenyl-2,3-dihydro-1H-pyrazol-4-yl}amino)-2,2-dimethyl-4-oxobutyl]-2-ethoxypyridine-3-carboxamide |

[Table 55-2]

| | |
|---|---|
| Example 14 | 5-Chloro-N-[4-({5-[2-(dimethylamino)ethyl]-1-methyl-3-oxo-2-phenyl-2,3-dihydro-1H-pyrazol-4-yl}amino)-2,2-dimethyl-4-oxobutyl]-2-methylbenzofuran-7-carboxamide |
| Example 15 | 5-Chloro-N-[4-({5-[2-(dimethylamino)ethyl]-1-methyl-3-oxo-2-phenyl-2,3-dihydro-1H-pyrazol-4-yl}amino)-2,2-dimethyl-4-oxobutyl]-2-(methoxymethyl)benzofuran-7-carboxamide |
| Example 16 | 7-Chloro-N-[4-({5-[2-(dimethylamino)ethyl]-1-methyl-3-oxo-2-phenyl-2,3-dihydro-1H-pyrazol-4-yl}amino)-2,2-dimethyl-4-oxobutyl]-2,3-dihydro-1,4-benzodioxine-5-carboxamide |
| Example 17 | 5-Chloro-N-[4-({5-[2-(diethylamino)ethyl]-1-methyl-3-oxo-2-phenyl-2,3-dihydro-1H-pyrazol-4-yl}amino)-2,2-dimethyl-4-oxobutyl]-2-ethoxybenzamide |
| Example 18 | 5-Chloro-N-[4-({5-[2-(isopropylamino)ethyl]-1-methyl-3-oxo-2-phenyl-2,3-dihydro-1H-pyrazol-4-yl}amino)-2,2-dimethyl-4-oxobutyl]-2-ethoxybenzamide |
| Example 19 | 5-Chloro-2-ethoxy-N-{4-[(5-{2-[isopropyl(methyl)amino]ethyl}-1-methyl-3-oxo-2-phenyl-2,3-dihydro-1H-pyrazol-4-yl)amino]-2,2-dimethyl-4-oxobutyl}benzamide |
| Example 20 | tert-Butyl N-{2-[4-({4-[(5-chloro-2-methoxybenzoyl)amino]-3,3-dimethylbutanoyl}amino)-2-methyl-5-oxo-1-phenyl-2,3-dihyro-1H-pyrazol-3-yl]ethyl}carbamate |
| Example 21 | N-(4-{[5-(2-aminoethyl)-1-methyl-3-oxo-2-phenyl-2,3-dihyro-1H-pyrazol-4-yl]amino-2,2-dimethyl-4-oxobutyl}-5-chloro-2-methoxybenzamide |
| Example 22 | 5-Chloro-N-[4-({5-[2-(dimethylamino)ethyl]-1-methyl-3-oxo-2-phenyl-2,3-dihydro-1H-pyrazol-4-yl}amino)-2,2-dimethyl-4-oxobutyl]-2-methoxybenzamide |
| Example 23 | N-(5-Chloro-2-ethoxyphenyl)-N’-{5-[2-(dimethylamino)ethyl]-1-methyl-3-oxo-2-phenyl-2,3-dihydro-1H-pyrazol-4-yl}-3,3-dimethylpentanediamide |
| Example 24 | N-(5-Chloro-2-ethoxy-4-fluorophenyl)-N’-{5-[2-(dimethylamino)ethyl]-1-methyl-3-oxo-2-phenyl-2,3-dihydro-1H-pyrazol-4-yl}-3,3-dimethylpentanediamide |
| Example 25 | N-(5-Chloro-2-ethoxy-3-pyridyl)-N’-{5-[2-(dimethylamino)ethyl]-1-methyl-3-oxo-2-phenyl-2,3-dihydro-1H-pyrazol-4-yl}-3,3-dimethylpentanediamide |
| Example 26 | 5-Chloro-N-[2,2-dimethyl-4-({1-methyl-3-oxo-2-phenyl-5-[2-(2,2,2-trifluoroethylamino)ethyl]-2,3-dihydro-1H-pyrazol-4-yl}amino)-4-oxobutyl]-2-ethoxybenzamide |
| Example 27 | 5-Chloro-N-[2,2-dimethyl-4-({1-methyl-3-oxo-2-phenyl-5-[2-(2,2,2-trifluoroethylamino)ethyl]-2,3-dihydro-1H-pyrazol-4-yl}amino)-4-oxobutyl]-2-ethoxy-4-fluorobenzamide |

[Table 55-3]

| | |
|---|---|
| Example 28 | 5-Chloro-N-[4-({5-[2-(dimethylamino)ethyl]1-methyl-3-oxo-2-phenyl-2,3-dihydro-1H-pyrazol-4-yl}amino)-2,2-dimethyl-4-oxobutyl]-2-(methoxymethoxy)benzamide |
| Example 29 | 5-Chloro-N-[4-({5-[2-(dimethylamino)ethyl]1-methyl-3-oxo-2-phenyl-2,3-dihydro-1H-pyrazol-4-yl}amino)-2,2-dimethyl-4-oxobutyl]-4-fluoro-2-(methoxymethoxy)benzamide |
| Example 30 | 5-Bromo-N-[4-({5-[2-(dimethylamino)ethyl]1-methyl-3-oxo-2-phenyl-2,3-dihydro-1H-pyrazol-4-yl}amino)-2,2-dimethyl-4-oxobutyl]-2-ethoxypyridine-3-carboxamide |
| Example 31 | N-{4-[(5-{2-[bis(trideuteriomethyl)amino]ethyl}-1-methyl-3-oxo-2-phenyl-2,3-dihydro-1H-pyrazol-4-yl)amino]-2,2-dimethyl-4-oxobutyl}-5-chloro-2-ethoxybenzamide |
| Example 32 | N-(5-chloro-2-ethoxyphenyl)-2-{1-[2-({5-[2-(dimethylamino)ethyl]-1-methyl-3-oxo-2-phenyl-2,3-dihydro-1H-pyrazol-4-yl}amino)-2-oxoethyl]cyclopentyl}acetamide |
| Example 33 | 5-Chloro-N-[2,2-dimethyl-4-({1-methyl-5-[2-(methylamino)ethyl]-3-oxo-2-phenyl-2,3-dihydro-1H-pyrazol-4-yl}amino)-4-oxobutyl]-2-ethoxybenzamide |
| Example 34 | 5-Chloro-N-[2,2-dimethyl-4-({1-methyl-5-[2-(methylamino)ethyl]-3-oxo-2-phenyl-2,3-dihydro-1H-pyrazol-4-yl}amino)-4-oxobutyl]-2-ethoxy-4-fluorobenzamide |

(continued)

| | |
|---|---|
| Example 35 | 5-Chloro-2-ethoxy-N-{4-[(5-{2-[ethyl(methyl)amino]ethyl}-1-methyl-3-oxo-2-phenyl-2,3-dihydro-1H-pyrazol-4-yl)amino]-2,2-dimethyl-4-oxobutyl}benzamide |
| Example 36 | 5-Chloro-2-ethoxy-N-{4-[(5-{2-[2-fluoroethyl(methyl)amino]ethyl}-1-methyl-3-oxo-2-phenyl-2,3-dihydro-1H-pyrazol-4-yl)amino]-2,2-dimethyl-4-oxobutyl}benzamide |
| Example 37 | 5-Chloro-2-ethoxy-4-fluoro-N-{4-[(5-{2-[2-fluoroethyl(methyl)amino]ethyl}-1-methyl-3-oxo-2-phenyl-2,3-dihydro-1H-pyrazol-4-yl)amino]-2,2-dimethyl-4-oxobutyl}benzamide |
| Example 38 | 5-Chloro-N-{2,2-dimethyl-4-[(1-methyl-5-{2-[methyl(2,2,2-trifluoroethyl)amino]ethyl}-3-oxo-2-phenyl-2,3-dihydro-1H-pyrazol-4-yl)amino]-4-oxobutyl}-2-ethoxy-4-fluorobenzamide |
| Example 39 | 5-Chloro-N-{2,2-dimethyl-4-[(1-methyl-5-{2-[methyl(2,2,2-trifluoroethyl)amino]ethyl}-3-oxo-2-phenyl-2,3-dihydro-1H-pyrazol-4-yl)amino]-4-oxobutyl}-2-ethoxybenzamide |
| Example 40 | 5-Chloro-N-[4-({5-[2-(dimethylamino)ethyl]-1-ethyl-3-oxo-2-phenyl-2,3-dihydro-1H-pyrazol-4-yl}amino)-2,2-dimethyl-4-oxobutyl]-2-ethoxybenzamide |
| Example 41 | 5-Chloro-N-[4-({5-[2-(dimethylamino)ethyl]-1-ethyl-3-oxo-2-phenyl-2,3-dihydro-1H-pyrazol-4-yl}amino)-2,2-dimethyl-4-oxobutyl]-2-ethoxy-4-fluorobenzamide |

[Table 55-4]

| | |
|---|---|
| Example 42 | 5-Chloro-N-[4-({5-[2-(dimethylamino)ethyl]-1-ethyl-3-oxo-2-phenyl-2,3-dihydro-1H-pyrazol-4-yl}amino)-2,2-dimethyl-4-oxobutyl]-2-ethoxypyridine-3-carboxamide |
| Example 43 | 5-Chloro-N-[4-({3-[2-(dimethylamino)ethyl]-5-oxo-1-phenyl-2-(trideuteriomethyl)-2,3-dihydro-1H-pyrazol-4-yl}amino)-2,2-dimethyl-4-oxobutyl]-2-ethoxybenzamide |
| Example 44 | 5-Bromo-N-[4-({3-[2-(dimethylamino)ethyl]-5-oxo-1-phenyl-2-(trideuteriomethyl)-2,3-dihydro-1H-pyrazol-4-yl}amino)-2,2-dimethyl-4-oxobutyl]-2-ethoxypyridine-3-carboxamide |
| Example 45 | 5-Chloro-N-[4-({3-[2-(dimethylamino)ethyl]-1-(2-fluorophenyl)-2-methyl-5-oxo-1,5-dihydro-1H-pyrazol-4-yl}amino)-2,2-dimethyl-4-oxobutyl]-2-ethoxybenzamide |
| Example 46 | 5-Chloro-N-[4-({3-[2-(dimethylamino)ethyl]-1-(2-fluorophenyl)-2-methyl-5-oxo-1,5-dihydro-1H-pyrazol-4-yl}amino)-2,2-dimethyl-4-oxobutyl]-2-methylbenzofuran-7-carboxamide |
| Example 47 | 5-Chloro-N-(4-{[1-(6-chloro-2-pyridyl)-3-(2-dimethylamino)ethyl]-2-methyl-5-oxo-1,5-dihydro-1H-pyrazol-4-yl}amino-2,2-dimethyl-4-oxobutyl)-2-ethoxybenzamide |
| Example 48 | 5-Chloro-N-[4-({5-[(2R)-2-(dimethylamino)propyl]-1-methyl-3-oxo-2-phenyl-2,3-dihydro-1H-pyrazol-4-yl}amino)-2,2-dimethyl-4-oxobutyl]-2-ethoxybenzamide |
| Example 49 | 5-Chloro-N-[4-({5-[(2R)-2-(dimethylamino)propyl]-1-methyl-3-oxo-2-phenyl-2,3-dihydro-1H-pyrazol-4-yl}amino)-2,2-dimethyl-4-oxobutyl]-2-ethoxy-4-fluorobenzamide |
| Example 50 | 5-Chloro-N-[4-({5-[(2R)-2-(dimethylamino)propyl]-1-methyl-3-oxo-2-phenyl-2,3-dihydro-1H-pyrazol-4-yl}amino)-2,2-dimethyl-4-oxobutyl]-2-methylbenzofuran-7-carboxamide |
| Example 51 | 5-Chloro-N-[4-({5-[(2S)-2-(dimethylamino)propyl]-1-methyl-3-oxo-2-phenyl-2,3-dihydro-1H-pyrazol-4-yl}amino)-2,2-dimethyl-4-oxobutyl]-2-ethoxybenzamide |
| Example 52 | 5-Chloro-N-[4-({5-[(2S)-2-(dimethylamino)propyl]-1-methyl-3-oxo-2-phenyl-2,3-dihydro-1H-pyrazol-4-yl}amino)-2,2-dimethyl-4-oxobutyl]-2-ethoxy-4-fluorobenzamide |
| Example 53 | 5-Chloro-N-[4-({5-[(2S)-2-(dimethylamino)propyl]-1-methyl-3-oxo-2-phenyl-2,3-dihydro-1H-pyrazol-4-yl}amino)-2,2-dimethyl-4-oxobutyl]-2-ethoxypyridine-3-carboxamide |
| Example 54 | 5-Chloro-N-[4-({5-[(2S)-2-(dimethylamino)propyl]-1-methyl-3-oxo-2-phenyl-2,3-dihydro-1H-pyrazol-4-yl}amino)-2,2-dimethyl-4-oxobutyl]-2-methylbenzofuran-7-carboxamide |
| Example 55 | N-(4-{[5-(2-Amino-2-methylpropyl)-1-methyl-3-oxo-2-phenyl-2,3-dihydro-1H-pyrazol-4-yl]amino}-2,2-dimethyl-4-oxobutyl)-5-chloro-2-ethoxybenzamide |

[Table 55-5]

| | |
|---|---|
| Example 56 | 5-Chloro-N-[4-({5-[2-(dimethylamino)-2-methylpropyl]-1-methyl-3-oxo-2-phenyl-2,3-dihydro-1H-pyrazol-4-yl}amino)-2,2-dimethyl-4-oxobutyl]-2-ethoxybenzamide |
| Example 57 | 5-Chloro-N-[4-({5-[2-(dimethylamino)-2-methylpropyl]-1-methyl-3-oxo-2-phenyl-2,3-dihydro-1H-pyrazol-4-yl}amino)-2,2-dimethyl-4-oxobutyl]-2-ethoxypyridine-3-carboxamide |
| Example 58 | N-(4-{[5-(2-amino-2-methylpropyl)-1-methyl-3-oxo-2-phenyl-2,3-dihyro-1H-pyrazol-4-yl]amino}-2,2-dimethyl-4-oxobutyl)-5-chloro-2-methylbenzofuran-7-carboxamide |
| Example 59 | 5-Chloro-N-[2,2-dimethyl-4-({1-methyl-5-[2-methyl-2-(methylamino)propyl]-3-oxo-2-phenyl-2,3-dihydro-1H-pyrazol-4-yl}amino)-4-oxobutyl]-2-ethoxybenzamide |
| Example 60 | 5-Bromo-N-[2,2-dimethyl-4-({1-methyl-5-[2-methyl-2-(methylamino)propyl]-3-oxo-2-phenyl-2,3-dihydro-1H-pyrazol-4-yl}amino)-4-oxobutyl]-2-ethoxypyridine-3-carboxamide |
| Example 61 | 5-Chloro-N-[2,2-dimethyl-4-({1-methyl-5-[2-methyl-2-(methylamino)propyl]-3-oxo-2-phenyl-2,3-dihydro-1H-pyrazol-4-yl}amino)-4-oxobutyl]-2-methylbenzofuran-7-carboxamide |
| Example 62 | 5-Chloro-N-[4-({5-[2-(dimethylamino)ethyl]-1-methyl-3-oxo-2-phenyl-2,3-dihydro-1H-pyrazolo-4-yl}amino)-2,2-bis(fluoromethyl)-4-oxobutyl]-2-ethoxybenzamide |
| Example 63 | 5-Chloro-N-[4-({5-[3-(dimethylamino)propyl]-1-methyl-3-oxo-2-phenyl-2,3-dihydro-1H-pyrazol-4-yl}amino)-2,2-dimethyl-4-oxobutyl]-2-ethoxybenzamide |
| Example 64 | 5-Chloro-N-[4-({5-[3-(dimethylamino)propyl]-1-methyl-3-oxo-2-phenyl-2,3-dihydro-1H-pyrazol-4-yl}amino)-2,2-dimethyl-4-oxobutyl]-2-ethoxy-4-fluorobenzamide monohydrochloride |
| Example 65 | 5-Chloro-N-[4-({5-[3-(dimethylamino)propyl]-1-methyl-3-oxo-2-phenyl-2,3-dihydro-1H-pyrazol-4-yl}amino)-2,2-dimethyl-4-oxobutyl]-2-methylbenzofuran-7-carboxamide |
| Example 66 | 5-Chloro-N-[4-({5-[2-(dimethylamino)-1-methylethyl]-1-methyl-3-oxo-2-phenyl-2,3-dihydro-1H-pyrazol-4-yl}amino)-2,2-dimethyl-4-oxobutyl]-2-ethoxybenzamide |
| Example 67 | 5-Chloro-N-[4-({5-[(1S)-2-(dimethylamino)-1-methylethyl]-1-methyl-3-oxo-2-phenyl-2,3-dihydro-1H-pyrazol-4-yl}amino)-2,2-dimethyl-4-oxobutyl]-2-ethoxybenzamide |
| Example 68 | 5-Chloro-N-[4-({5-[(1R)-2-(dimethylamino)-1-methylethyl]-1-methyl-3-oxo-2-phenyl-2,3-dihydro-1H-pyrazol-4-yl}amino)-2,2-dimethyl-4-oxobutyl]-2-ethoxybenzamide |
| Example 69 | 5-Chloro-N-[4-({5-[2-(dimethylamino)-1-methylethyl]-1-methyl-3-oxo-2-phenyl-2,3-dihydro-1H-pyrazol-4-yl}amino)-2,2-dimethyl-4-oxobutyl]-2-ethoxy-4-fluorobenzamide |

[Table 55-6]

| | |
|---|---|
| Example 70 | 5-Chloro-N-[4-({5-[(1R)-2-(dimethylamino)-1-methylethyl]-1-methyl-3-oxo-2-phenyl-2,3-dihydro-1H-pyrazol-4-yl}amino)-2,2-dimethyl-4-oxobutyl]-2-ethoxy-4-fluorobenzamide |
| Example 71 | 5-Chloro-N-[4-({5-[2-(dimethylamino)-1-methylethyl]-1-methyl-3-oxo-2-phenyl-2,3-dihydro-1H-pyrazol-4-yl}amino)-2,2-dimethyl-4-oxobutyl]-2-ethoxypyridine-3-carboxamide |
| Example 72 | 5-Chloro-N-[4-({5-[(1S)-2-(dimethylamino)-1-methylethyl]-1-methyl-3-oxo-2-phenyl-2,3-dihydro-1H-pyrazol-4-yl}amino)-2,2-dimethyl-4-oxobutyl]-2-ethoxypyridine-3-carboxamide |
| Example 73 | 5-Chloro-N-[4-({5-[(1R)-2-(dimethylamino)-1-methylethyl]-1-methyl-3-oxo-2-phenyl-2,3-dihydro-1H-pyrazol-4-yl}amino)-2,2-dimethyl-4-oxobutyl]-2-ethoxypyridine-3-carboxamide |
| Example 74 | 5-Bromo-N-[4-({5-[2-(dimethylamino)-1-methylethyl]-1-methyl-3-oxo-2-phenyl-2,3-dihydro-1H-pyrazol-4-yl}amino)-2,2-dimethyl-4-oxobutyl]-2-ethoxypyridine-3-carboxamide |
| Example 75 | 4,5-Dichloro-N-[4-({5-[2-(dimethylamino)-1-methylethyl]-1-methyl-3-oxo-2-phenyl-2,3-dihydro-1H-pyrazol-4-yl}amino)-2,2-dimethyl-4-oxobutyl]-2-(methoxymethoxy)benzamide |
| Example 76 | 5-Chloro-N-[2,2-dimethyl-4-({1-methyl-5-[1-methyl-2-(methylamino)ethyl]-3-oxo-2-phenyl-2,3-dihydro-1H-pyrazol-4-yl}amino)-4-oxobutyl]-2-ethoxybenzamide |

(continued)

| | |
|---|---|
| Example 77 | 5-Chloro-N-[2,2-dimethyl-4-({1-methyl-5-[1-methyl-2-(methylamino)ethyl]-3-oxo-2-phenyl-2,3-dihydro-1H-pyrazol-4-yl}amino)-4-oxobutyl]-2-ethoxypyridine-3-carboxamide |
| Example 78 | 5-Chloro-N-[2,2-dimethyl-4-({1-methyl-5-[1-methyl-2-(methylamino)ethyl]-3-oxo-2-phenyl-2,3-dihydro-1H-pyrazol-4-yl}amino)-4-oxobutyl]-2-methylbenzofuran-7-carboxamide |
| Example 79 | 5-Chloro-N-[4-({5-[2-(dimethylamino)-1-methoxyethyl]-1-methyl-3-oxo-2-phenyl-2,3-dihydro-1H-pyrazol-4-yl}amino)-2,2-dimethyl-4-oxobutyl]-2-ethoxybenzamide |
| Example 80 | 5-Chloro-N-[4-({5-[2-(dimethylamino)-1-methoxyethyl]-1-methyl-3-oxo-2-phenyl-2,3-dihydro-1H-pyrazol-4-yl}amino)-2,2-dimethyl-4-oxobutyl]-2-ethoxy-4-fluorobenzamide |
| Example 81 | 5-Chloro-N-[4-({5-[2-(dimethylamino)-1-methoxyethyl]-1-methyl-3-oxo-2-phenyl-2,3-dihydro-1H-pyrazol-4-yl}amino)-2,2-dimethyl-4-oxobutyl]-2-ethoxypyridine-3-carboxamide |
| Example 82 | (-)-5-Chloro-N-[4-({5-[2-(dimethylamino)-1-methoxyethyl]-1-methyl-3-oxo-2-phenyl-2,3-dihydro-1H-pyrazol-4-yl}amino)-2,2-dimethyl-4-oxobutyl]-2-ethoxypyridine-3-carboxamide |
| Example 83 | (+)-5-Chloro-N-[4-({5-[2-(dimethylamino)-1-methoxyethyl]-1-methyl-3-oxo-2-phenyl-2,3-dihydro-1H-pyrazol-4-yl}amino)-2,2-dimethyl-4-oxobutyl]-2-ethoxypyridine-3-carboxamide |

[Table 55-7]

| | |
|---|---|
| Example 84 | 5-Chloro-N-[4-({5-[2-(dimethylamino)-1,1-dimethylethyl]-1-methyl-3-oxo-2-phenyl-2,3-dihydro-1H-pyrazol-4-yl}amino)-2,2-dimethyl-4-oxobutyl]-2-ethoxybenzamide |
| Example 85 | (+)-5-Chloro-N-[2,2-dimethyl-4-({1-methyl-5-[1-(methylamino)ethyl]-3-oxo-2-phenyl-2,3-dihydro-1H-pyrazol-4-yl}amino)-4-oxobutyl]-2-ethoxypyridine-3-carboxamide |
| Example 86 | (-)-5-Chloro-N-[2,2-dimethyl-4-({1-methyl-5-[1-(methylamino)ethyl]-3-oxo-2-phenyl-2,3-dihydro-1H-pyrazol-4-yl}amino)-4-oxobutyl]-2-ethoxypyridine-3-carboxamide |
| Example 87 | 5-Chloro-N-[4-({5-[2-(dimethylamino)-1,2-dimethylpropyl]-1-methyl-3-oxo-2-phenyl-2,3-dihydro-1H-pyrazol-4-yl}amino)-2,2-dimethyl-4-oxobutyl]-2-ethoxybenzamide |
| Example 88 | 5-Chloro-N-[4-({5-[4-(dimethylamino)butyl]-1-methyl-3-oxo-2-phenyl-2,3-dihydro-1H-pyrazol-4-yl}amino)-2,2-dimethyl-4-oxobutyl]-2-ethoxybenzamide |
| Example 89 | 5-Chloro-N-[4-({5-[4-(dimethylamino)butyl]-1-methyl-3-oxo-2-phenyl-2,3-dihydro-1H-pyrazol-4-yl}amino)-2,2-dimethyl-4-oxobutyl]-2-ethoxy-4-fluorobenzamide |
| Example 90 | 5-Chloro-N-[4-({5-[4-(dimethylamino)butyl]-1-methyl-3-oxo-2-phenyl-2,3-dihydro-1H-pyrazol-4-yl}amino)-2,2-dimethyl-4-oxobutyl]-2-methylbenzofuran-7-carboxamide |
| Example 91 | 5-Chloro-N-[4-({5-[2-(dimethylamino)ethyl]-1-methyl-3-oxo-2-phenyl-2,3-dihydro-1H-pyrazol-4-yl}amino)-3-fluoro-2,2-dimethyl-4-oxobutyl]-2-ethoxybenzamide |
| Example 92 | 5-Chloro-N-{4-[(1,5-dimethyl-3-oxo-2-phenyl-2,3-dihydro-1H-pyrazol-4-yl)amino]-2,2-dimethyl-4-oxobutyl}-2-ethoxy-4-fluorobenzamide |
| Example 93 | 5-Chloro-N-{4-[(1,5-dimethyl-3-oxo-2-phenyl-2,3-dihydro-1H-pyrazol-4-yl)amino-2,2-dimethyl-4-oxobutyl]-2-ethoxybenzamide |
| Example 94 | 5-Chloro-N-{4-[(1,5-dimethyl-3-oxo-2-phenyl-2,3-dihydro-1H-pyrazol-4-yl)amino-2,2-dimethyl-4-oxobutyl]-2-isopropoxybenzamide |
| Example 95 | 5-Chloro-N-{4-[(1,5-dimethyl-3-oxo-2-phenyl-2,3-dihydro-1H-pyrazol-4-yl)amino-2,2-dimethyl-4-oxobutyl]-2-ethoxypyridine-3-carboxamide |
| Example 96 | 5-Chloro-2-(cyclopropoxy)-N-{4-[(1,5-dimethyl-3-oxo-2-phenyl-2,3-dihydro-1H-pyrazol-4-yl)amino-2,2-dimethyl-4-oxobutyl]benzamide |
| Example 97 | 5-Chloro-N-{4-[(1,5-dimethyl-3-oxo-2-phenyl-2,3-dihydro-1H-pyrazol-4-yl)amino]-2-hydroxy-2-methyl-4-oxobutyl}-4-fluoro-2-isopropoxybenzamide |

(continued)

| | |
|---|---|
| Example 98 | N-(5-Chloro-2-methoxyphenyl)-2-(2-{2-[(1,5-dimethyl-3-oxo-2-phenyl-2,3-dihydro-1H-pyrazol-4-yl)amino]-2-oxoethyl}-1,3-dioxolan-2-yl)acetamide |

[Table 55-8]

| | |
|---|---|
| Example 99 | N-(5-Chloro-2-methoxyphenyl)-N'-(1,5-dimethyl-3-oxo-2-phenyl-2,3-dihydro-1H-pyrazol-4-yl)-3-ethyl-3-methylpentanediamide |
| Example 100 | N-(5-Chloro-2-methoxyphenyl)-2-(1-{2-[(1,5-dimethyl-3-oxo-2-phenyl-2,3-dihydro-1H-pyrazol-4-yl)amino]-2-oxoethyl}cyclobutyl)acetamide |
| Example 101 | N-(5-Chloro-2-methoxyphenyl)-2-(1-{2-[(1,5-dimethyl-3-oxo-2-phenyl-2,3-dihydro-1H-pyrazol-4-yl)amino]-2-oxoethyl}cyclopropyl)acetamide |
| Example 102 | {1-[2-(5-Chloro-2-ethoxyaniline)-2-oxoethyl]-3-[(1,5-dimethyl-3-oxo-2-phenyl-2,3-dihydro-1H-pyrazol-4-yl)amino]-1-methyl-3-oxopropyl} acetate |
| Example 103 | N-(5-Chloro-2-ethoxyphenyl)-N'-(1,5-dimethyl-3-oxo-2-phenyl-2,3-dihydro-1H-pyrazol-4-yl)-3-hydroxy-3-methylpentanediamide |
| Example 104 | N-(5-Chloro-2-methoxyphenyl)-N'-(1,5-dimethyl-3-oxo-2-phenyl-2,3-dihydro-1H-pyrazol-4-yl)-3-methoxy-3-methylpentanediamide |
| Example 105 | 5-Chloro-N-{4-[(1,5-dimethyl-3-oxo-2-phenyl-2,3-dihydro-1H-pyrazol-4-yl)amino]-2,2-dimethyl-4-oxobutyl}-2-methyl-2,3-dihydrobenzofuran-7-carboxamide |
| Example 106 | 6-Chloro-N-{4-[(1,5-dimethyl-3-oxo-2-phenyl-2,3-dihydro-1H-pyrazol-4-yl)amino]-2,2-dimethyl-4-oxobutyl}chromane-8-carboxamide |
| Example 107 | 6-Chloro-N-{4-[(1,5-dimethyl-3-oxo-2-phenyl-2,3-dihydro-1H-pyrazol-4-yl)amino]-2,2-dimethyl-4-oxobutyl}-2-methylquinoline-8-carboxamide |
| Example 108 | 5-Chloro-N-{4-[(1,5-dimethyl-3-oxo-2-phenyl-2,3-dihydro-1H-pyrazol-4-yl)amino]-2,2-dimethyl-4-oxobutyl}-2-(oxetan-3-yloxy)benzamide |
| Example 109 | 6-Chloro-N-{4-[1,5-dimethyl-3-oxo-2-phenyl-2,3-dihydro-1H-pyrazol-4-yl]amino}-2,2-dimethyl-4-oxobutyl]-2-methyl-3,4-dihydro-2H-1,4-benzoxazine-8-carboxamide |
| Example 110 | 6-Chloro-N-{4-[(1,5-dimethyl-3-oxo-2-phenyl-2,3-dihydro-1H-pyrazol-4-yl)amino]-2,2-dimethyl-4-oxobutyl}-2,4-dimethyl-2,3-dihydro-1,4-benzoxazine-8-carboxamide |
| Example 111 | 6-Chloro-N-{4-[(1,5-dimethyl-3-oxo-2-phenyl-2,3-dihydro-1H-pyrazol-4-yl)amino]-2,2-dimethyl-4-oxobutyl}-2-ethyl-1,3-benzoxazole-4-carboxamide |
| Example 112 | 5-Chloro-N-[4-(1,5-dimethyl-3-oxo-2-phenyl-2,3-dihydro-1H-pyrazol-4-yl)amino]-3-hydroxy-2,2-dimethyl-4-oxobutyl]-2-methoxybenzamide |
| Example 113 | 5-Chloro-2-ethoxy-4-fluoro-N-(4-{[5-(methoxymethyl)-1-methyl-3-oxo-2-phenyl-2,3-dihydro-1H-pyrazol-4-yl]amino}-2,2-dimethyl-4-oxobutyl)benzamide |

[Table 55-9]

| | |
|---|---|
| Example 114 | 5-Chloro-2-ethoxy-N-(4-{[5-(methoxymethyl)-1-methyl-3-oxo-2-phenyl-2,3-dihydro-1H-pyrazol-4-yl]amino}-2,2-dimethyl-4-oxobutyl)benzamide |
| Example 115 | 5-Chloro-2-(methoxymethoxy)-N-(4-{[5-(methoxymethyl)-1-methyl-3-oxo-2-phenyl-2,3-dihydro-1H-pyrazol-4-yl]amino}-2,2-dimethyl-4-oxobutyl)benzamide |
| Example 116 | 5-Chloro-4-fluoro-2-(methoxymethoxy)-N-(4-{[5-(methoxymethyl)-1-methyl-3-oxo-2-phenyl-2,3-dihydro-1H-pyrazol-4-yl]amino}-2,2-dimethyl-4-oxobutyl)benzamide |
| Example 117 | 5-Chloro-2-ethoxy-N-(4-{[5-(methoxymethyl)-1-methyl-3-oxo-2-phenyl-2,3-dihydro-1H-pyrazol-4-yl]amino}-2,2-dimethyl-4-oxobutyl)pyridine-3-carboxamide |

(continued)

| | |
|---|---|
| Example 118 | N-(5-Chloro-2-ethoxy-3-pyridine)-N'-[5-(methoxymethyl)-1-methyl-3-oxo-2-phenyl-2,3-dihydro-1H-pyrazol-4-yl]-3,3-dimethylpentanediamide |
| Example 119 | N-(5-Chloro-2-ethoxy-4-fluorophenyl)-N'-[5-(methoxymethyl)-1-methyl-3-oxo-2-phenyl-2,3-dihydro-1H-pyrazol-4-yl]-3,3-dimethylpentanediamide |
| Example 120 | 5-Chloro-N-(4-{[5-(methoxymethyl)-1-methyl-3-oxo-2-phenyl-2,3-dihydro-1H-pyrazol-4-yl]amino}-2,2-dimethyl-4-oxobutyl)-2-methylbenzofuran-7-carboxamide |
| Example 121 | 5-Chloro-N-(4-{[5-(methoxymethyl)-1-methyl-3-oxo-2-phenyl-2,3-dihydro-1H-pyrazol-4-yl]amino}-2,2-dimethyl-4-oxobutyl)-2,2-dimethyl-3H-benzofuran-7-carboxamide |
| Example 122 | 5-Chloro-2-ethoxy-4-fluoro-N-(4-{[5-(1-methoxyethyl)-1-methyl-3-oxo-2-phenyl-2,3-dihydro-1H-pyrazol-4-yl]amino}-2,2-dimethyl-4-oxobutyl)benzamide |
| Example 122 | 5-Chloro-2-ethoxy-4-fluoro-N-(4-{[5-(1-methoxyethyl)-1-methyl-3-oxo-2-phenyl-2,3-dihydro-1H-pyrazol-4-yl]amino}-2,2-dimethyl-4-oxobutyl)benzamide |
| Example 123 | 5-Chloro-2-ethoxy-4-fluoro-N-(4-{[5-((1S)-methoxyethyl)-1-methyl-3-oxo-2-phenyl-2,3-dihydro-1H-pyrazol-4-yl]amino}-2,2-dimethyl-4-oxobutyl)benzamide |
| Example 124 | 5-Chloro-2-ethoxy-4-fluoro-N-(4-{[5-((1R)-1-methoxyethyl)-1-methyl-3-oxo-2-phenyl-2,3-dihydro-1H-pyrazol-4-yl]amino}-2,2-dimethyl-4-oxobutyl)benzamide |
| Example 125 | 5-Chloro-2-ethoxy-N-(4-{[5-(1-methoxyethyl)-1-methyl-3-oxo-2-phenyl-2,3-dihydro-1H-pyrazol-4-yl]amino}-2,2-dimethyl-4-oxobutyl)benzamide |
| Example 126 | N-(5-Chloro-2-ethoxy-3-pyridyl)-N'-[5-(1-methoxyethyl)-1-methyl-3-oxo-2-phenyl-2,3-dihydro-1H-pyrazol-4-yl]-3,3-dimethylpentanediamide |

[Table 55-10]

| | |
|---|---|
| Example 127 | 5-Chloro-N-{4-[(5-ethyl-1-methyl-3-oxo-2-phenyl-2,3-dihydro-1H-pyrazol-4-yl)amino]-2,2-dimethyl-4-oxobutyl}-2-(methoxymethoxy)benzamide |
| Example 128 | N-(5-Chloro-2-methoxyphenyl)-N'-(5-isopropyl-1-methyl-3-oxo-2-phenyl-2,3-dihydro-1H-pyrazol-4-yl)-3,3-dimethylpentanediamide |
| Example 129 | 5-Chloro-N-[4-({5-[4-(dimethylamino)-4-oxobutyl]-1-methyl-3-oxo-2-phenyl-2,3-dihydro-1H-pyrazol-4-yl}amino)-2,2-dimethyl-4-oxobutyl]-2-ethoxybenzamide |
| Example 130 | 5-Chloro-2-ethoxy-N-(4-{[5-(4-hydroxybutyl)-1-methyl-3-oxo-2-phenyl-2,3-dihydro-1H-pyrazol-4-yl]amino}-2,2-dimethyl-4-oxobutyl)benzamide |
| Example 131 | Methyl 2-[4-({4-[(5-chloro-2-ethoxybenzoyl)amino]-3,3-dimethylbutanoyl}amino)-2-methyl-5-oxo-1-phenyl-2,3-dihydro-1H-pyrazol-3-yl]acetate |
| Example 132 | N-(4-{[5-(2-Amino-2-oxoethyl)-1-methyl-3-oxo-2-phenyl-2,3-dihydro-1H-pyrazol-4-yl]amino}-2,2-dimethyl-4-oxobutyl)-5-chloro-2-ethoxybenzamide |
| Example 133 | 5-Chloro-N-(2,2-dimethyl-4-{[1-methyl-3-oxo-5-(3-oxo-3-pyrrolidin-1-ylpropyl)-2-phenyl-2,3-dihydro-1H-pyrazol-4-yl]amino}-4-oxobutyl)-2-ethoxybenzamide |
| Example 134 | 5-Chloro-N-[2,2-dimethyl-4-({1-methyl-5-[(1-methylazetidin-3-yl)oxymethyl]-3-oxo-2-phenyl-2,3-dihydro-1H-pyrazol-4-yl}amino)-4-oxobutyl-2-methoxybenzamide |
| Example 135 | 5-Chloro-N-(2,2-dimethyl-4-{[1-methyl-3-oxo-2-phenyl-5-(3-pyridyloxymethyl)pyrazol-4-yl]amino}-4-oxobutyl)-2-ethoxybenzamide |
| Example 136 | N-(5-Chloro-2-methoxyphenyl)-3,3-dimethyl-N'-(1-methyl-3-oxo-2-phenyl-2,3-dihydro-1H-pyrazol-4-yl)pentanediamide |
| Example 137 | 5-Chloro-2-ethoxy-N-{4-[(1-ethyl-5-methyl-3-oxo-2-phenyl-2,3-dihydro-1H-pyrazol-4-yl)amino]-2,2-dimethyl-4-oxobutyl}benzamide |

(continued)

| | |
|---|---|
| Example 138 | N-(5-Ch loro-4-fl uoro-2-m ethoxyphenyl)-3, 3-d i m ethyl-N'-(3-m ethyl-5-oxo-1-phenyl-2-propyl-1,5-dihydro-1H-pyrazol-4-yl)pentanediamide |
| Example 139 | 5-Chloro-N-(4-{[1-(2-hydroxyethyl)-5-methyl-3-oxo-2-phenyl-2,3-dihydro-1H-pyrazol-4-yl]amino}-2,2-dimethyl-4-oxobutyl)-2,2-dimethyl-3H-benzofuran-7-carboxamide |
| Example 140 | N-[4-({1-[2-(Azetidin-1-yl)ethyl]-5-methyl-3-oxo-2-phenyl-2,3-dihydro-1H-pyrazol-4-yl}amino)-2,2-dimethyl-4-oxobutyl]-5-chloro-2-ethoxypyridine-3-carboxamide |
| Example 141 | 5-Chloro-N-[4-({1-[2-(dimethylamino)ethyl]-5-methyl-3-oxo-2-phenyl-2,3-dihydro-1H-pyrazol-4-yl}amino)-2,2-dimethyl-4-oxobutyl]-2-ethoxypyridine-3-carboxamide |
| Example 142 | 5-Chloro-N-(2,2-dimethyl-4-{[5-methyl-1-(2-morpholinoethyl)-3-oxo-2-phenyl-2,3-dihydro-1H-pyrazol-4-yl]amino}-4-oxobutyl)-2-ethoxypyridine-3-carboxamide |

[Table 55-11]

| | |
|---|---|
| Example 143 | N-(5-Ch loro-4-fl uoro-2-m ethoxyphenyl)-3, 3-d i m ethyl-N'-(3-m ethyl-5-oxo-1-phenyl-2-vinyl-1,5-dihydro-1H-pyrazol-4-yl)pentanediamide |
| Example 144 | N-(5-Ch loro-4-fl uoro-2-m ethoxyphenyl)-3, 3-d i m ethyl-N'-(5-m ethyl-3-oxo-2-phenyl-2,3-dihydro-1H-pyrazol-4-yl)pentanediamide |
| Example 145 | 5-Chloro-N-(4-{[1,5-dimethyl-2-(m-toluyl)-3-oxopyrazol-4-yl]amino}-2,2-dimethyl-4-oxo-2,3-dihydro-1H-butyl)-2-methoxybenzamide |
| Example 146 | N-(5-Chloro-2-methoxyphenyl)-N'-[1-(3-methoxyphenyl)-2,3-dimethyl-5-oxo-1,5-dihydro-1H-pyrazol-4-yl]-3,3-dimethylpentanediamide |
| Example 147 | N-(5-Chloro-2-isopropoxy-phenyl)-N'-[1,5-dimethyl-3-oxo-2-[3-(trifluoromethyl)phenyl]-2,3-dihyro-1H-pyrazol-4-yl]-3,3-dimethylpentanediamide |
| Example 148 | N-(2,2-Bis(fluoromethyl)-4-{[5-(methoxymethyl)-1-methyl-3-oxo-2-phenyl-2,3-dihydro-1H-pyrazol-4-yl]amino}-4-oxobutyl)-5-chloro-2-ethoxybenzamide |
| Example 149 | 5-Chloro-N-(4-{[1,5-dimethyl-3-oxo-2-(2-pyridyl)-2,3-dihydro-1H-pyrazol-4-yl]amino}-2,2-dimethyl-4-oxobutyl)-2-ethoxybenzamide 0.625-hydrochloride |
| Example 150 | 5-Chloro-N-(4-{[1,5-dimethyl-3-oxo-2-(2-pyridyl)-2,3-dihydro-1H-pyrazol-4-yl]amino}-2,2-dimethyl-4-oxobutyl)-2-ethoxypyridine-3-carboxamide |
| Example 151 | 5-Chloro-N-(4-{[1,5-dimethyl-3-oxo-2-(2-pyridyl)-2,3-dihydro-1H-pyrazol-4-yl]amino}-2,2-dimethyl-4-oxobutyl)-2-methylbenzofuran-7-carboxamide |
| Example 152 | 5-Chloro-N-(4-{[1,5-dimethyl-2-(6-methyl-2-pyridyl)-3-oxo-2,3-dihydro-1H-pyrazol-4-yl]amino}-2,2-dimethyl-4-oxobutyl)-2-ethoxybenzamide |
| Example 153 | 5-Chloro-N-(4-{[1,5-dimethyl-2-(6-methyl-2-pyridyl)-3-oxo-2,3-dihydro-1H-pyrazol-4-yl]amino}-2,2-dimethyl-4-oxobutyl)-2-ethoxypyridine-3-carboxamide |
| Example 154 | N-[5-Chloro-2-(trifluoromethoxy)phenyl]-N'-[1,5-dimethyl-2-(6-methyl-2-pyridyl)-3-oxo-2,3-dihydro-1H-pyrazol-4-yl]-3,3-dimethylpentanediamide |
| Example 155 | 5-Chloro-N-(4-{[1,5-dimethyl-2-(6-methyl-2-pyridyl)-3-oxopyrazol-4-yl]amino}-2,2-dimethyl-4-oxobutyl)-2-methylbenzofuran-7-carboxamide |
| Example 156 | 5-Chloro-2-ethoxy-N-(4-{[1-isopropyl-5-methyl-3-oxo-2-(2-pyridyl)pyrazol-4-yl]amino}-2,2-dimethyl-4-oxobutyl)benzamide |
| Example 157 | 5-Chloro-2-ethoxy-N-(4-{[1-(4-methoxy-2-pyridyl)-2,3-dimethyl-5-oxopyrazol-4-yl]amino}-2,2-dimethyl-4-oxobutyl)benzamide |
| Example 158 | 5-Chloro-N-(2,2-dimethyl-4-{[1-methyl-3-oxo-2-phenyl-5-(3,3,3-trifluoropropyl)-2,3-dihydro-1H-pyrazol-4-yl]amino}-4-oxobutyl)-2-ethoxybenzamide |

[Table 55-12]

| | |
|---|---|
| Example 159 | 5-Chloro-2-ethoxy-4-fluoro-N-(4-{[5-(methoxymethyl)-1-methyl-3-oxo-2-phenyl-2,3-dihydro-1H-pyrazol-4-yl]amino}-2,2,3-trimethyl-4-oxobutyl)benzamide |
| Example 160 | N-(5-Chloro-2-methoxyphenyl)-N'-(1-cyclohexyl-2,3-dimethyl-5-oxo-1,5-dihydro-1H-pyrazol-4-yl)-3,3-dimethylpentanediamide |

[Test Example 1]

ATPase assay of TIP48/TIP49 complex

An ATPase assay of a TIP48/TIP49 complex was conducted using rTIP48 and rTIP49. Human TIP48 and TIP49 DNAs were purchased from Open Biosystems. Human TIP48 and TIP49 cDNAs cloned by PCR using the purchased DNAs were each integrated into plasmids for expression in E. *coli* and expressed in *E. coli* to obtain rTIP48 and rTIP49.

A test compound was dissolved at 10 mM using DMSO. The solution was diluted to 10 concentrations as 4-fold serial dilutions from 10 mM using DMSO.

A 2 x assay buffer (100 mM Tris-HCl, pH 7.5, 100 mM NaCl, 40 mM MgCl2, 2 mM DTT, 20 μM ATP, and 0.2 mg/mL BSA) was dispensed at 24.5 μL/well to a 384-well assay plate.

Test compound solutions were dispensed thereto at 1 μL/well. Subsequently, the solution in each well was mixed using a plate mixer.

rTIP48 and rTIP49 were diluted to a concentration of 50 μg/mL using sterilized water. The diluted solution was dispensed at 24.5 μL/well to the 384-well assay plate. Subsequently, the solution in each well was mixed using a plate mixer (final concentration: 24.5 μg/mL) (concentration of each test compound solution: 200 μM, 50 μM, 12.5 μM, 3.12 μM, 0.78 μM, 0.20 μM, 0.049 μM, 0.012 μM, 0.003 μM, and 0.0007 μM; DMSO concentration: 2%).

After the mixing using the plate mixer, the assay plate was left standing at room temperature for 1.5 hours.

5 μL of the solution in each well was transferred to a white assay plate, and ADP-Glo Reagent from ADP-Glo Kinase Assay (Promega Corp., catalog No. V9101) was dispensed thereto at 5 μL/well. After completion of the reaction, the plate was left standing at room temperature for 40 minutes. Kinase Detection Reagent was further dispensed thereto at 5 μL/well, and the plate was left standing at room temperature for 30 minutes. Then, luminescence intensity was measured using a plate reader.

The ATPase inhibitory activity was calculated according to the expression given below.

The luminescence intensity of a well supplemented with the test compound and the TIP48/TIP49 complex was defined as A. The luminescence intensity of a well supplemented with only the TIP48/TIP49 complex was defined as B. The luminescence intensity of a well supplemented with neither the test compound nor the TIP48/TIP49 complex was defined as C. The ratio between the test compound-supplemented group and the test compound-nonsupplemented group (T/C value) was determined according to the following calculation expression:

$$T/C = 100 \times [(A - C) / (B - C)].$$

The dose-response curve was calculated using GraphPad Prism 4 from the concentration of each of the serially diluted test compounds and the ATPase inhibitory activity (%) at this concentration. The concentration at which the ATPase activity was inhibited by 50% (IC50 value) was calculated and is shown in Table 56.

[Test Example 2]

Cell growth inhibition test using RAMOS cell

A cell growth inhibition test was carried out by treating human lymphoma cell line RAMOS cells with a test compound for a given period and then counting the number of viable cells by MTT assay or CellTiter-Glo Luminescent Cell Viability Assay (manufactured by Promega Corp.; hereinafter, referred to as ATP assay).

MTT assay

RAMOS cells were suspended in a medium (RPMI1640 medium containing 10% fetal bovine serum). The cell

suspension was inoculated at 5000 cells/150 μL/well to a 96-well multiwell plate. The test compound was dissolved in DMSO, and this solution was diluted with medium to prepare a sample solution (DMSO concentration: 1% or lower). Subsequently, test compound-free medium or the sample solution was added thereto at 50 μL/well. The MTT assay was carried out at the day of addition of the sample solution or the DMSO dilution to the cells and after culture at 37°C for 3 days under 5% CO2 of the cells supplemented with the sample solution or the medium. The MTT assay was carried out as follows.

A 5 mg/mL solution of MTT (3-(4,5-dimethylthiazol-2-yl)-2,5-diphenyltetrazolium bromide, Sigma-Aldrich Corp., M-2128) was prepared using a phosphate buffered solution (Dulbecco's phosphate-buffered saline). This MTT solution was added at 20 μL/well to the plate. Then, the plate was incubated at 37°C for 4 hours under 5% CO2. The plate was centrifuged at 1200 rpm for 5 minutes, and then, the culture supernatant was removed by suction using a dispenser. DMSO was added thereto at 150 μL/well to dissolve formed formazan. Luminescence from each well was rendered uniform by the stirring of the plate using a plate mixer. The absorbance of each well was measured using a plate reader under conditions involving OD of 540 nm and reference of 660 nm.

The number of viable cells and absorbance (hereinafter, referred to as an OD value) have been confirmed to correlate with each other. Therefore, the rate of cell growth was calculated according to the following expression: Rate of cell growth (T/C%) of a test compound-supplemented well = 100 x [(B - C) / (A-C)] wherein A represents the OD value (final culture date) of a test compound-free well; B represents the OD value (final culture date) of a test compound-containing well; and C represents the OD value (day of cell inoculation) of a well at the start of contact of the cells with the test compound.

T/C% at each concentration of the test compound was determined. The concentration at which the test compound offered 50% growth rate was calculated as a 50% growth inhibition concentration (GI50) value from the growth rates at two points interpolating 50% and the concentrations, and is shown in Table 56.

ATP assay

RAMOS cells were inoculated at 4000 cells/40 μL/well to a 384-well plate containing the compound prepared in advance at predetermined concentrations. The cells thus inoculated were cultured at 37°C for 3 days under 5% CO2. In order to measure a cell survival rate, CellTiter-Glo Reagent was added in an amount corresponding to 1/4 of the amount of the culture solution at the day of cell inoculation and at the final culture date and stirred at room temperature for 2 minutes. Then, luminescence intensity was measured using a plate reader. The cell survival rate was calculated according to the expression given below.

The number of viable cells and luminescence intensity have been confirmed to correlate with each other. Therefore, the rate of cell growth was calculated according to the following expression: Rate of cell growth (T/C%) of a test compound-supplemented well = 100 x [(B - C) / (A - C)] wherein A represents the luminescence intensity (final culture date) of a test compound-free well; B represents the luminescence intensity (final culture date) of a test compound-containing well; and C represents the luminescence intensity (day of cell inoculation) of a well at the start of contact of the cells with the test compound.

T/C% at each concentration of the test compound was determined. The concentration at which the test compound offered 50% growth rate was calculated as a GI50 value from the growth rates at two points interpolating 50% and the concentrations, and is shown in Table 56.

[Table 56-1]

| Example No. | TIP48/TIP49 IC50 (μM) | Ramos GI50 (MTT) (μM) | Ramos GI50 (ATP) (μM) |
|---|---|---|---|
| 1 | 0.17 | - | 0.13 |
| 2 | 0.16 | - | 0.25 |
| 3 | 0.054 | 0.85 | - |
| 4 | 0.048 | 0.17 | - |
| 5 | 0.17 | 0.18 | - |
| 6 | 0.15 | 0.17 | - |
| 7 | 0.11 | 0.47 | - |
| 8 | 0.096 | 0.17 | - |
| 9 | 0.21 | 0.48 | - |
| 10 | 0.014 | 0.045 | - |

(continued)

| Example No. | TIP48/TIP49 IC50 ($\mu$M) | Ramos GI50 (MTT) ($\mu M$) | Ramos GI50 (ATP) ($\mu$M) |
|---|---|---|---|
| 11 | 0.053 | 0.053 | 0.15 |
| 12 | 0.062 | 0.035 | 0.14 |
| 13 | 0.082 | 0.11 | 0.61 |
| 14 | 0.041 | 0.032 | 0.13 |
| 15 | 0.076 | - | 0.57 |
| 16 | 0.068 | - | 0.57 |
| 17 | 0.060 | 0.13 | - |
| 18 | 0.065 | 0.55 | - |
| 19 | 0.063 | 0.15 | - |
| 20 | 0.065 | 0.19 | - |
| 21 | 0.11 | 0.92 | - |
| 22 | 0.11 | 0.46 | - |
| 23 | 0.11 | 0.27 | - |
| 24 | 0.38 | 0.48 | - |
| 25 | 0.14 | 0.49 | - |
| 26 | 0.033 | - | 0.13 |
| 27 | 0.048 | - | 0.13 |
| 28 | 0.048 | - | 0.20 |
| 29 | 0.048 | - | 0.15 |
| 30 | 0.094 | - | 0.48 |
| 31 | 0.052 | - | 0.17 |
| 32 | 0.13 | - | 1.3 |
| 33 | 0.063 | 0.13 | - |
| 34 | 0.056 | - | 0.47 |
| 35 | 0.026 | 0.098 | - |
| 36 | 0.024 | - | 0.12 |
| 37 | 0.038 | - | 0.12 |

[Table 56-2]

| | | | |
|---|---|---|---|
| 38 | 0.77 | - | 0.21 |
| 39 | 0.082 | 0.099 | 0.24 |
| 40 | 0.033 | 0.027 | 0.12 |
| 41 | 0.020 | - | 0.098 |
| 42 | 0.080 | - | 0.15 |
| 43 | 0.060 | - | 0.19 |
| 44 | 0.15 | - | 0.47 |
| 45 | 0.077 | - | 0.48 |

(continued)

| | | | |
|---|---|---|---|
| 46 | 0.081 | - | 0.47 |
| 47 | 0.037 | - | 0.25 |
| 48 | 0.061 | - | 0.16 |
| 49 | 0.009 | - | 0.16 |
| 50 | 0.050 | - | 0.21 |
| 51 | 0.071 | - | 0.14 |
| 52 | 0.036 | - | 0.12 |
| 53 | 0.063 | - | 0.24 |
| 54 | 0.094 | - | 0.14 |
| 55 | 0.043 | - | 0.17 |
| 56 | 0.040 | - | 0.12 |
| 57 | 0.070 | - | 0.19 |
| 58 | 0.058 | - | 0.11 |
| 59 | 0.034 | - | 0.15 |
| 60 | 0.094 | - | 0.37 |
| 61 | 0.052 | - | 0.39 |
| 62 | 0.15 | - | 0.50 |
| 63 | 0.029 | 0.10 | - |
| 64 | 0.099 | - | 0.19 |
| 65 | 0.044 | - | 0.20 |
| 66 | 0.058 | - | 0.16 |
| 67 | 0.036 | - | 0.15 |
| 68 | 0.070 | - | 0.14 |
| 69 | 0.065 | - | 0.16 |
| 70 | 0.084 | - | 0.16 |
| 71 | 0.090 | - | 0.42 |
| 72 | 0.080 | - | 0.51 |
| 73 | 0.048 | - | 0.31 |
| 74 | 0.18 | - | 0.47 |
| 75 | 0.099 | - | 0.27 |
| 76 | 0.068 | - | 0.59 |
| 77 | 0.092 | - | 0.80 |
| 78 | 0.070 | - | 0.48 |

[Table 56-3]

| | | | |
|---|---|---|---|
| 79 | 0.051 | - | 0.14 |
| 80 | 0.14 | - | 0.17 |
| 81 | 0.21 | - | 0.41 |

(continued)

| | | | |
|---|---|---|---|
| 82 | 0.049 | - | 0.37 |
| 83 | 0.065 | - | 0.49 |
| 84 | 0.080 | - | 0.17 |
| 85 | 0.11 | - | 0.30 |
| 86 | 0.090 | - | 0.18 |
| 87 | 0.052 | - | 0.12 |
| 88 | 0.033 | 0.061 | - |
| 89 | 0.090 | - | 0.17 |
| 90 | 0.028 | - | 0.14 |
| 91 | 0.11 | - | 0.18 |
| 92 | 0.026 | - | 0.56 |
| 93 | 0.025 | 0.055 | - |
| 94 | 0.087 | 0.073 | - |
| 95 | 0.059 | 0.18 | - |
| 96 | 0.035 | 0.12 | - |
| 97 | 0.10 | 1.0 | - |
| 98 | 2.2 | 9.6 | - |
| 99 | 0.26 | 0.40 | - |
| 100 | 0.095 | 0.86 | - |
| 101 | 0.45 | 3.2 | - |
| 102 | 0.15 | 0.78 | - |
| 103 | 0.17 | 1.4 | - |
| 104 | 0.25 | 1.3 | - |
| 105 | 0.026 | 0.074 | - |
| 106 | 0.035 | 0.10 | - |
| 107 | 0.066 | 0.19 | - |
| 108 | 0.19 | 0.74 | - |
| 109 | 0.20 | 0.20 | - |
| 110 | 0.033 | 0.12 | - |
| 111 | 0.044 | 0.090 | - |
| 112 | 0.15 | 0.68 | - |
| 113 | 0.039 | 0.047 | 0.13 |
| 114 | 0.031 | 0.042 | 0.14 |
| 115 | 0.041 | - | 0.13 |
| 116 | 0.014 | - | 0.14 |
| 117 | 0.041 | 0.11 | - |
| 118 | 0.065 | 0.23 | 0.64 |
| 119 | 0.095 | 0.27 | - |

[Table 56-4]

| | | | |
|---|---|---|---|
| 120 | 0.024 | 0.051 | - |
| 121 | 0.061 | 0.14 | - |
| 122 | 0.46 | - | 0.14 |
| 123 | 0.039 | - | 0.12 |
| 124 | 0.038 | - | 0.14 |
| 125 | 0.036 | - | 0.14 |
| 126 | 0.056 | - | 0.58 |
| 127 | 0.055 | - | 0.13 |
| 128 | 0.19 | 0.59 | - |
| 129 | 0.052 | - | 0.19 |
| 130 | 0.054 | - | 0.064 |
| 131 | 0.062 | - | 0.13 |
| 132 | 0.10 | - | 0.12 |
| 133 | 0.081 | - | 0.50 |
| 134 | 0.22 | 0.80 | - |
| 135 | 0.10 | 0.12 | - |
| 136 | 0.31 | 1.7 | - |
| 137 | 0.038 | 0.048 | - |
| 138 | 0.18 | 0.33 | - |
| 139 | 0.025 | 0.065 | - |
| 140 | 0.25 | 0.24 | - |
| 141 | 0.16 | 0.23 | - |
| 142 | 0.061 | 0.18 | - |
| 143 | 0.26 | 1.2 | - |
| 144 | 1.5 | 11 | - |
| 145 | 0.042 | 0.77 | - |
| 146 | 0.45 | 1.3 | - |
| 147 | 0.081 | 0.040 | - |
| 148 | 0.083 | - | 0.23 |
| 149 | 0.10 | 0.19 | - |
| 150 | 0.13 | 0.35 | - |
| 151 | 0.040 | 0.13 | - |
| 152 | 0.057 | 0.081 | - |
| 153 | 0.098 | 0.21 | - |
| 154 | 0.082 | 0.11 | - |
| 155 | 0.059 | 0.059 | 0.19 |
| 156 | 0.048 | 0.10 | - |
| 157 | 0.14 | 0.12 | 0.43 |

(continued)

| | | | |
|---|---|---|---|
| 158 | 0.033 | - | 0.31 |
| 159 | 0.077 | - | 0.84 |
| 160 | 0.20 | 0.99 | - |

[Test Example 3]

Cell growth inhibition test

A cell growth inhibition test was carried out by treating a cultured cancer cell line with a test compound for a given period and then counting the number of viable cells by CellTiter-Glo Luminescent Cell Viability Assay (manufactured by Promega Corp.).

The following cells were inoculated to a 384-well plate containing the compound prepared in advance at predetermined concentrations.

Human bladder cancer cell line T24, human breast cancer cell lines MDA-MB-468, MCF7, HCC1954, MDA-MB-231, CAL-51, SK-BR-3, BT-20, and BT-474, human brain tumor and CNS (central nerve system) cell lines D283 Med, A-172, U251, and U-138 MG, human colorectal cancer cell lines HCT 116, RKO, COLO 205, SW620, HT-29, HCT-15, and SW948, human ovarian cancer cell lines OVCAR-5, RL952, ES-2, SK-OV-3, and AN3CA, human stomach cancer cell lines SNU-1, NCI-N87, and MKN45, a human head and neck cancer cell line FaDu, human kidney cancer cell lines 786-0, A-498, and ACHN, human leukemia cell lines MOLT-3, Kasumi-1, MOLT-4, NALM-6, MV-4-11, CCRF-CEM, and K-562, human multiple myeloma cell lines RPMI 8226 and U266B1, human lymphoma cell lines RAMOS, OCI-LY7, KARPAS 422, and DOHH-2, human liver cancer cell lines Hep G2 and HUH-7, human lung cancer cell lines COR-L23, NCI-H460, CALU-6, NCI-H1975, DMS 273, A549, and NCI-H146, human pancreatic cancer cell lines BxPC-3, CAPAN-2, and PANC-1, human prostate cancer cell lines DU 145 and PC-3, human skin cancer cell lines A-431, COLO 829, A375, and SK-MEL-28, and human bone and soft tissue tumor cell lines HT-1080, U2 OS, and SJSA-1 were separately inoculated at 200 to 4000 cells/well to a 384-well plate.

The cells thus inoculated were cultured at 37°C for 3 days under 5% CO2. In order to measure a cell survival rate, CellTiter-Glo Reagent was added in an amount corresponding to 1/4 of the amount of the culture solution at the day of cell inoculation and at the final culture date and stirred at room temperature for 2 minutes. Then, luminescence intensity was measured using a plate reader.

The cell survival rate was calculated according to the expression given below.

The number of viable cells and luminescence intensity have been confirmed to correlate with each other. Therefore, the rate of cell growth was calculated according to the following expression: Rate of cell growth (T/C%) of a test compound-supplemented well = 100 x [(B - C) / (A - C)] wherein A represents the luminescence intensity (final culture date) of a test compound-free well; B represents the luminescence intensity (final culture date) of a test compound-containing well; and C represents the luminescence intensity (day of cell inoculation) of a well at the start of contact of the cells with the test compound.

T/C% at the concentration of each of the serially diluted test compounds was determined. The concentration at which the test compound offered 50% growth rate was calculated as a GI50 value from the growth rates at two points interpolating 50% and the concentrations, and is shown in Table 57.

[Table 57-1]

| Cancer type | Cell line | Example 68 ($\mu$ M) | Example 73 ($\mu$ M) | Example 113 ($\mu$ M) |
|---|---|---|---|---|
| Bladder cancer | T24 | 0.65 | 0.85 | 0.49 |
| Breast cancer | MDA-MB-468 | 0.17 | 0.51 | 0.32 |
| | MCF7 | 0.53 | 0.98 | 0.75 |
| | HCCI954 | 0.34 | 0.92 | 0.72 |
| | MDA-MB-231 | 0.35 | 0.89 | 0.77 |
| | CAL-51 | 0.80 | 1.3 | 0.62 |
| | SK-BR-3 | 0.52 | 1.0 | 0.65 |
| | BT-20 | 0.59 | 1.3 | 0.85 |
| | BT-474 | 1.9 | 5.0 | 25 |
| Brain tumor | D283 Med | 0.13 | 0.31 | 0.23 |

(continued)

| Cancer type | Cell line | Example 68 ($\mu$ M) | Example 73 ($\mu$ M) | Example 113 ($\mu$ M) |
|---|---|---|---|---|
| Central Nerve System | A-172<br>U251<br>U-138 MG | 0.38<br>0.44<br>0.67 | 0.60<br>0.98<br>1.0 | 0.49<br>0.80<br>1.0 |
| Large intestine cancer | HCT 116<br>RKO<br>COLO 205<br>SW620<br>HT-29<br>HCT-15<br>SW948 | 0.18<br>0.24<br>0.31<br>0.84<br>0.51<br>2.7<br>3.9 | 0.46<br>0.57<br>0.85<br>1.6<br>1.2<br>4.0<br>4.2 | 0.23<br>0.31<br>0.72<br>0.91<br>0.92<br>0.80<br>1.4 |
| Ovary cancer | OVCAR-5<br>RL952<br>ES-2<br>SK-OV-3<br>AN3CA | 0.54<br>0.53<br>0.48<br>0.51<br>0.74 | 0.75<br>0.89<br>0.90<br>0.98<br>2.2 | 0.57<br>0.87<br>0.75<br>0.7<br>1.4 |
| Stomach cancer | SNU-1<br>NCI-N87<br>MKN45 | 0.23<br>0.27<br>0.48 | 0.71<br>0.66<br>1.1 | 0.58<br>0.53<br>1.0 |
| Head and neck cancer | FaDu | 0.21 | 0.46 | 0.35 |
| Kidney cancer | 786-0<br>A-498<br>ACHN | 0.68<br>0.54<br>1.8 | 0.92<br>0.94<br>2.5 | 0.32<br>0.43<br>0.97 |

[Table 57-2]

| | | | | |
|---|---|---|---|---|
| Leukemia | MOLT-3<br>KASUMI-1<br>MOLT-4<br>NALM-6<br>MV-4-11<br>CCRF-CEM<br>K-562 | 0.11<br>0.14<br>0.13<br>0.13<br>0.21<br>0.22<br>0.24 | 0.19<br>0.24<br>0.18<br>0.33<br>0.49<br>0.59<br>0.56 | 0.15<br>0.23<br>0.16<br>0.27<br>0.40<br>0.53<br>0.63 |
| Multiple myeloma | RPMI 8226<br>U266B1 | 0.14<br>0.22 | 0.18<br>0.62 | 0.16<br>0.44 |
| Lymphoma | RAMOS<br>OCI-LY7<br>KARPAS 422<br>DOHH-2 | 0.13<br>0.13<br>0.20<br>0.24 | 0.22<br>0.37<br>0.35<br>0.63 | 0.16<br>0.15<br>0.45<br>0.46 |
| Liver cancer | Hep G2<br>HUH-7 | 0.58<br>0.27 | 1.1<br>0.90 | 0.38<br>0.36 |
| Lung cancer | COR-L23<br>NCI-H460<br>CALU-6<br>NCI-H1975<br>DMS 273<br>A549 | 0.20<br>0.31<br>0.43<br>0.49<br>0.31<br>0.91 | 0.55<br>0.73<br>0.78<br>0.90<br>0.78<br>2.0 | 0.42<br>0.55<br>0.68<br>0.81<br>0.54<br>1.1 |

(continued)

| | | | | |
|---|---|---|---|---|
| | NCI-H146 | 1.1 | 2.7 | 2.0 |
| Pancreatic cancer | BxPC-3<br>CAPAN-2<br>PANC-1 | 0.22<br>0.64<br>0.90 | 0.44<br>0.98<br>2.0 | 0.37<br>0.75<br>1.2 |
| Prostate cancer | DU 145<br>PC-3 | 0.53<br>1.4 | 1.1<br>1.5 | 0.65<br>1.4 |
| Skin cancer | A-431<br>COLO 829<br>A375<br>SK-MEL-28 | 0.21<br>0.20<br>0.28<br>0.59 | 0.54<br>0.48<br>0.56<br>0.92 | 0.39<br>0.35<br>0.26<br>0.80 |
| Bone and soft tissue tumor | HT-1080<br>U2 OS<br>SJSA-1 | 0.22<br>0.53<br>0.75 | 0.52<br>0.92<br>1.3 | 0.35<br>0.64<br>0.87 |

[Test Example 4]

*In vivo* test of test compound: Antitumor test using mouse subcutaneous transplantation model

Lymphoma cell line RAMOS cells were suspended in a culture flask and then centrifuged to remove a supernatant. The cells were washed with PBS (phosphate buffered saline, Life Technologies Corp.) twice and then suspended in PBS. The cell suspension was subcutaneously transplanted at a dose of 1 x 107 cells/mouse to the right axillary regions of 6-week-old SCID mice (FOX CHASE SCID C.B.17/Icr-scid/scidJcl, CLEA Japan, Inc.). The test was conducted by using cancer-bearing mice having an estimated tumor volume (major axis x minor axis x minor axis / 2) of 200 to 350 mm3 and grouping the cancer-bearing mice without significant difference in average estimated tumor volume from a negative control group. The compound of Example 113 was suspended in a solvent (EtOH/DMA/Solutol HS-15/PEG400/5% HMPC). The suspension was orally administered to each mouse twice a day (BID) at a dose of 25, 50, 100, or 200 mg/kg for 4 consecutive days. On the next day, the tumor sizes were measured.

Liver cancer cell line HUH-7 cells were dissociated from a culture flask by trypsin treatment, then suspended in an RPMI1640 medium containing 10% FBS, and then centrifuged to remove the supernatant. The cells were washed with PBS twice and then suspended in BD Matrigel Basement Membrane Matrix (manufactured by BD Biosciences). The cell suspension was subcutaneously transplanted at a dose of 5 x 106 cells/mouse to the right axillary regions of 6-week-old nude mice (CAnN.Cg-Foxn1[nu]/CrlCrlj[Foxn1nu/Foxn1nu], Charles River Laboratories Japan, Inc.). The test was conducted by using cancer-bearing mice having an estimated tumor volume (major axis x minor axis x minor axis / 2) of 70 to 180 mm3 and grouping the cancer-bearing mice without significant difference in average estimated tumor volume from a negative control group. The compound of Example 113 was suspended in solvent (EtOH/DMA/Solutol HS-15/PEG400/5% HMPC). The suspension was orally administered to each mouse twice a day (BID) at a dose of 75 or 150 mg/kg for 4 consecutive days. Three days after the final administration date, the tumor sizes were measured.

Lung cancer cell line COR-L23 cells were dissociated from a culture flask by trypsin treatment, then suspended in an RPMI1640 medium containing 10% FBS, and then centrifuged to remove the supernatant. The cells were washed with this medium twice and then suspended in PBS. The cell suspension was subcutaneously transplanted at a dose of 5 x 106 cells/mouse to the right axillary regions of 6-week-old nude mice (BALB/cAJc1-nu/nu, CLEA Japan, Inc.). The test was conducted by using cancer-bearing mice having an estimated tumor volume (major axis x minor axis x minor axis / 2) of 100 to 150 mm3 and grouping the cancer-bearing mice without significant difference in average estimated tumor volume from a negative control group. The compound of Example 113 was suspended in solvent (EtOH/DMA/Solutol HS-15/PEG400/5% HMPC). The suspension was orally administered to each mouse once a day (QD) at a dose of 75, 150, or 300 mg/kg for 4 consecutive days. On the next day, the tumor sizes were measured.

Skin cancer cell line A375 cells were dissociated from a culture flask by trypsin treatment, then suspended in a DMEM medium (Life Technologies Corp.) containing 10% FBS, and then centrifuged to remove the supernatant. The cells were washed with this medium twice and then suspended in PBS. The cell suspension was subcutaneously transplanted at a dose of 3 x 106 cells/mouse to the right axillary regions of 6-week-old nude mice (BALB/cAJc1-nu/nu, CLEA Japan, Inc.). The test was conducted by using cancer-bearing mice having an estimated tumor volume (major axis x minor axis x minor axis / 2) of 75 to 150 mm3 and grouping the cancer-bearing mice without significant difference in average estimated tumor volume from a negative control group. The compound of Example 113 was suspended in

solvent (EtOH/DMA/Solutol HS-15/PEG400/5% HMPC). The suspension was orally administered to each mouse twice a day (BID) at a dose of 50, 100, or 200 mg/kg for 4 consecutive days. On the next day, the tumor sizes were measured.

The rate of tumor growth inhibition was also calculated in this test according to the following expression and is shown in a table.

Rate of tumor growth inhibition (%) = 100 - (Average value of the tumor volumes of the test compound administration group) / (Average value of the tumor volumes of the vehicle administration group) x 100. The results are shown in Table 58.

[Table 58]

| Cancer type | Cell line | Single dose (mg/kg) | Rate of tumor growth (%) inhibition (%) |
|---|---|---|---|
| Lymphoma | RAMOS | 200<br>100<br>50<br>25 | 75<br>72<br>56<br>35 |
| Liver cancer | HUH-7 | 150<br>75 | 63<br>46 |
| Skin cancer | A375 | 200<br>100<br>50 | 86<br>61<br>19 |
| Lung cancer | COR-L23 | 300<br>150<br>75 | 81<br>52<br>39 |

Industrial Applicability

The compound represented by the general formula (I) of the present invention or the pharmacologically acceptable salt thereof has an excellent inhibitory action on the ATPase activity of TIP48/TIP49 complex and as such, is useful as a therapeutic drug for tumors.

## Claims

**1.** A compound represented by the general formula (I) or a pharmacologically acceptable salt thereof:

[Formula 1]

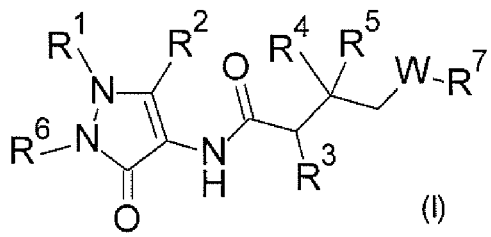

(I)

wherein

$R^1$ represents a hydrogen atom, a $C_1$-$C_6$ alkyl group optionally having 1 to 3 substituents independently selected from group A given below, a $C_2$-$C_6$ alkenyl group optionally having 1 to 3 substituents independently selected from group A given below, or a $C_2$-$C_6$ alkynyl group optionally having 1 to 3 substituents independently selected from group A given below;

$R^2$ represents a hydrogen atom, a halogen atom, a cyano group, a $C_2$-$C_6$ alkenyl group, a $C_1$-$C_6$ alkyl group, or-$CR^{21}R^{22}$-$(CR^{23}R^{24})_m$-$(CR^{25}R^{26})_n$-$(CR^{27}R^{28})_q$-$R^{29}$;

m, n, and q each independently represent an integer of 0 or 1, wherein
when m represents 0, n and q each represent 0, and when n represents 0, q represents 0;
$R^{21}$, $R^{22}$, $R^{23}$, $R^{24}$, $R^{25}$, $R^{26}$, $R^{27}$, and $R^{28}$ each independently represent a hydrogen atom, a hydroxy group, a halogen atom, a cyano group, a $C_1$-$C_6$ alkyl group, or a $C_1$-$C_6$ alkoxy group, or
$R^{21}$ together with $R^{22}$, $R^{23}$ together with $R^{24}$, $R^{25}$ together with $R^{26}$, and $R^{27}$ together with $R^{28}$ each independently optionally form an oxo group;
$R^{29}$ represents a halogen atom, a hydroxy group, a cyano group, a $C_1$-$C_6$ alkoxy group, $-NR^{291}R^{292}$, $-OR^{293}$, $-COR^{294}$, or $-SO_2R^{294}$;
$R^{291}$ and $R^{292}$ each independently represent a hydrogen atom, a $C_1$-$C_6$ alkylcarbonyl group optionally substituted by 1 to 3 halogen atoms, a $C_1$-$C_6$ alkoxycarbonyl group, a $C_3$-$C_6$ cycloalkyl group, a $C_1$-$C_6$ alkyl group optionally having 1 to 3 substituents independently selected from group B given below, or a phenyl-$C_1$-$C_6$ alkyl group optionally having, on the benzene ring, 1 to 3 substituents independently selected from group B given below;
$R^{293}$ represents a $C_1$-$C_6$ alkyl group, a $C_3$-$C_6$ cycloalkyl group, a 5- or 6-membered aromatic heterocyclic group optionally having, in the ring, 1 to 3 heteroatoms independently selected from the group consisting of a nitrogen atom, an oxygen atom, and a sulfur atom, or a 5- or 6-membered aliphatic heterocyclic group optionally having, in the ring, 1 to 3 heteroatoms independently selected from the group consisting of a nitrogen atom, an oxygen atom, and a sulfur atom, wherein
the 5- or 6-membered aromatic heterocyclic group and the 5- or 6-membered aliphatic heterocyclic group are each optionally substituted by 1 to 3 $C_1$-$C_6$ alkyl groups;
$R^{294}$ represents a hydroxy group, a $C_1$-$C_6$ alkoxy group, a phenyl group optionally having 1 to 3 substituents independently selected from group B given below, $-NR^{296}R^{297}$, or $-OR^{293}$;

$R^{296}$ and $R^{297}$ each independently represent a hydrogen atom, a $C_3$-$C_6$ cycloalkyl group, or a $C_1$-$C_6$ alkyl group optionally having 1 to 3 substituents independently selected from group B given below, or $R^{296}$ and $R^{297}$ optionally together form a 3-to 6-membered aliphatic heterocyclic ring optionally having, in the ring, 1 to 3 heteroatoms independently selected from the group consisting of a nitrogen atom, an oxygen atom, and a sulfur atom;

$R^3$ represents a hydrogen atom, a halogen atom, a hydroxy group, a $C_1$-$C_6$ alkyl group, or a $C_1$-$C_6$ alkoxy group;
$R^4$ and $R^5$ each independently represent a hydrogen atom, a hydroxy group, a halogen atom, a $C_1$-$C_6$ alkyl group optionally having 1 to 3 halogen atoms, a $C_1$-$C_6$ alkoxy group, or a $C_1$-$C_6$ alkylcarbonyloxy group, or
$R^4$ and $R^5$ optionally together form a 3- to 6-membered cycloalkyl ring, or
a 5- or 6-membered aliphatic heterocyclic ring optionally having, in the ring, 1 to 3 heteroatoms independently selected from the group consisting of a nitrogen atom, an oxygen atom, and a sulfur atom;
W represents any of the following $W^1$ to $W^3$:

[Formula 2]

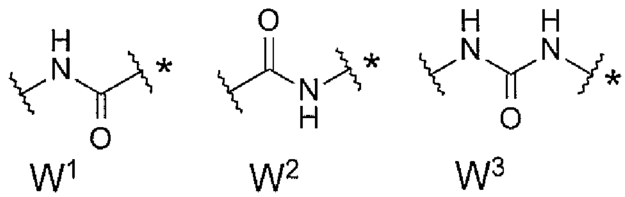

(* binds to $R^7$) ;
$R^6$ represents a phenyl group optionally having 1 to 5 substituents independently selected from group D given below, a $C_3$-$C_7$ cycloalkyl group optionally having 1 to 3 substituents independently selected from group D given below, or a 5- or 6-membered aromatic heterocyclic group optionally having, in the ring, 1 to 3 heteroatoms independently selected from the group consisting of a nitrogen atom, an oxygen atom, and a sulfur atom, wherein the 5- or 6-membered aromatic heterocyclic ring optionally has 1 to 4 substituents independently selected from group D given below; and
$R^7$ represents a phenyl group optionally having 1 to 5 substituents independently selected from group C given below, a naphthyl group optionally having 1 to 7 substituents independently selected from group C given below, a 5- or 6-membered aromatic heterocyclic group optionally having, in the ring, 1 to 3 heteroatoms independently selected from the group consisting of a nitrogen atom, an oxygen atom, and a sulfur atom, an 8-to 10-membered

bicyclic aromatic heterocyclic group optionally having, in the ring, 1 to 3 heteroatoms independently selected from the group consisting of a nitrogen atom, an oxygen atom, and a sulfur atom, or an 8- to 10-membered bicyclic partially unsaturated-ring aliphatic heterocyclic group optionally having, in the ring, 1 to 3 heteroatoms independently selected from the group consisting of a nitrogen atom, an oxygen atom, and a sulfur atom, wherein the 5- or 6-membered aromatic heterocyclic group, the 8-to 10-membered bicyclic aromatic heterocyclic group, and the bicyclic partially unsaturated-ring aliphatic heterocyclic group each optionally have 1 to 4 substituents independently selected from group C given below:

group A consists of a hydroxy group, a $C_1$-$C_6$ alkoxy group, an amino group, a $C_1$-$C_6$ alkylamino group, a di-$C_1$-$C_6$ alkylamino group, and a 5- or 6-membered aliphatic heterocyclic group optionally having, in the ring, 1 to 3 heteroatoms independently selected from the group consisting of a nitrogen atom, an oxygen atom, and a sulfur atom,
group B consists of a halogen atom, a hydroxy group, a cyano group, a $C_1$-$C_6$ alkyl group, and a $C_1$-$C_6$ alkoxy group,
group C consists of a halogen atom, a hydroxy group, a $C_1$-$C_6$ alkyl group, a $C_1$-$C_6$ alkoxy group optionally substituted by 1 to 3 halogen atoms, a $C_1$-$C_6$ alkoxy-$C_1$-$C_6$ alkyl group, a $C_3$-$C_6$ cycloalkoxy group, a $C_1$-$C_6$ alkoxy-$C_1$-$C_6$ alkoxy group, and an oxy group bonded to a 3- to 6-membered aliphatic heterocyclic group optionally having, in the ring, 1 to 3 heteroatoms independently selected from the group consisting of a nitrogen atom, an oxygen atom, and a sulfur atom, and
group D consists of a halogen atom, a $C_1$-$C_6$ alkyl group optionally substituted by 1 to 3 halogen atoms, and a $C_1$-$C_6$ alkoxy group.

**2.** The compound according to claim 1 or a pharmacologically acceptable salt thereof, wherein in the formula (I), $R^1$ represents a hydrogen atom, or a $C_1$-$C_6$ alkyl group optionally having 1 to 3 substituents independently selected from the group consisting of a hydroxy group and a di-$C_1$-$C_6$ alkylamino group.

**3.** The compound according to claim 1 or 2 or a pharmacologically acceptable salt thereof, wherein in the formula (I), $R^2$ represents a $C_1$-$C_6$ alkyl group or $-CR^{21a}R^{22a}-(CR^{23a}R^{24a})_{ma}-(CR^{25a}R^{26a})_{na}-(CR^{27a}R^{28a})_{qa}-R^{29a}$;
ma, na, and qa each independently represent an integer of 0 or 1, wherein when ma represents 0, na and qa each represent 0, and when na represents 0, qa represents 0;
$R^{21a}$, $R^{22a}$, $R^{23a}$, $R^{24a}$, $R^{25a}$, $R^{26a}$, $R^{27a}$, and $R^{28a}$ each independently represent a hydrogen atom, a $C_1$-$C_6$ alkyl group, or a $C_1$-$C_6$ alkoxy group;
$R^{29a}$ represents a halogen atom, a hydroxy group, a $C_1$-$C_6$ alkoxy group, $-NR^{291a}R^{292a}$, or $-COR^{294a}$;

$R^{291a}$ and $R^{292a}$ each independently represent a $C_1$-$C_6$ alkyl group optionally having 1 to 3 substituents independently selected from the group consisting of a halogen atom and a hydroxy group, or a hydrogen atom;
$R^{294a}$ represents a $C_1$-$C_6$ alkoxy group or $-NR^{296a}R^{297a}$; and
$R^{296a}$ and $R^{297a}$ each independently represent a hydrogen atom or a $C_1$-$C_6$ alkyl group, or
$R^{296a}$ and $R^{297a}$ optionally together form a 3-to 6-membered aliphatic heterocyclic ring optionally having, in the ring, 1 to 3 heteroatoms independently selected from the group consisting of a nitrogen atom, an oxygen atom, and a sulfur atom.

**4.** The compound according to any one of claims 1 to 3 or a pharmacologically acceptable salt thereof, wherein in the formula (I),
W represents any of the following $W^1$ and $W^2$:

[Formula 3]

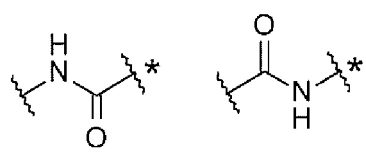

$W^1$ $W^2$

(* binds to $R^7$).

**5.** The compound according to any one of claims 1 to 4 or a pharmacologically acceptable salt thereof, wherein in the formula (I),
$R^6$ represents a phenyl group or a pyridyl group, wherein the phenyl group and the pyridyl group each optionally have one substituent independently selected from group E given below:
group E consists of a halogen atom, a $C_1$-$C_6$ alkyl group, a trifluoromethyl group, and a $C_1$-$C_6$ alkoxy group.

**6.** The compound according to any one of claims 1 to 5 or a pharmacologically acceptable salt thereof, wherein in the formula (I),
$R^7$ is represented by the following formula (II):

[Formula 4]

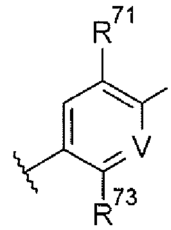
(II)

wherein

$R^{71}$ represents a halogen atom;
$R^{72}$ represents a hydrogen atom or a halogen atom;
$R^{73}$ represents a $C_1$-$C_6$ alkoxy group optionally substituted by 1 to 3 halogen atoms, a $C_1$-$C_6$ alkoxy-$C_1$-$C_6$ alkoxy group, or a $C_3$-$C_6$ cycloalkoxy group;
V represents a nitrogen atom or $CR^{74}$; and

$R^{74}$ represents a hydrogen atom, or
$R^{73}$ and $R^{74}$ optionally together form a pyridine ring,

a morpholine ring, a tetrahydrofuran ring, a tetrahydropyran ring, a dioxane ring, an oxazole ring, or a furan ring, wherein
the pyridine ring, the morpholine ring, the tetrahydrofuran ring, the tetrahydropyran ring, the dioxane ring, the oxazole ring, and the furan ring each optionally have, on the ring, 1 or 2 substituents independently selected from group F given below: group F consists of a $C_1$-$C_6$ alkyl group, a $C_1$-$C_6$ alkoxy group, a $C_1$-$C_6$ alkoxy-$C_1$-$C_6$ alkyl group, and a $C_1$-$C_6$ alkoxy-$C_1$-$C_6$ alkoxy group.

**7.** A compound represented by the general formula (III) or a pharmacologically acceptable salt thereof:

[Formula 5]

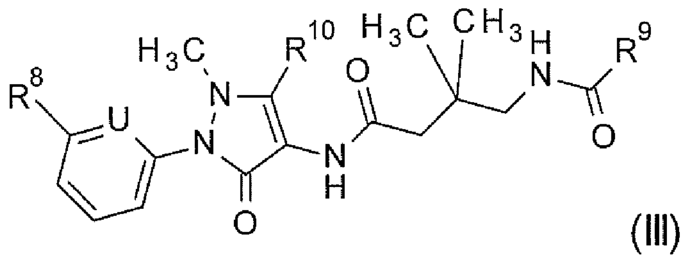
(III)

wherein

$R^8$ represents a hydrogen atom or a methyl group;
U represents CH or a nitrogen atom;
$R^9$ represents any of the following formulas (VII) to (IX) :

[Formula 6]

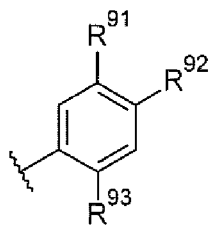
(VII)
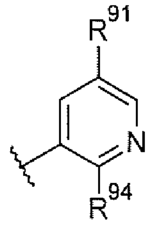
(VIII)
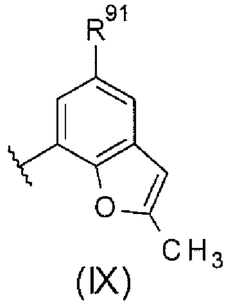
(IX)

wherein

$R^{91}$ represents a halogen atom;
$R^{92}$ represents a hydrogen atom or a halogen atom;
$R^{93}$ represents a methoxy group, an ethoxy group, or a 2-methoxyethoxy group; and
$R^{94}$ represents a methoxy group or an ethoxy group; and

$R^{10}$ represents a methyl group or any of the following formulas (IV) to (VI):

[Formula 7]

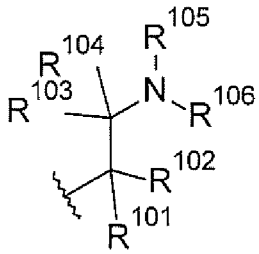
(IV)
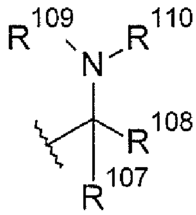
(V)
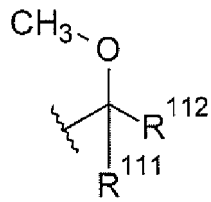
(VI)

wherein

$R^{101}$ and $R^{102}$ each independently represent a hydrogen atom, a methyl group, or a methoxy group; and
$R^{103}$, $R^{104}$, $R^{105}$, $R^{106}$, $R^{107}$, $R^{108}$, $R^{109}$, $R^{110}$, $R^{111}$, and $R^{112}$ each independently represent a hydrogen atom or a methyl group.

**8.** Any one compound selected from the following group or a pharmacologically acceptable salt thereof:

(+)-5-chloro-N-[2,2-dimethyl-4-({1-methyl-5-[1-(methylamino)ethyl]-3-oxo-2-phenyl-2,3-dihydro-1H-pyrazol-4-yl}amino)-4-oxobutyl]-2-ethoxypyridine-3-carboxamide,
(-)-5-chloro-N-[2,2-dimethyl-4-({1-methyl-5-[1-(methylamino)ethyl]-3-oxo-2-phenyl-2,3-dihydro-1H-pyrazol-4-yl}amino)-4-oxobutyl]-2-ethoxypyridine-3-carboxamide,
5-chloro-N-[4-({5-[2-(dimethylamino)ethyl]-1-methyl-3-oxo-2-phenyl-2,3-dihydro-1H-pyrazol-4-yl}amino)-2,2-dimethyl-4-oxobutyl]-2-ethoxybenzamide,
5-chloro-N-[4-({5-[(1R)-2-(dimethylamino)-1-methylethyl]-1-methyl-3-oxo-2-phenyl-2,3-dihydro-1H-pyrazol-4-yl}amino)-2,2-dimethyl-4-oxobutyl]-2-ethoxybenzamide, 5-chloro-N-[4-({5-[(1R)-2-(dimethylamino)-1-methylethyl]-1-methyl-3-oxo-2-phenyl-2,3-dihydro-1H-pyrazol-4-yl}amino)-2,2-dimethyl-4-oxobutyl]-2-ethoxypyridine-3-carboxamide,
5-chloro-2-ethoxy-4-fluoro-N-(4-{[5-(methoxymethyl)-1-methyl-3-oxo-2-phenyl-2,3-dihydro-1H-pyrazol-4-yl]amino}-2,2-dimethyl-4-oxobutyl)benzamide, 5-chloro-2-ethoxy-4-fluoro-N-(4-{[5-((1S)-1-methoxyethyl)-1-methyl-3-oxo-2-phenyl-2,3-dihydro-1H-pyrazol-4-yl]amino}-2,2-dimethyl-4-oxobutyl)benzamide, and

5-chloro-2-ethoxy-4-fluoro-N-(4-{[5-((1R)-1-methoxyethyl)-1-methyl-3-oxo-2-phenyl-2,3-dihydro-1H-pyrazol-4-yl]amino}-2,2-dimethyl-4-oxobutyl)benzamide.

**9.** A pharmaceutical composition comprising a compound according to any one of claims 1 to 8 or a pharmacologically acceptable salt thereof as an active ingredient.

**10.** An inhibitor of the ATPase activity of a TIP48/TIP49 complex comprising a compound according to any one of claims 1 to 8 or a pharmacologically acceptable salt thereof as an active ingredient.

**11.** An antitumor agent comprising a compound according to any one of claims 1 to 8 or a pharmacologically acceptable salt thereof as an active ingredient.

**12.** The antitumor agent according to claim 11, wherein the tumor is bladder cancer, breast cancer, brain tumor, colorectal cancer, ovary cancer, stomach cancer, head and neck cancer, kidney cancer, leukemia, multiple myeloma, lymphoma, liver cancer, lung cancer, pancreatic cancer, prostate cancer, skin cancer, or bone and soft tissue tumor.

**13.** A therapeutic agent for a tumor found to have an increased expression level of a TIP48/TIP49 complex, comprising a compound according to any one of claims 1 to 8 or a pharmacologically acceptable salt thereof as an active ingredient.

**14.** A therapeutic agent for a tumor that is treatable by inhibiting the ATPase activity of a TIP48/TIP49 complex, comprising a compound according to any one of claims 1 to 8 or a pharmacologically acceptable salt thereof as an active ingredient.

## INTERNATIONAL SEARCH REPORT

International application No.
PCT/JP2015/054318

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
C07D231/50, A61K31/4152, A61K31/4155, A61K31/423, A61K31/4439, A61K31/444, A61K31/4709, A61K31/5377, A61K31/538, A61P35/00, A61P43/00, C07D401/04, C07D401/12, C07D401/14, C07D403/12, C07D405/12, C07D413/12

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2015 |
| Kokai Jitsuyo Shinan Koho | 1971-2015 | Toroku Jitsuyo Shinan Koho | 1994-2015 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | WO 2004/106306 A1 (ASTON UNIVERSITY), 09 December 2004 (09.12.2004), & US 2007/0185115 A1 & GB 312368 D & EP 1628963 A1 & CA 2527194 A | 1-14 |
| A | WO 2010/075286 A1 (UNIVERSITY OF WASHINGTON), 01 July 2010 (01.07.2010), & US 2012/0046242 A1 | 1-14 |
| A | PATIL V et al, European Journal of Medicinal Chemistry, 2010, Vol.45, pp.4539-4544 | 1-14 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 13 April 2015 (13.04.15) | 28 April 2015 (28.04.15) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (July 2009)

**INTERNATIONAL SEARCH REPORT**

International application No.
PCT/JP2015/054318

Continuation of A. CLASSIFICATION OF SUBJECT MATTER
(International Patent Classification (IPC))

*C07D231/50*(2006.01)i, *A61K31/4152*(2006.01)i, *A61K31/4155*(2006.01)i, *A61K31/423*(2006.01)i, *A61K31/4439*(2006.01)i, *A61K31/444*(2006.01)i, *A61K31/4709*(2006.01)i, *A61K31/5377*(2006.01)i, *A61K31/538*(2006.01)i, *A61P35/00*(2006.01)i, *A61P43/00*(2006.01)i, *C07D401/04*(2006.01)i, *C07D401/12*(2006.01)i, *C07D401/14*(2006.01)i, *C07D403/12*(2006.01)i, *C07D405/12*(2006.01)i, *C07D413/12*(2006.01)i

(According to International Patent Classification (IPC) or to both national classification and IPC)

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 200710015 A
- WO 200967613 A
- US 200669093 B
- US 200772934 A1

### Non-patent literature cited in the description

- *Sci. Signal.,* 2013, vol. 12
- *Biochim. Biophys. Acta,* 2011, vol. 1815, 147-157
- *Mol. Cell,* 2000, vol. 5, 321-330
- *Hepatology,* 2009, vol. 50, 1871-1883
- *Oncol. Rep.,* 2012, vol. 28, 1619-1624
- *Jpn. J. Cancer Res.,* 2002, vol. 93, 894-901
- *Biochem. J.,* 2012, vol. 443, 549-559
- *J. Mol. Biol.,* 2007, vol. 366, 172-179
- **T.W. GREENE ; P.G.M. WUTS.** Protective Groups in Organic Synthesis. John Wiley & Sons, Inc, 2006
- *J. Org. Chem.,* 1985, vol. 50, 3627-3631
- *Tetrahedron Asymmetry,* 2010, vol. 21, 2124-2135
- Journal of the Chemical Society. Perkin Transactions, 1985, vol. 1, 1355-1362
- *J. Med. Chem.,* 2011, vol. 54, 7030-7054
- *European Journal of Organic Chemistry,* 2012, vol. 29, 5774-5788
- *Chem. & Pharm. Bull.,* 1996, vol. 44 (11), 2051-2060
- *J. Org. Chem.,* 2011, vol. 66, 5859-5865
- *J. Heterocycl. Chem.,* 2006, vol. 43, 1205-1215
- J. Chem. Soc. Perkin Trans, 1989, vol. 1, 265-278